(19) Europäisches Patentamt
European Patent Office
Office européen des brevets

(11) EP 4 763 843 A1

(12) EUROPEAN PATENT APPLICATION
published in accordance with Art. 153(4) EPC

(43) Date of publication:
**24.06.2026 Bulletin 2026/26**

(21) Application number: **24853790.4**

(22) Date of filing: **14.08.2024**

(51) International Patent Classification (IPC):
***C07D 413/06*** (2006.01) ***C07D 413/02*** (2006.01)
***C07D 487/04*** (2006.01) ***C07D 519/00*** (2006.01)
***A61K 31/41*** (2006.01) ***A61K 31/423*** (2006.01)
***A61K 31/428*** (2006.01) ***A61P 35/00*** (2006.01)

(52) Cooperative Patent Classification (CPC):
**A61K 31/41; A61K 31/423; A61K 31/428; A61P 35/00; C07D 413/02; C07D 413/06; C07D 487/04; C07D 519/00**

(86) International application number:
**PCT/CN2024/111949**

(87) International publication number:
**WO 2025/036382 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**14.08.2023 CN 202311021822**
**27.09.2023 CN 202311258886**
**29.12.2023 CN 202311862643**
**19.04.2024 CN 202410481362**

(71) Applicants:
- **Shanghai Henlius Biotech, Inc.**
  **Shanghai 201210 (CN)**
- **Shanghai Henlius Biologics Co., Ltd.**
  **Shanghai 201616 (CN)**
- **Shanghai Henlius Bioscience Co., Ltd.**
  **Shanghai (CN)**

(72) Inventors:
- **CHI, Yushi**
  **Shanghai 201616 (CN)**
- **LIU, Rui**
  **Shanghai 201616 (CN)**
- **ZHANG, Xiayan**
  **Shanghai 201616 (CN)**
- **SHAN, Yongqiang**
  **Shanghai 201616 (CN)**
- **TIAN, Xuemei**
  **Shanghai 201616 (CN)**

(74) Representative: **Plougmann Vingtoft a/s**
**Strandvejen 70**
**2900 Hellerup (DK)**

# (54) SULFONAMIDE COMPOUND, AND PREPARATION METHOD THEREFOR AND USE THEREOF

(57) The present disclosure relates to a substituted sulfonamide derivative of formula (I), a preparation method therefor, a pharmaceutical composition containing the compound, and the use thereof in the treatment and/or prevention of cancers in which lysine acetyltransferase 6A/B (KAT6A/B) plays a role.



Processed by Luminess, 75001 PARIS (FR)

## Description

### TECHNICAL FIELD

The present disclosure pertains to the field of pharmaceutics, and relates to a preparation method for a substituted sulfonamide compound and use thereof. Specifically, the present disclosure relates to a substituted sulfonamide compound of formula (I), a preparation method therefor, a pharmaceutical composition comprising the compound, and use thereof in the treatment and/or prevention of a cancer in which lysine acetyltransferase 6A/B (KAT6A/B) plays a role.

### BACKGROUND

Lysine acetyltransferases (KATs) use acetyl coenzyme A as an acetyl donor, catalyzing the acetylation of ε-amino groups of lysine residues on histones and non-histone proteins. Acetylation, as a reversible post-translational modification, affects protein function and gene expression through various mechanisms, including modulation of protein stability, enzymatic activity, subcellular localization, interactions with other types of post-translational modifications, and protein-DNA interactions.

Based on the homology of amino acid sequences, KATs can be divided into 4 subfamilies, namely the MYST family, the p300/CBP family, the SRC/p160 family, and the GNAT family. Among them, the largest subfamily is the MYST family, whose name is composed of the first letters of four early discovered members: MOZ, Ybf2, Sas2, and Tip60. The MYST family in mammals has a total of five members: KAT5 (TIP60), KAT6A (MOZ; MYST3), KAT6B (MORF; MYST4), KAT7 (HBO; MYST2), and KAT8 (MOF; MYST1), all of which have homologous genes in humans.

The members of the MYST family of proteins all contain a highly conserved MYST domain. This domain functions as an adapter protein that can interact with other proteins to form protein complexes. It has also been reported that the MYST domain may possess DNA-binding ability. In addition, zinc finger structures and chromatin-binding domains are also characteristic domains of this family of KATs. The MYST family of proteins regulates gene transcription activity by modulating the acetylation levels of lysine residues on histones (H2A, H3, H4). The downstream biological functions involved include the initiation of DNA replication, the regulation of gene expression, the repair of DNA damage, the modulation of Treg cells, the maintenance of cell stemness, the development and differentiation of the central nervous system and hematopoietic system, etc. Abnormal activation of KATs of the MYST family or related complexes thereof can easily lead to uncontrolled cell growth and apoptosis, thereby resulting in the occurrence of malignant tumors.

KAT6A (lysine acetyltransferase 6A, also known as MOZ) and KAT6B (lysine acetyltransferase 6B, also known as MORF), as important members of the MYST family, are primarily responsible for the acetylation modification of lysine 23 on histone H3 (H3K23). In addition, KAT6A is also capable of acetylating H3K9 and H3K14. The physiological and pathological functions regulated by KAT6A and KAT6B are quite extensive. They are involved in the development of the nervous system, the development of the cardiac septum, the proliferation and differentiation of hematopoietic progenitor cells, the maintenance of cell stemness, and the occurrence, development, and drug resistance generation of various tumors.

Abnormal alterations in the KAT6A gene are closely related to the occurrence and development of human tumors. In hematological tumors (e.g., acute myeloid leukemia), chromosomal translocations of KAT6A have been reported, forming fusion genes, such as KAT6A-CBP, KAT6A-TIF2, KAT6A-NcoA3, KAT6A-LEUTX, and KAT6A-EP300. Meanwhile, in a variety of solid tumors (e.g., breast cancer, ovarian cancer, cervical cancer, lung adenocarcinoma, colon cancer, and rectal cancer), KAT6A gene amplification has been discovered. Particularly in breast cancer, it has been found that 10%-15% of patients have a duplicated amplification region at 8p11-12, with KAT6A precisely located in this amplified region. Additionally, Chromosomal rearrangements of the KAT6B gene have been reported in acute myeloid leukemia (e.g., KAT6B-CBP), and up-regulated expression of KAT6B has also been observed in breast cancer. In tumor cells with KAT6A/B amplification, the gene expression level is closely related to the gene copy number. Studies have shown that in breast cancer cells with 8p11 amplification, the KAT6A protein localizes to the promoter region of the estrogen receptor ERα, promoting the expression of ERα. Conversely, shRNA-mediated knockdown of KAT6A reduces the mRNA and protein levels of ERα. These data indicate that KAT6A positively regulates the gene expression of ERα, and the growth of ER-positive breast cancer cells is dependent on the expression of this gene. Molecular genetic studies have shown that knockdown of KAT6A can induce down-regulation of ERα expression and inhibit the growth of T47D tumors *in vivo.* CTx-648, a small molecule inhibitor of KAT6A/B, has also shown excellent *in vivo* and *in vitro* anti-tumor activity against ER-positive breast cancer in preclinical studies. In addition to ER-positive breast cancer, KAT6A/B inhibitors have also demonstrated potential prospects for indication expansion in a variety of other solid tumors. For example, in glioma, KAT6A exerts a cancer-promoting function. It binds to TRIM24 to promote PI3K/AKT signal transduction activity, thereby promoting glioma cell proliferation and tumor growth.

Given the important role of KAT6A/B in human diseases such as cancer, inhibitors against KAT6A/B have broad

application prospects.

## SUMMARY

The present application relates to a compound of formula (I) as defined herein, which can act as a lysine acetyltransferase 6A/B (KAT6A/B) inhibitor. The present application is characterized by a method for treating and/or preventing a cancer in which lysine acetyltransferase 6A/B (KAT6A/B) plays a role, comprising administering to a subject in need thereof a therapeutically effective amount of the compound of formula (I) (including formulas II-1 to VII-3) as defined herein or a pharmaceutically acceptable salt, an ester, a stereoisomer, a tautomer, a polymorph, a hydrate, a solvate, an N-oxide, an isotopically labeled compound, a metabolite, or a prodrug thereof. The method of the present application can be used for treating a cancer in which lysine acetyltransferase 6A/B (KAT6A/B) plays a role by inhibiting the activity of lysine acetyltransferase 6A/B (KAT6A/B).

A first aspect of the present application relates to a compound of formula (I):

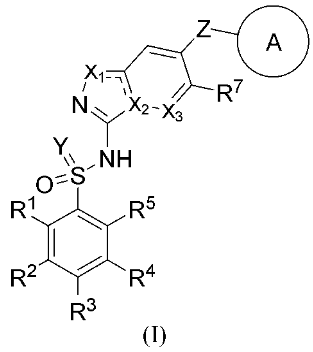

(I)

or a pharmaceutically acceptable salt, an ester, a stereoisomer, a tautomer, a polymorph, a hydrate, a solvate, an N-oxide, an isotopically labeled compound, a metabolite, or a prodrug thereof, wherein $R^1$, $R^2$, $R^3$, $R^4$, $R^5$, $R^7$, $X_1$, $X_2$, $X_3$, Y, ring A, and Z are as detailed herein below.

Another aspect of the present application relates to a pharmaceutical composition, comprising the compound of formula (I) or

the pharmaceutically acceptable salt, the ester, the stereoisomer, the tautomer, the polymorph, the hydrate, the solvate, the N-oxide, the isotopically labeled compound, the metabolite, or the prodrug thereof, and one or more pharmaceutically acceptable carriers, diluents, or excipients.

Another aspect of the present application relates to a method for inhibiting lysine acetyltransferase 6A/B (KAT6A/B). The method comprises administering to a subject in need thereof the compound of formula (I) or the pharmaceutically acceptable salt, the ester, the stereoisomer, the tautomer, the polymorph, the hydrate, the solvate, the N-oxide, the isotopically labeled compound, the metabolite, or the prodrug thereof, or the pharmaceutical composition comprising the same.

Another aspect of the present application relates to a method for treating a cancer in which lysine acetyltransferase 6A/B (KAT6A/B) plays a role. The method comprises administering to a subject in need thereof the compound of formula (I) or the pharmaceutically acceptable salt, the ester, the stereoisomer, the tautomer, the polymorph, the hydrate, the solvate, the N-oxide, the isotopically labeled compound, the metabolite, or the prodrug thereof, or the pharmaceutical composition comprising the same. The cancer is preferably selected from lung cancer, breast cancer, rectal cancer, colon cancer, esophageal cancer, gastric cancer, liver cancer, gallbladder cancer, cholangiocarcinoma, kidney cancer, bladder cancer, urothelial cancer, head and neck cancer, nasopharyngeal cancer, prostate cancer, cervical cancer/endometrial cancer, ovarian cancer, pancreatic cancer, melanoma, bone cancer, mesothelioma, gastrointestinal stromal tumor, sarcoma, brain glioma, thyroid cancer, salivary gland tumor, glioblastoma, neuroblastoma, gastric myxoma, lymphoma, leukemia, plasmacytoma, sinoatrial node tumor, and tenosynovial giant cell tumor, and more preferably selected from breast cancer, prostate cancer, lung cancer, pancreatic cancer, ovarian cancer, cervical cancer/endometrial cancer, bladder cancer, brain glioma, malignant lymphoma, liver cancer, and leukemia, wherein the breast cancer is preferably $ER^+$ breast cancer or $ER^+$/$HER2^-$ breast cancer, the lung cancer is preferably non-small cell lung cancer, and the prostate cancer is preferably castration-resistant prostate cancer.

The present application further provides compounds and compositions having significantly improved inhibitory activity against lysine acetyltransferase 6A/B (KAT6A/B), significantly improved pharmacodynamics and pharmacokinetics, and/or a significantly improved safety profile compared to known KAT6A/B inhibitors.

The details of the present application are set forth in the accompanying description below. Although methods and materials similar or equivalent to those described herein can be used in the practice or testing of the present application,

the illustrative methods and materials are now described. Other features, objectives, and advantages of the present application will be apparent from the specification and the claims. In the specification and the appended claims, the singular forms also include their plural forms, unless the context clearly indicates otherwise. Unless otherwise defined, all technical and scientific terms used herein have the same meanings as commonly understood by those of ordinary skill in the art to which the present application belongs. All patents and publications cited in this specification are incorporated herein by reference in their entirety.

The content of all references (including literature references, issued patents, published patent applications, and co-pending patent applications) cited throughout the present application is hereby expressly incorporated by reference in its entirety. Unless otherwise defined, all technical and scientific terms used herein have the meanings commonly known to those of ordinary skill in the art.

## BRIEF DESCRIPTION OF THE DRAWINGS

FIG. 1 shows the color development results of the effects of compounds on histone acetylation in estrogen receptor-positive breast cancer cells.

FIG. 2 shows the tumor volume change results from an anti-tumor experiment for compounds conducted in an $ER^+KAT6^{high}$ breast cancer cell-derived xenograft tumor model in Test Example 5.

FIG. 3 shows the mouse body weight change results from the anti-tumor experiment for the compounds conducted in the $ER^+KAT6^{high}$ breast cancer cell-derived xenograft tumor model in Test Example 5.

FIG. 4 shows the tumor volume change results from an anti-tumor experiment for compounds conducted in an $ER^+KAT6^{high}$ breast cancer cell-derived xenograft tumor model in Test Example 6.

FIG. 5 shows the mouse body weight change results from the anti-tumor experiment for the compounds conducted in the $ER^+KAT6^{high}$ breast cancer cell-derived xenograft tumor model in Test Example 6.

## DETAILED DESCRIPTION

### Definitions

Unless otherwise stated, the terms used in the specification and claims have the following meanings.

The term "alkyl" refers to a saturated aliphatic hydrocarbon group, which is a linear or branched group containing 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkyl), preferably alkyl containing 1 to 12 (e.g., 1, 2, 3, 4, 5, 6, 7, 8, 9, 10, 11, and 12) carbon atoms (i.e., $C_{1-12}$ alkyl), and more preferably alkyl containing 1 to 6 carbon atoms (i.e., $C_{1-6}$ alkyl). Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. Alkyl may be substituted or unsubstituted, and when it is substituted, the substitution may occur at any available point of attachment, and the substituent is preferably selected from one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "alkylene" refers to a saturated linear or branched aliphatic hydrocarbon group, which is a residue derived from the parent alkane by removal of two hydrogen atoms from the same carbon atom or two different carbon atoms, having 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkylene), preferably 1 to 12 carbon atoms (i.e., $C_{1-12}$ alkylene), and more preferably 1 to 6 carbon atoms (i.e., $C_{1-6}$ alkylene). Non-limiting examples include: methylene ($-CH_2-$), 1,1-ethylene ($-CH(CH_3)-$), 1,2-ethylene ($-CH_2CH_2-$), 1,1-propylene ($-CH(CH_2CH_3)-$), 1,2-propylene ($-CH_2CH(CH_3)-$), 1,3-propylene ($-CH_2CH_2CH_2-$), 1,4-butylene ($-CH_2CH_2CH_2CH_2-$), etc. Alkylene may be substituted or unsubstituted, and when it is substituted, the substitution may occur at any available point of attachment, and the substituent is preferably selected from one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "heteroalkylene" refers to alkylene in which one or more $-CH_2-$ are replaced by one or more groups selected from N, O, S, S(O), and $S(O)_2$, wherein the alkyl is as defined above. Heteroalkylene may be substituted or

unsubstituted, and when it is substituted, the substitution with a substituent may occur at any available point of attachment, and the substituent is preferably selected from one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "alkenyl" refers to an alkyl compound containing at least one carbon-carbon double bond in the molecule, wherein the alkyl is as defined above; the alkenyl has 2 to 12 (e.g., 2, 3, 4, 5, 6, 7, 8, 9, 10, 11, or 12) carbon atoms (i.e., $C_{2-12}$ alkenyl). The alkenyl is preferably alkenyl having 2 to 6 carbon atoms (i.e., $C_{2-6}$ alkenyl). Alkenyl may be substituted or unsubstituted, and when it is substituted, the substituent is preferably selected from one or more of alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "alkynyl" refers to an alkyl compound containing at least one carbon-carbon triple bond in the molecule, wherein the alkyl is as defined above; the alkynyl has 2 to 12 (e.g., 2, 3, 4, 5, 6, 7, 8, 9, 10, 11, or 12) carbon atoms (i.e., $C_{2-12}$ alkynyl). The alkynyl is preferably alkynyl having 2 to 6 carbon atoms (i.e., $C_{2-6}$ alkynyl). Alkynyl may be substituted or unsubstituted, and when it is substituted, the substituent is preferably selected from one or more of alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "cycloalkyl" refers to a saturated or partially unsaturated monocyclic or polycyclic hydrocarbon substituent; the cycloalkyl ring contains 3 to 20 (e.g., 3, 4, 5, 6, 7, 8, 9, 10, 11, 12, 13, 14, 15, 16, 17, 18, 19, or 20) carbon atoms (i.e., 3- to 20-membered cycloalkyl), preferably 3 to 12 (e.g., 3, 4, 5, 6, 7, 8, 9, 10, 11, or 12) carbon atoms (i.e., 3- to 12-membered cycloalkyl), preferably 3 to 8 (e.g., 3, 4, 5, 6, 7, and 8) carbon atoms (i.e., 3- to 8-membered cycloalkyl), further preferably 4 to 7 (e.g., 4, 5, 6, and 7) carbon atoms (i.e., 4- to 7-membered cycloalkyl), and more preferably 3 to 6 (e.g., 3, 4, 5, and 6) carbon atoms (i.e., 3- to 6-membered cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl includes spirocycloalkyl, fused cycloalkyl, and bridged cycloalkyl.

The term "spirocycloalkyl" refers to a 5- to 20-membered (e.g., 5, 6, 7, 8, 9, 10, 11, 12, 13, 14, 15, 16, 17, 18, 19 or 20 ring atoms, i.e., 5- to 20-membered spirocycloalkyl) polycyclic group in which the monocyclic rings share one carbon atom (referred to as a spiro atom), and it may contain one or more double bonds. It is preferably 6- to 14-membered (i.e., 6- to 14-membered spirocycloalkyl), and is more preferably 7- to 10-membered (e.g., 7-, 8-, 9-, or 10-membered, i.e., 7- to 10-membered spirocycloalkyl). According to the number of spiro atoms shared between the rings, spirocycloalkyl may be monospirocycloalkyl, bispirocycloalkyl, or polyspirocycloalkyl, preferably monospirocycloalkyl and bispirocycloalkyl, and more preferably 3-membered/5-membered, 3-membered/6-membered, 4-membered/4-membered, 4-membered/5-membered, 4-membered/6-membered, 5-membered/5-membered, or 5-membered/6-membered monospirocycloalkyl. Non-limiting examples of spirocycloalkyl include:

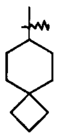 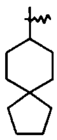 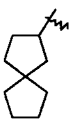 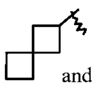 and 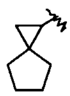 .

The term "fused cycloalkyl" refers to a 5- to 20-membered (e.g., 5, 6, 7, 8, 9, 10, 11, 12, 13, 14, 15, 16, 17, 18, 19, or 20 ring atoms, i.e., 5- to 20-membered fused cycloalkyl) all-carbon polycyclic group in which each of the rings in the system shares a pair of adjacent carbon atoms with the other rings in the system, wherein one or more of the rings may contain one or more double bonds. It is preferably 6- to 14-membered (i.e., 6- to 14-membered fused cycloalkyl), and is more preferably 7- to 10-membered (e.g., 7-, 8-, 9-, or 10-membered, i.e., 7- to 10-membered fused cycloalkyl). According to the number of constituent rings, it may be bicyclic, tricyclic, tetracyclic, or polycyclic fused cycloalkyl, preferably bicyclic or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic fused cycloalkyl. Non-limiting examples of fused cycloalkyl include:

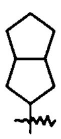 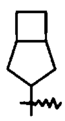 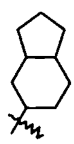 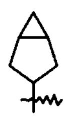 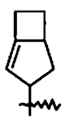 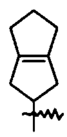 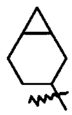 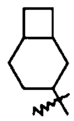 and 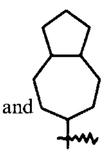 .

The term "bridged cycloalkyl" refers to a 5- to 20-membered (e.g., 5, 6, 7, 8, 9, 10, 11, 12, 13, 14, 15, 16, 17, 18, 19,

or 20 carbon atoms, i.e., 5- to 20-membered bridged cycloalkyl) all-carbon polycyclic group in which any two of the rings share two carbon atoms that are not directly connected, and it may contain one or more double bonds. It is preferably 6- to 14-membered (i.e., 6- to 14-membered bridged cycloalkyl), and is more preferably 7- to 10-membered (e.g., 7-, 8-, 9-, or 10-membered, i.e., 7- to 10-membered bridged cycloalkyl). According to the number of constituent rings, it may be bicyclic, tricyclic, tetracyclic, or polycyclic bridged cycloalkyl, preferably bicyclic, tricyclic, or tetracyclic bridged cycloalkyl, and more preferably bicyclic or tricyclic bridged cycloalkyl. Non-limiting examples of bridged cycloalkyl include:

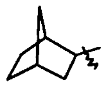 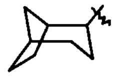 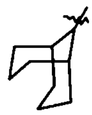 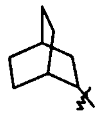 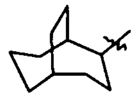

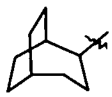 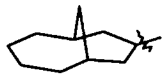 and 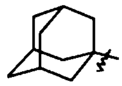 .

The cycloalkyl ring includes those in which the cycloalkyl described above (including monocyclic cycloalkyl, spirocycloalkyl, fused cycloalkyl, and bridged cycloalkyl) is fused to an aryl, heteroaryl, or heterocycloalkyl ring, wherein the ring attached to the parent structure is cycloalkyl. Non-limiting examples include

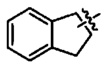 , 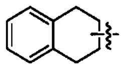 , 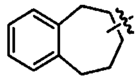 ,

etc.;

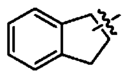

or

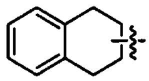

is preferred.

Cycloalkyl may be substituted or unsubstituted, and when it is substituted, the substitution may occur at any available point of attachment, and the substituent is preferably selected from one or more of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples of alkoxy include: methoxy, ethoxy, propoxy, and butoxy. Alkoxy may be optionally substituted or unsubstituted, and when it is substituted, the substituent is preferably one or more groups independently selected from a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "heterocyclyl" refers to a saturated or partially unsaturated monocyclic or polycyclic substituent containing 3 to 20 ring atoms (e.g.,

3, 4, 5, 6, 7, 8, 9, 10, 11, 12, 13, 14, 15, 16, 17, 18, 19, or 20 ring atoms, i.e. 3-to 20-membered heterocyclyl), wherein one or more of the ring atoms are heteroatoms selected from nitrogen, oxygen, and sulfur (the sulfur may be optionally substituted with oxo

(i.e., forming sulfoxide or sulfone)), but excluding a ring moiety of -O-O-, -O-S-, or -S-S-, and the other ring atoms are carbon. Preferably, it contains 3 to 12 (e.g., 3, 4, 5, 6, 7, 8, 9, 10, 11, and 12) ring atoms (i.e., 3- to 12-membered heterocyclyl), 1-4 (e.g., 1, 2, 3, and 4) of which are heteroatoms; more preferably, it contains 3 to 8 (e.g., 3, 4, 5, 6, 7, and 8) ring atoms (i.e., 3-to 8-membered heterocyclyl), 1-3 (e.g., 1, 2, and 3) of which are heteroatoms; further preferably, it contains 4 to 7 (e.g., 4, 5, 6, and 7) ring atoms (i.e., 4- to 7-membered heterocyclyl), 1-3 (e.g., 1, 2, and 3) of which are heteroatoms; more preferably, it contains 3 to 6 (e.g., 3, 4, 5, and 6) ring atoms (i.e., 3- to 6-membered heterocyclyl), 1-3 (e.g., 1, 2, and 3) of which are heteroatoms; most preferably, it contains 5 or 6 ring atoms (i.e., 5- or 6-membered

heterocyclyl), 1-2 (e.g., 1 or 2) of which are heteroatoms. Non-limiting examples of monocyclic heterocyclyl include pyrrolidinyl, tetrahydropyranyl, 1,2,3,6-tetrahydropyridinyl, piperidinyl, piperazinyl, morpholinyl, thiomorpholinyl, homopiperazinyl, etc. Polycyclic heterocyclyl includes spiroheterocyclyl, fused heterocyclyl, and bridged heterocyclyl.

The term "spiroheterocyclyl" refers to a 5- to 20-membered (e.g., 5, 6, 7, 8, 9, 10, 11, 12, 13, 14, 15, 16, 17, 18, 19, or 20 ring atoms, i.e., 5- to 20-membered spiroheterocyclyl) polycyclic heterocyclyl group in which the monocyclic rings share one atom (referred to as a spiro atom), wherein one or more of the ring atoms are heteroatoms selected from nitrogen, oxygen, and sulfur (the sulfur may be optionally substituted with oxo (i.e., forming sulfoxide or sulfone)), and the other ring atoms are carbon. It may contain one or more double bonds. It is preferably 6- to 14-membered (i.e., 6- to 14-membered spiroheterocyclyl), and is more preferably 7- to 10-membered (e.g., 7-, 8-, 9-, or 10-membered, i.e., 7- to 10-membered spiroheterocyclyl). According to the number of spiro atoms shared between the rings, spiroheterocyclyl may be monospiroheterocyclyl, bispiroheterocyclyl, or polyspiroheterocyclyl, preferably monospiroheterocyclyl and bispiroheterocyclyl, and more preferably 3-membered/5-membered, 3-membered/6-membered, 4-membered/4-membered, 4-membered/5-membered, 4-membered/6-membered, 5-membered/5-membered, or 5-membered/6-membered monospiroheterocyclyl. Non-limiting examples of spiroheterocyclyl include:

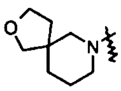 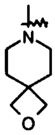 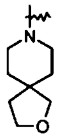 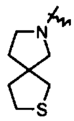 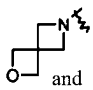 and 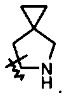.

The term "fused heterocyclyl" refers to a 5- to 20-membered (e.g., 5, 6, 7, 8, 9, 10, 11, 12, 13, 14, 15, 16, 17, 18, 19, or 20 ring atoms, i.e., 5- to 20-membered fused heterocyclyl) polycyclic heterocyclyl group in which each of the rings in the system shares a pair of adjacent atoms with the other rings in the system, wherein one or more of the rings may contain one or more double bonds; one or more of the ring atoms are heteroatoms selected from nitrogen, oxygen, and sulfur (the sulfur may be optionally substituted with oxo (i.e., forming sulfoxide or sulfone)), and the other ring atoms are carbon. It is preferably 6- to 14-membered (i.e., 6- to 14-membered fused heterocyclyl), and is more preferably 7- to 10-membered (e.g., 7-, 8-, 9-, or 10-membered, i.e., 7- to 10-membered fused heterocyclyl). According to the number of constituent rings, it may be bicyclic, tricyclic, tetracyclic, or polycyclic fused heterocyclyl, preferably bicyclic or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic fused heterocyclyl. Non-limiting examples of fused heterocyclyl include:

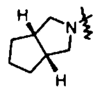 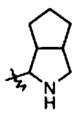 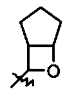 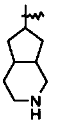 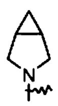 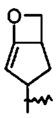 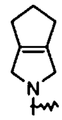 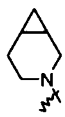

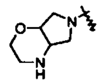 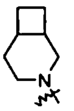 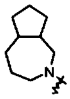 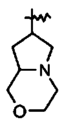 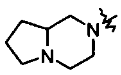 and 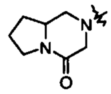.

The term "bridged heterocyclyl" refers to a 5- to 14-membered (e.g., 5, 6, 7, 8, 9, 10, 11, 12, 13, or 14 ring atoms, i.e., 5- to 14-membered bridged heterocyclyl) polycyclic heterocyclyl group in which any two of the rings share two atoms that are not directly connected, and it may contain one or more double bonds, wherein one or more of the ring atoms are heteroatoms selected from nitrogen, oxygen, and sulfur (the sulfur may be optionally substituted with oxo (i.e., forming sulfoxide or sulfone)), and the other ring atoms are carbon. It is preferably 6- to 14-membered (i.e., 6- to 14-membered bridged heterocyclyl), and is more preferably 7- to 10-membered (e.g., 7-, 8-, 9-, or 10-membered, i.e., 7- to 10-membered bridged heterocyclyl). According to the number of constituent rings, it may be bicyclic, tricyclic, tetracyclic, or polycyclic bridged heterocyclyl, preferably bicyclic, tricyclic, or tetracyclic bridged heterocyclyl, and more preferably bicyclic or tricyclic bridged heterocyclyl. Non-limiting examples of bridged heterocyclyl include:

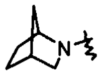 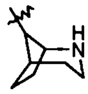 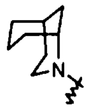 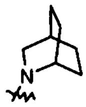

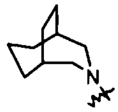 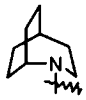 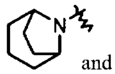 and 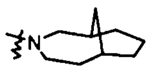

The heterocyclyl ring includes those in which the heterocyclyl described above (including monocyclic heterocyclyl, spiroheterocyclyl, fused heterocyclyl, and bridged heterocyclyl) is fused to an aryl, heteroaryl, or cycloalkyl ring, wherein the ring attached to the parent structure is heterocyclyl; its non-limiting examples include:

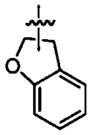、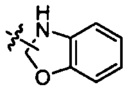、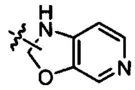 and 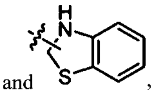, etc.

Heterocyclyl may be substituted or unsubstituted, and when it is substituted, the substitution may occur at any available point of attachment, and the substituent is preferably selected from one or more of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "aryl" refers to a 6- to 14-membered (e.g., 6, 7, 8, 9, 10, 11, 12, 13 or 14 ring atoms, i.e., 6- to 14-membered aryl) all-carbon monocyclic or fused polycyclic (fused polycyclic describes the rings sharing a pair of adjacent carbon atoms) group having a conjugated π-electron system, preferably 6- to 10-membered (i.e., 6- to 10-membered aryl), such as phenyl and naphthyl. The aryl ring includes those in which the aryl ring described above is fused to a heteroaryl, heterocyclyl, or cycloalkyl ring, wherein the ring attached to the parent structure is the aryl ring; its non-limiting examples include:

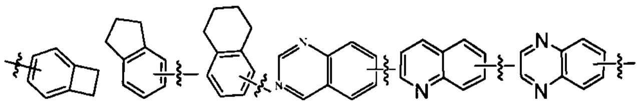

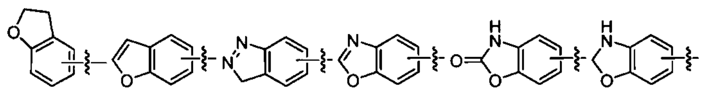

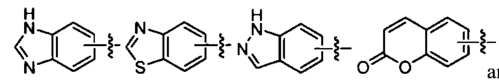 and 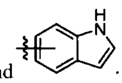.

Aryl may be substituted or unsubstituted, and when it is substituted, the substitution may occur at any available point of attachment, and the substituent is preferably selected from one or more of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "heteroaryl" refers to a heteroaromatic system containing 1 to 4 (e.g., 1, 2, 3, and 4) heteroatoms and 5 to 14 ring atoms (e.g., 5, 6, 7, 8, 9, 10, 11, 12, 13, or 14 ring atoms, i.e., 5- to 14-membered heteroaryl), wherein the heteroatoms are selected from oxygen, sulfur, and nitrogen. The heteroaryl is preferably 5- to 10-membered (e.g., 5-, 6-, 7-, 8-, 9- or 10-membered, i.e., 5- to 10-membered heteroaryl), and is more preferably 5- or 6-membered (i.e., 5- or 6-membered heteroaryl), such as furanyl, thienyl, pyridinyl, pyrrolyl, N-alkylpyrrolyl, pyrimidinyl, pyrazinyl, pyridazinyl, imidazolyl, pyrazolyl, triazolyl, tetrazolyl, etc. The heteroaryl ring includes those in which the heteroaryl ring described above is fused to an aryl, heterocyclyl, or cycloalkyl ring, wherein the ring attached to the parent structure is the heteroaryl ring; its non-limiting examples include:

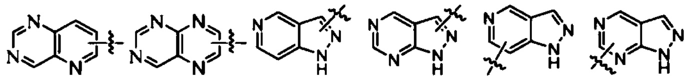

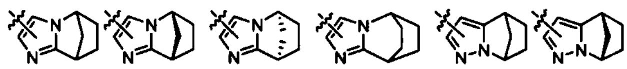

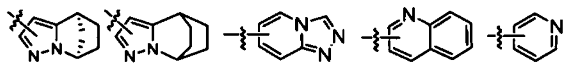

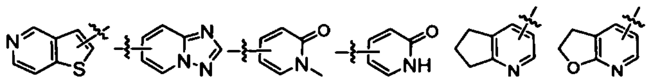

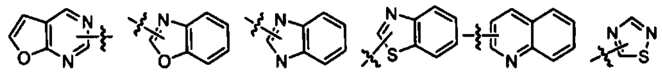

and

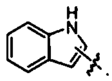.

Heteroaryl may be substituted or unsubstituted, and when it is substituted, the substitution may occur at any available point of attachment, and the substituent is preferably selected from one or more of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The cycloalkyl, heterocyclyl, aryl, and heteroaryl described above include residues derived from the parent ring by removal of one hydrogen atom from a ring atom, or residues derived from the parent ring by removal of two hydrogen atoms from the same ring atom or two different ring atoms, i.e., "divalent cycloalkyl", "divalent heterocyclyl" (e.g.,

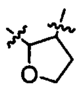, 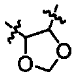,

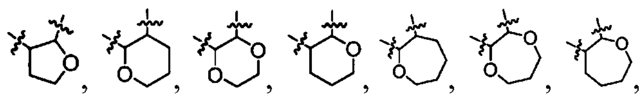,

etc.), "arylene", and "heteroarylene".

The term "cycloalkylalkyl" refers to alkyl substituted with one or more cycloalkyl groups, wherein the cycloalkyl and alkyl are as defined above.

The term "heterocyclylalkyl" refers to alkyl substituted with one or more heterocyclyl groups, wherein the heterocyclyl and alkyl are as defined above.

The term "heteroarylalkyl" refers to alkyl substituted with one or more heteroaryl groups, wherein the heteroaryl and alkyl are as defined above.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "alkoxyalkyl" refers to alkyl substituted with one or more alkoxy groups, wherein the alkyl and alkoxy are as defined above.

The term "hydroxyalkyl" refers to alkyl substituted with one or more hydroxyl groups, wherein the alkyl is as defined above.

The term "halogen" refers to fluorine, chlorine, bromine, or iodine.

The term "hydroxyl" refers to -OH.

The term "sulfhydryl" refers to -SH.

The term "amino" refers to $-NH_2$.

The term "cyano" refers to -CN.

The term "nitro" refers to $-NO_2$.

The term "oxo" refers to "=O".

The term "carbonyl" refers to C=O.

The term "aldehyde group" refers to -C(O)H.

The term "carboxyl" refers to -C(O)OH.

The term "carboxylate ester group" refers to -C(O)O(alkyl) or -C(O)O(cycloalkyl), wherein the alkyl and cycloalkyl are as defined above.

In another aspect, the compounds of the present disclosure may have particular geometric or stereoisomeric forms. The present disclosure contemplates all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present disclosure. Additional asymmetric carbon atoms may be present in the substituents such as alkyl. All such isomers and mixtures thereof are included within the scope of the present disclosure. Optically active (R)- and (S)-enantiomers and D- and L-isomers can be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), the compound reacts with an appropriate optically active acid or base to form a diastereomeric salt, which is then subjected to diastereomer resolution through conventional methods well known in the art to acquire the pure enantiomer. Furthermore, separation of enantiomers and diastereomers is generally accomplished by chromatography using a chiral stationary phase, optionally in combination with chemical derivatization (e.g., carbamate formation from amines).

In the chemical structure of the compound of the present disclosure, the bond 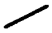 indicates an unspecified configuration; that is, if chiral isomers exist in the chemical structure, the bond 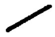 may be 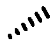 or 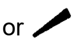, or may include both the configurations. In the chemical structure of the compound of the present disclosure, the bond 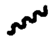 does not specify a configuration; that is, the chemical structure may be in a Z configuration or an E configuration, or may include both the configurations.

In addition, the compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as prototropic tautomers) include interconversion via proton migration, such as keto-enol isomerization and imine-enamine isomerization. An example of a lactam-lactim equilibrium is present between A and B as shown below.

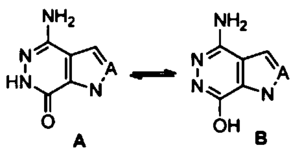

A B

All compounds in the present disclosure can be drawn as form A or form B. All tautomeric forms are within the scope of the present disclosure. The nomenclature of the compounds does not exclude any tautomers.

The present disclosure also includes some isotopically labeled compounds of the present disclosure that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, $^{36}Cl$, etc.

The compound of the present disclosure may contain an unnatural proportion of atomic isotope at one or more of the atoms that constitute the compound. For example, the compound may be labeled with a radioisotope, such as tritium ($^{3}H$). Hydrogen may be replaced by deuterium to form a deuterated drug. The bond formed between deuterium and carbon is firmer than that formed between ordinary hydrogen and carbon. Compared to an undeuterated drug, the deuterated drug has the advantages of reduced toxic and side effects, increased stability, enhanced efficacy, prolonged biological half-life,

etc. All isotopic variations of the compounds of the present disclosure, whether radioactive or not, are encompassed within the scope of the present disclosure.

Furthermore, substitution with heavier isotopes such as deuterium (i.e., $^{2}H$) may provide certain therapeutic advantages (e.g., increased *in vivo* half-life or reduced dose requirement) resulting from greater metabolic stability and hence may be preferred in certain circumstances in which deuterium substitution may be partial or complete, wherein partial deuterium substitution refers to substitution of at least one hydrogen with at least one deuterium.

Unless otherwise specified, when a position is specifically designated as deuterium (D), that position shall be understood as deuterium with an abundance that is at least 1000 times greater than the natural abundance of deuterium (which is 0.015%) (i.e., at least 10% deuterium incorporation). The deuterium in the compounds of the examples with an abundance greater than the natural abundance of deuterium may be deuterium with an abundance that is at least 1000 times, at least 2000 times, at least 3000 times, at least 4000 times, at least 5000 times, at least 6000 times, or higher times the natural abundance. The present disclosure also includes various deuterated forms of the compound of formula (I). Each available hydrogen atom attached to a carbon atom may be independently replaced by a deuterium atom. Those skilled in the art are able to synthesize the deuterated forms of the compound of formula (I) with reference to the relevant literature. Commercially available deuterated starting materials can be used in preparing the deuterated forms of the compound of formula (I), or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, etc.

"Optional" or "optionally" means that the event or circumstance subsequently described may, but does not necessarily, occur. This description includes instances where the event or circumstance occurs or does not occur. For example, "heterocyclyl group optionally substituted with alkyl" means that alkyl may, but does not necessarily, exist. This description includes the instance where the heterocyclyl group is substituted with alkyl and the instance where it is not.

"Substituted" means that one or more, preferably 1-5, and more preferably 1-3, hydrogen atoms in the group are independently substituted with a corresponding number of substituents. Those skilled in the art are able to determine (experimentally or theoretically) possible or impossible substitutions without undue effort. For example, it may be unstable when an amino or hydroxyl group having free hydrogen is bound to a carbon atom having an unsaturated (e.g., olefinic) bond.

"Pharmaceutical composition" refers to a mixture containing one or more of the compounds or the physiologically/pharmaceutically acceptable salts or prodrugs thereof described herein, and other chemical components, as well as other components such as physiologically/pharmaceutically acceptable carriers and excipients. The pharmaceutical composition is intended to promote administration to an organism and facilitate the absorption of the active ingredient so that it can exert its biological activity.

"Pharmaceutically acceptable salt" refers to a salt of the compound of the present disclosure, which, when used in a mammal, possesses safety

and effectiveness and has the intended biological activity. The salt may be separately prepared during the final isolation and purification of the compound, or by reacting a suitable group with a suitable base or acid. Bases commonly used to form pharmaceutically acceptable salts include inorganic bases, such as sodium hydroxide and potassium hydroxide, and organic bases, such as ammonia. Acids commonly used to form pharmaceutically acceptable salts include inorganic acids and organic acids.

For drugs or pharmacologically active agents, the term "therapeutically effective amount", "inhibitory effective amount", or "prophylactically effective amount" refers to an amount of the drug or agent sufficient to achieve, or partially achieve, the desired effect. The determination of the effective amount varies from person to person. It depends on the age and general condition of a subject, as well as the specific active substance used. The appropriate effective amount in a case may be determined by those skilled in the art in the light of routine tests.

The term "pharmaceutically acceptable" as used herein refers to those compounds, materials, compositions, and/or dosage forms that are, within the scope of sound medical judgment, suitable for use in contact with the tissues of patients without excessive toxicity, irritation, allergic reaction, or other problems or complications, and are commensurate with a reasonable benefit/risk ratio and effective for the intended use.

As used herein, the singular forms "a", "an", and "the" include plural references and vice versa, unless the context clearly indicates otherwise.

When the term "about" or "approximately" is applied to parameters such as pH, concentration, temperature, etc., it means that the parameter may vary by $\pm 10\%$, and sometimes more preferably within $\pm 5\%$. As will be understood by those skilled in the art, when the parameters are not critical, the numbers are generally given for illustrative purposes only and are not intended to be limiting.

It should be noted that in the case where the structure of a compound described in this specification is not consistent with the chemical formula, the structure shall prevail.

## Compounds of the Present Disclosure

In some aspects, the present disclosure provides a compound of formula (I) or a pharmaceutically acceptable salt, an ester, a stereoisomer, a tautomer, a polymorph, a hydrate, a solvate, an N-oxide, an isotopically labeled compound, a metabolite, or a prodrug thereof:

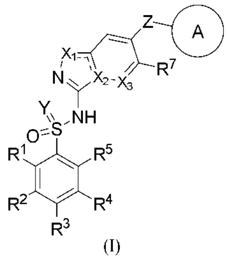

(I)

wherein:

the dashed line is an optional chemical bond;
$R^1$, $R^2$, and $R^3$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
$R^4$ and $R^5$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, $C_{3-6}$ halocycloalkyl, $C_{3-6}$ cycloalkoxy, $C_{3-6}$ halocycloalkoxy, 6- to 10-membered aryl, 5- to 10-membered heteroaryl, and $C_{1-6}$ hydroxyalkyl, or $R^4$ and $R^5$, together with the carbon atoms to which they are attached, form 4- to 10-membered heterocyclyl, 5- to 6-membered heteroaryl, 7- to 12-membered spiroheterocyclyl, or 8- to 15-membered fused heterocyclyl, wherein the 4- to 10-membered heterocyclyl, 5- to 6-membered heteroaryl, 7- to 12-membered spiroheterocyclyl, or 8- to 15-membered fused heterocyclyl is optionally substituted with one or more substituents selected from: hydroxyl, amino, halogen, $C_{1-6}$ alkyl, $C_{1.6}$ haloalkyl, $C_{1-6}$ hydroxyalkyl, $C_{1-6}$ alkoxy, $C_{1-6}$ haloalkoxy, $-C(O)C_{1-6}$ alkyl, $-C(O)C_{3-6}$ cycloalkyl, $-C(O)C_{1-6}$ haloalkyl, $-C(O)C_{3-6}$ halocycloalkyl, $-C(O)OC_{1-6}$ alkyl, $-S(O)_2C_{1-6}$ alkyl, - $S(O)_2C_{3-6}$ cycloalkyl, $-S(O)_2C_{1-6}$ haloalkyl, $-S(O)_2C_{3-6}$ halocycloalkyl, $-PO(C_{1-6}$ alkyl$)_2$, $-PO(C_{3-6}$ cycloalkyl$)_2$, $-PO(C_{1-6}$ haloalkyl$)_2$, $-PO(C_{3-6}$ halocycloalkyl$)_2$, oxo, $=CH_2$, $=CHC_{1-6}$ alkyl, $=C(C_{1-6}$ alkyl$)_2$, $=CHC_{1-6}$ haloalkyl, $=C(C_{1-6}$ haloalkyl$)_2$, $-NHC_{1-6}$ alkyl, $-N(C_{1-6}$ alkyl$)_2$, cyano, benzyl, $C_{3-6}$ cycloalkyl, $C_{3-6}$ heterocyclyl, $C_{3-6}$ halocycloalkyl, $C_{3-6}$ haloheterocyclyl, $C_{3-6}$ cycloalkoxy, $C_{3-6}$ halocycloalkoxy, 6- to 10-membered aryl, 5- to 10-membered heteroaryl, and $C_{2-6}$ alkenyl;
ring A is selected from 3- to 8-membered heterocyclyl, 6- to 10-membered aryl, and 5-to 10-membered heteroaryl and 6- to 12-membered fused heterocyclyl containing at least one nitrogen atom; ring A is optionally substituted with one or more substituents selected from: halogen, amino, hydroxyl, cyano, $C_{1-6}$ alkyl, $C_{1-6}$ haloalkyl, $C_{1-6}$ alkoxy, 3- to 8-membered cycloalkoxy, $C_{1-6}$ haloalkoxy, 3- to 8-membered halocycloalkoxy, oxo, and $C_{2-6}$ alkenyl;
$X_1$ is O, S, C, or N;
$X_2$ is N or C;
$X_3$ is N or $CR_6$;
$R^6$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-8}$ cycloalkyl, $C_{3-8}$ halocycloalkyl, $C_{3-8}$ cycloalkoxy, and $C_{3-8}$ halocycloalkoxy;
$R^7$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-8}$ cycloalkyl, $C_{3-8}$ halocycloalkyl, $C_{3-8}$ cycloalkoxy, and $C_{3-8}$ halocycloalkoxy; and
Y is O or NH; and
Z is $-CH_2-$, $-CF_2-$, -C(O)-, O, -S(O)-, $-S(O)_2-$, or -NH-.

In some embodiments of the compound of formula (I), $X_1$ is O. In some embodiments of the compound of formula (I), $X_1$ is S. In some embodiments of the compound of formula (I), $X_1$ is C. In some embodiments of the compound of formula (I), $X_1$ is N.
In some embodiments of the compound of formula (I), $X_2$ is N. In some embodiments of the compound of formula (I), $X_2$ is C.
In some embodiments of the compound of formula (I), $X_3$ is N.
In some embodiments of the compound of formula (I), $X_3$ is $CR_6$, wherein $R^6$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-8}$ cycloalkyl, $C_{3-8}$ halocycloalkyl, $C_{3-8}$ cycloalkoxy, and $C_{3-8}$ halocycloalkoxy.

In some embodiments of the compound of formula (I), Z is $-CH_2-$. Alternatively, in some embodiments of the compound of formula (I), Z is $-CF_2-$. Alternatively, in some embodiments of the compound of formula (I), Z is -C(O)-. Alternatively, in some embodiments of the compound of formula (I), Z is O. Alternatively, in some embodiments of the compound of formula (I), Z is -S(O)-. Alternatively, in some embodiments of the compound of formula (I), Z is $-S(O)_2-$. Alternatively, in some embodiments of the compound of formula (I), Z is -NH-.

In some aspects, the present disclosure provides a compound of formula (II-1) or a pharmaceutically acceptable salt, an ester, a stereoisomer,

a tautomer, a polymorph, a hydrate, a solvate, an N-oxide, an isotopically labeled compound, a metabolite, or a prodrug thereof,

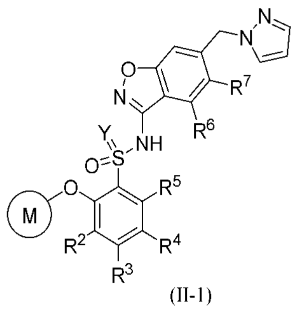

(II-1)

wherein:

$R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;

$R^4$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, $C_{3-6}$ halocycloalkyl, $C_{3-6}$ cycloalkoxy, $C_{3-6}$ halocycloalkoxy, and $C_{1-6}$ hydroxyalkyl;

$R^5$ is selected from a hydrogen atom, $C_{1-4}$ alkoxy, $C_{1-4}$ haloalkoxy, $C_{4-6}$ cycloalkoxy, and $C_{4-6}$ halocycloalkoxy;

$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;

ring M is selected from 4- to 7-membered cycloalkyl and 4- to 7-membered halocycloalkyl;

Y is O or NH.

In some embodiments of the compound of formula (II-1), $R^2$ and $R^3$ are identical or different and are each independently selected from a hydrogen atom, halogen, $C_{1-4}$ alkyl, and $C_{1-4}$ alkoxy. Preferably, in some embodiments of the compound of formula (II-1), $R^2$ and $R^3$ are identical or different and are each independently selected from a hydrogen atom and halogen. More preferably, in some embodiments of the compound of formula (II-1), both $R^2$ and $R^3$ are hydrogen atoms.

In some embodiments of the compound of formula (II-1), $R^4$ is selected from a hydrogen atom, $C_{1-6}$ alkyl, $C_{3-6}$ heterocyclyl, and $C_{1-6}$ hydroxyalkyl. Preferably, in some embodiments of the compound of formula (II-1), $R^4$ is selected from a hydrogen atom, ethyl, isopropyl, and *tert*-butyl. More specifically, in some embodiments of the compound of formula (II-1), $R^4$ is a hydrogen atom. In some other embodiments of the compound of formula (II-1), $R^4$ is ethyl. In some other embodiments of the compound of formula (II-1), $R^4$ is isopropyl. In some other embodiments of the compound of formula (II-1), $R^4$ is *tert*-butyl.

In some embodiments of the compound of formula (II-1), $R^5$ is selected from a hydrogen atom, methoxy, difluoromethoxy, trifluoromethoxy, $-OCH_2CHF_2$, cyclobutyloxy, and

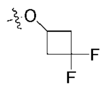

.

Preferably, in some embodiments of the compound of formula (II-1), $R^5$ is a hydrogen atom or methoxy. More specifically, in some embodiments of the compound of formula (II-1), $R^5$ is a hydrogen atom. In some other embodiments of the compound of formula (II-1), $R^5$ is methoxy.

In some embodiments of the compound of formula (II-1), $R^6$ and $R^7$ are identical or different and are each independently selected from a hydrogen atom, a chlorine atom, a fluorine atom, methoxy, ethoxy, isopropoxy, difluoromethoxy, and cyclopropyl.

In some embodiments of the compound of formula (II-1), ring M is selected from cyclobutyl and

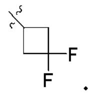

More specifically, in some embodiments of the compound of formula (II-1), ring M is cyclobutyl. In some other embodiments of the compound of formula (II-1), ring M is

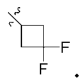

In some embodiments of the compound of formula (II-1), Y is O. In some other embodiments of the compound of formula (II-1), Y is NH.

In some aspects, the present disclosure provides a compound of formula (II-2) or a pharmaceutically acceptable salt, an ester, a stereoisomer, a tautomer, a polymorph, a hydrate, a solvate, an N-oxide, an isotopically labeled compound, a metabolite, or a prodrug thereof,

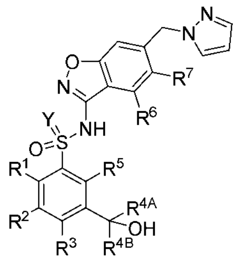

(II-2)

wherein:

$R^1$ is selected from $C_{1-4}$ alkoxy, $C_{1-4}$ haloalkoxy, $C_{3-6}$ cycloalkoxy, and $C_{3-6}$ halocycloalkoxy;
$R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;
$R^{4A}$ and $R^{4B}$ are identical or different and are each independently selected from $C_{1-6}$ alkyl and $C_{1-6}$ haloalkyl;
$R^5$ is selected from a hydrogen atom, $C_{1-4}$ alkoxy, $C_{1-4}$ haloalkoxy, $C_{4-6}$ cycloalkoxy, and $C_{4-6}$ halocycloalkoxy;
$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
ring M is selected from 4- to 7-membered cycloalkyl and 4- to 7-membered halocycloalkyl;
Y is O or NH.

In some embodiments of the compound of formula (II-2), $R^1$ is selected from methoxy, difluoromethoxy, trifluoromethoxy, $-OCH_2CHF_2$, cyclobutyloxy, and

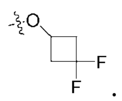

In some embodiments of the compound of formula (II-2), $R^2$ and $R^3$ are identical or different and are each independently selected from a hydrogen atom, halogen, $C_{1-4}$ alkyl, and $C_{1-4}$ alkoxy. Preferably, in some embodiments of the compound of formula (II-2), $R^2$ and $R^3$ are identical or different and are each independently selected from a hydrogen atom and halogen. More preferably, in some embodiments of the compound of formula (II-2), both $R^2$ and $R^3$ are hydrogen atoms.

In some embodiments of the compound of formula (II-2), both $R^{4A}$ and $R^{4B}$ are methyl.

In some embodiments of the compound of formula (II-2), $R^5$ is selected from a hydrogen atom, methoxy, difluoromethoxy, trifluoromethoxy, $-OCH_2CHF_2$, cyclobutyloxy, and

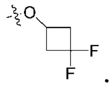

Preferably, in some embodiments of the compound of formula (II-2), $R^5$ is selected from a hydrogen atom and methoxy. More specifically, in some embodiments of the compound of formula (II-2), $R^5$ is a hydrogen atom. In some other embodiments of the compound of formula (II-2), $R^5$ is methoxy.

In some embodiments of the compound of formula (II-2), $R^6$ and $R^7$ are identical or different and are each independently selected from a hydrogen atom, a chlorine atom, a fluorine atom, methoxy, ethoxy, isopropoxy, difluoromethoxy, and cyclopropyl.

In some embodiments of the compound of formula (II-2), ring M is cyclobutyl. In some other embodiments of the compound of formula (II-2), ring M is

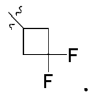

In some embodiments of the compound of formula (II-2), Y is O. In some other embodiments of the compound of formula (II-2), Y is NH.

In some aspects, the present disclosure provides a compound of formula (II-3) or a pharmaceutically acceptable salt, an ester, a stereoisomer, a tautomer, a polymorph, a hydrate, a solvate, an N-oxide, an isotopically labeled compound, a metabolite, or a prodrug thereof,

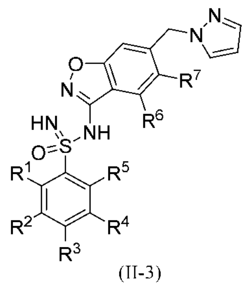

(II-3)

wherein:

$R^1$ is selected from $C_{1-4}$ alkoxy, $C_{1-4}$ haloalkoxy, $C_{3-6}$ cycloalkoxy, and $C_{3-6}$ halocycloalkoxy;
$R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;
$R^4$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, $C_{3-6}$ halocycloalkyl, $C_{3-6}$ cycloalkoxy, $C_{3-6}$ halocycloalkoxy, and $C_{1-6}$ hydroxyalkyl;
$R^5$ is selected from a hydrogen atom, $C_{1-4}$ alkoxy, $C_{1-4}$ haloalkoxy, $C_{4-6}$ cycloalkoxy, and $C_{4-6}$ halocycloalkoxy;
$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy.

In some embodiments of the compound of formula (II-3), $R^1$ is selected from methoxy, difluoromethoxy, trifluoromethoxy, $-OCH_2CHF_2$, cyclobutyloxy, and

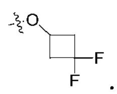

In some embodiments of the compound of formula (II-3), $R^2$ and $R^3$ are identical or different and are each independently selected from a hydrogen atom, halogen, $C_{1-4}$ alkyl, and $C_{1-4}$ alkoxy. Preferably, in some embodiments of the compound of formula (II-3), $R^2$ and $R^3$ are identical or different and are each independently selected from a hydrogen atom and halogen. More preferably, in some embodiments of the compound of formula (II-3), both $R^2$ and $R^3$ are hydrogen atoms.

In some embodiments of the compound of formula (II-3), $R^4$ is selected from a hydrogen atom, $C_{1-6}$ alkyl, $C_{3-6}$

heterocyclyl, and $C_{1-6}$ hydroxyalkyl. Preferably, in some embodiments of the compound of formula (II-3), $R^4$ is selected from a hydrogen atom, ethyl, isopropyl, and *tert*-butyl.

In some embodiments of the compound of formula (II-3), $R^5$ is selected from a hydrogen atom, methoxy, difluoromethoxy, trifluoromethoxy, $-OCH_2CHF_2$, cyclobutyloxy, and

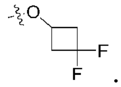

Preferably, in some embodiments of the compound of formula (II-3), $R^5$ is a hydrogen atom or methoxy.

In some embodiments of the compound of formula (II-3), $R^6$ and $R^7$ are identical or different and are each independently selected from a hydrogen atom, a chlorine atom, a fluorine atom, methoxy, ethoxy, isopropoxy, difluoromethoxy, and cyclopropyl.

In some aspects, the present disclosure provides a compound of formula (III) or a pharmaceutically acceptable salt, an ester, a stereoisomer, a tautomer, a polymorph, a hydrate, a solvate, an N-oxide, an isotopically labeled compound, a metabolite, or a prodrug thereof,

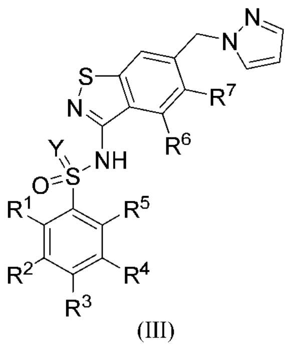

(III)

wherein:

- $R^1$, $R^2$, and $R^3$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
- $R^4$ and $R^5$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, $C_{3-6}$ cycloalkoxy, and $C_{1-6}$ hydroxyalkyl;
- $R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
- Y is O or NH.

In some embodiments of the compound of formula (III), $R^1$ is selected from $C_{1-6}$ alkoxy, $C_{1-6}$ haloalkoxy, $C_{3-6}$ cycloalkoxy, and $C_{3-6}$ halocycloalkoxy.

In some embodiments of the compound of formula (III), $R^2$ and $R^3$ are identical or different and are each independently selected from a hydrogen atom, halogen, and $C_{1-6}$ alkyl.

Preferably, in some embodiments of the compound of formula (III), $R^1$ is methoxy, and both $R^2$ and $R^3$ are hydrogen atoms.

In some embodiments of the compound of formula (III), $R^4$ is selected from a hydrogen atom, $C_{1-6}$ alkyl, $C_{3-6}$ heterocyclyl, and $C_{1-6}$ hydroxyalkyl.

Preferably, in some embodiments of the compound of formula (III), $R^4$ is selected from a hydrogen atom, ethyl, isopropyl, *tert*-butyl, and

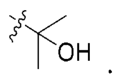

More preferably, in some embodiments of the compound of formula (III), $R^4$ is a hydrogen atom.

In some embodiments of the compound of formula (III), $R^5$ is selected from a hydrogen atom, $C_{1-4}$ alkoxy, $C_{1-4}$ haloalkoxy, $C_{3-6}$ cycloalkoxy, and $C_{3-6}$ halocycloalkoxy. Preferably, in some embodiments of the compound of formula (III),

$R^5$ is selected from a hydrogen atom, methoxy, difluoromethoxy, trifluoromethoxy, $-OCH_2CHF_2$, cyclobutyloxy, and

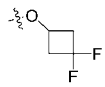.

More specifically, in some embodiments of the compound of formula (III), $R^5$ is a hydrogen atom. In some other embodiments of the compound of formula (III), $R^5$ is methoxy.

In some embodiments of the compound of formula (III), $R^6$ and $R^7$ are identical or different and are each independently selected from a hydrogen atom, a chlorine atom, a fluorine atom, methoxy, ethoxy, isopropoxy, difluoromethoxy, and cyclopropyl.

In some embodiments of the compound of formula (III), Y is O. In some other embodiments of the compound of formula (III), Y is NH.

In some aspects, the present disclosure provides a compound of formula (IV) or a pharmaceutically acceptable salt, an ester, a stereoisomer, a tautomer, a polymorph, a hydrate, a solvate, an N-oxide, an isotopically labeled compound, a metabolite, or a prodrug thereof,

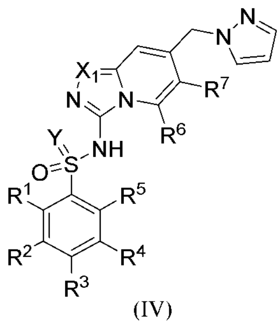

(IV)

wherein:

- $R^1$, $R^2$, and $R^3$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
- $R^4$ and $R^5$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, $C_{3-6}$ cycloalkoxy, and $C_{1-6}$ hydroxyalkyl;
- $R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
- $X_1$ is C or N;
- Y is O or NH.

In some embodiments of the compound of formula (IV), $R^1$ is selected from $C_{1-6}$ alkoxy, $C_{1-6}$ haloalkoxy, $C_{3-6}$ cycloalkoxy, and $C_{3-6}$ halocycloalkoxy. Preferably, in some embodiments of the compound of formula (IV), $R^1$ is methoxy.

In some embodiments of the compound of formula (IV), $R^2$ and $R^3$ are identical or different and are each independently selected from a hydrogen atom, halogen, and $C_{1-6}$ alkyl. Preferably, in some embodiments of the compound of formula (IV), both $R^2$ and $R^3$ are hydrogen atoms.

In some embodiments of the compound of formula (IV), $R^4$ is selected from a hydrogen atom, $C_{1-6}$ alkyl, $C_{3-6}$ heterocyclyl, and $C_{1-6}$ hydroxyalkyl. Preferably, in some embodiments of the compound of formula (IV), $R^4$ is selected from a hydrogen atom, ethyl, isopropyl, *tert*-butyl, and

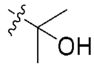.

More preferably, in some embodiments of the compound of formula (IV), $R^4$ is a hydrogen atom.

In some embodiments of the compound of formula (IV), $R^5$ is selected from a hydrogen atom, $C_{1-4}$ alkoxy, $C_{1-4}$ haloalkoxy, $C_{3-6}$ cycloalkoxy, and $C_{3-6}$ halocycloalkoxy. Preferably, in some embodiments of the compound of formula (IV), $R^5$ is selected from a hydrogen atom, methoxy, difluoromethoxy, trifluoromethoxy, $-OCH_2CHF_2$, cyclobutyloxy, and

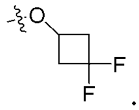

More specifically, in some embodiments of the compound of formula (IV), $R^5$ is a hydrogen atom. In some other embodiments of the compound of formula (IV), $R^5$ is methoxy.

In some embodiments of the compound of formula (IV), $R^6$ and $R^7$ are identical or different and are each independently selected from a hydrogen atom, a chlorine atom, a fluorine atom, methoxy, ethoxy, isopropoxy, difluoromethoxy, and cyclopropyl.

In some embodiments of the compound of formula (IV), $X_1$ is C. In some other embodiments of the compound of formula (IV), $X_1$ is N.

In some embodiments of the compound of formula (IV), Y is O. In some other embodiments of the compound of formula (IV), Y is NH.

In some aspects, the present disclosure provides a compound of formula (V) or a pharmaceutically acceptable salt, an ester, a stereoisomer, a tautomer, a polymorph, a hydrate, a solvate, an N-oxide, an isotopically labeled compound, a metabolite, or a prodrug thereof,

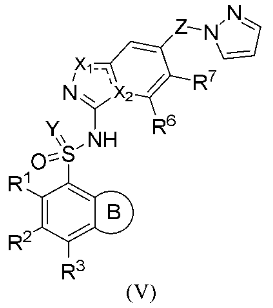

(V)

wherein:

the dashed line is an optional chemical bond;

$R^1$, $R^2$, and $R^3$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;

ring B is selected from 4- to 10-membered heterocyclyl, 5- to 6-membered heteroaryl, and 7- to 12-membered spiroheterocyclyl; ring B is optionally substituted with one or more substituents selected from: hydroxyl, amino, halogen, $C_{1-6}$ alkyl, $C_{1-6}$ haloalkyl, $C_{1-6}$ alkoxy, $C_{1-6}$ haloalkoxy, $-C(O)C_{1-6}$ alkyl, $-C(O)C_{3-6}$ cycloalkyl, $-C(O)C_{1-6}$ haloalkyl, $-C(O)C_{3-6}$ halocycloalkyl, $-C(O)OC_{1-6}$ alkyl, $-S(O)_2C_{1-6}$ alkyl, $-S(O)_2C_{3-6}$ cycloalkyl, $-S(O)_2C_{1-6}$ haloalkyl, $-S(O)_2C_{3-6}$ halocycloalkyl, $-PO(C_{1-6}$ alkyl$)_2$, $-PO(C_{3-6}$ cycloalkyl$)_2$, $-PO(C_{1-6}$ haloalkyl$)_2$, $-PO(C_{3-6}$ halocycloalkyl$)_2$, oxo, $=CH_2$, $=CHC_{1-6}$ alkyl, $=C(C_{1-6}$ alkyl$)_2$, $=CHC_{1-6}$ haloalkyl, $=C(C_{1-6}$ haloalkyl$)_2$, $-NHC_{1-6}$ alkyl, $-N(C_{1-6}$ alkyl$)_2$, cyano, benzyl, $C_{3-6}$ cycloalkyl, $C_{3-6}$ heterocyclyl, $C_{3-6}$ halocycloalkyl, $C_{3-6}$ cycloalkoxy, $C_{3-6}$ halocycloalkoxy, 6- to 10-membered aryl, 5- to 10-membered heteroaryl, and $C_{2-6}$ alkenyl.

$R^6$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-8}$ cycloalkyl, $C_{3-8}$ halocycloalkyl, $C_{3-8}$ cycloalkoxy, and $C_{3-8}$ halocycloalkoxy;

$R^7$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-8}$ cycloalkyl, $C_{3-8}$ halocycloalkyl, $C_{3-8}$ cycloalkoxy, and $C_{3-8}$ halocycloalkoxy;

$X_1$ is O, S, C, or N;

$X_2$ is N or C;

Y is O or NH;

Z is $-CH_2-$, $-CF_2-$, -C(O)-, O, -S(O)-, $-S(O)_2-$, or -NH-.

In some embodiments of the compound of formula (V), $X_1$ is O. In some embodiments of the compound of formula (V), $X_1$ is S. In some embodiments of the compound of formula (V), $X_1$ is C. In some embodiments of the compound of formula (V), $X_1$ is N.

In some embodiments of the compound of formula (V), $X_2$ is N. In some other embodiments of the compound of formula (V), $X_2$ is C.

In some embodiments of the compound of formula (V), $X_1$ is O or S; $X_2$ is C. Preferably, in some embodiments of

the compound of formula (V), $X_1$ is O; $X_2$ is C.

In some embodiments of the compound of formula (V), Y is O. In some other embodiments of the compound of formula (V), Y is NH.

In some embodiments of the compound of formula (V), Z is $-CH_2-$. Alternatively, in some embodiments of the compound of formula (V), Z is $-CF_2-$. Alternatively, in some embodiments of the compound of formula (V), Z is -C(O)-. Alternatively, in some embodiments of the compound of formula (V), Z is O. Alternatively, in some embodiments of the compound of formula (V), Z is -S(O)-. Alternatively, in some embodiments of the compound of formula (V), Z is $-S(O)_2-$. Alternatively, in some embodiments of the compound of formula (V), Z is -NH-.

In some embodiments of the compound of formula (V), $R^1$ is selected from a hydrogen atom, $C_{1-4}$ alkoxy, $C_{1-4}$ haloalkoxy, $C_{4-6}$ cycloalkoxy, and $C_{4-6}$ halocycloalkoxy. Preferably, in some embodiments of the compound of formula (V), $R^1$ is selected from a hydrogen atom, methoxy, difluoromethoxy, trifluoromethoxy, $-OCH_2CHF_2$, cyclobutyloxy, and

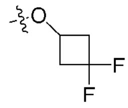.

More specifically, in some embodiments of the compound of formula (V), $R^1$ is cyclobutyloxy. In some other embodiments of the compound of formula (V), $R^1$ is methoxy.

In some embodiments of the compound of formula (V), $R^2$ and $R^3$ are each independently selected from a hydrogen atom, halogen, $C_{1-4}$ alkyl, and $C_{1-4}$ alkoxy. Preferably, in some embodiments of the compound of formula (V), both $R^2$ and $R^3$ are hydrogen atoms.

In some embodiments of the compound of formula (V), $R^6$ and $R^7$ are identical or different and are each independently selected from a hydrogen atom, a chlorine atom, a fluorine atom, methoxy, ethoxy, isopropoxy, difluoromethoxy, and cyclopropyl. Preferably, in some embodiments of the compound of formula (V), $R^6$ and $R^7$ are identical or different and are each independently selected from a hydrogen atom, a chlorine atom, a fluorine atom, and methoxy. More preferably, in some embodiments of the compound of formula (V), $R^6$ is methoxy. In some embodiments of the compound of formula (V), $R^7$ is a hydrogen atom. In some other embodiments of the compound of formula (V), $R^7$ is a fluorine atom.

In some embodiments of the compound of formula (V), ring B is

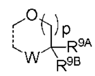,

wherein the dashed line represents a chemical bond shared by ring B and the benzene ring; W is selected from -O-, $-NR^{9C}-$, $-CR^{9D}R^{9E}-$,

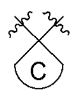, 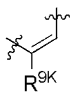, 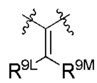,

and -C(O)-, and W is connected to a carbon atom ortho to the carbon atom where $R^3$ is located on the benzene ring; p is 0, 1, or 2; $R^{9A}$ and $R^{9B}$ are identical or different and are each independently selected from a hydrogen atom, hydroxyl, oxo, halogen, $C_{1-6}$ alkyl, $C_{1-6}$ haloalkyl, $C_{1-6}$ alkoxy, $C_{1-6}$ haloalkoxy, $-C(O)C_{1-6}$ alkyl, - $C(O)C_{1-6}$ haloalkyl, cyano, $C_{3-6}$ cycloalkyl, $C_{3-6}$ halocycloalkyl, $C_{3-6}$ cycloalkoxy, and $C_{3-6}$ halocycloalkoxy; $R^{9C}$ is selected from a hydrogen atom, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, $C_{3-6}$ halocycloalkoxy, - $C(O)C_{1-6}$ alkyl, $-C(O)C_{3-6}$ cycloalkyl, $-C(O)C_{1-6}$ haloalkyl, $-C(O)C_{3-6}$ halocycloalkyl, $-C(O)OC_{1-6}$ alkyl, $-S(O)_2C_{1-6}$ alkyl, $-S(O)_2C_{3-6}$ cycloalkyl, $-S(O)_2C_{1-6}$ haloalkyl, $-S(O)_2C_{3-6}$ halocycloalkyl, - $PO(C_{1-6}$ alkyl$)_2$, $-PO(C_{3-6}$ cycloalkyl$)_2$, $-PO(C_{1-6}$ haloalkyl$)_2$, $-PO(C_{3-6}$ halocycloalkyl$)_2$, benzyl, 6- to 10-membered aryl, 5- to 10-membered heteroaryl, and $C_{2-6}$ alkenyl; $R^{9D}$ and $R^{9E}$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $-C(O)C_{1-6}$ alkyl, $-C(O)C_{1-6}$ haloalkyl, cyano, - $NHC_{1-6}$ alkyl, $-N(C_{1-6}$ alkyl$)_2$, $C_{3-6}$ cycloalkyl, $C_{3-6}$ halocycloalkyl, $C_{3-6}$ cycloalkoxy, $C_{3-6}$ halocycloalkoxy, and hydroxyl; ring C is selected from 3- to 8-membered cycloalkyl, 3- to 8-membered heterocyclyl, 6- to 12-membered spirocyclyl, 6- to 12-membered spiroheterocyclyl, and 5- to 12-membered bridged heterocyclyl; ring C is optionally substituted with one or more substituents selected from halogen, hydroxyl, amino, $C_{1-6}$ alkyl, and $C_{1-6}$ haloalkyl; $R^{9K}$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, 6- to 10-membered aryl, 5- to 10-membered heteroaryl, and hydroxyl, wherein the $C_{3-6}$ cycloalkyl and $C_{3-6}$ heterocyclyl are optionally substituted with one or more substituents selected from: halogen, hydroxyl, methyl, and methoxy; $R^{9M}$ and $R^{9L}$

are identical or different and are each independently selected from a hydrogen atom, $C_{1-6}$ alkyl, and $C_{1-6}$ haloalkyl. Preferably, W is selected from -O-, $-NR^{9C}-$,

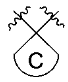, 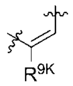,

and -C(O)-.Relatively preferably, W is selected from -O-, $-NR^{9C}-$,

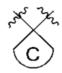, and 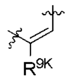.

More preferably, W is selected from - O- and $-NR^{9C}-$. In some embodiments of the compound of formula (V), W is -O-. In some embodiments of the compound of formula (V), W is $-NR^{9C}-$.

Preferably, $R^{9A}$ and $R^{9B}$ are identical or different and are each independently selected from a hydrogen atom, a fluorine atom, and methyl. More preferably, both $R^{9A}$ and $R^{9B}$ are hydrogen atoms.

Preferably, $R^{9C}$ is selected from a hydrogen atom, $C_{1-6}$ alkyl, $C_{1-6}$ haloalkoxy, $-C(O)C_{1-6}$ alkyl, $-C(O)OC_{1-6}$ alkyl, $-S(O)_2C_{1-6}$ alkyl, heteroaryl, and

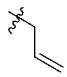.

More preferably, $R^{9C}$ is selected from a hydrogen atom, methyl, deuterated methyl, ethyl, trifluoroethyl, benzyl, $-C(O)OCH_3$, $- C(O)CH_3$, and $-S(O)_2CH_3$.

Preferably, $R^{9D}$ and $R^{9E}$ are identical or different and are each independently selected from a hydrogen atom, a fluorine atom, methyl, and hydroxyl. More preferably, $R^{9D}$ and $R^{9E}$ are identical or different and are each independently selected from a hydrogen atom, methyl, and hydroxyl.

Preferably, ring C is selected from

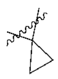, 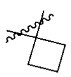, 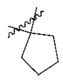, and 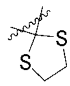,

and ring C is optionally substituted with one or more substituents selected from halogen, hydroxyl, amino, $C_{1-6}$ alkyl, and $C_{1-6}$ haloalkyl.More preferably, ring C is selected from

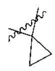, 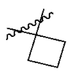, 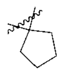, and 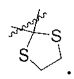.

Preferably, in some embodiments of the compound of formula (V), p is 2. In some embodiments of the compound of formula (V), p is 0 or 1. In some embodiments of the compound of formula (V), p is 1. In some other embodiments of the compound of formula (V), p is 0.

In some aspects, the present disclosure provides a compound of formula (VI-1) or a pharmaceutically acceptable salt, an ester, a stereoisomer, a tautomer, a polymorph, a hydrate, a solvate, an N-oxide, an isotopically labeled compound, a metabolite, or a prodrug thereof,

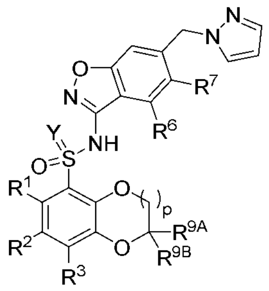

(VI-1)

wherein:

$R^1$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
$R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;
$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
$R^{9A}$ and $R^{9B}$ are identical or different and are each independently selected from a hydrogen atom, hydroxyl, oxo, halogen, $C_{1-6}$ alkyl, $C_{1-6}$ haloalkyl, $C_{1-6}$ alkoxy, $C_{1-6}$ haloalkoxy, - $C(O)C_{1-6}$ alkyl, $-C(O)C_{1-6}$ haloalkyl, cyano, $C_{3-6}$ cycloalkyl, $C_{3-6}$ halocycloalkyl, $C_{3-6}$ cycloalkoxy, and $C_{3-6}$ halocycloalkoxy;
Y is O or NH;
p is 0, 1, or 2.

In some embodiments of the compound of formula (VI-1), $R^1$ is selected from a hydrogen atom, $C_{1-4}$ alkoxy, $C_{1-4}$ haloalkoxy, $C_{4-6}$ cycloalkoxy, and $C_{4-6}$ halocycloalkoxy. Preferably, in some embodiments of the compound of formula (VI-1), $R^1$ is selected from a hydrogen atom, methoxy, difluoromethoxy, trifluoromethoxy, $-OCH_2CHF_2$, cyclobutyloxy, and

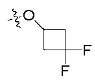.

More specifically, in some embodiments of the compound of formula (VI-1), $R^1$ is cyclobutyloxy. In some other embodiments of the compound of formula (VI-1), $R^1$ is methoxy.

In some embodiments of the compound of formula (VI-1), $R^2$ and $R^3$ are each independently selected from a hydrogen atom, halogen, $C_{1-4}$ alkyl, and $C_{1-4}$ alkoxy. Preferably, in some embodiments of the compound of formula (VI-1), both $R^2$ and $R^3$ are hydrogen atoms.

In some embodiments of the compound of formula (VI-1), $R^6$ and $R^7$ are identical or different and are each independently selected from a hydrogen atom, a chlorine atom, a fluorine atom, methoxy, ethoxy, isopropoxy, difluoromethoxy, and cyclopropyl. Preferably, in some embodiments of the compound of formula (VI-1), $R^6$ and $R^7$ are identical or different and are each independently selected from a hydrogen atom, a chlorine atom, a fluorine atom, and methoxy. More preferably, in some embodiments of the compound of formula (VI-1), $R^6$ is methoxy. In some embodiments of the compound of formula (VI-1), $R^7$ is a hydrogen atom. In some other embodiments of the compound of formula (VI-1), $R^7$ is a fluorine atom.

In some embodiments of the compound of formula (VI-1), $R^{9A}$ and $R^{9B}$ are identical or different and are each independently selected from a hydrogen atom, a fluorine atom, and methyl. Preferably, in some embodiments of the compound of formula (VI-1), both $R^{9A}$ and $R^{9B}$ are hydrogen atoms.

In some embodiments of the compound of formula (VI-1), Y is O. In some other embodiments of the compound of formula (VI-1), Y is NH.

In some embodiments of the compound of formula (VI-1), p is 2. In some embodiments of the compound of formula (VI-1), p is 0 or 1. In some embodiments of the compound of formula (VI-1), p is 1. In some other embodiments of the compound of formula (VI-1), p is 0.

In some aspects, the present disclosure provides a compound of formula (VI-2) or a pharmaceutically acceptable salt, an ester, a stereoisomer, a tautomer, a polymorph, a hydrate, a solvate, an N-oxide, an isotopically labeled compound, a metabolite, or a prodrug thereof,

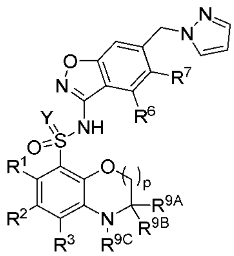

(VI-2)

wherein:

$R^1$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
$R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;
$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
$R^{9A}$ and $R^{9B}$ are identical or different and are each independently selected from a hydrogen atom, hydroxyl, oxo, halogen, $C_{1-6}$ alkyl, $C_{1-6}$ haloalkyl, $C_{1-6}$ alkoxy, $C_{1-6}$ haloalkoxy, - $C(O)C_{1-6}$ alkyl, -$C(O)C_{1-6}$ haloalkyl, cyano, $C_{3-6}$ cycloalkyl, $C_{3-6}$ halocycloalkyl, $C_{3-6}$ cycloalkoxy, and $C_{3-6}$ halocycloalkoxy;
$R^{9C}$ is selected from a hydrogen atom, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, $C_{3-6}$ halocycloalkoxy, - $C(O)C_{1-6}$ alkyl, -$C(O)C_{3-6}$ cycloalkyl, -$C(O)C_{1-6}$ haloalkyl, -$C(O)C_{3-6}$ halocycloalkyl, -$C(O)OC_{1-6}$ alkyl, -$S(O)_2C_{1-6}$ alkyl, -$S(O)_2C_{3-6}$ cycloalkyl, -$S(O)_2C_{1-6}$ haloalkyl, -$S(O)_2C_{3-6}$ halocycloalkyl, - $PO(C_{1-6}$ alkyl$)_2$, -$PO(C_{3-6}$ cycloalkyl$)_2$, -$PO(C_{1-6}$ haloalkyl$)_2$, -$PO(C_{3-6}$ halocycloalkyl$)_2$, benzyl, 6- to 10-membered aryl, 5- to 10-membered heteroaryl, and $C_{2-6}$ alkenyl;
Y is O or NH;
p is 0, 1, or 2.

In some embodiments of the compound of formula (VI-2), $R^1$ is selected from a hydrogen atom, $C_{1-4}$ alkoxy, $C_{1-4}$ haloalkoxy, $C_{4-6}$ cycloalkoxy, and $C_{4-6}$ halocycloalkoxy. Preferably, in some embodiments of the compound of formula (VI-2), $R^1$ is selected from a hydrogen atom, methoxy, difluoromethoxy, trifluoromethoxy, -$OCH_2CHF_2$, cyclobutyloxy, and

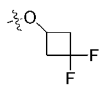
.

More specifically, in some embodiments of the compound of formula (VI-2), $R^1$ is cyclobutyloxy. In some other embodiments of the compound of formula (VI-2), $R^1$ is methoxy.

In some embodiments of the compound of formula (VI-2), $R^2$ and $R^3$ are each independently selected from a hydrogen atom, halogen, $C_{1-4}$ alkyl, and $C_{1-4}$ alkoxy. Preferably, in some embodiments of the compound of formula (VI-2), both $R^2$ and $R^3$ are hydrogen atoms.

In some embodiments of the compound of formula (VI-2), $R^6$ and $R^7$ are identical or different and are each independently selected from a hydrogen atom, a chlorine atom, a fluorine atom, methoxy, ethoxy, isopropoxy, difluoromethoxy, and cyclopropyl. Preferably, in some embodiments of the compound of formula (VI-2), $R^6$ and $R^7$ are identical or different and are each independently selected from a hydrogen atom, a chlorine atom, a fluorine atom, and methoxy. More preferably, in some embodiments of the compound of formula (VI-2), $R^6$ is methoxy. In some embodiments of the compound of formula (VI-2), $R^7$ is a hydrogen atom. In some other embodiments of the compound of formula (VI-2), $R^7$ is a fluorine atom.

In some embodiments of the compound of formula (VI-2), $R^{9A}$ and $R^{9B}$ are identical or different and are each independently selected from a hydrogen atom, a fluorine atom, and methyl. Preferably, in some embodiments of the compound of formula (VI-2), both $R^{9A}$ and $R^{9B}$ are hydrogen atoms.

In some embodiments of the compound of formula (VI-2), $R^{9C}$ is selected from a hydrogen atom, $C_{1-6}$ alkyl, $C_{1-6}$ haloalkoxy, -$C(O)C_{1-6}$ alkyl, -$C(O)OC_{1-6}$ alkyl, -$S(O)_2C_{1-6}$ alkyl, heteroaryl, and

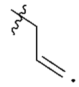

Preferably, in some embodiments of the compound of formula (VI-2), $R^{9C}$ is selected from a hydrogen atom, methyl, deuterated methyl, ethyl, trifluoroethyl, benzyl, - $C(O)OCH_3$, $-C(O)CH_3$, and $-S(O)_2CH_3$.

In some embodiments of the compound of formula (VI-2), $R^{9C}$ is selected from a hydrogen atom, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, $C_{3-6}$ halocycloalkoxy, 6- to 10-membered aryl, 5- to 10-membered heteroaryl, and $C_{2-6}$ alkenyl; in some embodiments of the compound of formula (VI-2), $R^{9C}$ is selected from $C_{1-6}$ alkyl, heteroaryl, and

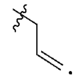

Preferably, in some embodiments of the compound of formula (VI-2), $R^{9C}$ is methyl.

In some embodiments of the compound of formula (VI-2), $R^{9C}$ is selected from - $C(O)C_{1-6}$ alkyl, $-C(O)C_{3-6}$ cycloalkyl, $-C(O)C_{1-6}$ haloalkyl, $-C(O)C_{3-6}$ halocycloalkyl, $-C(O)OC_{1-6}$ alkyl, $-S(O)_2C_{1-6}$ alkyl, $-S(O)_2C_{3-6}$ cycloalkyl, $-S(O)_2C_{1-6}$ haloalkyl, $-S(O)_2C_{3-6}$ halocycloalkyl, - $PO(C_{1-6}$ alkyl$)_2$, $-PO(C_{3-6}$ cycloalkyl$)_2$, $-PO(C_{1-6}$ haloalkyl$)_2$, $-PO(C_{3-6}$ halocycloalkyl$)_2$, and benzyl; in some embodiments of the compound of formula (VI-2), $R^{9C}$ is selected from a hydrogen atom, $C_{1-6}$ haloalkoxy, $-C(O)C_{1-6}$ alkyl, $-C(O)OC_{1-6}$ alkyl, and $-S(O)_2C_{1-6}$ alkyl. Preferably, in some embodiments of the compound of formula (VI-2), $R^{9C}$ is selected from a hydrogen atom, deuterated methyl, ethyl, trifluoroethyl, benzyl, $-C(O)OCH_3$, $-C(O)CH_3$, and - $S(O)_2CH_3$. In some embodiments of the compound of formula (VI-2), Y is O. In some other embodiments of the compound of formula (VI-2), Y is NH.

In some embodiments of the compound of formula (VI-2), p is 2. In some embodiments of the compound of formula (VI-2), p is 0 or 1. In some embodiments of the compound of formula (VI-2), p is 1. In some other embodiments of the compound of formula (VI-2), p is 0.

In some aspects, the present disclosure provides a compound of formula (VI-3) or a pharmaceutically acceptable salt, an ester, a stereoisomer, a tautomer, a polymorph, a hydrate, a solvate, an N-oxide, an isotopically labeled compound, a metabolite, or a prodrug thereof,

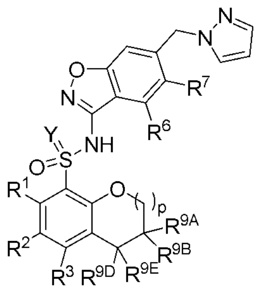

(VI-3)

wherein:

- $R^1$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
- $R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;
- $R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
- $R^{9A}$ and $R^{9B}$ are identical or different and are each independently selected from a hydrogen atom, hydroxyl, oxo, halogen, $C_{1-6}$ alkyl, $C_{1-6}$ haloalkyl, $C_{1-6}$ alkoxy, $C_{1-6}$ haloalkoxy, - $C(O)C_{1-6}$ alkyl, $-C(O)C_{1-6}$ haloalkyl, cyano, $C_{3-6}$ cycloalkyl, $C_{3-6}$ halocycloalkyl, $C_{3-6}$ cycloalkoxy, and $C_{3-6}$ halocycloalkoxy;
- $R^{9D}$ and $R^{9E}$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $-C(O)C_{1-6}$ alkyl, $-C(O)C_{1-6}$ haloalkyl, cyano, $-NHC_{1-6}$ alkyl, $-N(C_{1-6}$ alkyl$)_2$, $C_{3-6}$ cycloalkyl, $C_{3-6}$ halocycloalkyl, $C_{3-6}$ cycloalkoxy, $C_{3-6}$ halocycloalkoxy, and hydroxyl;
- Y is O or NH;

p is 0, 1, or 2.

In some embodiments of the compound of formula (VI-3), $R^1$ is selected from a hydrogen atom, $C_{1-4}$ alkoxy, $C_{1-4}$ haloalkoxy, $C_{4-6}$ cycloalkoxy, and $C_{4-6}$ halocycloalkoxy. Preferably, in some embodiments of the compound of formula (VI-3), $R^1$ is selected from a hydrogen atom, methoxy, difluoromethoxy, trifluoromethoxy, $-OCH_2CHF_2$, cyclobutyloxy, and

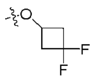.

More specifically, in some embodiments of the compound of formula (VI-3), $R^1$ is cyclobutyloxy. In some other embodiments of the compound of formula (VI-3), $R^1$ is methoxy.

In some embodiments of the compound of formula (VI-3), $R^2$ and $R^3$ are each independently selected from a hydrogen atom, halogen, $C_{1-4}$ alkyl, and $C_{1-4}$ alkoxy. Preferably, in some embodiments of the compound of formula (VI-3), both $R^2$ and $R^3$ are hydrogen atoms.

In some embodiments of the compound of formula (VI-3), $R^6$ and $R^7$ are identical or different and are each independently selected from a hydrogen atom, a chlorine atom, a fluorine atom, methoxy, ethoxy, isopropoxy, difluoromethoxy, and cyclopropyl. Preferably, in some embodiments of the compound of formula (VI-3), $R^6$ and $R^7$ are identical or different and are each independently selected from a hydrogen atom, a chlorine atom, a fluorine atom, and methoxy. More preferably, in some embodiments of the compound of formula (VI-3), $R^6$ is methoxy. In some embodiments of the compound of formula (VI-3), $R^7$ is a hydrogen atom. In some other embodiments of the compound of formula (VI-3), $R^7$ is a fluorine atom.

In some embodiments of the compound of formula (VI-3), $R^{9A}$ and $R^{9B}$ are identical or different and are each independently selected from a hydrogen atom, a fluorine atom, and methyl. Preferably, in some embodiments of the compound of formula (VI-3), both $R^{9A}$ and $R^{9B}$ are hydrogen atoms.

In some embodiments of the compound of formula (VI-3), $R^{9D}$ and $R^{9E}$ are identical or different and are each independently selected from a hydrogen atom, a fluorine atom, methyl, and hydroxyl. In some embodiments of the compound of formula (VI-3), $R^{9D}$ and $R^{9E}$ are identical or different and are each independently selected from a fluorine atom and methyl. Specifically, in some embodiments of the compound of formula (VI-3), both $R^{9D}$ and $R^{9E}$ are fluorine atoms or methyl.

In some embodiments of the compound of formula (VI-3), Y is O. In some other embodiments of the compound of formula (VI-3), Y is NH.

In some embodiments of the compound of formula (VI-3), p is 2. In some embodiments of the compound of formula (VI-3), p is 0 or 1. In some embodiments of the compound of formula (VI-3), p is 1. In some other embodiments of the compound of formula (VI-3), p is 0.

In some aspects, the present disclosure provides a compound of formula (VI-4) or a pharmaceutically acceptable salt, an ester, a stereoisomer, a tautomer, a polymorph, a hydrate, a solvate, an N-oxide, an isotopically labeled compound, a metabolite, or a prodrug thereof,

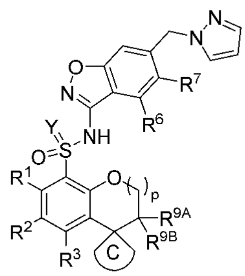

(VI-4)

wherein:

- $R^1$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
- $R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;
- $R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;

$R^{9A}$ and $R^{9B}$ are identical or different and are each independently selected from a hydrogen atom, hydroxyl, oxo, halogen, $C_{1-6}$ alkyl, $C_{1-6}$ haloalkyl, $C_{1-6}$ alkoxy, $C_{1-6}$ haloalkoxy, - $C(O)C_{1-6}$ alkyl, $-C(O)C_{1-6}$ haloalkyl, cyano, $C_{3-6}$ cycloalkyl, $C_{3-6}$ halocycloalkyl, $C_{3-6}$ cycloalkoxy, and $C_{3-6}$ halocycloalkoxy;

ring C is selected from 3- to 8-membered cycloalkyl, 3- to 8-membered heterocyclyl, 6-to 12-membered spirocyclyl, 6- to 12-membered spiroheterocyclyl, and 5- to 12-membered bridged heterocyclyl; ring C is optionally substituted with one or more substituents selected from halogen, hydroxyl, amino, $C_{1-6}$ alkyl, and $C_{1-6}$ haloalkyl;

Y is O or NH;

p is 0, 1, or 2.

In some embodiments of the compound of formula (VI-4), $R^1$ is selected from a hydrogen atom, $C_{1-4}$ alkoxy, $C_{1-4}$ haloalkoxy, $C_{4-6}$ cycloalkoxy, and $C_{4-6}$ halocycloalkoxy. Preferably, in some embodiments of the compound of formula (VI-4), $R^1$ is selected from a hydrogen atom, methoxy, difluoromethoxy, trifluoromethoxy, $-OCH_2CHF_2$, cyclobutyloxy, and

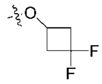.

More specifically, in some embodiments of the compound of formula (VI-4), $R^1$ is cyclobutyloxy. In some other embodiments of the compound of formula (VI-4), $R^1$ is methoxy.

In some embodiments of the compound of formula (VI-4), $R^2$ and $R^3$ are each independently selected from a hydrogen atom, halogen, $C_{1-4}$ alkyl, and $C_{1-4}$ alkoxy. Preferably, in some embodiments of the compound of formula (VI-4), both $R^2$ and $R^3$ are hydrogen atoms.

In some embodiments of the compound of formula (VI-4), $R^6$ and $R^7$ are identical or different and are each independently selected from a hydrogen atom, a chlorine atom, a fluorine atom, methoxy, ethoxy, isopropoxy, difluoromethoxy, and cyclopropyl. Preferably, in some embodiments of the compound of formula (VI-4), $R^6$ and $R^7$ are identical or different and are each independently selected from a hydrogen atom, a chlorine atom, a fluorine atom, and methoxy. More preferably, in some embodiments of the compound of formula (VI-4), $R^6$ is methoxy. In some embodiments of the compound of formula (VI-4), $R^7$ is a hydrogen atom. In some other embodiments of the compound of formula (VI-4), $R^7$ is a fluorine atom.

In some embodiments of the compound of formula (VI-4), $R^{9A}$ and $R^{9B}$ are identical or different and are each independently selected from a hydrogen atom, a fluorine atom, and methyl. Preferably, in some embodiments of the compound of formula (VI-4), both $R^{9A}$ and $R^{9B}$ are hydrogen atoms.

In some embodiments of the compound of formula (VI-4), ring C is selected from

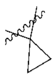,

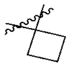, 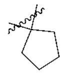, and 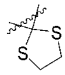,

and ring C is optionally substituted with one or more substituents selected from halogen, hydroxyl, amino, $C_{1-6}$ alkyl, and $C_{1-6}$ haloalkyl.Preferably, ring C is selected from

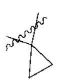, 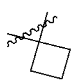, 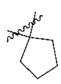, and 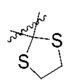.

In some embodiments of the compound of formula (VI-4), Y is O. In some other embodiments of the compound of formula (VI-4), Y is NH.

In some embodiments of the compound of formula (VI-4), p is 2. In some embodiments of the compound of formula (VI-4), p is 0 or 1. In some embodiments of the compound of formula (VI-4), p is 1. In some other embodiments of the compound of formula (VI-4), p is 0.

In some aspects, the present disclosure provides a compound of formula (VII-1) or a pharmaceutically acceptable salt, an ester, a stereoisomer, a tautomer, a polymorph, a hydrate, a solvate, an N-oxide, an isotopically labeled compound, a metabolite, or a prodrug thereof,

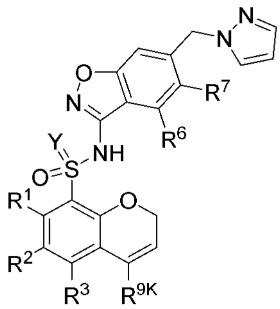

(VII-1)

wherein:

$R^1$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
$R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;
$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
$R^{9K}$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, 6- to 10-membered aryl, 5- to 10-membered heteroaryl, and hydroxyl, wherein the $C_{3-6}$ cycloalkyl and $C_{3-6}$ heterocyclyl are optionally substituted with one or more substituents selected from: halogen, hydroxyl, methyl, and methoxy;
Y is O or NH.

In some embodiments of the compound of formula (VII-1), $R^1$ is selected from a hydrogen atom, $C_{1-4}$ alkoxy, $C_{1-4}$ haloalkoxy, $C_{4-6}$ cycloalkoxy, and $C_{4-6}$ halocycloalkoxy. Preferably, in some embodiments of the compound of formula (VII-1), $R^1$ is selected from a hydrogen atom, methoxy, difluoromethoxy, trifluoromethoxy, $-OCH_2CHF_2$, cyclobutyloxy, and

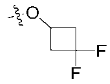

.

More preferably, in some embodiments of the compound of formula (VII-1), $R^1$ is methoxy.

In some embodiments of the compound of formula (VII-1), $R^2$ and $R^3$ are identical or different and are each independently selected from a hydrogen atom, halogen, $C_{1-4}$ alkyl, and $C_{1-4}$ alkoxy. Preferably, in some embodiments of the compound of formula (VII-1), $R^2$ and $R^3$ are identical or different and are each independently selected from a hydrogen atom and halogen. More preferably, in some embodiments of the compound of formula (VII-1), both $R^2$ and $R^3$ are hydrogen atoms.

In some embodiments of the compound of formula (VII-1), $R^6$ and $R^7$ are identical or different and are each independently selected from a hydrogen atom, a chlorine atom, a fluorine atom, methoxy, ethoxy, isopropoxy, difluoromethoxy, and cyclopropyl. In some embodiments of the compound of formula (VII-1), $R^6$ is methoxy. In some embodiments of the compound of formula (VII-1), $R^7$ is a hydrogen atom. In some other embodiments of the compound of formula (VII-1), $R^7$ is a fluorine atom.

In some embodiments of the compound of formula (VII-1), $R^{9K}$ is selected from a hydrogen atom, halogen, $C_{1-6}$ alkyl, $C_{1-6}$ haloalkyl, and hydroxyl. Preferably, in some embodiments of the compound of formula (VII-1), $R^{9K}$ is selected from a hydrogen atom, methyl, and hydroxyl.

In some embodiments of the compound of formula (VII-1), Y is O. In some other embodiments of the compound of formula (VII-1), Y is NH.

In some aspects, the present disclosure provides a compound of formula (VII-2) or a pharmaceutically acceptable salt, an ester, a stereoisomer, a tautomer, a polymorph, a hydrate, a solvate, an N-oxide, an isotopically labeled compound, a metabolite, or a prodrug thereof,

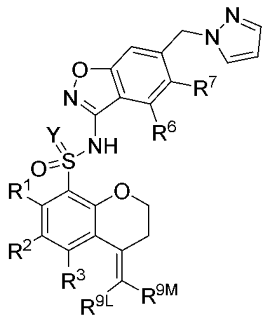

(VII-2)

wherein:

- $R^1$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
- $R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;
- $R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
- $R^{9M}$ and $R^{9L}$ are identical or different and are each independently selected from a hydrogen atom, $C_{1-6}$ alkyl, and $C_{1-6}$ haloalkyl;
- Y is O or NH.

In some embodiments of the compound of formula (VII-2), $R^1$ is selected from a hydrogen atom, $C_{1-4}$ alkoxy, $C_{1-4}$ haloalkoxy, $C_{4-6}$ cycloalkoxy, and $C_{4-6}$ halocycloalkoxy. Preferably, in some embodiments of the compound of formula (VII-2), $R^1$ is selected from a hydrogen atom, methoxy, difluoromethoxy, trifluoromethoxy, $-OCH_2CHF_2$, cyclobutyloxy, and

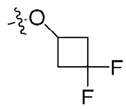.

More preferably, in some embodiments of the compound of formula (VII-2), $R^1$ is methoxy.

In some embodiments of the compound of formula (VII-2), $R^2$ and $R^3$ are identical or different and are each independently selected from a hydrogen atom, halogen, $C_{1-4}$ alkyl, and $C_{1-4}$ alkoxy. Preferably, in some embodiments of the compound of formula (VII-2), $R^2$ and $R^3$ are identical or different and are each independently selected from a hydrogen atom and halogen. More preferably, in some embodiments of the compound of formula (VII-2), both $R^2$ and $R^3$ are hydrogen atoms.

In some embodiments of the compound of formula (VII-2), $R^6$ and $R^7$ are identical or different and are each independently selected from a hydrogen atom, a chlorine atom, a fluorine atom, methoxy, ethoxy, isopropoxy, difluoromethoxy, and cyclopropyl. In some embodiments of the compound of formula (VII-2), $R^6$ is methoxy. In some embodiments of the compound of formula (VII-2), $R^7$ is a hydrogen atom. In some other embodiments of the compound of formula (VII-2), $R^7$ is a fluorine atom.

In some embodiments of the compound of formula (VII-2), $R^{9M}$ and $R^{9L}$ are identical or different and are each independently selected from a hydrogen atom and $C_{1-6}$ alkyl. Preferably, in some embodiments of the compound of formula (VII-2), $R^{9M}$ and $R^{9L}$ are identical or different and are each independently selected from a hydrogen atom, a fluorine atom, and methyl.

In some embodiments of the compound of formula (VII-2), Y is O. In some other embodiments of the compound of formula (VII-2), Y is NH.

In some aspects, the present disclosure provides a compound of formula (VII-3) or a pharmaceutically acceptable salt, an ester, a stereoisomer, a tautomer, a polymorph, a hydrate, a solvate, an N-oxide, an isotopically labeled compound, a metabolite, or a prodrug thereof,

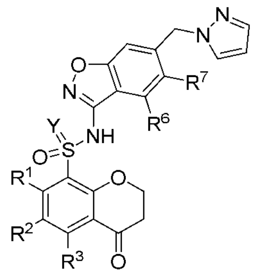

(VII-3)

wherein:

$R^1$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
$R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;
$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
Y is O or NH.

In some embodiments of the compound of formula (VII-3), $R^1$ is selected from a hydrogen atom, $C_{1-4}$ alkoxy, $C_{1-4}$ haloalkoxy, $C_{4-6}$ cycloalkoxy, and $C_{4-6}$ halocycloalkoxy. Preferably, in some embodiments of the compound of formula (VII-3), $R^1$ is selected from a hydrogen atom, methoxy, difluoromethoxy, trifluoromethoxy, $-OCH_2CHF_2$, cyclobutyloxy, and

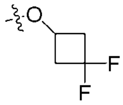.

More preferably, in some embodiments of the compound of formula (VII-3), $R^1$ is methoxy.

In some embodiments of the compound of formula (VII-3), $R^2$ and $R^3$ are identical or different and are each independently selected from a hydrogen atom, halogen, $C_{1-4}$ alkyl, and $C_{1-4}$ alkoxy. Preferably, in some embodiments of the compound of formula (VII-3), $R^2$ and $R^3$ are identical or different and are each independently selected from a hydrogen atom and halogen. More preferably, in some embodiments of the compound of formula (VII-3), both $R^2$ and $R^3$ are hydrogen atoms.

In some embodiments of the compound of formula (VII-3), $R^6$ and $R^7$ are identical or different and are each independently selected from a hydrogen atom, a chlorine atom, a fluorine atom, methoxy, ethoxy, isopropoxy, difluoromethoxy, and cyclopropyl. In some embodiments of the compound of formula (VII-3), $R^6$ is methoxy. In some embodiments of the compound of formula (VII-3), $R^7$ is a hydrogen atom. In some other embodiments of the compound of formula (VII-3), $R^7$ is a fluorine atom.

In some embodiments of the compound of formula (VII-3), Y is O. In some other embodiments of the compound of formula (VII-3), Y is NH.

In some embodiments, the present disclosure provides compounds listed in Table 1 below or pharmaceutically acceptable salts, esters, stereoisomers, tautomers, polymorphs, hydrates, solvates, N-oxides, isotopically labeled compounds, metabolites, or prodrugs thereof.

Table 1. Typical compounds of the present disclosure, including but not limited to:

| Compound No. | Structural formula | Chemical name |
|---|---|---|
| 1 | 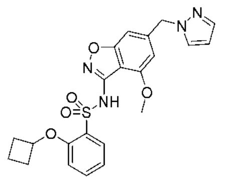 | *N*-(6-((1*H*-Pyrazol-1-yl)methyl)-4-methoxybenzo[*d*]isoxazol-3-yl)-2-cyclobutoxybenzenesulfonamide |

(continued)

| Compound No. | Structural formula | Chemical name |
|---|---|---|
| 2 | 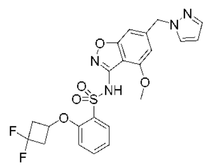 | *N*-(6-((1*H*-Pyrazol-1-yl)methyl)-4-methoxybenzo[*d*]isoxazol-3-yl)-2-(3,3-difluorocyclobutoxy)benzenesulfonamide |
| 3 | 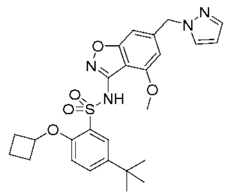 | *N*-(6-((1*H*-Pyrazol-1-yl)methyl)-4-methoxybenzo[*d*]isoxazol-3-yl)-5-(*tert*-butyl)-2-cyclobutoxybenzenesulfonamide |
| 4 | 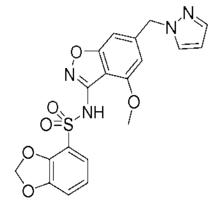 | *N*-(6-((1*H*-Pyrazol-1-yl)methyl)-4-methoxybenzo[*d*]isoxazol-3-yl)benzo[*d*][1,3]dioxole-4-sulfonamide |
| 5 | 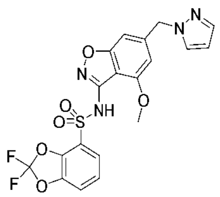 | *N*-(6-((1*H*-Pyrazol-1-yl)methyl)-4-methoxybenzo[*d*]isoxazol-3-yl)-2,2-difluorobenzo[*d*][1,3]dioxole-4-sulfonamide |
| 6 | 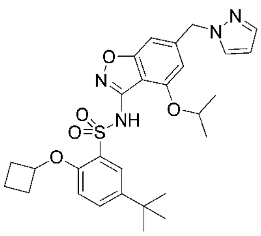 | *N*-(6-((1*H*-Pyrazol-1-yl)methyl)-4-isopropoxybenzo[*d*]isoxazol-3-yl)-5-(*tert*-butyl)-2-cyclobutoxybenzenesulfonamide |
| 7 | 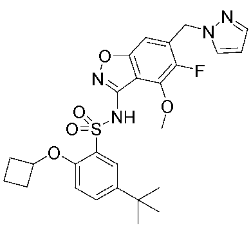 | *N*-(6-((1*H*-Pyrazol-1-yl)methyl)-5-fluoro-4-methoxybenzo[*d*]isoxazol-3-yl)-5-(*tert*-butyl)-2-cyclobutoxybenzenesulfonamide |
| 8 | 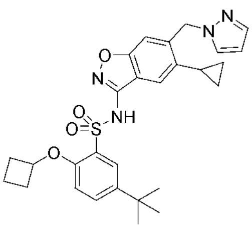 | *N*-(6-((1*H*-Pyrazol-1-yl)methyl)-5-cyclopropylbenzo[*d*]isoxazol-3-yl)-5-(*tert*-butyl)-2-cyclobutoxybenzenesulfonamide |

(continued)

| Compound No. | Structural formula | Chemical name |
|---|---|---|
| 9 | 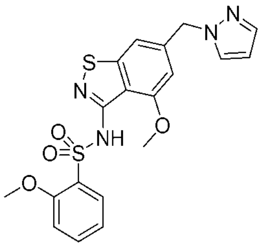 | *N*-(6-((1*H*-Pyrazol-1-yl)methyl)-4-methoxybenzo[*d*]isothiazol-3-yl)-2-methoxybenzenesulfonamide |
| 10 | 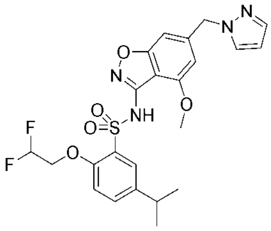 | *N*-(6-((1*H*-Pyrazol-1-yl)methyl)-4-methoxybenzo[*d*]isoxazol-3-yl)-2-(2,2-difluoroethoxy)-5-isopropylbenzenesulfonamide |
| 11 | 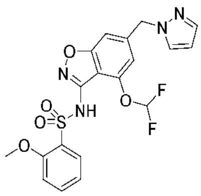 | *N*-(6-((1*H*-Pyrazol-1-yl)methyl)-4-(difluoromethoxy)benzo[*d*]isoxazol-3-yl)-2-methoxybenzenesulfonamide |
| 12 | 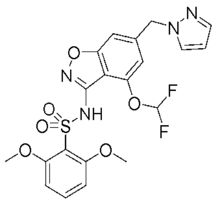 | *N*-(6-((1*H*-Pyrazol-1-yl)methyl)-4-(difluoromethoxy)benzo[*d*]isoxazol-3-yl)-2,6-dimethoxybenzenesulfonamide |
| 13 | 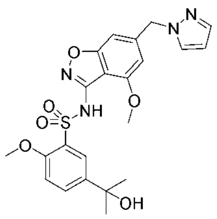 | *N*-(6-((1*H*-Pyrazol-1-yl)methyl)-4-methoxybenzo[*d*]isoxazol-3-yl)-5-(2-hydroxypropan-2-yl)-2-methoxybenzenesulfonamide |
| 14 | 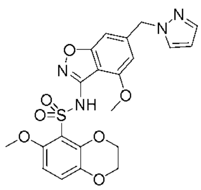 | *N*-(6-((1*H*-Pyrazol-1-yl)methyl)-4-methoxybenzo[*d*]isoxazol-3-yl)-6-methoxy-2,3-dihydrobenzo[*b*][1,4]dioxine-5-sulfonamide |
| 15 | 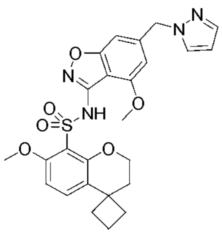 | *N*-(6-((1*H*-Pyrazol-1-yl)methyl)-4-methoxybenzo[*d*]isoxazol-3-yl)-7-methoxyspiro[chromane-4,1'-cyclobutane]-8-sulfonamide |

(continued)

| Compound No. | Structural formula | Chemical name |
|---|---|---|
| 16 | 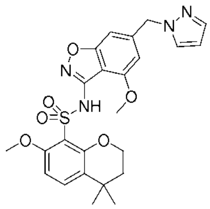 | *N*-(6-((1*H*-Pyrazol-1-yl)methyl)-4-methoxybenzo[*d*]isoxazol-3-yl)-7-methoxy-4,4-dimethylchromane-8-sulfonamide |
| 17 | 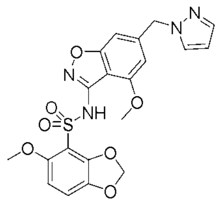 | *N*-(6-((1*H*-Pyrazol-1-yl)methyl)-4-methoxybenzo[*d*]isoxazol-3-yl)-5-methoxybenzo[*d*][1,3]dioxole-4-sulfonamide |
| 18 | 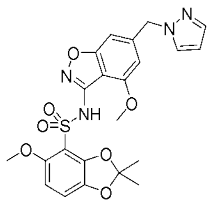 | *N*-(6-((1*H*-Pyrazol-1-yl)methyl)-4-methoxybenzo[*d*]isoxazol-3-yl)-5-methoxy-2,2-dimethylbenzo[*d*][1,3]dioxole-4-sulfonamide |
| 19 | 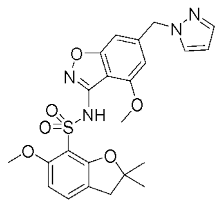 | *N*-(6-((1*H*-Pyrazol-1-yl)methyl)-4-methoxybenzo[*d*]isoxazol-3-yl)-6-methoxy-2,2-dimethyl-2,3-dihydrobenzofuran-7-sulfonamide |
| 20 | 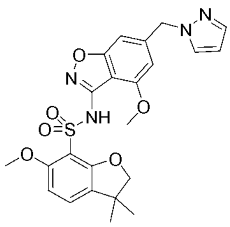 | *N*-(6-((1*H*-Pyrazol-1-yl)methyl)-4-methoxybenzo[*d*]isoxazol-3-yl)-6-methoxy-3,3-dimethyl-2,3-dihydrobenzofuran-7-sulfonamide |
| 21 | 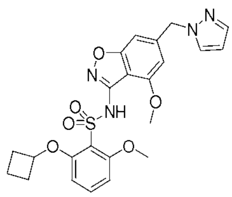 | *N*-(6-((1*H*-Pyrazol-1-yl)methyl)-4-methoxybenzo[*d*]isoxazol-3-yl)-2-cyclobutoxy-6-methoxybenzenesulfonamide |
| 22 | 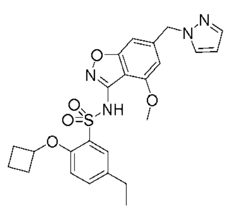 | *N*-(6-((1*H*-Pyrazol-1-yl)methyl)-4-methoxybenzo[*d*]isoxazol-3-yl)-2-cyclobutoxy-5-ethylbenzenesulfonamide |

(continued)

| Compound No. | Structural formula | Chemical name |
|---|---|---|
| 23 | 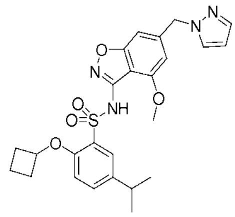 | *N*-(6-((1*H*-Pyrazol-1-yl)methyl)-4-methoxybenzo[*d*]isoxazol-3-yl)-2-cyclobutoxy-5-isopropylbenzenesulfonamide |
| 24 | 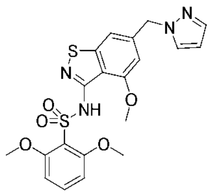 | *N*-(6-((1*H*-Pyrazol-1-yl)methyl)-4-methoxybenzo[*d*]isothiazol-3-yl)-2,6-dimethoxybenzenesulfonamide |
| 25 | 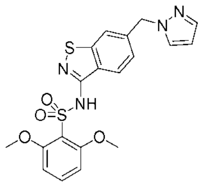 | *N*-(6-((1*H*-Pyrazol-1-yl)methyl)benzo[*d*]isothiazol-3-yl)-2,6-dimethoxybenzenesulfonamide |
| 26 | 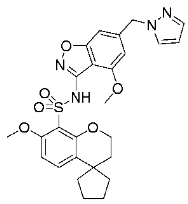 | *N*-(6-((1*H*-Pyrazol-1-yl)methyl)-4-methoxybenzo[*d*]isoxazol-3-yl)-7-methoxyspiro[chromane-4,1'-cyclopentane]-8-sulfonamide |
| 27 | 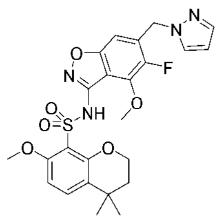 | *N*-(6-((1*H*-Pyrazol-1-yl)methyl)-5-fluoro-4-methoxybenzo[*d*]isoxazol-3-yl)-7-methoxy-4,4-dimethylchromane-8-sulfonamide |
| 28 | 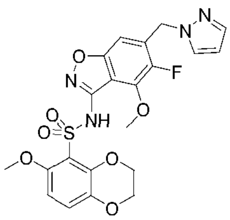 | *N*-(6-((1*H*-Pyrazol-1-yl)methyl)-5-fluoro-4-methoxybenzo[*d*]isoxazol-3-yl)-6-methoxy-2,3-dihydrobenzo[*b*][1,4]dioxine-5-sulfonamide |
| 29 | 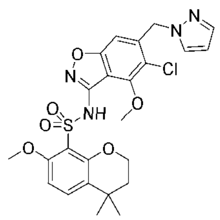 | *N*-(6-((1*H*-Pyrazol-1-yl)methyl)-5-chloro-4-methoxybenzo[*d*]isoxazol-3-yl)-7-methoxy-4,4-dimethylchromane-8-sulfonamide |

(continued)

| Compound No. | Structural formula | Chemical name |
|---|---|---|
| 30 | 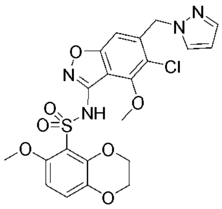 | *N*-(6-((1*H*-Pyrazol-1-yl)methyl)-5-chloro-4-methoxybenzo[*d*]isoxazol-3-yl)-6-methoxy-2,3-dihydrobenzo[*b*][1,4]dioxine-5-sulfonamide |
| 31 | 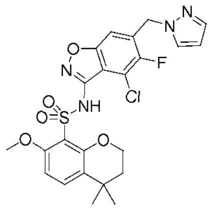 | *N*-(6-((1*H*-Pyrazol-1-yl)methyl)-4-chloro-5-fluorobenzo[*d*]isoxazol-3-yl)-7-methoxy-4,4-dimethylchromane-8-sulfonamide |
| 32 | 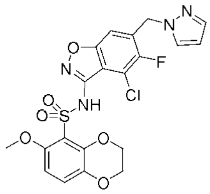 | *N*-(6-((1*H*-Pyrazol-1-yl)methyl)-4-chloro-5-fluorobenzo[*d*]isoxazol-3-yl)-6-methoxy-2,3-dihydrobenzo[*b*][1,4]dioxine-5-sulfonamide |
| 33 | 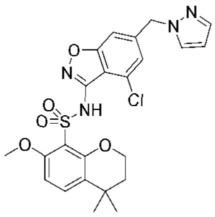 | *N*-(6-((1*H*-Pyrazol-1-yl)methyl)-4-chlorobenzo[*d*]isoxazol-3-yl)-7-methoxy-4,4-dimethylchromane-8-sulfonamide |
| 34 | 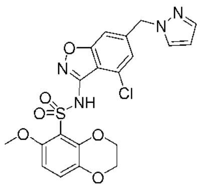 | *N*-(6-((1*H*-Pyrazol-1-yl)methyl)-4-chlorobenzo[*d*]isoxazol-3-yl)-6-methoxy-2,3-dihydrobenzo[*b*][1,4]dioxine-5-sulfonamide |
| 35 | 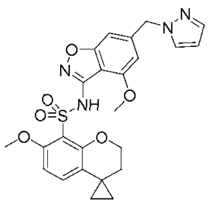 | *N*-(6-((1*H*-Pyrazol-1-yl)methyl)-4-methoxybenzo[*d*]isoxazol-3-yl)-7-methoxyspiro[chromane-4,1'-cyclopropane]-8-sulfonamide |
| 36 | 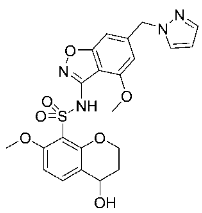 | *N*-(6-((1*H*-Pyrazol-1-yl)methyl)-4-methoxybenzo[*d*]isoxazol-3-yl)-4-hydroxy-7-methoxychromane-8-sulfonamide |

(continued)

| Compound No. | Structural formula | Chemical name |
|---|---|---|
| 37 | 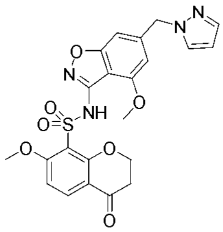 | *N*-(6-((1*H*-Pyrazol-1-yl)methyl)-4-methoxybenzo[*d*]isoxazol-3-yl)-7-methoxy-4-oxochromane-8-sulfonamide |
| 38 | 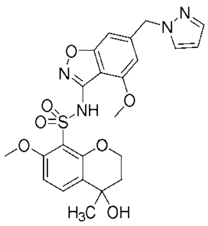 | *N*-(6-((1*H*-Pyrazol-1-yl)methyl)-4-methoxybenzo[*d*]isoxazol-3-yl)-4-hydroxy-7-methoxy-4-methylchromane-8-sulfonamide |
| 39 | 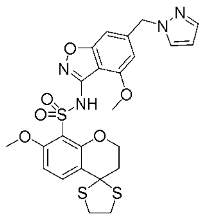 | *N*-(6-((1*H*-Pyrazol-1-yl)methyl)-4-methoxybenzo[*d*]isoxazol-3-yl)-7-methoxyspiro[chromane-4,2'-[1,3]dithiolane]-8-sulfonamide |
| 40 | 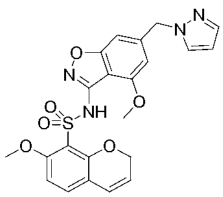 | *N*-(6-((1*H*-Pyrazol-1-yl)methyl)-4-methoxybenzo[*d*]isoxazol-3-yl)-7-methoxy-2*H*-chromene-8-sulfonamide |
| 41 | 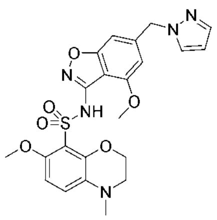 | *N*-(6-((1*H*-Pyrazol-1-yl)methyl)-4-methoxybenzo[*d*]isoxazol-3-yl)-7-methoxy-4-methyl-3,4-dihydro-2*H*-benzo[*b*][1,4]oxazine-8-sulfonamide |
| 42 | 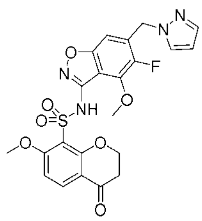 | *N*-(6-((1*H*-Pyrazol-1-yl)methyl)-5-fluoro-4-methoxybenzo[*d*]isoxazol-3-yl)-7-methoxy-4-oxochromane-8-sulfonamide |
| 43 | 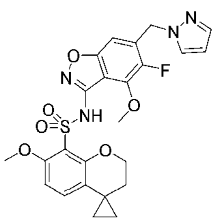 | *N*-(6-((1*H*-Pyrazol-1-yl)methyl)-5-fluoro-4-methoxybenzo[*d*]isoxazol-3-yl)-7-methoxyspiro[chromane-4,1'-cyclopropane]-8-sulfonamide |

(continued)

| Compound No. | Structural formula | Chemical name |
|---|---|---|
| 44 | 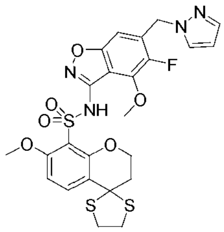 | *N*-(6-((1*H*-Pyrazol-1-yl)methyl)-5-fluoro-4-methoxybenzo[*d*]isoxazol-3-yl)-7-methoxyspiro[chromane-4,2'-[1,3]dithiolane]-8-sulfonamide |
| 45 | 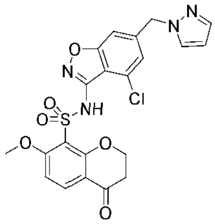 | *N*-(6-((1*H*-Pyrazol-1-yl)methyl)-4-chlorobenzo[*d*]isoxazol-3-yl)-7-methoxy-4-oxochromane-8-sulfonamide |
| 46 | 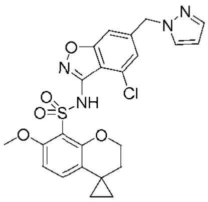 | *N*-(6-((1*H*-Pyrazol-1-yl)methyl)-4-chlorobenzo[*d*]isoxazol-3-yl)-7-methoxyspiro[chromane-4,1'-cyclopropane]-8-sulfonamide |
| 47 | 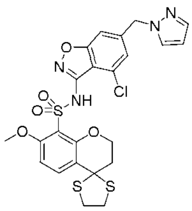 | *N*-(6-((1*H*-Pyrazol-1-yl)methyl)-4-chlorobenzo[*d*]isoxazol-3-yl)-7-methoxyspiro[chromane-4,2'-[1,3]dithiolane]-8-sulfonamide |
| 48 | 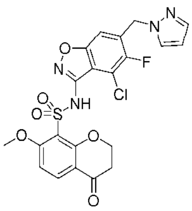 | *N*-(6-((1*H*-Pyrazol-1-yl)methyl)-4-chloro-5-fluorobenzo[*d*]isoxazol-3-yl)-7-methoxy-4-oxochromane-8-sulfonamide |
| 49 | 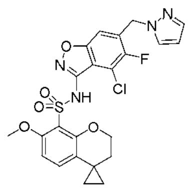 | *N*-(6-((1*H*-Pyrazol-1-yl)methyl)-4-chloro-5-fluorobenzo[*d*]isoxazol-3-yl)-7-methoxyspiro[chromane-4,1'-cyclopropane]-8-sulfonamide |
| 50 | 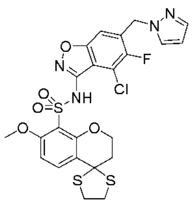 | *N*-(6-((1*H*-Pyrazol-1-yl)methyl)-4-chloro-5-fluorobenzo[*d*]isoxazol-3-yl)-7-methoxyspiro[chromane-4,2'-[1,3]dithiolane]-8-sulfonamide |

(continued)

| Compound No. | Structural formula | Chemical name |
|---|---|---|
| 51 | 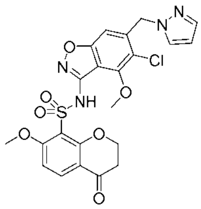 | *N*-(6-((1*H*-Pyrazol-1-yl)methyl)-5-chloro-4-methoxybenzo[*d*]isoxazol-3-yl)-7-methoxy-4-oxochromane-8-sulfonamide |
| 52 | 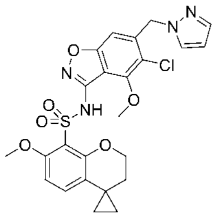 | *N*-(6-((1*H*-Pyrazol-1-yl)methyl)-5-chloro-4-methoxybenzo[*d*]isoxazol-3-yl)-7-methoxyspiro[chromane-4,1'-cyclopropane]-8-sulfonamide |
| 53 | 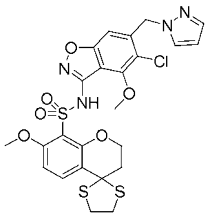 | *N*-(6-((1*H*-Pyrazol-1-yl)methyl)-5-chloro-4-methoxybenzo[*d*]isoxazol-3-yl)-7-methoxyspiro[chromane-4,2'-[1,3]dithiolane]-8-sulfonamide |
| 54 | 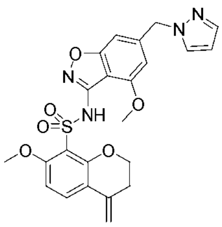 | *N*-(6-((1*H*-Pyrazol-1-yl)methyl)-4-methoxybenzo[*d*]isoxazol-3-yl)-7-methoxy-4-methylenechromane-8-sulfonamide |
| 55 | 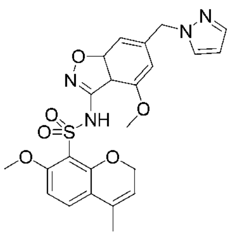 | *N*-(6-((1*H*-Pyrazol-1-yl)methyl)-4-methoxy-3*a*,7*a*-dihydrobenzo[*d*]isoxazol-3-yl)-7-methoxy-4-methyl-2*H*-chromene-8-sulfonamide |
| 56 | 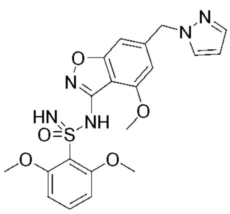 | *N*-(6-((1*H*-Pyrazol-1-yl)methyl)-4-methoxybenzo[*d*]isoxazol-3-yl)-2,6-dimethoxybenzenesulfonimidamide |
| 57 | 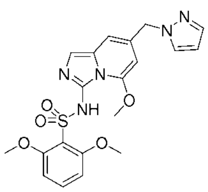 | *N*-(7-((1*H*-Pyrazol-1-yl)methyl)-5-methoxyimidazo[1,5-a]pyridin-3-yl)-2,6-dimethoxybenzenesulfonamide |

(continued)

| Compound No. | Structural formula | Chemical name |
|---|---|---|
| 58 | 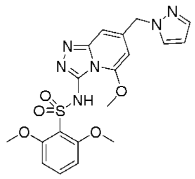 | *N*-(7-((1*H*-Pyrazol-1-yl)methyl)-5-methoxy-[1,2,4]triazolo[4,3-*a*]pyridin-3-yl)-2,6-dimethoxybenzenesulfonamide |
| 59 | 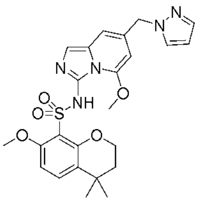 | *N*-(7-((1*H*-Pyrazol-1-yl)methyl)-5-methoxyimidazo[1,5-*a*]pyridin-3-yl)-7-methoxy-4,4-dimethylchromane-8-sulfonamide |
| 60 | 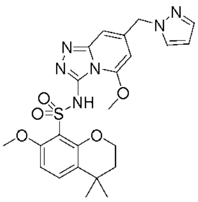 | *N*-(7-((1*H*-Pyrazol-1-yl)methyl)-5-methoxy-[1,2,4]triazolo[4,3-*a*]pyridin-3-yl)-7-methoxy-4,4-dimethylchromane-8-sulfonamide |
| 61 | 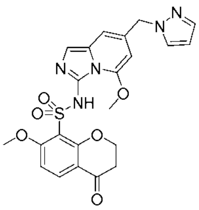 | *N*-(7-((1*H*-Pyrazol-1-yl)ymethyl)-5-methoxyimidazo[1,5-a]pyridin-3-yl)-7-methoxy-4-oxochromane-8-sulfonamide |
| 62 | 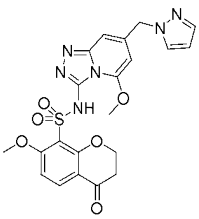 | *N*-(7-((1*H*-Pyrazol-1-yl)methyl)-5-methoxy-[1,2,4]triazolo[4,3-*a*]pyridin-3-yl)-7-methoxy-4-oxochromane-8-sulfonamide |
| 63 | 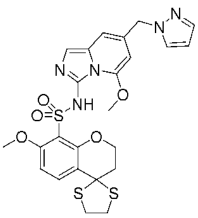 | *N*-(7-((1*H*-Pyrazol-1-yl)methyl)-5-methoxyimidazo[1,5-*a*]pyridin-3-yl)-7-methoxyspiro[chromane-4,2'-[1,3]dithiolane]-8-sulfonamide |
| 64 | 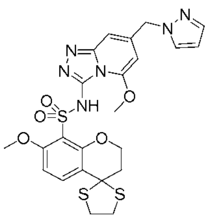 | *N*-(7-((1*H*-Pyrazol-1-yl)methyl)-5-methoxy-[1,2,4]triazolo[4,3-*a*]pyridin-3-yl)-7-methoxyspiro[chromane-4,2'-[1,3]dithiolane]-8-sulfonamide |

(continued)

| Compound No. | Structural formula | Chemical name |
|---|---|---|
| 65 | 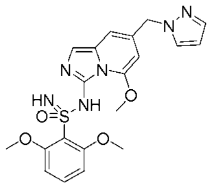 | *N*-(7-((1*H*-Pyrazol-1-yl)methyl)-5-methoxyimidazo[1,5-*a*]pyridin-3-yl)-2,6-dimethoxybenzenesulfonimidamide |
| 66 | 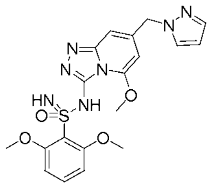 | *N*-(7-((1*H*-Pyrazol-1-yl)methyl)-5-methoxy-[1,2,4]triazolo[4,3-*a*]pyridin-3-yl)-2,6-dimethoxybenzenesulfonimidamide |
| 67 | 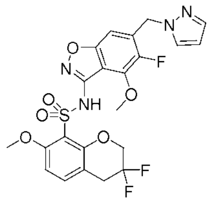 | *N*-(6-((1*H*-Pyrazol-1-yl)methyl)-5-fluoro-4-methoxybenzo[*d*]isoxazol-3-yl)-3,3-difluoro-7-methoxychromane-8-sulfonamide |
| 68 | 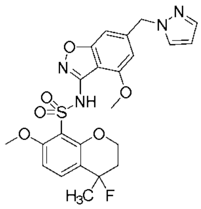 | *N*-(6-((1*H*-Pyrazol-1-yl)methyl)-4-methoxybenzo[*d*]isoxazol-3-yl)-4-fluoro-7-methoxy-4-methylchromane-8-sulfonamide |
| 69 | 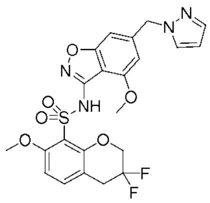 | *N*-(6-((1*H*-Pyrazol-1-yl)methyl)-4-methoxybenzo[*d*]isoxazol-3-yl)-3,3-difluoro-7-methoxychromane-8-sulfonamide |
| 70 | 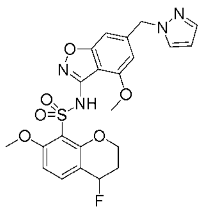 | *N*-(6-((1*H*-Pyrazol-1-yl)methyl)-4-methoxybenzo[*d*]isoxazol-3-yl)-4-fluoro-7-methoxychromane-8-sulfonamide |
| 71 | 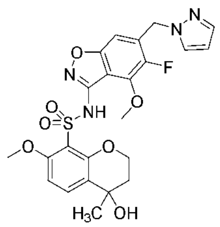 | *N*-(6-((1*H*-Pyrazol-1-yl)methyl)-5-fluoro-4-methoxybenzo[*d*]isoxazol-3-yl)-4-hydroxy-7-methoxy-4-methylchromane-8-sulfonamide |

(continued)

| Compound No. | Structural formula | Chemical name |
|---|---|---|
| 72 | 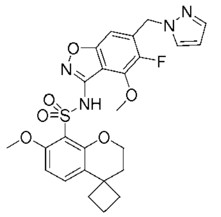 | *N*-(6-((1*H*-Pyrazol-1-yl)methyl)-5-fluoro-4-methoxybenzo[*d*]isoxazol-3-yl)-7-methoxyspiro[chromane-4,1'-cyclobutane]-8-sulfonamide |
| 73 | 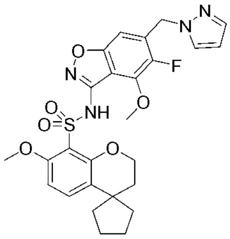 | *N*-(6-((1*H*-Pyrazol-1-yl)methyl)-5-fluoro-4-methoxybenzo[*d*]isoxazol-3-yl)-7-methoxyspiro[chromane-4,1'-cyclopentane]-8-sulfonamide |
| 74 | 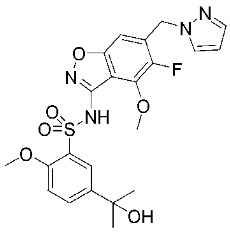 | *N*-(6-((1*H*-Pyrazol-1-yl)methyl)-5-fluoro-4-methoxybenzo[*d*]isoxazol-3-yl)-5-(2-hydroxypropan-2-yl)-2-methoxybenzenesulfonamide |
| 75 | 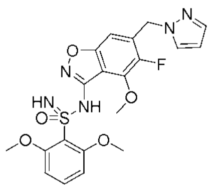 | *N*-(6-((1*H*-Pyrazol-1-yl)methyl)-5-fluoro-4-methoxybenzo[*d*]isoxazol-3-yl)-2,6-dimethoxybenzenesulfonimidamide |
| 76 | 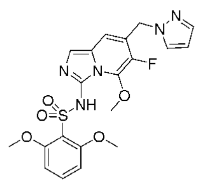 | *N*-(7-((1*H*-Pyrazol-1-yl)methyl)-6-fluoro-5-methoxyimidazo[1,5-*a*]pyridin-3-yl)-2,6-dimethoxybenzenesulfonamide |
| 77 | 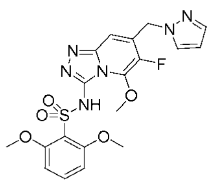 | *N*-(7-((1*H*-Pyrazol-1-yl)methyl)-6-fluoro-5-methoxy-[1,2,4]triazolo[4,3-*a*]pyridin-3-yl)-2,6-dimethoxybenzenesulfonamide |
| 78 | 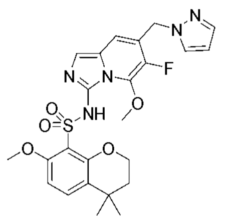 | *N*-(7-((1*H*-Pyrazol-1-yl)methyl)-6-fluoro-5-methoxyimidazo[1,5-*a*]pyridin-3-yl)-7-methoxy-4,4-dimethylchromane-8-sulfonamide |

(continued)

| Compound No. | Structural formula | Chemical name |
|---|---|---|
| 79 | 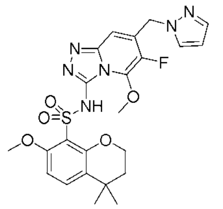 | *N*-(7-((1*H*-Pyrazol-1-yl)methyl)-6-fluoro-5-methoxy-[1,2,4]triazolo[4,3-*a*]pyridin-3-yl)-7-methoxy-4,4-dimethylchromane-8-sulfonamide |
| 80 | 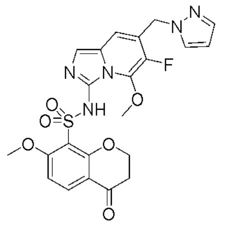 | *N*-(7-((1*H*-Pyrazol-1-yl)methyl)-6-fluoro-5-methoxyimidazo[1,5-*a*]pyridin-3-yl)-7-methoxy-4-oxochromane-8-sulfonamide |
| 81 | 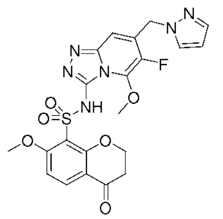 | *N*-(7-((1*H*-Pyrazol-1-yl)methyl)-6-fluoro-5-methoxy-[1,2,4]triazolo[4,3-*a*]pyridin-3-yl)-7-methoxy-4-oxochromane-8-sulfonamide |
| 82 | 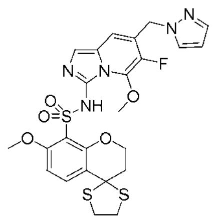 | *N*-(7-((1*H*-Pyrazol-1-yl)methyl)-6-fluoro-5-methoxyimidazo[1,5-*a*]pyridin-3-yl)-7-methoxyspiro[chromane-4,2'-[1,3]dithiolane]-8-sulfonamide |
| 83 | 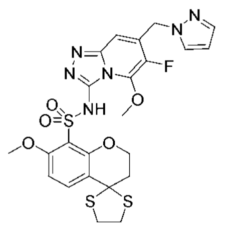 | *N*-(7-((1*H*-Pyrazol-1-yl)methyl)-6-fluoro-5-methoxy-[1,2,4]triazolo[4,3-*a*]pyridin-3-yl)-7-methoxyspiro[chromane-4,2'-[1,3]dithiolane]-8-sulfonamide |
| 84 | 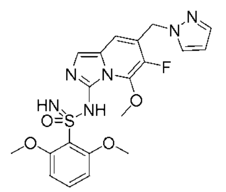 | *N*-(7-((1*H*-Pyrazol-1-yl)methyl)-6-fluoro-5-methoxyimidazo[1,5-*a*]pyridin-3-yl)-2,6-dimethoxybenzenesulfonimidamide |
| 85 | 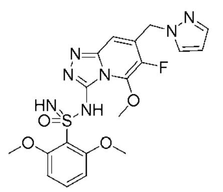 | *N*-(7-((1*H*-Pyrazol-1-yl)methyl)-6-fluoro-5-methoxy-[1,2,4]triazolo[4,3-*a*]pyridin-3-yl)-2,6-dimethoxybenzenesulfonimidamide |

(continued)

| Compound No. | Structural formula | Chemical name |
|---|---|---|
| 86 | 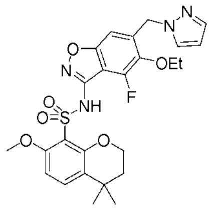 | *N*-(6-((1*H*-Pyrazol-1-yl)methyl)-5-ethoxy-4-fluorobenzo[*d*]isoxazol-3-yl)-7-methoxy-4,4-dimethylchromane-8-sulfonamide |
| 87 | 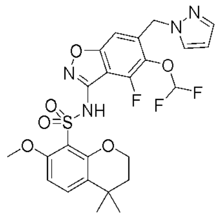 | *N*-(6-((1*H*-Pyrazol-1-yl)methyl)-5-(difluoromethoxy)-4-fluorobenzo[*d*]isoxazol-3-yl)-7-methoxy-4,4-dimethylchromane-8-sulfonamide |
| 88 | 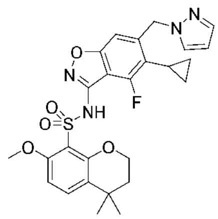 | *N*-(6-((1*H*-Pyrazol-1-yl)methyl)-5-cyclopropyl-4-fluorobenzo[*d*]isoxazol-3-yl)-7-methoxy-4,4-dimethylchromane-8-sulfonamide |
| 89 | 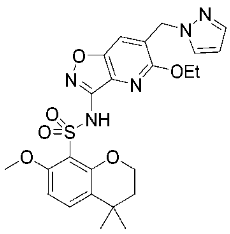 | *N*-(6-((1*H*-Pyrazol-1-yl)methyl)-5-ethoxyisoxazolo[4,5-b]pyridin-3-yl)-7-methoxy-4,4-dimethylchromane-8-sulfonamide |
| 90 | 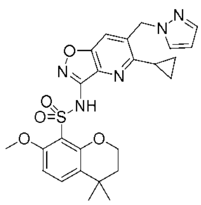 | *N*-(6-((1*H*-Pyrazol-1-yl)methyl)-5-cyclopropylisoxazolo[4,5-*b*]pyridin-3-yl)-7-methoxy-4,4-dimethylchromane-8-sulfonamide |
| 91 | 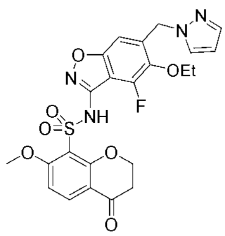 | *N*-(6-((1*H*-Pyrazol-1-yl)methyl)-5-ethoxy-4-fluorobenzo[*d*]isoxazol-3-yl)-7-methoxy-4-oxochromane-8-sulfonamide |
| 92 | 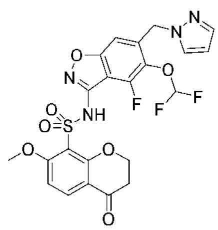 | *N*-(6-((1*H*-Pyrazol-1-yl)methyl)-5-(difluoromethoxy)-4-fluorobenzo[*d*]isoxazol-3-yl)-7-methoxy-4-oxochromane-8-sulfonamide |

(continued)

| Compound No. | Structural formula | Chemical name |
|---|---|---|
| 93 | 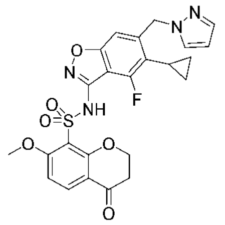 | *N*-(6-((1*H*-Pyrazol-1-yl)methyl)-5-cyclopropyl-4-fluorobenzo[*d*]isoxazol-3-yl)-7-methoxy-4-oxochromane-8-sulfonamide |
| 94 | 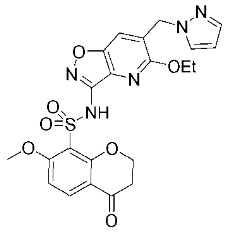 | *N*-(6-((1*H*-Pyrazol-1-yl)methyl)-5-ethoxyisoxazolo[4,5-b]pyridin-3-yl)-7-methoxy-4-oxochromane-8-sulfonamide |
| 95 | 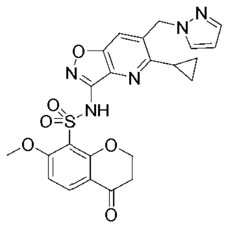 | *N*-(6-((1*H*-Pyrazol-1-yl)methyl)-5-cyclopropylisoxazolo[4,5-*b*]pyridin-3-yl)-7-methoxy-4-oxochromane-8-sulfonamide |
| 96 | 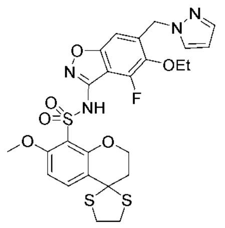 | *N*-(6-((1*H*-Pyrazol-1-yl)methyl)-5-ethoxy-4-fluorobenzo[*d*]isoxazol-3-yl)-7-methoxyspiro[chromane-4,2'-[1,3]dithiolane]-8-sulfonamide |
| 97 | 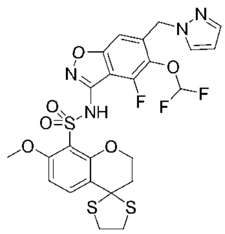 | *N*-(6-((1*H*-Pyrazol-1-yl)methyl)-5-(difluoromethoxy)-4-fluorobenzo[*d*]isoxazol-3-yl)-7-methoxyspiro[chromane-4,2'-[1,3]dithiolane]-8-sulfonamide |
| 98 | 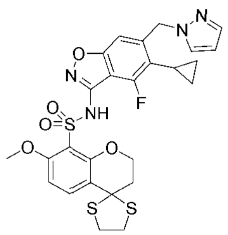 | *N*-(6-((1*H*-Pyrazol-1-yl)methyl)-5-cyclopropyl-4-fluorobenzo[*d*]isoxazol-3-yl)-7-methoxyspiro[chromane-4,2'-[1,3]dithiolane]-8-sulfonamide |

(continued)

| Compound No. | Structural formula | Chemical name |
|---|---|---|
| 99 | 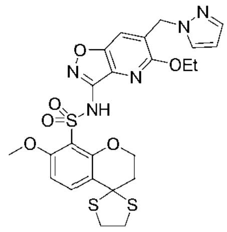 | *N*-(6-((1*H*-Pyrazol-1-yl)methyl)-5-ethoxyisoxazolo[4,5-*b*]pyridin-3-yl)-7-methoxyspiro[chromane-4,2'-[1,3]dithiolane]-8-sulfonamide |
| 100 | 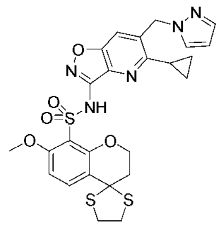 | *N*-(6-((1*H*-Pyrazol-1-yl)methyl)-5-cyclopropylisoxazolo[4,5-b]pyridin-3-yl)-7-methoxyspiro[chromane-4,2'-[1,3]dithiolane]-8-sulfonamide |
| 101 | 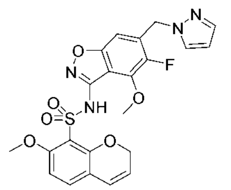 | *N*-(6-((1*H*-Pyrazol-1-yl)methyl)-5-fluoro-4-methoxybenzo[*d*]isoxazol-3-yl)-7-methoxy-2*H*-chromene-8-sulfonamide |
| 102 | 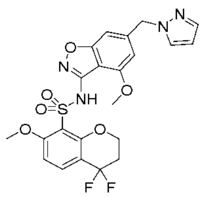 | *N*-(6-((1*H*-Pyrazol-1-yl)methyl)-4-methoxybenzo[*d*]isoxazol-3-yl)-4,4-difluoro-7-methoxychromane-8-sulfonamide |
| 103 | 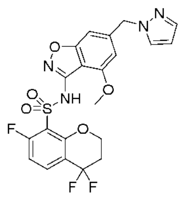 | *N*-(6-((1*H*-Pyrazol-1-yl)methyl)-4-methoxybenzo[*d*]isoxazol-3-yl)-4,4,7-trifluorochromane-8-sulfonamide |
| 104 | 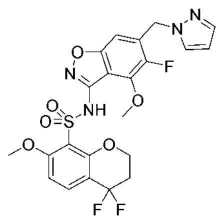 | *N*-(6-((1*H*-Pyrazol-1-yl)methyl)-5-fluoro-4-methoxybenzo[*d*]isoxazol-3-yl)-4,4-difluoro-7-methoxychromane-8-sulfonamide |
| 105 | 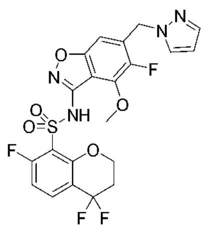 | *N*-(6-((1*H*-Pyrazol-1-yl)methyl)-5-fluoro-4-methoxybenzo[*d*]isoxazol-3-yl)-4,4,7-trifluorochromane-8-sulfonamide |

(continued)

| Compound No. | Structural formula | Chemical name |
|---|---|---|
| 106 | 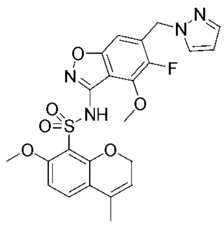 | *N*-(6-((1*H*-Pyrazol-1-yl)methyl)-5-fluoro-4-methoxybenzo[*d*]isoxazol-3-yl)-7-methoxy-4-methyl-2*H*-chromene-8-sulfonamide |
| 107 | 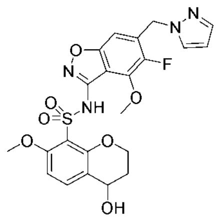 | *N*-(6-((1*H*-Pyrazol-1-yl)methyl)-5-fluoro-4-methoxybenzo[*d*]isoxazol-3-yl)-4-hydroxy-7-methoxychromane-8-sulfonamide |
| 108 | 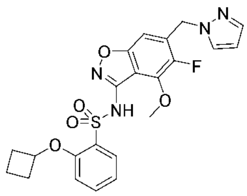 | *N*-(6-((1*H*-Pyrazol-1-yl)methyl)-5-fluoro-4-methoxybenzo[*d*]isoxazol-3-yl)-2-cyclobutoxybenzenesulfonamide |
| 109 | 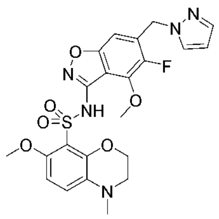 | *N*-(6-((1*H*-Pyrazol-1-yl)methyl)-5-fluoro-4-methoxybenzo[*d*]isoxazol-3-yl)-7-methoxy-4-methyl-3,4-dihydro-2*H*-benzo[*b*][1,4]oxazine-8-sulfonamide |
| 110 | 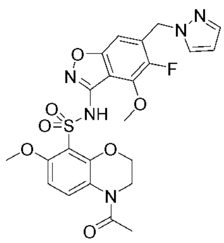 | *N*-(6-((1*H*-Pyrazol-1-yl)methyl)-5-fluoro-4-methoxybenzo[*d*]isoxazol-3-yl)-4-acetyl-7-methoxy-3,4-dihydro-2*H*-benzo[*b*][1,4]oxazine-8-sulfonamide |
| 111 | 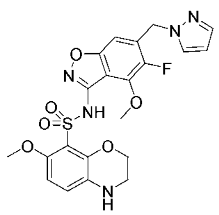 | *N*-(6-((1*H*-Pyrazol-1-yl)methyl)-5-fluoro-4-methoxybenzo[*d*]isoxazol-3-yl)-7-methoxy-3,4-dihydro-2*H*-benzo[*b*][1,4]oxazine-8-sulfonamide |
| 112 | 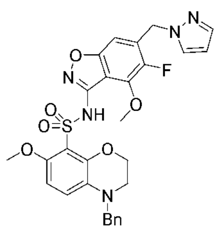 | *N*-(6-((1*H*-Pyrazol-1-yl)methyl)-5-fluoro-4-methoxybenzo[*d*]isoxazol-3-yl)-4-benzyl-7-methoxy-3,4-dihydro-2*H*-benzo[*b*][1,4]oxazine-8-sulfonamide |

(continued)

| Compound No. | Structural formula | Chemical name |
|---|---|---|
| 113 | 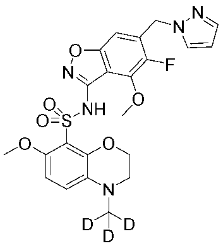 | *N*-(6-((1*H*-Pyrazol-1-yl)methyl)-5-fluoro-4-methoxybenzo[*d*]isoxazol-3-yl)-7-methoxy-4-(methyl-$d_3$)-3,4-dihydro-2*H*-benzo[*b*][1,4]oxazine-8-sulfonamide |
| 114 | 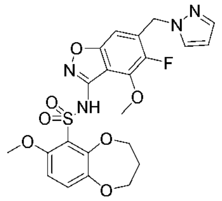 | *N*-(6-((1*H*-Pyrazol-1-yl)methyl)-5-fluoro-4-methoxybenzo[*d*]isoxazol-3-yl)-7-methoxy-3,4-dihydro-2*H*-benzo[*b*][1,4]dioxepine-6-sulfonamide |
| 115 | 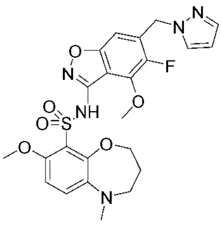 | *N*-(6-((1*H*-Pyrazol-1-yl)methyl)-5-fluoro-4-methoxybenzo[*d*]isoxazol-3-yl)-8-methoxy-5-methyl-2,3,4,5-tetrahydrobenzo[*b*][1,4]oxazepine-9-sulfonamide |
| 116 | 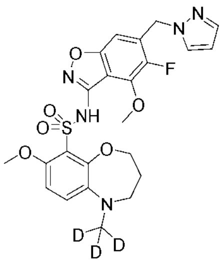 | *N*-(6-((1*H*-Pyrazol-1-yl)methyl)-5-fluoro-4-methoxybenzo[*d*]isoxazol-3-yl)-8-methoxy-5-(methyl-$d_3$)-2,3,4,5-tetrahydrobenzo[*b*][1,4]oxazepine-9-sulfonamide |
| 117 | 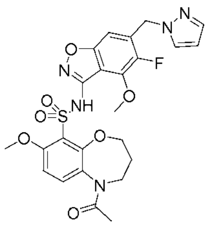 | *N*-(6-((1*H*-Pyrazol-1-yl)methyl)-5-fluoro-4-methoxybenzo[*d*]isoxazol-3-yl)-5-acetyl-8-methoxy-2,3,4,5-tetrahydrobenzo[*b*][1,4]oxazepine-9-sulfonamide |
| 118 | 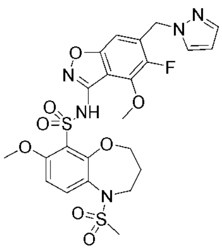 | *N*-(6-((1*H*-Pyrazol-1-yl)methyl)-5-fluoro-4-methoxybenzo[*d*]isoxazol-3-yl)-8-methoxy-5-(methylsulfonyl)-2,3,4,5-tetrahydrobenzo[*b*][1,4]oxazepine-9-sulfonamide |

(continued)

| Compound No. | Structural formula | Chemical name |
|---|---|---|
| 119 | 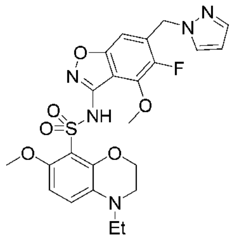 | *N*-(6-((1*H*-Pyrazol-1-yl)methyl)-5-fluoro-4-methoxybenzo[*d*]isoxazol-3-yl)-4-ethyl-7-methoxy-3,4-dihydro-2*H*-benzo[*b*][1,4]oxazine-8-sulfonamide |
| 120 | 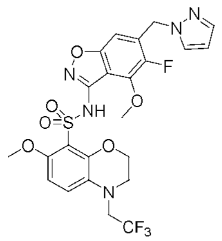 | *N*-(6-((1*H*-Pyrazol-1-yl)methyl)-5-fluoro-4-methoxybenzo[*d*]isoxazol-3-yl)-7-methoxy-4-(2,2,2-trifluoroethyl)-3,4-dihydro-2*H*-benzo[*b*][1,4]oxazine-8-sulfonamide |
| 121 | 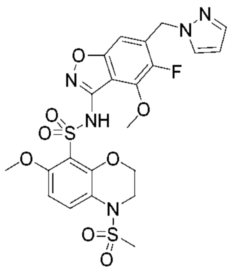 | *N*-(6-((1*H*-Pyrazol-1-yl)methyl)-5-fluoro-4-methoxybenzo[*d*]isoxazol-3-yl)-7-methoxy-4-(methylsulfonyl)-3,4-dihydro-2*H*-benzo[*b*][1,4]oxazine-8-sulfonamide |
| 122 | 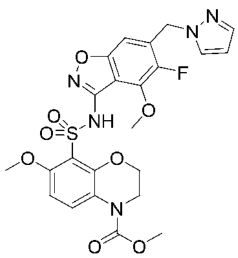 | Methyl 8-(*N*-(6-((1*H*-pyrazol-1-yl)methyl)-5-fluoro-4-methoxybenzo[*d*]isoxazol-3-yl)sulfamoyl)-7-methoxy-3,4-dihydro-4*H*-benzo[*b*][1,4]oxazine-4-carboxylate |
| 123 | 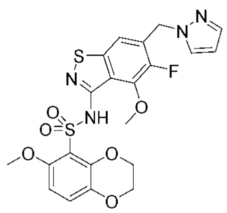 | *N*-(6-((1*H*-Pyrazol-1-yl)methyl)-5-fluoro-4-methoxybenzo[*d*]isothiazol-3-yl)-6-methoxy-2,3-dihydrobenzo[*b*][1,4]dioxine-5-sulfonamide |
| 124 | 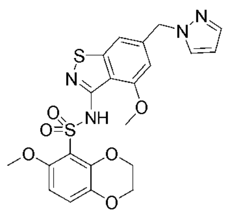 | *N*-(6-((1*H*-Pyrazol-1-yl)methyl)-4-methoxybenzo[*d*]isothiazol-3-yl)-6-methoxy-2,3-dihydrobenzo[*b*][1,4]dioxine-5-sulfonamide |

(continued)

| Compound No. | Structural formula | Chemical name |
|---|---|---|
| 125 | 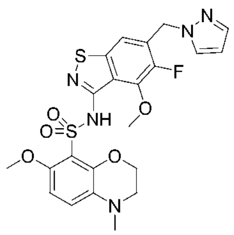 | *N*-(6-((1*H*-Pyrazol-1-yl)methyl)-5-fluoro-4-methoxybenzo[*d*]isothiazol-3-yl)-7-methoxy-4-methyl-3,4-dihydro-2*H*-benzo[*b*][1,4]oxazine-8-sulfonamide |
| 126 | 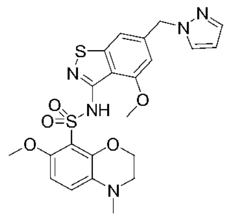 | *N*-(6-((1*H*-Pyrazol-1-yl)methyl)-4-methoxybenzo[*d*]isothiazol-3-yl)-7-methoxy-4-methyl-3,4-dihydro-2*H*-benzo[*b*][1,4]oxazine-8-sulfonamide |
| 127 | 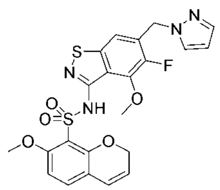 | *N*-(6-((1*H*-Pyrazol-1-yl)methyl)-5-fluoro-4-methoxybenzo[*d*]isothiazol-3-yl)-7-methoxy-2*H*-chromene-8-sulfonamide |
| 128 | 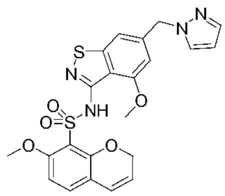 | *N*-(6-((1*H*-Pyrazol-1-yl)methyl)-4-methoxybenzo[*d*]isothiazol-3-yl)-7-methoxy-2*H*-chromene-8-sulfonamide |
| 129 | 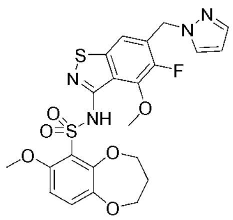 | *N*-(6-((1*H*-Pyrazol-1-yl)methyl)-5-fluoro-4-methoxybenzo[*d*]isothiazol-3-yl)-7-methoxy-3,4-dihydro-2*H*-benzo[*b*][1,4]dioxepine-6-sulfonamide |
| 130 | 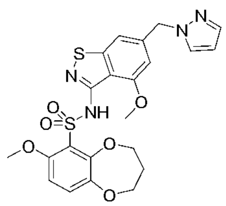 | *N*-(6-((1*H*-Pyrazol-1-yl)methyl)-4-methoxybenzo[*d*]isothiazol-3-yl)-7-methoxy-3,4-dihydro-2*H*-benzo[*b*][1,4]dioxepine-6-sulfonamide |
| 131 | 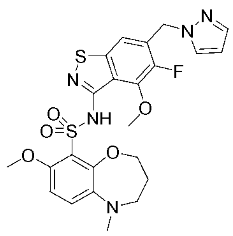 | *N*-(6-((1*H*-Pyrazol-1-yl)methyl)-5-fluoro-4-methoxybenzo[*d*]isothiazol-3-yl)-8-methoxy-5-methyl-2,3,4,5-tetrahydrobenzo[*b*][1,4]oxazepine-9-sulfonamide |

(continued)

| Compound No. | Structural formula | Chemical name |
|---|---|---|
| 132 | 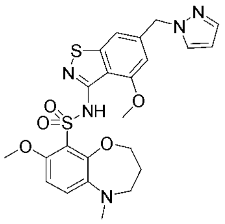 | *N*-(6-((1*H*-Pyrazol-1-yl)methyl)-4-methoxybenzo[*d*]isothiazol-3-yl)-8-methoxy-5-methyl-2,3,4,5-tetrahydrobenzo[*b*][1,4]oxazepine-9-sulfonamide |

The compounds of formula (I) (including compounds of any applicable sub-formulas as described herein or compounds listed in Table 1) may exist in the form of individual enantiomers, diastereomers, atropisomers, and/or geometric isomers (if applicable), or mixtures of stereoisomers (including racemic mixtures and mixtures enriched in one or more stereoisomers). In some embodiments, where applicable, the compounds of formula (I) (including compounds of any applicable sub-formulas described herein or compounds listed in Table 1) may exist as mixtures of atropisomers in any ratio (including about 1:1). In some embodiments, where applicable, the compounds of formula (I) (including compounds of any applicable sub-formulas described herein or compounds listed in Table 1) may exist as isolated individual atropisomers, which are substantially free of other atropisomers (e.g., by weight, by HPLC area, or both, having less than 20%, less than 10%, less than 5%, less than 1%, or undetectable amounts of other atropisomers).

In some embodiments, the compounds described above are isotopically substituted. Preferably, in some embodiments, the isotopic substitution is a substitution with a deuterium atom.

Surprisingly, the compounds of the present disclosure exhibit significantly improved inhibitory activity against KAT6A/B, significantly improved pharmacodynamics and pharmacokinetics, and/or a significantly improved safety profile compared to KAT6A/B inhibitors known in the prior art.

**Synthetic Methods for Compounds of the Present Disclosure**

In view of the present disclosure, those skilled in the art can readily synthesize the compounds of the present disclosure. Exemplary syntheses are shown in the "Examples" section.

The following synthetic methods for the compounds are exemplary. By using appropriate synthetic starting materials or intermediates, those skilled in the art can similarly apply the synthetic methods to synthesize other compounds.

To achieve the objectives of the present disclosure, the present disclosure adopts the following technical solutions:

A method for preparing the compound of formula (I) or the pharmaceutically acceptable salt thereof of the present disclosure, comprising the following steps:

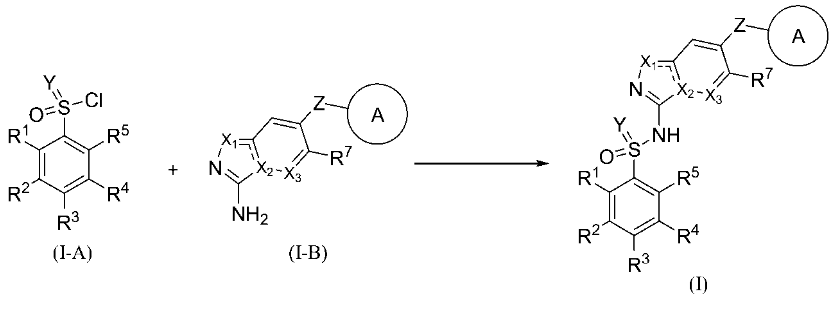

reacting a compound of formula (I-A) or a salt thereof with a compound of formula (I-B) or a salt thereof in the presence of a base to give the compound of formula (I) or the pharmaceutically acceptable salt thereof,

wherein:

the dashed line is an optional chemical bond, and

ring A, $R^1$, $R^2$, $R^3$, $R^4$, $R^5$, $R^7$, $X_1$, $X_2$, $X_3$, Y, and Z are as defined in the compound of formula (I) described above; or

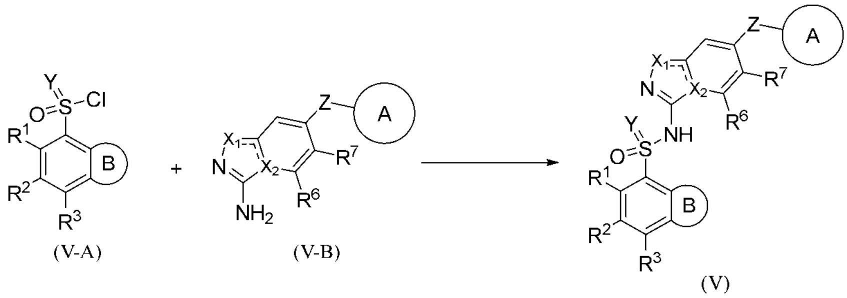

(V-A) (V-B) (V)

reacting a compound of formula (V-A) or a salt thereof with a compound of formula (V-B) or a salt thereof in the presence of a base to give a compound of formula (V) or a pharmaceutically acceptable salt thereof,
wherein:
the dashed line is an optional chemical bond, and
ring A, ring B, $R^1$, $R^2$, $R^3$, $R^6$, $R^7$, $X_1$, $X_2$, Y, and Z are as defined in the compound of formula (V) described above; or

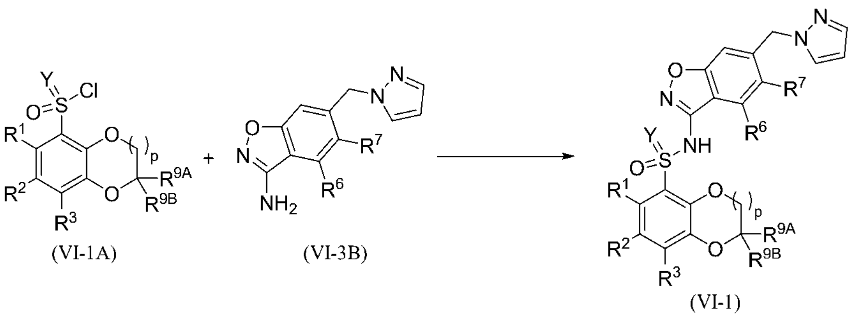

(VI-1A) (VI-3B) (VI-1)

reacting a compound of formula (VI-1A) or a salt thereof with a compound of formula (VI-3B) or a salt thereof in the presence of a base to give a compound of formula (VI-1) or a pharmaceutically acceptable salt thereof,
wherein:
$R^1$, $R^2$, $R^3$, $R^6$, $R^7$, $R^{9A}$ $R^{9B}$, Y, and p are as defined in the compound of formula (VI-1) described above; or

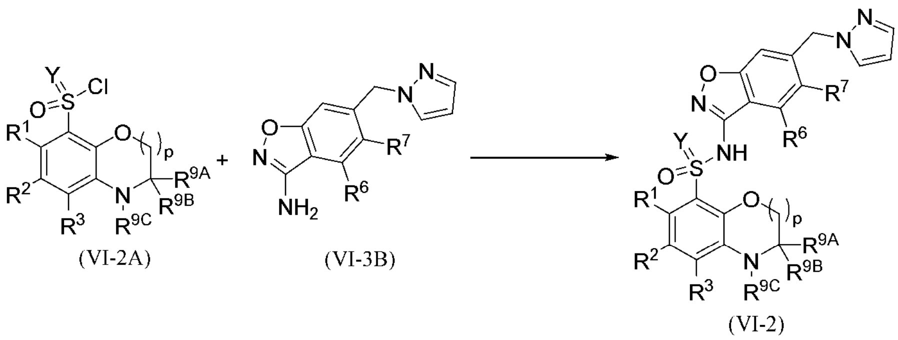

(VI-2A) (VI-3B) (VI-2)

reacting a compound of formula (VI-2A) or a salt thereof with a compound of formula (VI-3B) or a salt thereof in the presence of a base to give a compound of formula (VI-2) or a pharmaceutically acceptable salt thereof,
wherein:
$R^1$, $R^2$, $R^3$, $R^6$, $R^7$, $R^{9A}$, $R^{9B}$, $R^{9C}$, Y, and p are as defined in the compound of formula (VI-2) described above; or

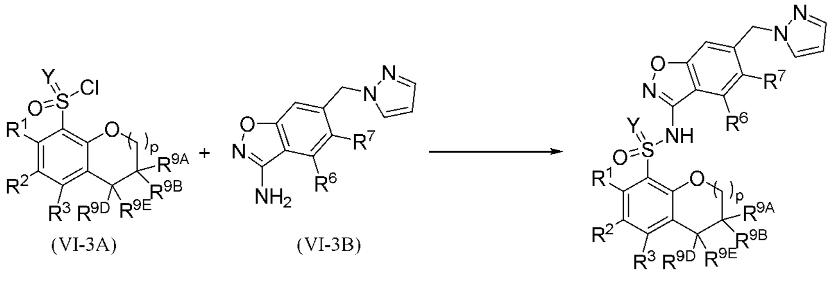

(VI-3A) (VI-3B) (VI-3)

reacting a compound of formula (VI-3A) or a salt thereof with a compound of formula (VI-3B) or a salt thereof in the presence of a base to give a compound of formula (VI-3) or a pharmaceutically acceptable salt thereof,
wherein:
$R^1$, $R^2$, $R^3$, $R^6$, $R^7$, $R^{9A}$, $R^{9B}$, $R^{9D}$, $R^{9E}$, Y, and p are as defined in the compound of formula (VI-3) described above; or

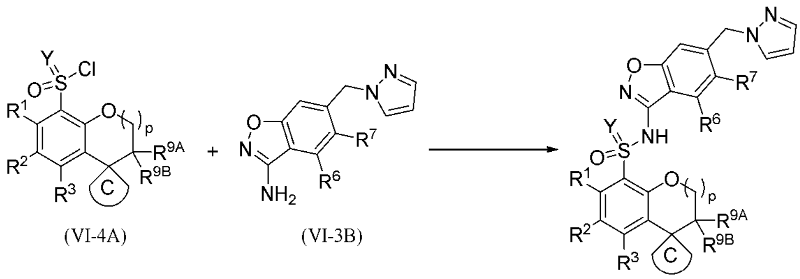

(VI-4A) (VI-3B) (VI-4)

reacting a compound of formula (VI-4A) or a salt thereof with a compound of formula (VI-3B) or a salt thereof in the presence of a base to give a compound of formula (VI-4) or a pharmaceutically acceptable salt thereof,
wherein:
ring C, $R^1$, $R^2$, $R^3$, $R^6$, $R^7$, $R^{9A}$, $R^{9B}$, Y, and p are as defined in the compound of formula (VI-4) described above; or

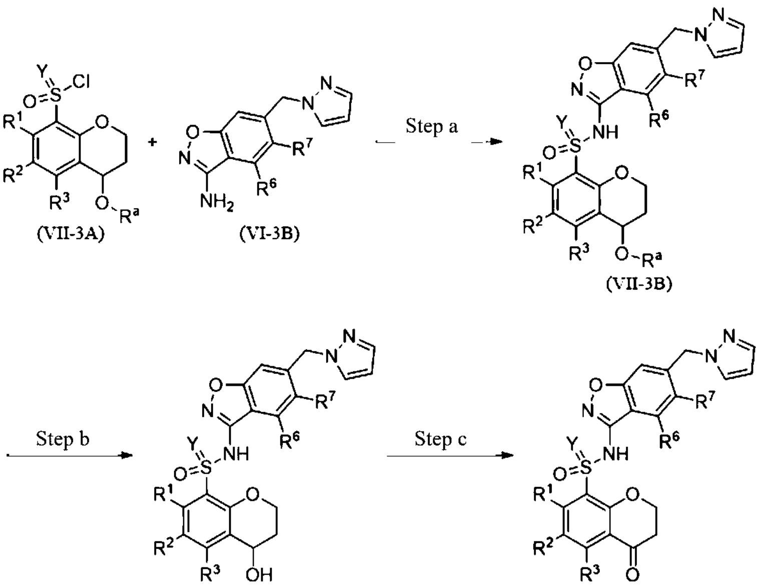


(VII-3A) (VI-3B) (VII-3B) (VII-3C) (VII-3)

step a: reacting a compound of formula (VII-3A) or a salt thereof with a compound of formula (VI-3B) or a salt thereof in the presence of a base to give a compound of formula (VII-3B) or a pharmaceutically acceptable salt thereof;
step b: reacting the compound of formula (VII-3B) or the salt thereof with a deprotecting reagent to remove a hydroxyl protecting group to give a compound of formula (VII-3C) or a pharmaceutically acceptable salt thereof; and
step c: reacting the compound of formula (VII-3C) or the salt thereof with an oxidizing reagent for oxidation to give a

compound of formula (VII-3) or a pharmaceutically acceptable salt thereof;
wherein:
$R^1$, $R^2$, $R^3$, $R^6$, $R^7$, and Y are as defined in the compound of formula (VII-3) described above;
$R^a$ is the hydroxyl protecting group, preferably (trimethylsilyl)ethoxymethyl;
the deprotecting reagent in step b is an acid or a fluorine-containing reagent, preferably trifluoroacetic acid or tetrabutylammonium fluoride;
the oxidizing reagent in step c is a reagent for oxidizing a hydroxyl group to a ketone, preferably manganese dioxide or Dess-Martin reagent.

The base used in the reactions of the above schemes is selected from: triethylamine, diisopropylethylamine, pyridine, 2,4-dimethylpyridine, 2,6-dimethylpyridine, n-butyllithium, lithium bis(trimethylsilyl)amide, sodium hydride, sodium hydroxide, cesium carbonate, and potassium carbonate.

The reactions of the above schemes are preferably carried out in a solvent selected from: ethylene glycol dimethyl ether, methanol, ethanol, acetonitrile, n-butanol, toluene, tetrahydrofuran, dichloromethane, dimethyl sulfoxide, 1,4-dioxane, N,N-dimethylformamide, N,N-dimethylacetamide, 1,2-dibromoethane, toluene, pyridine, and a mixture thereof.

It will be apparent to those skilled in the art that conventional protecting groups may be required to prevent certain functional groups from undergoing undesired reactions. Suitable protecting groups for various functional groups and suitable conditions for protecting and deprotecting particular functional groups are well known in the art. For example, many protecting groups are described in "Protective Groups in Organic Synthesis", 4th ed. P.G.M. Wuts; T.W. Greene, John Wiley, 2007, and the references cited therein. The reagents used in the reactions described herein are generally known compounds or can be prepared by known procedures or obvious modifications thereof. For example, some reagents suitable for use in the reactions described herein can be prepared by following the corresponding procedures described in WO2019/103952, the content of which is incorporated herein by reference in its entirety. Furthermore, many reagents are available from commercial suppliers, such as Aldrich Chemical Co. (Milwaukee, Wisconsin, USA) and Sigma (St. Louis, Missouri, USA). Other reagents can be prepared by procedures described in standard reference books or obvious modifications thereof, such as: Fieser and Fieser's Reagents for Organic Synthesis, Volumes 1-15 (John Wiley and Sons, 1991); Rodd's Chemistry of Carbon Compounds, Volumes 1-5 and Supplemental (Elsevier Science Publishers, 1989); Organic Reactions, Volumes 1-40 (John Wiley and Sons, 1991); March's Advanced Organic Chemistry, (Wiley, 7th ed.); and Larock's Comprehensive Organic Transformations (Wiley-VCH, 1999); as well as any updates available as of the present application.

## Pharmaceutical Composition

Certain embodiments relate to a pharmaceutical composition comprising one or more of the compounds of the present disclosure.

The pharmaceutical composition may optionally comprise a pharmaceutically acceptable excipient. In some embodiments, the pharmaceutical composition comprises the compound of the present disclosure (e.g., the compound of formula (I) to (VII-3), any one of Compound Nos. 1-132, or a pharmaceutically acceptable salt thereof) and a pharmaceutically acceptable excipient. Pharmaceutically acceptable excipients are known in the art. Non-limiting suitable excipients include, for example, encapsulating materials or additives, such as absorption enhancers, antioxidants, binders, buffers, carriers, coating agents, colorants, diluents, disintegrants, emulsifiers, extenders, fillers, flavoring agents, humectants, lubricants, perfumes, preservatives, propellants, releasing agents, sterilizing agents, sweeteners, solubilizers, wetting agents, and mixtures thereof. See also Remington's The Science and Practice of Pharmacy, 21st ed., A.R. Gennaro (Lippincott, Williams & Wilkins, Baltimore, Md., 2005; incorporated herein by reference), which discloses various excipients for formulating pharmaceutical compositions and known techniques for preparing pharmaceutical compositions.

The pharmaceutical composition may comprise any one or more of the compounds of the present disclosure. For example, in some embodiments, the pharmaceutical composition comprises, for example, a therapeutically effective amount of the compound of formula (I) to (VII-3), any one of Compound Nos. 1-132, or a pharmaceutically acceptable salt thereof. In any of the embodiments described herein, the pharmaceutical composition may comprise a therapeutically effective amount of a compound selected from Compound Nos. 1-132, or a pharmaceutically acceptable salt thereof.

The pharmaceutical composition may also be formulated for delivery via any known route of delivery, including but not limited to oral delivery, parenteral delivery, inhalation, etc.

In some embodiments, the pharmaceutical composition may be formulated for oral administration. Oral formulations may be presented as: discrete
units, such as capsules, pills, cachets, lozenges, or tablets, each containing a predetermined amount of the active compound; powders or granules; solutions or suspensions in aqueous or non-aqueous liquids; or oil-in-water or water-in-oil emulsions. Excipients for preparing orally administered compositions are known in the art. Non-limiting suitable excipients include, for example, agar, alginic acid, aluminum hydroxide, benzyl alcohol, benzyl benzoate, 1,3-butanediol, carbomer, castor oil, cellulose, cellulose acetate, cocoa butter, corn starch, corn oil, cottonseed oil, crospovidone, diglycerides, ethanol, ethyl cellulose, ethyl laurate, ethyl oleate, fatty acid esters, gelatin, germ oil, glucose, glycerol, peanut oil, hydroxypropyl methylcellulose, isopropanol, isotonic saline, lactose, magnesium hydroxide, magnesium stearate, malt, mannitol, monoglycerides, olive oil, potassium phosphate salts, potato starch, povidone, propylene glycol, Ringer's solution, safflower oil, sesame oil, sodium carboxymethyl cellulose, sodium phosphate salts, sodium lauryl sulfate, sodium sorbitol, soybean oil, stearic acid, stearyl fumarate, sucrose, surfactants, talc, tragacanth, tetrahydrofurfuryl alcohol, triglycerides, water, and mixtures thereof.

In some embodiments, the pharmaceutical composition is formulated for parenteral administration (e.g., intravenous injection or infusion, or subcutaneous or intramuscular injection). Parenteral formulations may be, for example, aqueous solutions, suspensions, or emulsions. Excipients for preparing parenteral formulations are known in the art. Non-limiting suitable excipients include, for example, 1,3-butanediol, castor oil, corn oil, cottonseed oil, glucose, germ oil, peanut oil, liposomes, oleic acid, olive oil, Ringer's solution, safflower oil, sesame oil, soybean oil, U.S.P. or isotonic sodium chloride solution, water, and mixtures thereof.

In some embodiments, the pharmaceutical composition is formulated for inhalation administration. For example, inhalable formulations may be formulated as nasal sprays, dry powders, or as aerosols that can be administered via metered dose inhalers. Excipients for preparing inhalation formulations are known in the art. Non-limiting suitable excipients include, for example, lactose, talc, silicic acid, aluminum hydroxide, calcium silicate, and polyamide powder, as well as mixtures of these substances. Sprays may also contain propellants, such as chlorofluorocarbons and volatile unsubstituted hydrocarbons, for example, butane and propane.

The pharmaceutical composition may comprise various amounts of the compound of the present disclosure, depending on various factors, such as the intended use, potency, and selectivity of the compound. In some embodiments, the pharmaceutical composition comprises a therapeutically effective amount of the compound of the present disclosure (e.g., the compound of formula I to VII-3, any one of Compound Nos. 1-132, or a pharmaceutically acceptable salt thereof). In some embodiments, the pharmaceutical composition comprises a therapeutically effective amount of the compound of the present disclosure and a pharmaceutically acceptable excipient. As used herein, the therapeutically effective amount of the compound of the present disclosure is an amount effective for treating a cancer as described herein, such as lung cancer, breast cancer, rectal cancer, colon cancer, esophageal cancer, gastric cancer, liver cancer, gallbladder cancer, cholangiocarcinoma, kidney cancer, bladder cancer, urothelial cancer, head and neck cancer, nasopharyngeal cancer, prostate cancer, cervical cancer/endometrial cancer, ovarian cancer, pancreatic cancer, melanoma, bone cancer, mesothelioma, gastrointestinal stromal tumor, sarcoma, brain glioma, thyroid cancer, salivary gland tumor, glioblastoma, neuroblastoma, gastric myxoma, lymphoma, leukemia, plasmacytoma, sinoatrial node tumor, or tenosynovial giant cell tumor. This amount may depend on the recipient of treatment, the cancer being treated and the severity thereof, the composition containing the compound, the time of administration, the route of administration, the duration of treatment, the potency of the compound (e.g., the potency for inhibiting KAT6A/B), its clearance rate, and whether another drug is co-administered.

For veterinary use, the compounds of the present disclosure may be administered in appropriately acceptable formulations according to normal veterinary practice. A veterinarian can readily determine the most appropriate administration regimen and route of administration for a particular animal.

In some embodiments, all necessary ingredients for treating such diseases using the compound of the present disclosure, either alone or in combination with another agent or intervention conventionally used for treating a cancer associated with KAT6A/B, can be packaged into a kit. Specifically, in some embodiments, the present disclosure provides a kit for therapeutic intervention for a disease, comprising a packaged drug set, wherein the drug set comprises the compound disclosed herein, as well as a buffer and other components for preparing the drug in a deliverable form, and/or a device for delivering such drugs, and/or any agent for combination therapy with the compound of the present disclosure, and/or instructions for use packaged together with the drug for treating the disease. The instructions for use may be fixed in any tangible medium, such as printed paper or a computer-readable magnetic or optical medium, or may indicate reference to a remote computer data source, such as a World Wide Web webpage accessible via the Internet.

### Treatment Methods/Uses

The compounds of the present disclosure can be used as therapeutically active substances for treating and/or preventing cancers associated with KAT6A/B. In particular, the compounds of the present disclosure, by acting on KAT6A/B to mediate signal transduction, can be used to treat disorders associated with the modulation of KAT6A/B function, especially the inhibition of KAT6A/B function. Such disorders include diseases in which the pathogenic mechanisms are associated with these cytokines mediated by KAT6A/B, and they encompass any one of those diseases known in the art and those diseases described herein.

In some embodiments, the present disclosure provides a method for inhibiting KAT6A/B, comprising contacting a cell with an effective amount of one or more of the compounds of the present disclosure (e.g., the compound of formula I to VII-3, any one of Compound Nos. 1-132, or a pharmaceutically acceptable salt thereof).

In some embodiments, the present disclosure provides a method for inhibiting KAT6A/B function in a subject in need, comprising administering to the subject an effective amount of one or more of the compounds of the present disclosure (e.g., the compound of formula I to VII-3, any one of Compound Nos. 1-132, or a pharmaceutically acceptable salt thereof).

In some embodiments, the present disclosure provides a method for treating or preventing a KAT6A/B-mediated cancer in a subject in need, comprising administering to the subject an effective amount of one or more of the compounds of the present disclosure (e.g., the compound of formula I to VII-3, any one of Compound Nos. 1-132, or a pharmaceutically acceptable salt thereof). Suitable KAT6A/B-mediated cancers that can be treated using the method described herein include any of those cancers known in the art. Exemplary KAT6A/B-mediated cancers that can be treated using the method described herein also include, but are not limited to, lung cancer, breast cancer, rectal cancer, colon cancer, esophageal cancer, gastric cancer, liver cancer, gallbladder cancer, cholangiocarcinoma, kidney cancer, bladder cancer, urothelial cancer, head and neck cancer, nasopharyngeal cancer, prostate cancer, cervical cancer/endometrial cancer, ovarian cancer, pancreatic cancer, melanoma, bone cancer, mesothelioma, gastrointestinal stromal tumor, sarcoma, brain glioma, thyroid cancer, salivary gland tumor, glioblastoma, neuroblastoma, gastric myxoma, lymphoma, leukemia, plasmacytoma, sinoatrial node tumor, and tenosynovial giant cell tumor described herein.

In some embodiments, the present disclosure provides a method for treating or preventing, for example, the cancer described herein, in a subject in need, comprising administering to the subject an effective amount of one or more of the compounds of the present disclosure (e.g., the compound of formula I to VII-3, any one of Compound Nos. 1-132, or a pharmaceutically acceptable salt thereof).

In some embodiments, the present disclosure provides a method for treating or preventing a cancer in a subject in need, comprising administering to the subject an effective amount of one or more of the compounds of the present disclosure (e.g., the compound of formula I to VII-3, any one of Compound Nos. 1-132, or a pharmaceutically acceptable salt thereof), wherein the cancer may be one or more cancers selected from: lung cancer, breast cancer, rectal cancer, colon cancer, esophageal cancer, gastric cancer, liver cancer, gallbladder cancer, cholangiocarcinoma, kidney cancer, bladder cancer, urothelial cancer, head and neck cancer, nasopharyngeal cancer, prostate cancer, cervical cancer/-endometrial cancer, ovarian cancer, pancreatic cancer, melanoma, bone cancer, mesothelioma, gastrointestinal stromal tumor, sarcoma, brain glioma, thyroid cancer, salivary gland tumor, glioblastoma, neuroblastoma, gastric myxoma, lymphoma, leukemia, plasmacytoma, sinoatrial node tumor, and tenosynovial giant cell tumor, wherein the breast cancer is preferably $ER^+$ breast cancer or $ER^+/HER2^-$ breast cancer, the lung cancer is preferably non-small cell lung cancer, and the prostate cancer is preferably castration-resistant prostate cancer.

The administration described herein is not limited to any particular route of administration. For example, in some embodiments, the administration may be oral, nasal, transdermal, pulmonary, inhalation, buccal, sublingual, intraperitoneal, subcutaneous, intramuscular, intravenous, rectal, intrapleural, intrathecal, and parenteral. In some embodiments, the administration is oral administration.

The administration regimen, including the dose, can vary and can be adjusted, which may depend on the recipient of treatment, the cancer being treated and the severity thereof, the composition containing the compound, the time of administration, the route of administration, the duration of treatment, the potency of the compound, its clearance rate, and whether another drug is co-administered.

## Examples

The present disclosure is further described with reference to the following examples; however, these examples are not intended to limit the scope of the present disclosure.

The structures of the compounds were determined by nuclear magnetic resonance (NMR) spectroscopy or/and mass spectrometry (MS). The NMR shifts ($\delta$) are given in $10^{-6}$ (ppm). The NMR analyses were performed using a Bruker AVANCE NEO 500M nuclear magnetic resonance system, with dimethyl sulfoxide-D6 (DMSO-$d_6$), chloroform-D ($CDCl_3$), and methanol-D4 ($CD_3OD$) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters; MS model: Waters ACQuity Qda Detector/Waters SQ Detector), and THERMO Ultimate 3000-Q Exactive (manufacturer: THERMO; MS model: THERMO Q Exactive).

The high-performance liquid chromatography (HPLC) analyses were performed using Agilent HPLC 1200DAD, Agilent HPLC 1200VWD, and Waters HPLC e2695-2489 high-performance liquid chromatographs.

The chiral HPLC analyses were performed using an Agilent 1260 DAD high-performance liquid chromatograph.

The preparative high-performance liquid chromatography was performed using Waters 2767, Waters 2767-SQ

Detecor2, Shimadzu LC-20AP, and Gilson-281 preparative chromatographs.

The preparative chiral chromatography was performed using a Shimadzu LC-20AP preparative chromatograph.

The CombiFlash preparative flash chromatograph used was CombiFlash Rf200 (TELEDYNE ISCO).

The thin-layer chromatography (TLC) silica gel plates used were Yantai Huanghai HSGF254 silica gel plates. The silica gel plates used in the TLC analyses had a layer thickness of 0.15 mm-0.2 mm, and those used in the TLC separation and purification for products had a layer thickness of 0.4 mm-0.5 mm.

Silica gel column chromatography generally used 200-300 mesh or 300-400 mesh silica gel as the carrier.

The mean kinase inhibition rates and $IC_{50}$ values were measured using a NovoStar microplate reader (BMG, Germany).

The known starting materials in the present disclosure may be synthesized by using or according to methods known in the art, or may be purchased from companies such as Shanghai Titan Scientific, ABCR GmbH & Co. KG, Acros Organics, Aldrich Chemical Company, Accela ChemBio Inc., Bide Pharmatech, etc.

In the examples, the reactions could all be performed under an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenation apparatus and a Qinglan QL-500 hydrogen generator, or an HC2-SS hydrogenation apparatus.

The hydrogenation reactions generally involved 3 cycles of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The monitoring of reaction progress in the examples was performed using thin-layer chromatography (TLC). The developing solvents used in the reactions, the eluent systems used in the column chromatography purification of compounds, and the developing solvent systems used in the thin-layer chromatography include: A: n-hexane/ethyl acetate system, and B: dichloromethane/methanol system. The volume ratio of the solvents was adjusted depending on the polarity of the compound, or a small amount of a basic or acidic reagent such as triethylamine and acetic acid could be added for adjustment.

**Example 1**

Synthesis of Intermediate 1

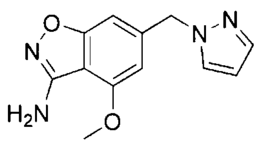

**Intermediate 1**

6-((1*H*-Pyrazol-1-yl)methyl)-4-methoxybenzo[*d*]isoxazol-3-amine

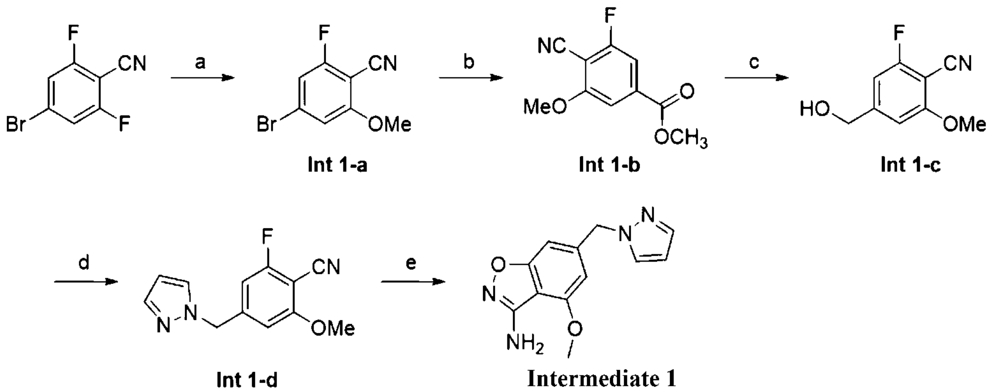


Int 1-a Int 1-b Int 1-c

Int 1-d Intermediate 1

Step a:

4-Bromo-2-fluoro-6-methoxybenzonitrile

A sodium methoxide solution (9.92 g, 55.09 mmol, 30 wt% in methanol) was added to a solution of 4-bromo-2,6-difluorobenzonitrile (10.00 g, 45.87 mmol) in tetrahydrofuran (60 mL) at 0 °C. The reaction mixture was stirred at 25 °C for 16 h, diluted with water, and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 20:1) to give compound **Int 1-a** (2.72 g, yield: 25.8%).

$^1$H NMR (300 MHz, $CDCl_3$): $\delta$ 7.02-6.99 (m, 1H), 6.94 (s, 1H), 3.96 (s, 3H).

Step b:

Methyl 4-cyano-3-fluoro-5-methoxybenzoate

Triethylamine (14 mL, 100.07 mmol) and [1,1'-bis(diphenylphosphino)ferrocene]palladium(II) dichloride (2.55 g, 3.49 mmol) were added to a solution of compound **Int 1-a** (8.00 g, 34.78 mmol) in methanol (80 mL). The reaction system was stirred at 80 °C for 16 h under a carbon monoxide atmosphere (3.0 MPa). After the reaction was completed, the reaction mixture was cooled to room temperature, diluted with water, and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 20:1) to give compound **Int 1-b** (6.43 g, yield: 88.4%).

$^1$HNMR (300 MHz, $CDCl_3$): $\delta$7.46-7.38 (m, 2H), 4.01 (s, 3H), 3.95 (s, 3H).

Step c:

2-Fluoro-4-(hydroxymethyl)-6-methoxybenzonitrile

Lithium borohydride (0.74 g, 33.98 mmol) was added to a solution of compound **Int 1-b** (3.57 g, 17.07 mmol) in tetrahydrofuran (30 mL) at 0 °C. The reaction system was heated to 80 °C and then stirred for 2 h. After the reaction was completed, the reaction mixture was cooled to room temperature, then quenched with a saturated aqueous sodium bicarbonate solution (20 mL), washed with water (20 mL), and extracted with ethyl acetate (20 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 4:1) to give compound **Int 1-c** (2.14 g, yield: 69.2%).

$^1$HNMR (300 MHz, DMSO-$d_6$): $\delta$7.05 (s, 1H), 6.98 (d, 1H), 5.60 (t, 1H), 4.57 (d, 2H), 3.94 (s, 3H).

Step d:

4-((1*H*-Pyrazol-1-yl)methyl)-2-fluoro-6-methoxybenzonitrile

Cesium carbonate (4.61 g, 14.15 mmol) was added to a solution of compound **Int 1-c** (2.14 g, 11.81 mmol) and

1-(methylsulfonyl)-1*H*-pyrazole (2.07 g, 14.16 mmol) in tetrahydrofuran (30 mL). The reaction system was heated to 70 °C and stirred for 1 h. After the reaction was completed, the reaction mixture was cooled to room temperature, then diluted with water, and extracted with ethyl acetate (25 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 2:1) to give compound **Int 1-d** (2.15 g, yield: 78.7%).

MS m/z (ESI): 232.0 $[M+H]^+$.

Step e:

6-((1*H*-Pyrazol-1-yl)methyl)-4-methoxybenzo[*d*]isoxazol-3-amine

Tetramethylguanidine (8.5 mL, 67.60 mmol) was added dropwise to compound **Int 1-d** (2.59 g, 11.20 mmol) and acetohydroxamic acid (2.52 g, 33.57 mmol) in a mixed solution (*N,N*-dimethylformamide and water in a ratio of 9:1, 30 mL). The reaction system was heated to 60 °C and stirred for 7 h. After the reaction was completed, the reaction mixture was cooled to room temperature, then diluted with water (50 mL), and extracted with ethyl acetate (30 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 2:1) to give compound **intermediate 1** (2.17 g, yield: 79.3%).

$^1$HNMR (300 MHz, DMSO-$d_6$): δ 7.87 (d, 1H), 7.50 (d, 1H), 6.69 (s, 1H), 6.63 (s, 1H), 6.30 (t, 1H), 5.95 (s, 2H), 5.41 (s, 2H), 3.85 (s, 3H).

MS m/z (ESI): 245.1 $[M+H]^+$.

**Example 2**

Synthesis of Intermediate 2

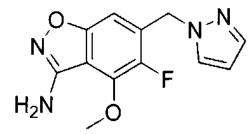

**Intermediate 2**

6-((1*H*-Pyrazol-1-yl)methyl)-5-fluoro-4-methoxybenzo[d]isoxazol-3-amine

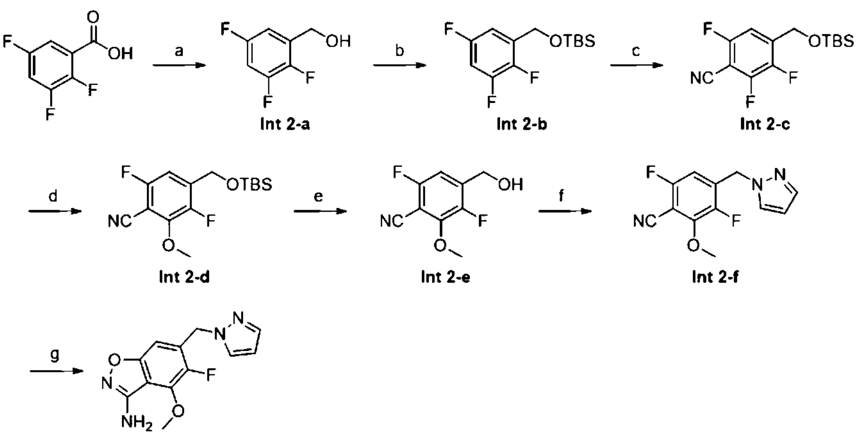


**Intermediate 2**

Step a:

(2,3,5-Trifluorophenyl)methanol

A solution of borane in tetrahydrofuran (1.0 M in tetrahydrofuran, 123 mL, 122.66 mmol) was added to a solution of 2,3,5-trifluorobenzoic acid (7.20 g, 40.89 mmol) in tetrahydrofuran (70 mL) at 25 °C. The reaction system was heated to 50 °C and stirred for 4 h. After the reaction was completed, the mixture was cooled to room temperature, quenched with a 1 N aqueous hydrochloric acid solution (50 mL), and extracted with ethyl acetate (500 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 9:1) to give compound **Int 2-a** (6.03 g, yield: 91.0%).
$^1$H NMR (400 MHz, $CDCl_3$): δ7.01-6.97 (m, 1H), 6.89-6.82 (m, 1H), 4.79 (s, 2H).

Step b:

*tert*-Butyldimethyl((2,3,5-trifluorobenzyl)oxy)silane

Compound **Int 2-a** (7.48 g, 46.14 mmol), *tert*-butyldimethylsilyl chloride (8.34 g, 55.33 mmol), triethylamine (7.00 g, 69.18 mmol), and 4-dimethylaminopyridine (0.56 g, 4.58 mmol) were dissolved in a solution of dichloromethane (80 mL). The reaction system was stirred at 25 °C for 16 h. After the reaction was completed, the reaction mixture was cooled to room temperature, diluted with water, and extracted with dichloromethane (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 20:1) to give compound **Int 2-b** (11.40 g, yield: 89.4%).
$^1$H NMR (400 MHz, $CDCl_3$): δ7.02-6.98 (m, 1H), 6.82-6.77 (m, 1H), 4.79 (s, 2H), 0.95 (s, 9H), 0.13 (s, 6H).

Step c:

4-(((*tert*-Butyldimethylsilyl)oxy)methyl)-2,3,6-trifluorobenzonitrile

Lithium diisopropylamide (2.0 M in *n*-hexane, 18.50 mL, 37.00 mmol) was added to a solution of compound **Int 2-b** (6.80 g, 24.60 mmol) in tetrahydrofuran (80 mL) at -78 °C. After the reaction system was stirred at -78 °C for 1 h, 4-toluenesulfonyl cyanide (4.90 g, 27.04 mmol) was added to the system, and stirring was continued for 2 h. After the reaction was completed, the reaction mixture was warmed to room temperature, quenched with a saturated ammonium chloride solution, and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 9:1) to give compound **Int 2-c** (3.60 g, yield: 48.5%).
$^1$H NMR (400 MHz, $CDCl_3$): δ7.23-7.19 (m, 1H), 4.83 (s, 2H), 0.95 (s, 9H), 0.14 (s, 6H).

Step d:

4-(((*tert*-Butyldimethylsilyl)oxy)methyl)-3,6-difluoro-2-methoxybenzonitrile

Compound **Int 2-c** (3.60 g, 11.94 mmol) and sodium methoxide (1.93 g, 35.72 mmol) were dissolved in a solution of tetrahydrofuran (40 mL). The reaction system was heated to 80 °C and stirred for 4 h. After the reaction was completed, the reaction mixture was warmed to room temperature, diluted with water, and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **Int 2-d** (1.60 g, yield: 42.7%).
$^1$H NMR (400 MHz, $CDCl_3$): δ6.91-6.87 (m, 1H), 4.78 (s, 2H), 4.14 (d, 3H), 0.95 (s, 9H), 0.13 (s, 6H).

Step e:

3,6-Difluoro-4-(hydroxymethyl)-2-methoxybenzonitrile

Tetrabutylammonium fluoride (2.67 g, 10.21 mmol) was added to a solution of compound **Int 2-d** (1.60 g, 5.10 mmol) in tetrahydrofuran (20 mL). The reaction system was stirred at 25 °C for 1 h. After the reaction was completed, the reaction mixture was diluted with water (20 mL) and extracted with ethyl acetate (30 mL × 3). The organic layers were

combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **Int 2-e** (0.79 g, yield: 77.7%).

$^1$H NMR (400 MHz, $CDCl_3$): $\delta$7.06-7.03 (m, 1H), 4.81 (s, 2H), 4.15 (d, 3H).

Step f:

4-((1*H*-Pyrazol-1-yl)methyl)-3,6-difluoro-2-methoxybenzonitrile

Cesium carbonate (2.58 g, 7.92 mmol) was added to a solution of compound **Int 2-e** (0.79 g, 3.97 mmol) and 1-(methylsulfonyl)-1*H*-pyrazole (0.70 g, 4.76 mmol) in acetonitrile (20 mL). The reaction system was heated to 75 °C and stirred for 4 h. After the reaction was completed, the reaction mixture was cooled to room temperature, then diluted with water, and extracted with ethyl acetate (25 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **Int 2-f** (0.81 g, yield: 81.9%).

MS m/z (ESI): 250.0 $[M+H]^+$.

Step g:

6-((1*H*-Pyrazol-1-yl)methyl)-5-fluoro-4-methoxybenzo[*d*]isoxazol-3-amine

Compound **Int 2-f** (810 mg, 3.25 mmol), acetohydroxamic acid (732 mg, 9.75 mmol), and 2-*tert*-butyl-1,1,3,3-tetramethylguanidine (557 mg, 3.25 mmol) were dissolved in acetonitrile (15 mL). The reaction system was heated to 80 °C and stirred for 16 h. After the reaction was completed, the reaction mixture was cooled to room temperature and concentrated under reduced pressure. The resulting crude product was purified by flash silica gel column chromatography (petroleum ether:ethyl acetate = 2:1) to give compound **intermediate 2** (440 mg, yield: 51.6%).

$^1$HNMR (400 MHz, DMSO-$d_6$): $\delta$7.86 (d, 1H), 7.54 (d, 1H), 6.72 (d, 1H), 6.31 (t, 1H), 6.11 (s, 2H), 5.47 (s, 2H), 4.06 (d, 3H).

**Example 3**

Synthesis of Intermediate 3

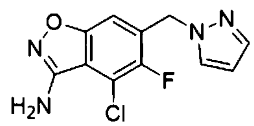

**Intermediate 3**

6-((1*H*-Pyrazol-1-yl)methyl)-4-chloro-5-fluorobenzo[*d*]isoxazol-3-amine

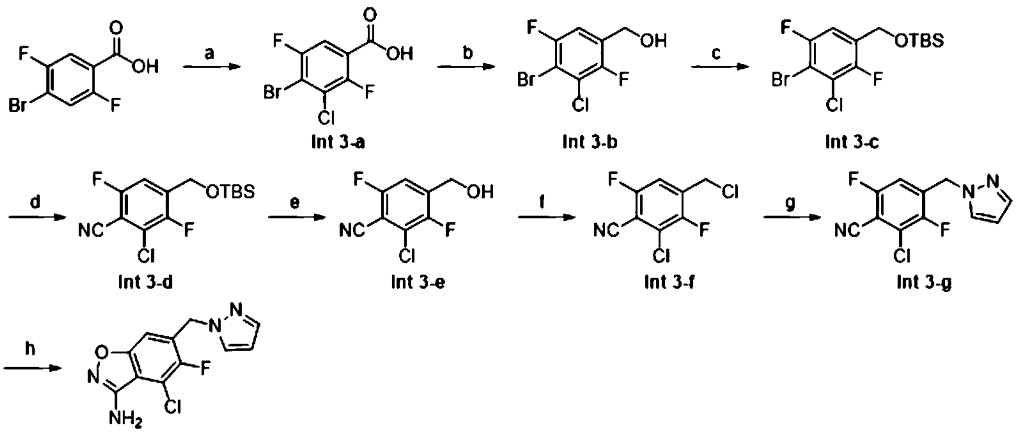


Int 3-a Int 3-b Int 3-c Int 3-d Int 3-e Int 3-f Int 3-g

Intermediate 3

Step a:

4-Bromo-3-chloro-2,5-difluorobenzoic acid

Under a nitrogen atmosphere, at 80 °C, *N*-chlorosuccinimide (5.63 g, 42.16 mmol) was added to a solution of 4-bromo-2,5-difluorobenzoic acid (5.00 g, 21.10 mmol) in 98% concentrated sulfuric acid (30 mL). The reaction system was then stirred at 80 °C for 16 h. After the reaction was completed, the mixture was poured into ice water, and the resulting mixture was concentrated under reduced pressure. The resulting filter cake was purified by flash C18 reversed-phase column chromatography (methanol:water (0.1% trifluoroacetic acid) = 3:2) to give compound **Int 3-a** (2.30 g, yield: 40.2%).
$^1$HNMR (400 MHz, DMSO-$d_6$): $\delta$14.05 (brs, 1H), 7.80 (dd, 1H).

Step b:

(4-Bromo-3-chloro-2,5-difluorophenyl)methanol

Under a nitrogen atmosphere, at 0 °C, a solution of borane in tetrahydrofuran (1.0 M in tetrahydrofuran, 28.74 mL, 28.74 mmol) was added dropwise to a solution of compound **Int 3-a** (3.90 g, 14.37 mmol) in tetrahydrofuran (60 mL). The reaction system was heated to 50 °C and stirred for 2.5 h. After the reaction was completed, the reaction system was quenched with methanol at 0 °C and extracted with ethyl acetate (200 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 9:1) to give compound **Int 3-b** (3.26 g, yield: 88.1%).
$^1$HNMR (400 MHz, DMSO-$d_6$): $\delta$7.44 (dd, 1H), 5.61 (t, 1H), 4.56 (d, 2H).

Step c:

((4-Bromo-3-chloro-2,5-difluorobenzyl)oxy)(*tert*-butyl)dimethylsilane

*tert*-Butyldimethylsilyl chloride (2.10 g, 13.93 mmol) was added to a solution of compound **Int 3-b** (3.26 g, 12.66 mmol) and pyrazole (1.72 g, 25.32 mmol) in dichloromethane (60 mL) at 25 °C. The reaction system was stirred at 25 °C for 2 h. After the reaction was completed, the reaction system was poured into ice water, and the resulting mixture was extracted with ethyl acetate (200 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether) to give compound **Int 3-c** (4.56 g, yield: 96.9%).
$^1$HNMR (400 MHz, DMSO-$d_6$): $\delta$7.38 (dd, 1H), 4.75 (s, 2H), 0.89 (s, 9H), 0.10 (s, 6H).

Step d:

4-(((*tert*-Butyldimethylsilyl)oxy)methyl)-2-chloro-3,6-difluorobenzonitrile

Under a nitrogen atmosphere, compound **Int 3-c** (4.65 g, 12.51 mmol) and copper(I) cyanide (5.60 g, 62.55

mmol) were dissolved in a solution of *N,N*-dimethylformamide (25 mL). The reaction system was heated to 140 °C and stirred for 16 h. After the reaction was completed, the reaction system was quenched with water and extracted with ethyl acetate (200 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 9:1) to give compound **Int 3-d** (1.89 g, yield: 47.5%).

$^1$HNMR (400 MHz, DMSO-$d_6$): 7.48 (dd, 1H), 4.86 (s, 2H), 0.91 (s, 9H), 0.12 (s, 6H).

Step e:

2-Chloro-3,6-difluoro-4-(hydroxymethyl)benzonitrile

Tetrabutylammonium fluoride (2.18 g, 8.33 mmol) was added to a solution of compound **Int 3-d** (1.89 g, 5.95 mmol) in tetrahydrofuran (38 mL) at 0 °C. The reaction system was stirred at 0 °C for 1 h. After the reaction was completed, the reaction system was poured into ice water, and the resulting mixture was extracted with ethyl acetate (150 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 4:1) to give compound **Int 3-e** (0.84 g, yield: 69.4%).

$^1$HNMR (400 MHz, DMSO-$d_6$): 7.55 (dd, 1H), 5.81 (t, 1H), 4.65 (d, 2H).

Step f:

2-Chloro-4-(chloromethyl)-3,6-difluorobenzonitrile

Under a nitrogen atmosphere, *N,N*-dimethylformamide (0.15 g, 2.04 mmol) was added to a solution of compound **Int 3-e** (4.15 g, 20.39 mmol) and thionyl chloride (12.13 g, 101.96 mmol) in dichloromethane (40 mL). The reaction system was heated to 40 °C and stirred for 3 h. After the reaction was completed, the reaction system was diluted with water and extracted with ethyl acetate (300 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 9:1) to give compound **Int 3-f** (4.35 g, yield: 96.1%).

$^1$HNMR (400 MHz, DMSO-$d_6$): 7.84 (dd, 1H), 4.88 (s, 2H).

Step g:

4-((1*H*-Pyrazol-1-yl)methyl)-2-chloro-3,6-difluorobenzonitrile

Under a nitrogen atmosphere, compound **Int 3-f** (2.25 g, 10.13 mmol), pyrazole (0.76 g, 11.16 mmol), 18-crown-6 (0.80 g, 3.03 mmol), and potassium carbonate (2.80 g, 20.26 mmol) were dissolved in acetonitrile (24 mL). The reaction system was heated to 80 °C and stirred for 1.5 h. After the reaction was completed, the reaction mixture was cooled to room temperature, then diluted with water (50 mL), and extracted with ethyl acetate (150 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 9:1) to give compound **Int 3-g** (1.00 g, yield: 38.9%).

$^1$HNMR (400 MHz, DMSO-$d_6$): $\delta$ 7.89 (d, 1H), 7.53 (d, 1H), 7.21 (dd, 1H), 6.33 (t, 1H), 5.54 (s, 2H).

Step h:

6-((1*H*-Pyrazol-1-yl)methyl)-4-chloro-5-fluorobenzo[d]isoxazol-3-amine

Compound **Int 3-g** (21.34 g, 5.28 mmol), acetohydroxamic acid (1.19 g, 15.85 mmol), and potassium carbonate (2.19 g, 15.85 mmol) were dissolved in a mixed solution (*N,N*-dimethylformamide and water in a ratio of 5:1, 24 mL). The reaction system was heated to 80 °C and stirred for 1.5 h. After the reaction was completed, the reaction mixture was cooled to room temperature and poured into ice water, and the resulting mixture was extracted with ethyl acetate (150 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 9:1) to give compound

**intermediate 3** (0.94 g, yield: 69.1%).
$^1$HNMR (400 MHz, DMSO-$d_6$): $\delta$7.87 (d, 1H), 7.50 (d, 1H), 7.18 (d, 1H), 6.31 (t, 1H), 6.24 (s, 2H), 5.52 (s, 2H).
MS m/z (ESI): 267.2 [M+H]$^+$.

**Example 4**

Synthesis of Intermediate 4

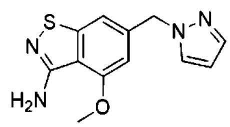

**Intermediate 4**

6-((1*H*-Pyrazol-1-yl)methyl)-4-methoxybenzo[*d*]isothiazol-3-amine

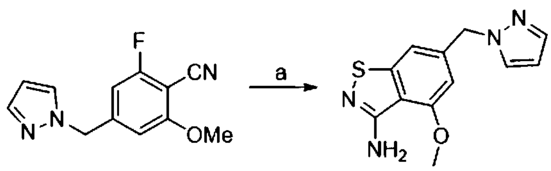


**Int 1-d** **Intermediate 4**

Step a:

6-((1*H*-Pyrazol-1-yl)methyl)-4-methoxybenzo[*d*]isothiazol-3-amine

Under a nitrogen atmosphere, sodium sulfide (157 mg, 2.02 mmol) was added to a solution of compound **Int 1-d** (466 mg, 2.02 mmol) in dimethyl sulfoxide (15 mL). The reaction system was heated to 70 °C and stirred for 7 h. After the reaction was completed, the reaction system was cooled to 0 °C. Subsequently, aqueous ammonia (6 mL) and an aqueous sodium hypochlorite solution (6 mL) were slowly added to the reaction system, and stirring was continued for 16 h. The reaction mixture was diluted with water (100 mL) and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:methanol = 9:1) to give compound **intermediate 4** (210 mg, yield: 40.0%).
$^1$HNMR (300 MHz, DMSO-$d_6$): $\delta$7.87 (d, 1H), 7.49 (d, 1H), 7.13 (s, 1H), 6.74 (s, 1H), 6.50 (s, 2H), 6.30 (t, 1H), 5.42 (s, 2H), 3.90 (s, 3H), 2.54 (s, 2H).

**Example 5**

Synthesis of Intermediate 5

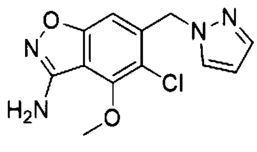

**Intermediate 5**

6-((1*H*-Pyrazol-1-yl)methyl)-5-chloro-4-methoxybenzo[*d*]isoxazol-3-amine

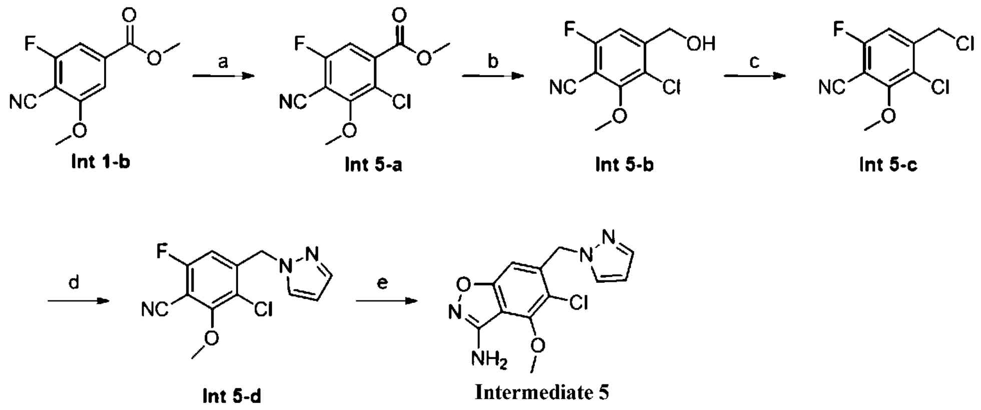

Int 1-b
Int 5-a
Int 5-b
Int 5-c
Int 5-d
Intermediate 5

Step a:

Methyl 2-chloro-4-cyano-5-fluoro-3-methoxybenzoate

Compound **Int 1-b** (2.10 g, 10.04 mmol), trichloroisocyanuric acid (1.16 g, 4.99 mmol), and trifluoromethanesulfonic acid (0.09 mL, 1.00 mmol) were dissolved in a solution of hexafluoroisopropanol (20 mL). The reaction system was heated to 60 °C and stirred for 40 h. After the reaction was completed, the mixture was cooled to room temperature, quenched with water, and extracted with ethyl acetate (150 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:dichloromethane = 4:1) to give compound **Int 5-a** (1.45 g, yield: 59.3%).

$^1$H NMR (400 MHz, $CDCl_3$): $\delta$7.34 (d, 1H), 4.14 (s, 3H), 3.97 (s, 3H).

Step b:

3-Chloro-6-fluoro-4-(hydroxymethyl)-2-methoxybenzonitrile

Lithium borohydride (2.0 M in tetrahydrofuran, 6.6 mL, 13.20 mmol) was added dropwise to a solution of compound **Int 5-a** (1.60 g, 6.57 mmol) in tetrahydrofuran (50 mL) at 0 °C. Subsequently, the reaction system was heated to 50 °C and then stirred for 1 h. After the reaction was completed, the reaction mixture was cooled to room temperature, then quenched with a saturated aqueous ammonium chloride solution, and extracted with ethyl acetate (150 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 4:1) to give compound **Int 5-b** (1.37 g, yield: 96.7%).

$^1$HNMR (400 MHz, DMSO-$d_6$): $\delta$7.28 (d, 1H), 4.82 (d, 2H), 4.10 (s, 3H).

Step c:

3-Chloro-4-(chloromethyl)-6-fluoro-2-methoxybenzonitrile

Thionyl chloride (10 mL) and one drop of *N,N*-dimethylformamide were added to a solution of compound **Int 5-b** (610 mg, 2.83 mmol) in dichloromethane (10 mL). The reaction system was heated to 40 °C and stirred for 3 h. After the reaction was completed, the reaction mixture was cooled to room temperature, then diluted with a saturated aqueous sodium bicarbonate solution, and extracted with dichloromethane (50 mL × 3). The organic layers were combined, washed with a saturated aqueous NaCl solution, and dried over anhydrous sodium sulfate. After filtration, the mother liquor was concentrated under reduced pressure to give compound **Int 5-c** (580 mg, yield: 87.7%).

$^1$HNMR (400 MHz, DMSO-$d_6$): $\delta$7.19 (d, 1H), 7.67 (s, 2H), 7.13 (s, 3H).

Step d:

4-((1*H*-Pyrazol-1-yl)methyl)-3-chloro-6-fluoro-2-methoxybenzonitrile

Compound **Int 5-c** (960 mg, 4.10 mmol), pyrazole (838 mg, 12.31 mmol), cesium fluoride (1.87 g, 12.31 mmol), and tetrabutylammonium iodide (152 mg, 0.41 mmol) were dissolved in a solution of acetonitrile (40 mL). The reaction system was heated to 40 °C and stirred for 16 h. After the reaction was completed, the mixture was poured into ice water, and the resulting mixture was extracted with ethyl acetate (100 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 6:1) to give compound **Int 5-d** (770 mg, yield: 70.7%).

$^1$HNMR (400 MHz, DMSO-$d_6$): δ7.90 (d, 1H), 7.57 (d, 1H), 6.55 (d, 1H), 6.36 (t, 1H), 5.55 (s, 2H), 4.07 (s, 3H).

Step e:

6-((1*H*-Pyrazol-1-yl)methyl)-5-chloro-4-methoxybenzo[*d*]isoxazol-3-amine

Compound **Int 5-d** (1.61 g, 6.06 mmol), acetohydroxamic acid (1.37 g, 18.25 mmol), and potassium carbonate (2.53 g, 18.31 mmol) were dissolved in a mixed solution (*N*,*N*-dimethylformamide and water in a ratio of 10:1, 55 mL). The reaction system was heated to 70 °C and stirred for 16 h. After the reaction was completed, the reaction mixture was cooled to room temperature, then diluted with water (50 mL), and extracted with ethyl acetate (150 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 4:1) to give **intermediate 5** (1.10 g, yield: 65.1%).

$^1$HNMR (400 MHz, DMSO-$d_6$): δ7.88 (d, 1H), 7.55 (d, 1H), 6.60 (s, 1H), 6.34 (t, 1H), 6.20 (s, 2H), 5.53 (s, 2H), 3.94 (s, 3H).

**Example 6**

Synthesis of Intermediate 6

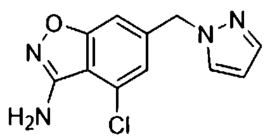

**Intermediate 6**

6-((1*H*-Pyrazol-1-yl)methyl)-4-chlorobenzo[*d*]isoxazol-3-amine

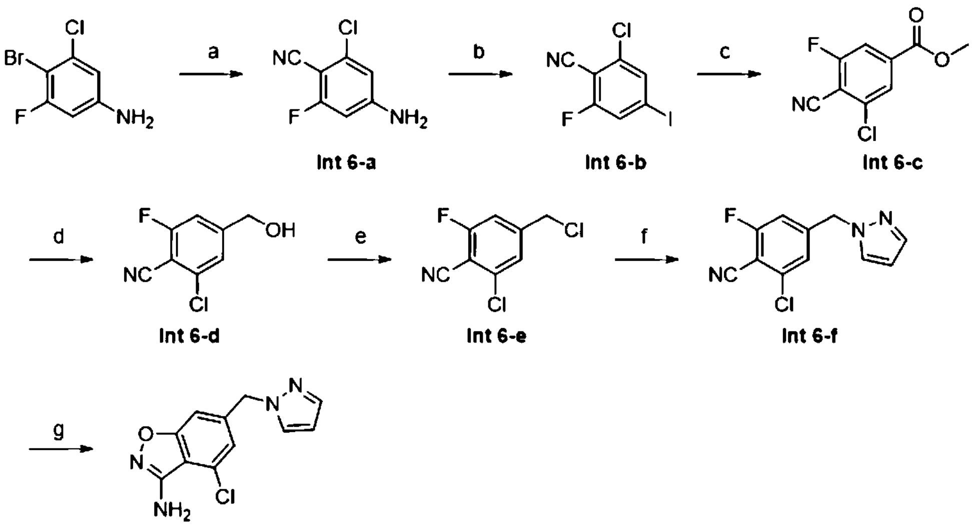


Int 6-a Int 6-b Int 6-c Int 6-d Int 6-e Int 6-f

**Intermediate 6**

Step a:

4-Amino-2-chloro-6-fluorobenzonitrile

Under a nitrogen atmosphere, 4-bromo-3-chloro-5-fluoroaniline (21.70 g, 96.68 mmol) and copper(I) cyanide (8.66 g, 96.69 mmol) were dissolved in a solution of *N*-methylpyrrolidinone (100 mL). The reaction system was heated to 190 °C and stirred for 2 h. After the reaction was completed, the reaction mixture was cooled to room temperature, quenched with 14% aqueous ammonia, and extracted with ethyl acetate (300 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 1:1) to give compound **Int 6-a** (16.40 g, yield: 99.5%).
$^1H$ NMR (300 MHz, DMSO-$d_6$): $\delta$6.87 (s, 2H), 6.60 (d, 1H), 6.44 (dd, 1H).

Step b:

2-Chloro-6-fluoro-4-iodobenzonitrile

An aqueous solution (100 mL) of sodium nitrite (4.45 g, 64.46 mmol) was slowly added to a solution of compound **Int 6-a** (10.00 g, 58.63 mmol) and 98% concentrated sulfuric acid (9.4 mL, 175.80 mmol) in acetonitrile (300 mL) and water (90 mL) at 0 °C. The reaction system was stirred at 10 °C for 15 min, then warmed to room temperature, and stirred for another 2 h. After the reaction was completed, the mixture was poured into an aqueous solution (200 mL), and the resulting mixture was extracted with ethyl acetate (300 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 20:1) to give compound **Int 6-b** (12.00 g, yield: 72.7%).
$^1H$ NMR (300 MHz, $CDCl_3$): $\delta$7.73 (s, 1H), 7.55 (dd, 1H).

Step c:

Methyl 3-chloro-4-cyano-5-fluorobenzoate

Triethylamine (15.21 g, 150.30 mmol) and [1,1'-bis(diphenylphosphino)ferrocene]palladium(II) dichloride (3.64 g, 4.97 mmol) were added to a solution of compound **Int 6-b** (14.10 g, 50.10 mmol) in methanol (140 mL). The reaction system was stirred at 40 °C for 18 h under a carbon monoxide atmosphere (2.0 MPa). After the reaction was completed, the reaction mixture was cooled to room temperature, diluted with water, and extracted with ethyl acetate (300 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 92:8) to give compound **Int 6-c** (8.40 g, yield: 78.5%).

$^1$HNMR (300 MHz, $CDCl_3$): $\delta$7.99 (s, 1H), 7.77 (dd, 1H), 3.98 (s, 3H).

Step d:

2-Chloro-6-fluoro-4-(hydroxymethyl)benzonitrile

Under a nitrogen atmosphere, a solution of lithium borohydride (39.3 mL, 78.60 mmol) in tetrahydrofuran (100 mL) was added to a solution of compound **Int 6-c** (8.40 g, 39.33 mmol) in tetrahydrofuran (100 mL). The reaction system was stirred at 25 °C for 1 h. After the reaction was completed, the reaction mixture was quenched with a saturated aqueous ammonium chloride solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 7:3) to give compound **Int 6-d** (5.00 g, yield: 68.5%).

$^1$HNMR (300 MHz, $CDCl_3$): $\delta$7.34 (s, 1H), 7.18 (dd, 1H), 4.78 (s, 2H), 1.86 (s, 1H).

Step e:

2-Chloro-4-(chloromethyl)-6-fluorobenzonitrile

Under a nitrogen atmosphere, a solution of thionyl chloride (6.02 g, 50.61 mmol) in dichloromethane (10 mL) was added dropwise to a solution of compound **Int 6-d** (4.70 g, 25.33 mmol) and *N*,*N*-dimethylformamide (0.02 g, 0.25 mmol) in dichloromethane (70 mL). The reaction system was heated to 40 °C and stirred for 1 h. After the reaction was completed, the mixture was poured into ice water, and the resulting mixture was extracted with dichloromethane (200 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 92:8) to give compound **Int 6-e** (4.74 g, yield: 91.7%).

$^1$HNMR (400 MHz, $CDCl_3$): $\delta$7.37 (s, 1H), 7.20 (dd, 1H), 4.55 (s, 2H).

Step f:

4-((1*H*-Pyrazol-1-yl)methyl)-2-chloro-6-fluorobenzonitrile

Compound **Int 6-e** (4.70 g, 23.04 mmol), pyrazole (4.70 g, 69.04 mmol), cesium fluoride (10.48 g, 68.99 mmol), and tetrabutylammonium iodide (0.85 g, 2.30 mmol) were dissolved in a solution of acetonitrile (100 mL). The reaction system was heated to 50 °C and stirred for 18 h. After the reaction was completed, the reaction mixture was quenched with water and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 82:18) to give compound **Int 6-f** (3.61 g, yield: 66.5%).

$^1$HNMR (400 MHz, DMSO-$d_6$): $\delta$7.91 (d, 1H), 7.54 (d, 1H), 7.35 (s, 1H), 7.28 (d, 1H), 6.34 (t, 1H), 5.48 (s, 2H).

Step g:

6-((1*H*-Pyrazol-1-yl)methyl)-4-chlorobenzo[*d*]isoxazol-3-amine

Compound **Int 6-f** (3.61 g, 15.32 mmol), acetohydroxamic acid (3.45 g, 45.96 mmol), and tetramethylguanidine (10.57 g, 91.77 mmol) were dissolved in a mixed solution (*N*,*N*-dimethylformamide and water in a ratio of 10:1, 55 mL). The reaction system was heated to 80 °C and stirred for 18 h. After the reaction was completed, the reaction mixture was cooled to room temperature, then diluted with water (100 mL), and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:2) to give **intermediate 6** (2.90 g, yield: 71.0%).

$^1$HNMR (300 MHz, DMSO-$d_6$): $\delta$7.90 (d, 1H), 7.51 (d, 1H), 7.24 (s, 1H), 7.10 (s, 1H), 6.31 (t, 1H), 6.19 (s, 2H), 5.46 (s, 2H).

**Example 7**

Synthesis of Compound 1

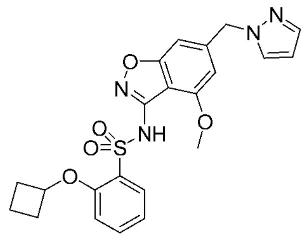

**1**

*N*-(6-((1*H*-Pyrazol-1-yl)methyl)-4-methoxybenzo[*d*]isoxazol-3-yl)-2-cyclobutoxybenzenesulfonamide

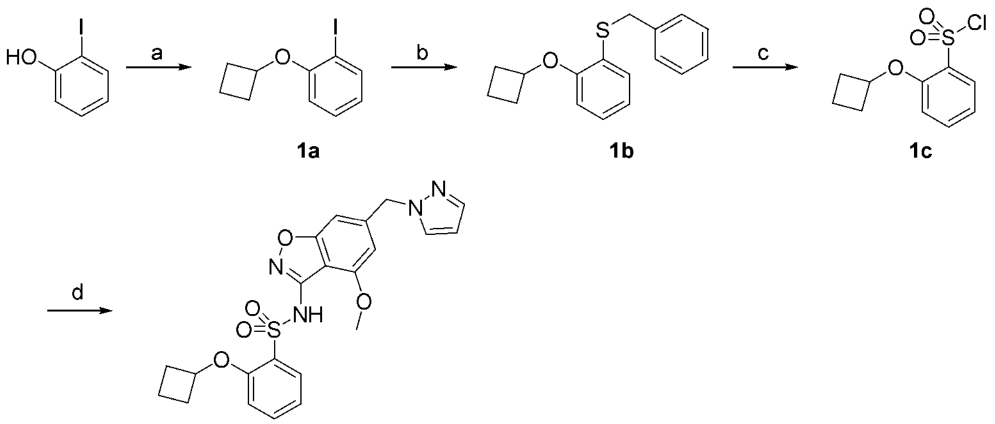


Step a:

1-Cyclobutoxy-2-iodobenzene

Under a nitrogen atmosphere, potassium carbonate (5.30 g, 38.35 mmol) was added to a solution of 2-iodophenol (2.80 g, 12.73 mmol) and cyclobutyl bromide (3.41 g, 25.26 mmol) in *N*,*N*-dimethylformamide (40 mL), and the reaction system was heated to 80 °C and stirred for 16 h. After the reaction was completed, the reaction mixture was cooled to room temperature, diluted with water (100 mL), and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **1a** (2.60 g, yield: 74.5%).

$^1$H NMR (300 MHz, $CDCl_3$): $\delta$7.81 (d, 1H), 7.32-7.27 (m, 1H), 6.76-6.71 (m, 2H), 4.76-4.68 (m, 1H), 2.57-2.48 (m, 2H), 2.38-2.25 (m, 2H), 1.99-1.89 (m, 1H), 1.96-1.62 (m, 1H).

Step b:

Benzyl(2-cyclobutoxyphenyl)sulfane

Under a nitrogen atmosphere, benzyl mercaptan (1.77 g, 14.25 mmol), cesium carbonate (6.18 g, 18.97 mmol), 1,4-diazabicyclo[2.2.2]octane (0.11 g, 0.98 mmol), and copper(I) iodide (0.91 g, 4.78 mmol) were added to a solution of compound **1a** (2.60 g, 9.49 mmol) in *N*,*N*-dimethylformamide (50 mL). The reaction system was heated to 100 °C and stirred for 16 h. After the reaction was completed, the reaction mixture was cooled to room temperature, diluted with water (100 mL), and extracted with ethyl acetate (100 mL × 3). The organic layers were combined, washed with a saturated aqueous NaCl solution, and dried over anhydrous sodium sulfate. After filtration, the mother liquor was concentrated under

reduced pressure. The resulting crude product was purified by flash silica gel column chromatography (petroleum ether:ethyl acetate = 2:1) to give compound **1b** (2.10 g, yield: 81.9%).

MS m/z (ESI): 271.0 $[M+H]^+$.

Step c:

2-Cyclobutoxybenzenesulfonyl chloride

*N*-Chlorosuccinimide (3.10 g, 23.22 mmol) was added to compound **1b** (2.10 g, 7.77 mmol) in a mixed solution (glacial acetic acid and water in a ratio of 10:1, 33 mL) at 0 °C. The reaction system was stirred at 25 °C for 12 h. Subsequently, the reaction mixture was cooled to 0 °C with ice, stirred for 1 h, then filtered under reduced pressure, and dried under vacuum to give compound **1c** (1.36 g, yield: 71.0%).

$^1$HNMR (300 MHz, $CDCl_3$): $\delta$ 7.59-7.49 (m, 1H), 7.43-7.34 (m, 2H), 7.03-6.91 (m, 1H), 4.85-4.73 (m, 1H), 2.53-2.35 (m, 2H), 2.36-2.16 (m, 2H), 1.95-1.80 (m, 1H), 1.77-1.57 (m, 1H).

Step d:

*N*-(6-((1*H*-Pyrazol-1-yl)methyl)-4-methoxybenzo[*d*]isoxazol-3-yl)-2-cyclobutoxybenzenesulfonamide

Compound **1c** (870 mg, 3.53 mmol) and **intermediate 1** (250 mg, 1.02 mmol) were dissolved in pyridine (2 mL). The reaction system was heated to 100 °C and stirred for 16 h. After the reaction was completed, the reaction mixture was cooled to room temperature and concentrated under reduced pressure. The resulting crude product was purified by high-performance liquid chromatography (Welchrom C18, 20 $\times$ 150 mm, 5 $\mu$m; mobile phase A: 5 mM aqueous ammonium bicarbonate solution, mobile phase B: acetonitrile; gradient ratio: phase B 5%-95%) to give compound **1** (61 mg, yield: 13.1%).

$^1$HNMR (400 MHz, DMSO-$d_6$): $\delta$9.82 (s, 1H), 7.87-7.84 (m, 2H), 7.58-7.50 (t, 1H), 7.49 (d, 1H), 7.10-7.06 (t, 1H), 6.97-6.95 (d, 1H), 6.83 (s, 1H), 6.75 (s, 1H), 6.30-6.29 (t, 1H), 5.44 (s, 2H), 4.73-4.69 (m, 1H), 3.81 (s, 3H), 2.26-2.22 (m, 2H), 1.82-1.77 (m, 2H), 1.57-1.49 (m, 2H).

MS m/z (ESI): 455.0 $[M+H]^+$.

**Example 8**

Synthesis of Compound 2

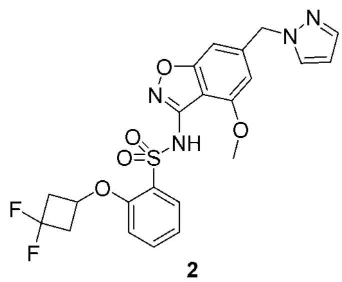

**2**

*N*-(6-((1*H*-Pyrazol-1-yl)methyl)-4-methoxybenzo[*d*]isoxazol-3-yl)-2-(3,3-difluorocyclobutoxy)benzenesulfonamide

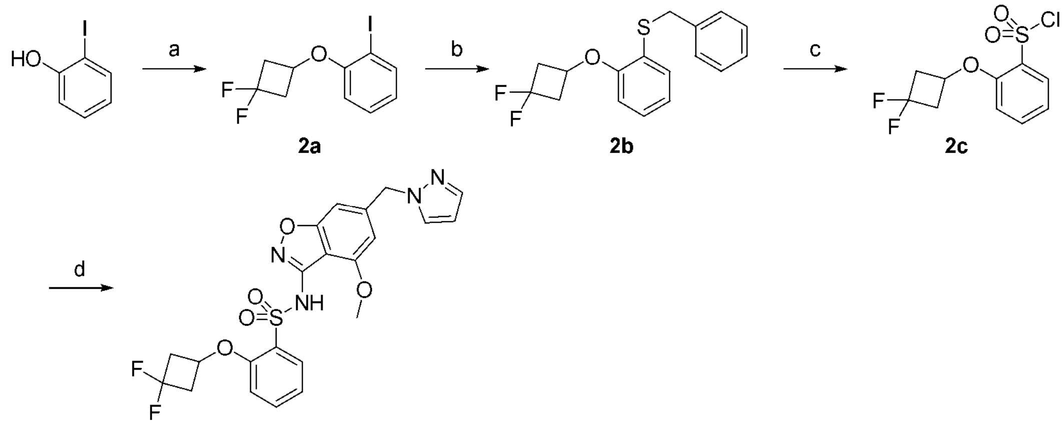


2a 2b 2c 2

Step a:

1-(3,3-Difluorocyclobutoxy)-2-iodobenzene

Under a nitrogen atmosphere, triphenylphosphine (10.93 g, 41.67 mmol) and diisopropyl azodicarboxylate (5.62 g, 27.79 mmol) were added to a solution of 2-iodophenol (4.89 g, 22.23 mmol) and 3,3-difluorocyclobutanol (2.00 g, 18.50 mmol) in toluene (150 mL). The reaction system was heated to 110 °C and stirred for 16 h. After the reaction was completed, the reaction mixture was cooled to room temperature, diluted with water (150 mL), and extracted with ethyl acetate (150 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 2:1) to give compound **2a** (2.70 g, yield: 47.1%).

$^1$H NMR (300 MHz, $CDCl_3$): $\delta$7.81-7.78 (m, 1H), 7.37-7.33 (m, 1H), 6.92-6.90 (m, 1H), 6.81-6.77 (m, 1H), 4.85-4.81 (m, 1H), 3.32-3.19 (m, 2H), 2.76-2.64 (m, 2H).

Step b:

Benzyl(2-(3,3-difluorocyclobutoxy)phenyl)sulfane

Under a nitrogen atmosphere, benzyl mercaptan (1.63 g, 13.12 mmol), cesium carbonate (5.70 g, 17.49 mmol), 1,4-diazabicyclo[2.2.2]octane (0.10 g, 0.87 mmol), and copper(I) iodide (0.84 g, 4.37 mmol) were added to a solution of compound **2a** (2.70 g, 8.71 mmol) in *N*,*N*-dimethylformamide (50 mL). The reaction system was heated to 100 °C and stirred for 16 h. After the reaction was completed, the reaction mixture was cooled to room temperature, diluted with water (100 mL), and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 2:1) to give compound **2b** (2.00 g, yield: 75.0%).

$^1$H NMR (300 MHz, $CDCl_3$): $\delta$7.40-7.28 (m, 6H), 7.27-7.16 (m, 1H), 6.99-6.88 (m, 2H), 4.84-4.79 (m, 1H), 4.19 (s, 2H), 3.29-3.19 (m, 2H), 2.76-2.66 (m, 2H).

Step c:

2-(3,3-Difluorocyclobutoxy)benzenesulfonyl chloride

*N*-Chlorosuccinimide (2.62 g, 19.62 mmol) was added to compound **2b** (2.00 g, 6.53 mmol) in a mixed solution (glacial acetic acid and water in a ratio of 10:1, 33 mL) at 0 °C. The reaction system was stirred at 25 °C for 12 h. Subsequently, the reaction mixture was cooled to 0 °C with ice, stirred for 1 h, then filtered under reduced pressure, and dried under vacuum to give compound **2c** (1.20 g, yield: 65.0%).

$^1$HNMR (300 MHz, $CDCl_3$): $\delta$8.08-8.06 (d, 1H), 7.77-7.72 (m, 1H), 7.26-7.21 (m, 1H), 7.00-6.98 (d, 1H), 4.94 (m, 1H), 3.33-3.20 (m, 2H), 3.10-2.94 (m, 2H).

Step d:

*N*-(6-((1*H*-Pyrazol-1-yl)methyl)-4-methoxybenzo[*d*]isoxazol-3-yl)-2-(3,3-difluorocyclobutoxy)benzenesulfonamide

Compound **2c** (1.20 g, 4.24 mmol) and **intermediate 1** (200 mg, 0.82 mmol) were dissolved in pyridine (2 mL). The reaction system was heated to 100 °C and stirred for 16 h. After the reaction was completed, the reaction mixture was cooled to room temperature and concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **2** (20 mg, yield: 5.0%).

$^1$HNMR (400 MHz, DMSO-$d_6$): $\delta$10.35 (s, 1H), 7.99-7.77 (m, 2H), 7.69-7.41 (m, 2H), 7.24-6.98 (m, 2H), 6.78 (s, 1H), 6.70 (s, 1H), 6.37-6.21 (m, 1H), 5.43 (s, 2H), 4.90-4.74 (m, 1H), 3.78 (s, 3H), 3.17-2.92 (m, 2H), 2.74-2.56 (m, 2H).

MS m/z (ESI): 491.0 [M+H]$^+$.

**Example 9**

Synthesis of Compound 3

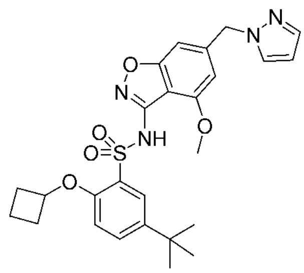

**3**

*N*-(6-((1*H*-Pyrazol-1-yl)methyl)-4-methoxybenzo[*d*]isoxazol-3-yl)-5-(*tert*-butyl)-2-cyclobutoxybenzenesulfonamide

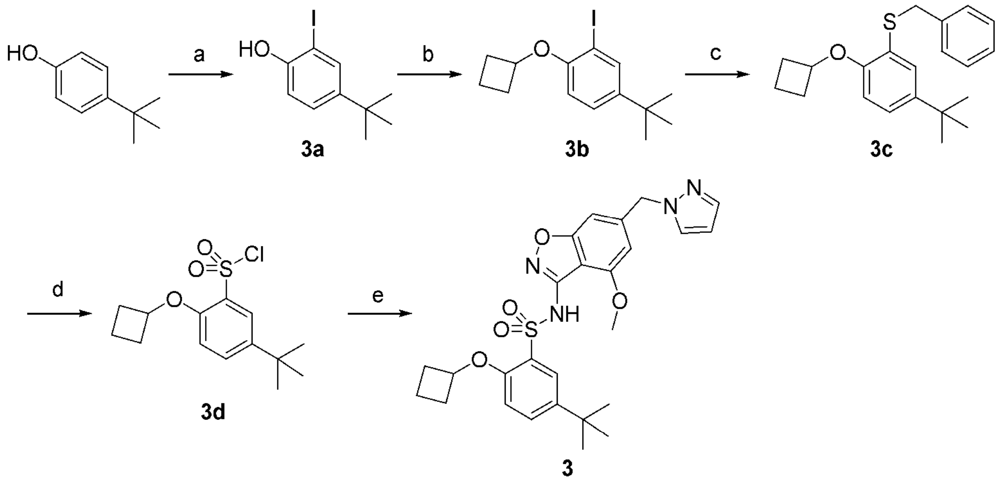


Step a:

4-(*tert*-Butyl)-2-iodophenol

*p*-Toluenesulfonic acid monohydrate (6.33 g, 33.28 mmol) was added to a solution of 4-(*tert*-butyl)phenol (5.00 g, 33.28 mmol) in acetonitrile (75 mL) at 20 °C. After the mixture was reacted for 30 min, *N*-iodosuccinimide (7.49 g, 33.29 mmol) was added, and the reaction system was stirred for 16 h. After the reaction was completed, the reaction mixture was quenched with an aqueous sodium sulfite solution (100 mL) and acidified with a hydrochloric acid solution (1.0 M), and then the phases were separated. The organic phase was collected, and the aqueous phase was extracted with ethyl acetate

(100 mL × 2). The organic layers were combined and dried over anhydrous sodium sulfate. After filtration, the mother liquor was concentrated under reduced pressure. The resulting crude product was purified by flash silica gel column chromatography (petroleum ether:ethyl acetate = 20:1) to give compound **3a** (8.20 g, yield: 89.2%).

$^1$H NMR (300 MHz, $CDCl_3$): $\delta$ 7.62 (d, 1H), 7.27-7.24 (m, 1H), 6.91 (d, 1H), 5.17 (s, 1H), 1.29 (s, 9H).

Step b:

4-(*tert*-Butyl)-1-cyclobutoxy-2-iodobenzene

Bromocyclobutane (2.92 g, 21.63 mmol) was added to a solution of compound **3a** (2.00 g, 7.24 mmol) and potassium carbonate (2.99 g, 21.63 mmol) in *N*,*N*-dimethylformamide (35 mL) at 20 °C. The reaction system was heated to 70 °C and stirred for 16 h. After the reaction was completed, the reaction mixture was cooled to room temperature, diluted with water (50 mL), and extracted with ethyl acetate (30 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 20:1) to give compound **3b** (2.20 g, yield: 92.0%).

$^1$H NMR (300 MHz, $CDCl_3$): $\delta$7.75 (d, 1H), 7.74-7.22 (m, 1H), 6.59 (d, 1H), 4.67-4.60 (m, 1H), 2.48-2.41 (m, 2H), 2.28-2.19 (m, 2H), 1.91-1.82 (m, 1H), 1.73-1.61 (m, 1H), 1.27 (s, 9H).

Step c:

Benzyl(5-(*tert*-butyl)-2-cyclobutoxyphenyl)sulfane

Under a nitrogen atmosphere, benzyl mercaptan (5.42 g, 43.64 mmol), cesium carbonate (14.21 g, 43.61 mmol), 1,4-diazabicyclo[2.2.2]octane (0.12 g, 1.06 mmol), and copper(I) iodide (0.21 g, 1.10 mmol) were added to a solution of compound **3b** (7.20 g, 21.80 mmol) in *N*,*N*-dimethylformamide (35 mL). The reaction system was heated to 100 °C and stirred for 16 h. After the reaction was completed, the reaction mixture was cooled to room temperature, diluted with water (200 mL), and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 10:1) to give compound **3c** (1.90 g, yield: 26.7%).

$^1$H NMR (400 MHz, $CDCl_3$): $\delta$7.40-7.24 (m, 6H), 7.20-7.19 (m, 1H), 6.69 (d, 1H), 4.70-4.62 (m, 1H), 4.10 (s, 2H), 2.50-2.42 (m, 2H), 2.28-2.18 (m, 2H), 1.90-1.83 (m, 1H), 1.72-1.64 (m, 1H), 1.20 (s, 9H).

Step d:

5-(*tert*-Butyl)-2-cyclobutoxybenzenesulfonyl chloride

*N*-Chlorosuccinimide (3.69 g, 27.63 mmol) was added to compound **3c** (1.50 g, 4.59 mmol) in a mixed solution (glacial acetic acid and water in a ratio of 10:1, 18 mL) at 0 °C. The reaction system was stirred at 10 °C for 1 h. After the reaction was completed, the mixture was quenched with water (20 mL) and filtered under reduced pressure. The filter cake was washed with water (3 mL × 5) and dried under vacuum to give compound **3d** (1.00 g, yield: 71.9%).

$^1$HNMR (300 MHz, $CDCl_3$): $\delta$7.80 (d, 1H), 7.32-7.29 (m, 1H), 6.65 (d, 1H), 4.74-4.65 (m, 1H), 2.55-2.45 (m, 2H), 2.36-2.22 (m, 2H), 1.97-1.87 (m, 1H), 1.78-1.61 (m, 1H), 1.32 (s, 9H).

Step e:

*N*-(6-((1*H*-Pyrazol-1-yl)methyl)-4-methoxybenzo[*d*]isoxazol-3-yl)-5-(*tert*-butyl)-2-cyclobutoxybenzenesulfonamide

Compound **3d** (743 mg, 2.45 mmol) was added to a solution of **intermediate 1** (100 mg, 0.41 mmol) in pyridine (4 mL) at 10 °C. The reaction system was heated to 100 °C and stirred for 16 h. After the reaction was completed, the reaction mixture was cooled to room temperature and concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **3** (64 mg, yield: 37.9%).

$^1$HNMR (400 MHz, $CDCl_3$): $\delta$8.10 (d, 1H), 8.00 (s, 1H), 7.56 (d, 1H), 7.49-7.46 (m, 1H), 7.42(d, 1H), 6.77 (s, 1H), 6.69 (d, 1H), 6.44 (s, 1H), 6.32 (t, 1H), 5.38 (s, 2H), 4.72-4.69 (m, 1H), 3.94 (s, 3H), 2.42-2.38 (m, 2H), 2.15-2.10 (m, 2H), 1.80-1.63 (m, 2H), 1.31 (s, 9H).

MS m/z (ESI): 511.1 $[M+H]^+$.

**Example 10**

Synthesis of Compound 4

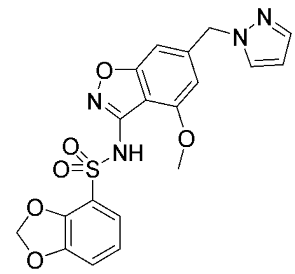

**4**

*N*-(6-((1*H*-Pyrazol-1-yl)methyl)-4-methoxybenzo[*d*]isoxazol-3-yl)benzo[*d*][1,3]dioxole-4-sulfonamide

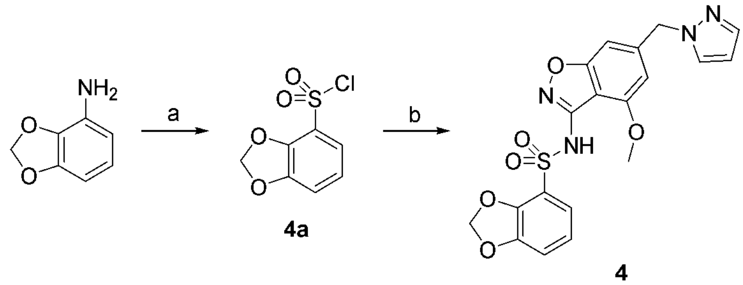


**4a**

**4**

Step a:

Benzo[*d*][1,3]dioxole-4-sulfonyl chloride

Thionyl chloride (5.3 mL, 73.06 mmol) was added dropwise to water (30 mL) at 25 °C, and the mixture was stirred for 6 h for later use. 4-Amino-1,3-benzodioxole (1.00 g, 7.29 mmol) was added dropwise to concentrated hydrochloric acid (7 mL) at 0 °C to give a hydrochloride, and then a solution of sodium nitrite (650 mg, 9.42 mmol) in water (2 mL) was added dropwise to the hydrochloride system. Subsequently, copper(I) chloride (70 mg, 0.71 mmol) was added to an aqueous solution containing thionyl chloride at a temperature below 5 °C, and the prepared hydrochloride solution was slowly added. The mixture was stirred at 40 °C for 16 h and then filtered under reduced pressure. The filter cake was purified by flash silica gel column chromatography (petroleum ether:ethyl acetate = 10:1) to give compound **4a** (680 mg, yield: 42.3%).

Step b:

*N*-(6-((1*H*-Pyrazol-1-yl)methyl)-4-methoxybenzo[*d*]isoxazol-3-yl)benzo[*d*][1,3]dioxole-4-sulfonamide

Compound **4a** (72 mg, 0.33 mmol) was added to a solution of **intermediate 1** (80 mg, 0.33 mmol) in pyridine (2 mL). The reaction system was heated to 120 °C and stirred for 5 h. Compound **4a** (72 mg, 0.33 mmol) was then added, and the mixture was stirred for 2 h. Subsequently, the last portion of compound **4a** (72 mg, 0.33 mmol) was added, and stirring was continued for 2 h. After the reaction was completed, the reaction mixture was cooled to room temperature and concentrated under reduced pressure. The resulting mixture was purified by flash silica gel column chromatography (petroleum ether:ethyl acetate = 10:1) to give a crude compound. The resulting crude product was purified by high-performance liquid chromatography (Welchrom C18, 20 × 150 mm, 5 μm; mobile phase A: 5 mM aqueous ammonium

bicarbonate solution, mobile phase B: acetonitrile; gradient ratio: phase B 5%-95%) to give compound **4** (21 mg, yield: 15.0%).

$^1$HNMR (400 MHz, DMSO-$d_6$): $\delta$ 7.85 (d, 1H), 7.49 (d, 1H), 7.30-7.19 (m, 1H), 7.19-6.94 (m, 2H), 6.91-6.82 (m, 1H), 6.71 (s, 1H), 6.62 (s, 1H), 6.29 (t, 1H), 6.02 (s, 2H), 5.40 (s, 2H), 3.80 (s, 3H).

MS m/z (ESI): 428.9 $[M+H]^+$.

**Example 11**

Synthesis of Compound 5

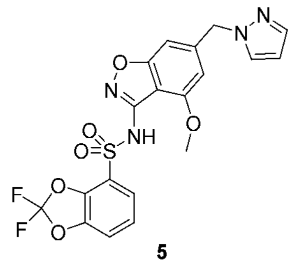

**5**

*N*-(6-((1*H*-Pyrazol-1-yl)methyl)-4-methoxybenzo[*d*]isoxazol-3-yl)-2,2-difluorobenzo[*d*][1,3]dioxole-4-sulfonamide

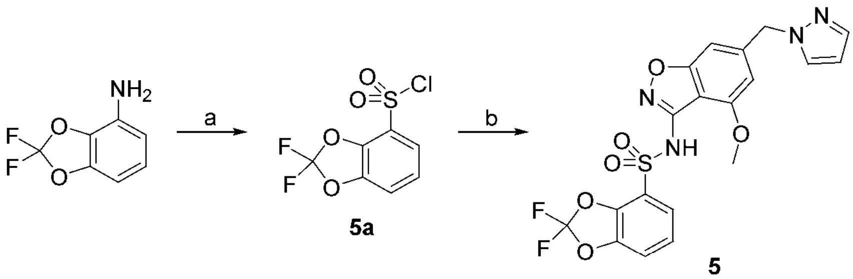


Step a:

2,2-Difluorobenzo[*d*][1,3]dioxole-4-sulfonyl chloride

Thionyl chloride (5.96 mL, 82.16 mmol) was added dropwise to water (30 mL) at 25 °C, and the mixture was stirred for 6 h for later use. 4-Amino-2,2-difluoro-1,3-benzodioxole (1.00 g, 5.78 mmol) was added dropwise to concentrated hydrochloric acid (7 mL) at 0 °C to give a hydrochloride, and then a solution of sodium nitrite (523 mg, 7.58 mmol) in water (2 mL) was added dropwise to the hydrochloride system. Subsequently, copper(I) chloride (10 mg, 0.10 mmol) was added to an aqueous solution containing thionyl chloride at a temperature below 5 °C, and the prepared hydrochloride solution was slowly added. The mixture was stirred at 0 °C for 2 h and then extracted with dichloromethane (50 mL × 2). The organic phases were combined and concentrated under reduced pressure to give compound **5a** (1.00 g, yield: 67.5%).

Step b:

*N*-(6-((1*H*-Pyrazol-1-yl)methyl)-4-methoxybenzo[*d*]isoxazol-3-yl)-2,2-difluorobenzo[*d*][1,3]dioxole-4-sulfonamide

A solution of compound **5a** (157 mg, 0.61 mmol) in pyridine (0.5 mL) was added to a solution of **intermediate 1** (100 mg, 0.41 mmol) in pyridine (2 mL). The reaction system was heated to 80 °C and stirred for 2 h. Compound **5a** (157 mg, 0.61 mmol) was then added, and the mixture was stirred for 2 h. After the reaction was completed, the reaction mixture was cooled to room temperature and concentrated under reduced pressure. The resulting mixture was purified by preparative thin-layer chromatography (dichloromethane:methanol = 20:1) to give a crude compound. The resulting crude product was purified by high-performance liquid chromatography (Welchrom C18, 20 × 150 mm, 5 μm; mobile phase A: 5 mM

aqueous ammonium bicarbonate solution, mobile phase B: acetonitrile; gradient ratio: phase B 5%-95%) to give compound **5** (30 mg, yield: 15.8%).

$^1$HNMR (400 MHz, DMSO-$d_6$): $\delta$7.84 (d, 1H), 7.57-7.44 (m, 2H), 7.30-7.18 (m, 1H), 7.15-7.05 (m, 1H), 7.05-6.94 (m, 1H), 6.67 (s, 1H), 6.59 (s, 1H), 6.29 (t, 1H), 5.39 (s, 2H), 3.80 (s, 3H).

MS m/z (ESI): 464.9 [M+H]$^+$.

**Example 12**

Synthesis of Compound 6

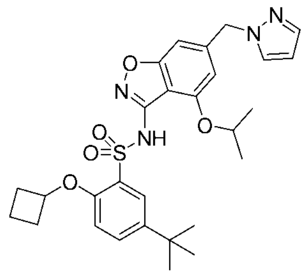

**6**

*N*-(6-((1*H*-Pyrazol-1-yl)methyl)-4-isopropoxybenzo[*d*]isoxazol-3-yl)-5-(*tert*-butyl)-2-cyclobutoxybenzenesulfonamide

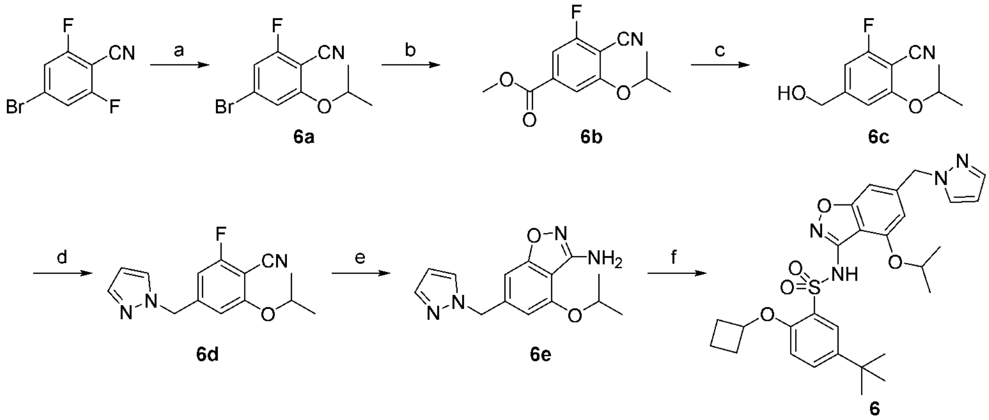


Step a:

4-Bromo-2-fluoro-6-isopropoxybenzonitrile

Under a nitrogen atmosphere, 4-bromo-2,6-difluorobenzonitrile (2.40 g, 11.01 mmol) was added to a solution of sodium metal (280 mg, 11.67 mmol) in isopropanol (30 mL) at 0 °C, and the reaction mixture was stirred at 20 °C for 16 h. Then, the reaction mixture was concentrated under reduced pressure. The resulting crude product was purified by flash silica gel column chromatography (petroleum ether:ethyl acetate = 100:1) to give compound **6a** (1.70 g, yield: 59.8%).

$^1$H NMR (400 MHz, $CDCl_3$): $\delta$6.95 (d, 1H), 6.9 (s, 1H), 4.66-4.63 (m, 1H), 1.41 (d, 6H).

Step b:

Methyl 4-cyano-3-fluoro-5-isopropoxybenzoate

Triethylamine (4.6 mL, 34.09 mmol) and [1,1'-bis(diphenylphosphino)ferrocene]palladium(II) dichloride (0.24 g, 0.33 mmol) were added to a solution of compound **6a** (1.70 g, 6.59 mmol) in methanol (20 mL). The reaction system was

stirred at 80 °C for 16 h under a carbon monoxide atmosphere (1 atm). After the reaction was completed, the reaction mixture was cooled to room temperature and concentrated under reduced pressure. The resulting crude product was purified by flash silica gel column chromatography (petroleum ether:ethyl acetate = 100:1) to give compound **6b** (1.20 g, yield: 76.8%).

$^{1}$HNMR (400 MHz, $CDCl_3$): $\delta$7.40 (s, 1H), 7.37 (d, 1H), 4.66-4.63 (m, 1H), 3.96 (s, 3H), 1.44 (d, 6H).

Step c:

2-Fluoro-4-(hydroxymethyl)-6-isopropoxybenzonitrile

Lithium borohydride (0.40 g, 18.37 mmol) was added in portions to a solution of compound **6b** (1.10 g, 4.64 mmol) in tetrahydrofuran (10 mL) at 10 °C. The reaction system was heated to 70 °C and then stirred for 2 h. After the reaction was completed, the reaction mixture was cooled to room temperature, then quenched with diluted hydrochloric acid (1.0 M), and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 20:1) to give compound 6c (907 mg, yield: 93.5%).

$^{1}$HNMR (300 MHz, $CDCl_3$): $\delta$7.31 (s, 1H), 6.82 (s, 1H), 6.77 (d, 1H), 4.77 (s, 2H), 4.73-4.69 (m, 1H), 1.45 (d, 6H).

Step d:

4-((1*H*-Pyrazol-1-yl)methyl)-2-fluoro-6-isopropoxybenzonitrile

Cesium carbonate (1.09 g, 3.35 mmol) was added to a solution of compound **6c** (586 mg, 2.80 mmol) and 1-(methylsulfonyl)-1*H*-pyrazole (491 mg, 3.36 mmol) in acetonitrile (10 mL) at 20 °C. The reaction system was heated to 70 °C and stirred for 1 h. After the reaction was completed, the reaction mixture was cooled to room temperature, then diluted with water, and extracted with ethyl acetate (25 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 5:1 to 2:1) to give compound **6d** (700 mg, yield: 96.4%).

$^{1}$HNMR (400 MHz, $CDCl_3$): $\delta$ 7.60 (d, 1H), 7.45 (d, 1H), 6.48 (d, 1H), 6.45 (s, 1H), 6.36 (t, 1H), 5.32 (s, 2H), 4.61-4.49 (m, 1H), 1.35 (d, 6H).

Step e:

6-((1*H*-Pyrazol-1-yl)methyl)-4-isopropoxybenzo[*d*]isoxazol-3-amine

Tetramethylguanidine (1.87 g, 16.20 mmol) was added dropwise to compound **6d** (700 mg, 2.70 mmol) and acetohydroxamic acid (608 mg, 8.10 mmol) in a mixed solution (*N*,*N*-dimethylformamide and water in a ratio of 9:1, 8 mL). The reaction system was heated to 60 °C and stirred for 7 h. After the reaction was completed, the reaction mixture was cooled to room temperature, then diluted with water, and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 1:1) to give compound **6e** (500 mg, yield: 68.0%).

$^{1}$HNMR (400 MHz, DMSO-$d_6$): $\delta$7.86 (d, 1H), 7.50 (d, 1H), 6.67 (s, 1H), 6.60 (s, 1H), 6.29 (t, 1H), 5.80 (s, 2H), 5.40 (s, 2H), 4.72-4.66 (m, 1H), 1.34 (d, 6H).

Step f:

*N*-(6-((1*H*-Pyrazol-1-yl)methyl)-4-isopropoxybenzo[*d*]isoxazol-3-yl)-5-(*tert*-butyl)-2-cyclobutoxybenzenesulfonamide

Compound **3d** (336 mg, 1.11 mmol) was added to a solution of compound **6e** (100 mg, 0.37 mmol) in pyridine (4 mL) at 10 °C. The reaction system was heated to 100 °C and stirred for 16 h. After the reaction was completed, the reaction mixture was cooled to room temperature and concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **6** (20 mg, yield: 10.1%).

$^{1}$HNMR (400 MHz, DMSO-$d_6$): $\delta$9.62 (s, 1H), 7.86 (d, 1H), 7.83 (d, 1H), 7.57 (d, 1H), 7.48 (d, 1H),

6.88 (s, 1H), 6.83 (d, 1H), 6.68 (s, 1H), 6.29 (t, 1H), 5.43 (s, 2H), 4.64-4.53 (m, 1H), 4.49-4.46 (m, 1H), 2.08-2.03 (m, 2H), 1.53-1.31 (m, 4H), 1.27 (s, 9H), 1.09 (d, 6H).
MS m/z (ESI): 539.2 [M+H]$^+$.

**Example 13**

Synthesis of Compound 7

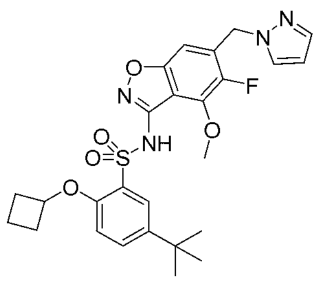

**7**

*N*-(6-((1*H*-Pyrazol-1-yl)methyl)-5-fluoro-4-methoxybenzo[*d*]isoxazol-3-yl)-5-(*tert*-butyl)-2-cyclobutoxybenzenesulfonamide

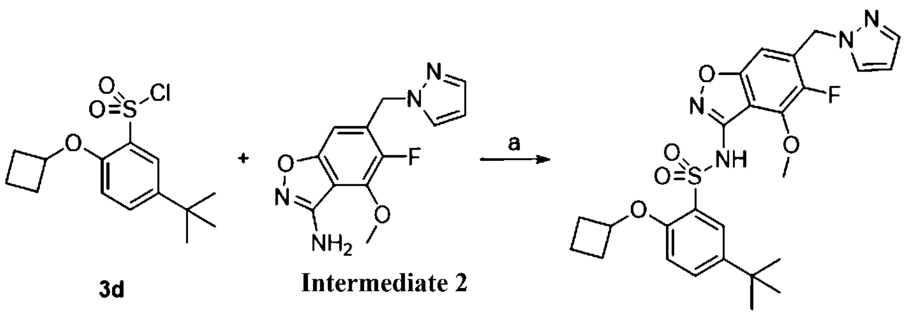


Step a:

*N*-(6-((1*H*-Pyrazol-1-yl)methyl)-5-fluoro-4-methoxybenzo[*d*]isoxazol-3-yl)-5-(*tert*-butyl)-2-cyclobutoxybenzenesulfonamide

Sodium hydride (60%, 37 mg, 0.93 mmol) was added to a solution of **intermediate 2** (80 mg, 0.31 mmol) in *N*,*N*-dimethylformamide (2 mL) at 0 °C. The reaction system was stirred at 0 °C for 10 min. Then, compound **3d** (139 mg, 0.46 mmol) was added to the system, and stirring was continued for 20 min. After the reaction was completed, the reaction mixture was warmed to room temperature, quenched with a saturated aqueous ammonium chloride solution (10 mL), and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 9:1) to give compound **7** (22 mg, yield: 13.6%).
$^1$HNMR (400 MHz, DMSO-$d_6$): $\delta$10.36 (s, 1H), 7.96-7.75(m, 2H), 7.68-7.55 (m, 1H), 7.49 (d, 1H), 6.95 (d, 1H), 6.90 (d, 1H), 6.30 (t, 1H), 5.50 (s, 2H), 4.66-4.62 (m, 1H), 3.94 (d, 3H), 2.20-2.16 (m, 2H), 1.74-1.57 (m, 2H), 1.57-1.48 (m, 2H), 1.26 (s, 9H).
MS m/z (ESI): 529.0 [M+H]$^+$.

**Example 14**

Synthesis of Compound 8

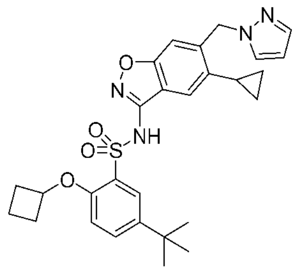

**8**

*N*-(6-((1*H*-Pyrazol-1-yl)methyl)-5-cyclopropylbenzo[*d*]isoxazol-3-yl)-5-(*tert*-butyl)-2-cyclobutoxybenzenesulfonamide

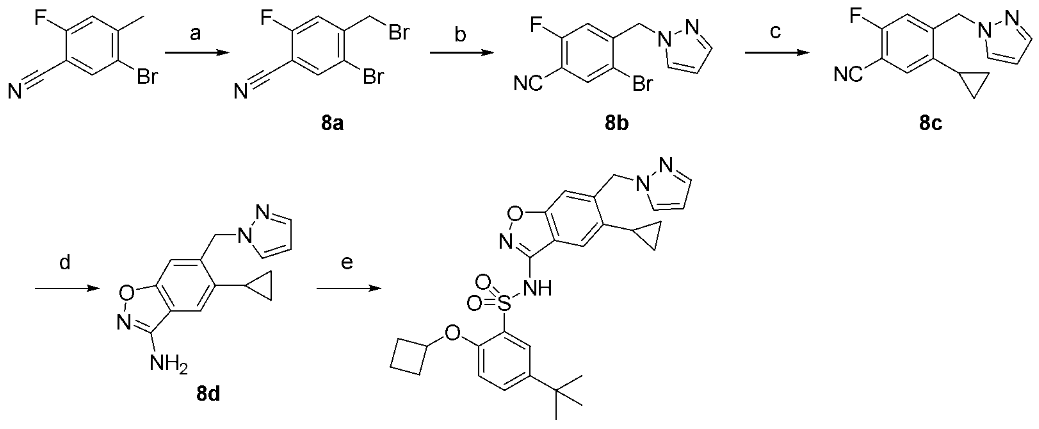


Step a:

5-Bromo-4-(bromomethyl)-2-fluorobenzonitrile

1,3-Dibromo-5,5-dimethylimidazolidine-2,4-dione (0.67 g, 2.34 mmol) and azobisisobutyronitrile (0.10 g, 0.61 mmol) were added to a solution of 5-bromo-2-fluoro-4-methylbenzonitrile (1.00 g, 4.67 mmol) in acetonitrile (10 mL). The reaction system was heated to 80 °C and stirred for 16 h. After the reaction was completed, the reaction mixture was cooled to room temperature and concentrated under reduced pressure. The resulting crude product was purified by flash silica gel column chromatography (petroleum ether:ethyl acetate = 9:1) to give compound **8a** (0.85 g, yield: 62.1%).
$^1$H NMR (400 MHz, $CDCl_3$): $\delta$7.83 (d, 1H), 7.37 (d, 1H), 4.53 (s, 2H).

Step b:

4-((1*H*-Pyrazol-1-yl)methyl)-5-bromo-2-fluorobenzonitrile

Pyrazole (0.25 g, 3.67 mmol) and cesium carbonate (1.33 g, 4.08 mmol) were added to a solution of compound **8a** (1.00 g, 3.41 mmol) in acetonitrile (16 mL). The reaction system was stirred at 25 °C for 5 h. After the reaction was completed, the mixture was diluted with water and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 5:1) to give compound **8b** (0.73 g, yield: 76.3%).
$^1$H NMR (300 MHz, $CDCl_3$): $\delta$7.82 (d, 1H), 7.64 (d, 1H), 7.52 (d, 1H), 6.50 (d, 1H), 6.39 (t, 1H), 5.42 (s, 2H).
MS m/z (ESI): 281.0 [M+H]$^+$.

Step c:

4-((1*H*-Pyrazol-1-yl)methyl)-5-cyclopropyl-2-fluorobenzonitrile

Under a nitrogen atmosphere, cesium carbonate (1.58 g, 4.85 mmol), cyclopropylboronic acid (0.42 g, 4.89 mmol), tris(dibenzylideneacetone)dipalladium (0.16 g, 0.17 mmol), and 2-(di-tert-butylphosphino)biphenyl (0.14 g, 0.47 mmol) were added to a solution of compound **8b** (0.68 g, 2.43 mmol) in 1,4-dioxane (15 mL). The reaction system was heated to 90 °C and stirred for 16 h. After the reaction was completed, the mixture was diluted with water and extracted with ethyl acetate (80 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 5:1) to give compound **8c** (0.54 g, yield: 92.2%).

$^1$HNMR (300 MHz, $CDCl_3$): $\delta$7.62 (d, 1H), 7.46 (d, 1H), 7.33 (d, 1H), 6.49 (d, 1H), 6.38 (t, 1H), 5.56 (s, 2H), 1.87-1.80 (m, 1H), 1.07-1.03 (m, 2H), 0.71-0.67 (m, 2H).

MS m/z (ESI): 240.1 $[M-H]^+$.

Step d:

6-((1*H*-Pyrazol-1-yl)methyl)-5-cyclopropylbenzo[*d*]isoxazol-3-amine

Acetohydroxamic acid (0.46 g, 6.13 mmol) and tetramethylguanidine (2.08 g, 18.06 mmol) were added to compound **8c** (0.49 g, 2.03 mmol) in a mixed solution (acetonitrile and water in a ratio of 9:1, 10 mL). The reaction system was heated to 60 °C and stirred for 16 h under a nitrogen atmosphere. After the reaction was completed, the reaction mixture was cooled to room temperature, then diluted with water, and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 1:1) to give compound **8d** (0.35 g, yield: 67.8%).

$^1$HNMR (400 MHz, DMSO-$d_6$): $\delta$7.84 (d, 1H), 7.58 (s, 1H), 7.53 (d, 1H), 6.71 (s, 1H), 6.36-6.28 (m, 3H), 5.64 (s, 2H), 2.10-1.99 (m, 1H), 1.02-0.90 (m, 2H), 0.69-0.56 (m, 2H).

MS m/z (ESI): 255.2 $[M+H]^+$.

Step e:

*N*-(6-((1*H*-Pyrazol-1-yl)methyl)-5-cyclopropylbenzo[*d*]isoxazol-3-yl)-5-(*tert*-butyl)-2-cyclobutoxybenzenesulfonamide

Compound **8d** (100 mg, 0.39 mmol) and compound **3d** (357 mg, 1.18 mmol) were dissolved in pyridine (6 mL). The reaction system was heated to 120 °C and stirred for 56 h under a nitrogen atmosphere. After the reaction was completed, the reaction mixture was cooled to room temperature and concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **8** (35 mg, yield: 17.1%).

$^1$HNMR (400 MHz, DMSO-$d_6$): $\delta$11.48 (s, 1H), 7.88-7.76 (m, 3H), 7.56 (d, 1H), 7.52 (d, 1H), 6.86 (d, 1H), 6.82 (s, 1H), 6.32 (t, 1H), 5.65 (s, 2H), 4.74-4.60 (m, 1H), 2.25-2.12 (m, 2H), 2.12-2.10 (m, 1H), 1.78-1.64 (m, 2H), 1.53-1.37 (m, 2H), 1.25 (s, 9H), 1.02-0.93(m, 2H), 0.64-0.54 (m, 2H).

MS m/z (ESI): 521.1 $[M+H]^+$.

**Example 15**

Synthesis of Compound 9

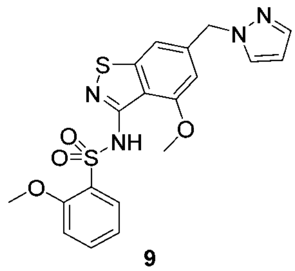

9

*N*-(6-((1*H*-Pyrazol-1-yl)methyl)-4-methoxybenzo[*d*]isothiazol-3-yl)-2-methoxybenzenesulfonamide

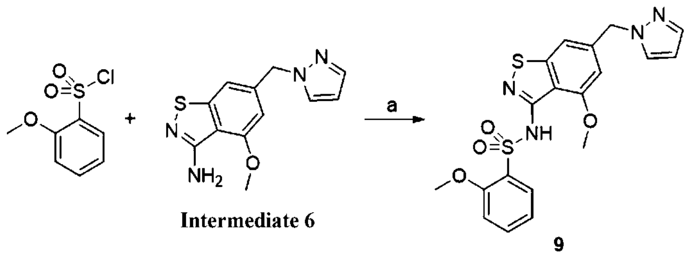


9

Step a:

*N*-(6-((1*H*-Pyrazol-1-yl)methyl)-4-methoxybenzo[*d*]isothiazol-3-yl)-2-methoxybenzenesulfonamide

Under a nitrogen atmosphere, 2-methoxybenzenesulfonyl chloride (64 mg, 0.31 mmol) was added to a solution of **intermediate 6** (80 mg, 0.31 mmol) in pyridine (5 mL), and the reaction system was heated to 70 °C and stirred for 16 h. After the reaction was completed, the reaction mixture was cooled to room temperature and concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **9** (23 mg, yield: 17.4%).

$^1$HNMR (400 MHz, DMSO-$d_6$): $\delta$9.41 (brs, 1H), 7.91-7.88 (m, 2H), 7.62-7.58 (m, 1H), 7.49 (d, 1H), 7.28 (s, 1H), 7.15-7.09 (m, 2H), 6.94 (s, 1H), 6.30 (t, 1H), 5.45 (s, 2H), 4.05 (s, 3H), 3.78 (s, 3H).

MS m/z (ESI): 430.9 [M+H]$^+$.

**Example 16**

Synthesis of Compound 10

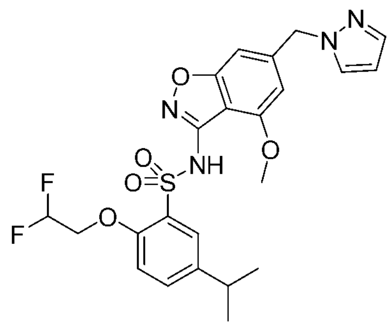

10

*N*-(6-((1*H*-Pyrazol-1-yl)methyl)-4-methoxybenzo[*d*]isoxazol-3-yl)-2-(2,2-difluoroethoxy)-5-isopropylbenzenesulfonamide

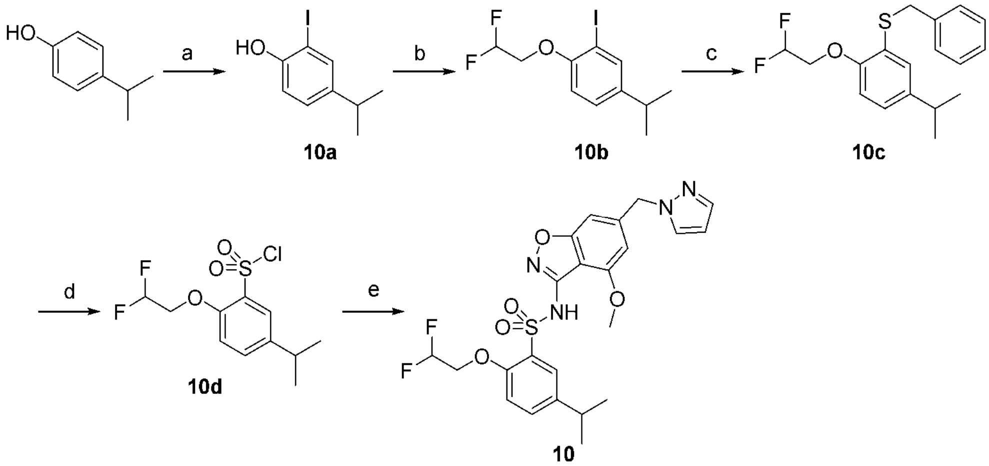


Step a:

2-Iodo-4-isopropylphenol

*p*-Toluenesulfonic acid (0.17 g, 0.99 mmol) and *N*-iodosuccinimide (2.25 g, 9.99 mmol) were added to a solution of 4-isopropylphenol (1.36 g, 9.99 mmol) in dichloromethane (40 mL) at 10 °C. The reaction system was stirred for 16 h. After the reaction was completed, the mixture was diluted with water and extracted with dichloromethane (150 mL × 3). The organic layers were combined and dried over anhydrous sodium sulfate. After filtration, the mother liquor was concentrated under reduced pressure. The resulting crude product was purified by flash silica gel column chromatography (petroleum ether:ethyl acetate = 30:1) to give compound **10a** (1.72 g, yield: 65.7%).

$^1$H NMR (400 MHz, $CDCl_3$): $\delta$7.49 (d, 1H), 7.11-7.08 (m, 1H), 6.91 (d, 1H), 5.13 (s, 1H), 2.83-2.80 (m, 1H), 1.21 (s, 3H), 1.20 (s, 3H).

Step b:

1-(2,2-Difluoroethoxy)-2-iodo-4-isopropylbenzene

Compound **10a** (1.60 g, 6.10 mmol) and potassium carbonate (1.69 g, 12.20 mmol) were dissolved in a solution of *N*,*N*-dimethylformamide (15 mL). The reaction system was stirred at 25 °C for 5 min, and then 1,1-difluoro-2-iodoethane (1.29 g, 6.72 mmol) was added. The reaction system was heated to 100 °C and stirred for 16 h. After the reaction was completed, the reaction mixture was cooled to room temperature, diluted with water (50 mL), and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 20:1) to give compound **10b** (1.80 g, yield: 90.4%).

$^1$H NMR (400 MHz, $CDCl_3$): $\delta$7.64 (d, 1H), 7.17-7.14 (m, 1H), 6.76 (d, 1H), 6.29-5.99 (m, 1H), 4.23-4.16 (m, 2H), 2.86-2.79 (m, 1H), 1.22 (s, 3H), 1.20 (s, 3H).

Step c:

Benzyl(2-(2,2-difluoroethoxy)-5-isopropylphenyl)sulfane

Under a nitrogen atmosphere, benzyl mercaptan (0.78 g, 6.20 mmol), potassium carbonate (1.33 g, 9.60 mmol), 1,4-diazabicyclo[2.2.2]octane (0.05 g, 0.48 mmol), and copper(I) iodide (0.05 g, 0.24 mmol) were added to a solution of compound **10b** (1.56 g, 4.78 mmol) in *N*,*N*-dimethylformamide (15 mL). The reaction system was heated to 100 °C and stirred for 16 h. After the reaction was completed, the reaction mixture was cooled to room temperature, diluted with water (50 mL), and extracted with dichloromethane (100 mL × 3). The organic layers were combined, washed with a saturated

aqueous NaCl solution, and dried over anhydrous sodium sulfate. After filtration, the mother liquor was concentrated under reduced pressure. The resulting crude product was purified by flash silica gel column chromatography (petroleum ether:dichloromethane = 30:1) to give compound **10c** (1.49 g, yield: 96.6%).

$^1$H NMR (400 MHz, $CDCl_3$) $\delta$ 7.32-7.27 (m, 3H), 7.26-7.19 (m, 2H), 7.10 (d, 1H), 7.04-7.01 (m, 1H), 6.77 (d, 1H), 6.26-5.97 (m, 1H), 4.23-4.15 (m, 2H), 4.08 (s, 2H), 2.82-2.75 (m, 1H), 1.16 (s, 3H), 1.15 (s, 3H).

MS m/z (ESI): 321.1 $[M-H]^+$.

Step d:

2-(2,2-Difluoroethoxy)-5-isopropylbenzenesulfonyl chloride

*N*-Chlorosuccinimide (3.69 g, 27.60 mmol) was added to compound **10c** (1.49 g, 4.62 mmol) in a mixed solution (glacial acetic acid and water in a ratio of 10:1, 22 mL) at 25 °C. The reaction system was stirred at 25 °C for 2 h. After the reaction was completed, water (100 mL) was added for dilution, and the mixture was extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 10:1) to give compound **10d** (1.12 g, yield: 81.1%).

$^1$HNMR (400 MHz, $CDCl_3$): $\delta$7.82 (d, 1H), 7.56-7.54 (m, 1H), 7.04 (d, 1H), 6.37-6.07 (m, 1H), 4.42-4.34 (m, 2H), 3.00-2.93 (m, 1H), 1.28 (s, 3H), 1.26 (s, 3H).

Step e:

*N*-(6-((1*H*-Pyrazol-1-yl)methyl)-4-methoxybenzo[*d*]isoxazol-3-yl)-2-(2,2-difluoroethoxy)-5-isopropylbenzenesulfonamide

**Intermediate 1** (50 mg, 0.20 mmol) and compound **10d** (306 mg, 1.02 mmol) were dissolved in a solution of pyridine (5 mL). The reaction system was heated to 120 °C and stirred for 24 h. After the reaction was completed, the reaction mixture was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **10** (57 mg, yield: 55.0%).

$^1$HNMR (400 MHz, DMSO-$d_6$): $\delta$9.93 (s, 1H), 7.87 (d, 1H), 7.69 (d, 1H), 7.52-7.48 (m, 2H), 7.22 (s, 1H), 6.82-6.73 (m, 2H), 6.52-6.23 (m, 2H), 5.43 (s, 2H), 4.41-4.35 (m, 2H), 3.80 (s, 3H), 2.95-2.91 (m, 1H), 1.18 (s, 3H), 1.16 (s, 3H).

MS m/z (ESI): 507.0 $[M+H]^+$.

**Example 17**

Synthesis of Compound 11

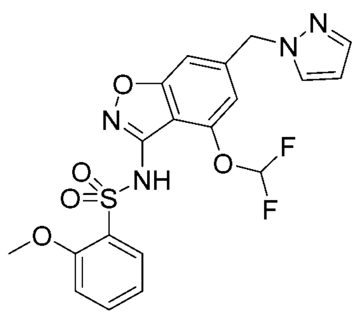

**11**

*N*-(6-((1*H*-Pyrazol-1-yl)methyl)-4-(difluoromethoxy)benzo[*d*]isoxazol-3-yl)-2-methoxybenzenesulfonamide

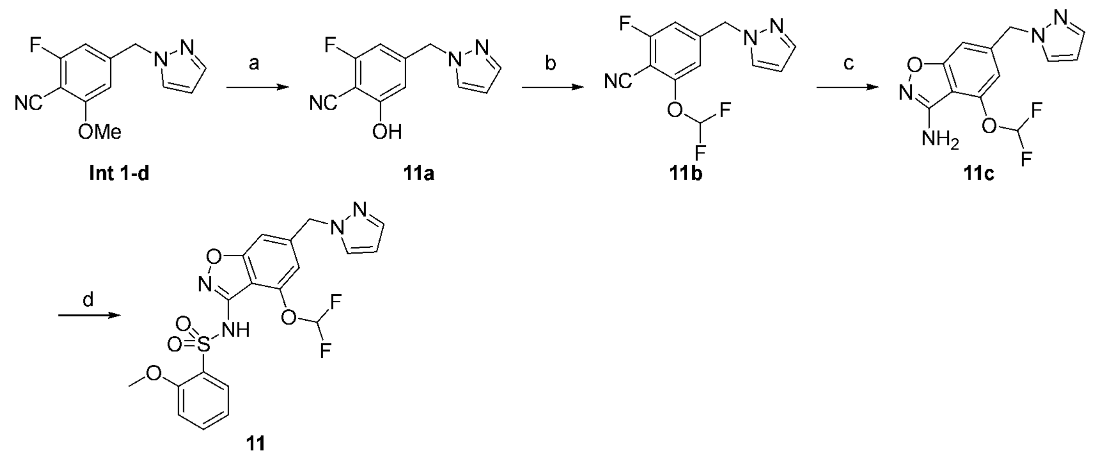


Int 1-d 11a 11b 11c

11

Step a:

4-((1*H*-Pyrazol-1-yl)methyl)-2-fluoro-6-hydroxybenzonitrile

Compound **Int 1-d** (1.10 g, 4.76 mmol) was dissolved in a solution of pyridine in hydrochloric acid (7 mL). The reaction system was heated to 180 °C and stirred for 2.5 h. After the reaction was completed, the reaction mixture was cooled to room temperature and poured into ice water, and the resulting mixture was extracted with ethyl acetate (20 mL × 3). The organic layers were combined, washed with a saturated aqueous NaCl solution, and dried over anhydrous sodium sulfate. After filtration, the mother liquor was concentrated under reduced pressure to give compound **11a** (0.78 g, yield: 75.5%).

MS m/z (ESI): 218.0 $[M+H]^+$.

Step b:

4-((1*H*-Pyrazol-1-yl)methyl)-2-(difluoromethoxy)-6-fluorobenzonitrile

Sodium chlorodifluoroacetate (821 mg, 5.31 mmol) and potassium carbonate (745 mg, 5.39 mmol) were added to compound **11a** (780 mg, 3.59 mmol) in a mixed solution (*N*,*N*-dimethylformamide and water in a ratio of 30:1, 20 mL). The reaction system was heated to 120 °C and stirred for 4 h under a nitrogen atmosphere. After the reaction was completed, the reaction mixture was cooled to room temperature, diluted with water, and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:tetrahydrofuran = 4:1) to give compound **11b** (650 mg, yield: 67.7%).

$^1H$ NMR (300 MHz, $CDCl_3$): $\delta$8.11 (d, 1H), 7.98 (d, 1H), 7.78-6.91 (m, 3H), 6.87 (t, 1H), 5.88 (s, 2H).

Step c:

6-((1*H*-Pyrazol-1-yl)methyl)-4-(difluoromethoxy)benzo[*d*]isoxazol-3-amine

Compound **11b** (0.65 g, 2.43 mmol), acetohydroxamic acid (0.55 g, 7.30 mmol), and tetramethylguanidine (1.68 g, 14.60 mmol) were dissolved in a mixed solution (acetonitrile and water in a ratio of 15:1, 5 mL). The reaction system was heated to 60 °C and stirred for 7 h under a nitrogen atmosphere. After the reaction was completed, the reaction mixture was cooled to room temperature and then concentrated under reduced pressure. The resulting crude product was purified by flash silica gel column chromatography (dichloromethane:methanol = 20:1) to give compound **11c** (0.42 g, yield: 61.6%).

MS m/z (ESI): 281.0 $[M+H]^+$.

Step d:

*N*-(6-((1*H*-Pyrazol-1-yl)methyl)-4-(difluoromethoxy)benzo[*d*]isoxazol-3-yl)-2-methoxybenzenesulfonamide

Under a nitrogen atmosphere, at 0 °C, sodium hydride (60%, 71 mg, 1.78 mmol) was added to a solution of **11c** (100 mg, 0.36 mmol) in N,N-dimethylformamide (4 mL), and the reaction system was stirred for 10 min. Then, 2-methoxybenzenesulfonyl chloride (111 mg, 0.54 mmol) was added to the system. The mixture was stirred at 0 °C for 1 h, then warmed to 25 °C, and stirred for another 1 h. After the reaction was completed, the mixture was quenched with water and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **11** (28 mg, yield: 17.4%).

$^1$HNMR (400 MHz, DMSO-$d_6$): $\delta$10.67 (s, 1H), 7.97-7.87 (m, 1H), 7.77 (d, 1H), 7.75-7.58 (m, 1H), 7.51 (s, 1H), 6.37-6.92 (m, 5H), 6.37-6.28 (m, 1H), 5.50 (s, 2H), 3.75 (s, 3H).

MS m/z (ESI): 450.9 [M+H]$^+$.

**Example 18**

Synthesis of Compound 12

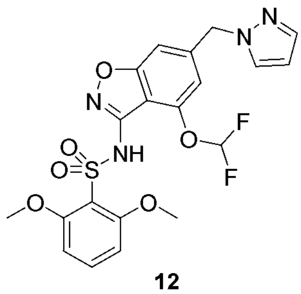

**12**

*N*-(6-((1*H*-Pyrazol-1-yl)methyl)-4-(difluoromethoxy)benzo[*d*]isoxazol-3-yl)-2,6-dimethoxybenzenesulfonamide

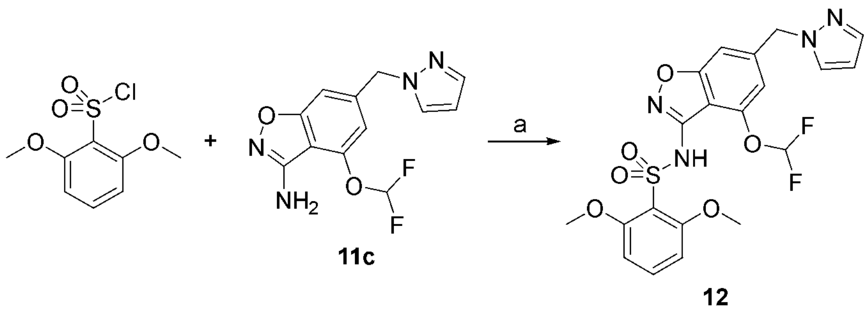


Step a:

*N*-(6-((1*H*-Pyrazol-1-yl)methyl)-4-(difluoromethoxy)benzo[*d*]isoxazol-3-yl)-2,6-dimethoxybenzenesulfonamide

Under a nitrogen atmosphere, at 0 °C, sodium hydride (60%, 71 mg, 1.78 mmol) was added to a solution of **11c** (100 mg, 0.36 mmol) in N,N-dimethylformamide (4 mL), and the reaction system was stirred for 10 min. Then, 2,6-dimethoxybenzenesulfonyl chloride (127 mg, 0.53 mmol) was added to the system. The mixture was stirred at 0 °C for 1 h, then warmed to 25 °C, and stirred for another 1 h. After the reaction was completed, the mixture was quenched with water and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150

mm, 5 μm; mobile phase A: 5 mM aqueous ammonium bicarbonate solution, mobile phase B: acetonitrile; gradient ratio: phase B 5%-95%) to give compound **12** (54 mg, yield: 31.5%).

$^1$HNMR (400 MHz, DMSO-$d_6$): $\delta$10.22 (s, 1H), 7.89 (d, 1H), 7.51-7.11 (m, 4H), 7.00 (s, 1H), 6.77 (d, 2H), 6.36-6.26 (m, 1H), 5.50 (s, 2H), 3.74 (s, 6H).

MS m/z (ESI): 481.0 [M+H]$^+$.

**Example 19**

Synthesis of Compound 13

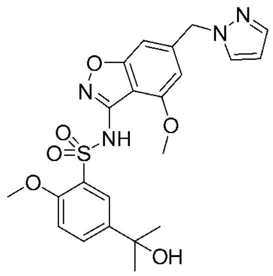

**13**

*N*-(6-((1*H*-Pyrazol-1-yl)methyl)-4-methoxybenzo[*d*]isoxazol-3-yl)-5-(2-hydroxypropan-2-yl)-2-methoxybenzenesulfonamide

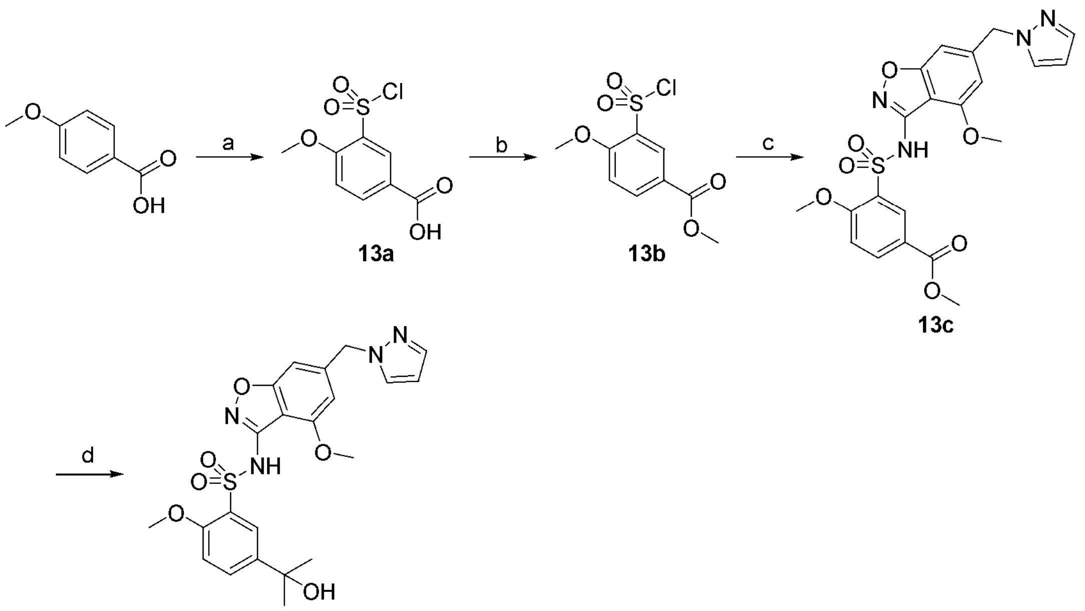


**13a** **13b** **13c**

**13**

Step a:

3-(Chlorosulfonyl)-4-methoxybenzoic acid

*p*-Methoxybenzoic acid (500 mg, 3.29 mmol) was dissolved in chlorosulfonic acid (2 mL). The reaction system was heated to 65 °C and stirred for 5 h under a nitrogen atmosphere. After the reaction was completed, the reaction mixture was slowly poured into ice water, and the resulting mixture was filtered under reduced pressure to give compound **13a** (610 mg, yield: 74.1%).

$^1$H NMR (400 MHz, DMSO-$d_6$): $\delta$13.64 (s, 1H), 8.30 (d, 1H), 7.92-7.88 (m, 1H), 7.06 (d, 1H), 3.83 (s, 3H).
MS m/z (ESI): 248.9 [M-H]$^+$.

Step b:

Methyl 3-(chlorosulfonyl)-4-methoxybenzoate

Under a nitrogen atmosphere, compound **13a** (4.00 g, 15.96 mmol) was dissolved in thionyl chloride (80 mL) at 20 °C, and the reaction system was stirred at 20 °C for 18 h. Subsequently, methanol (10 mL) was added to the reaction system, and stirring was continued for 2 h. After the reaction was completed, the mixture was diluted with water and extracted with ethyl acetate (150 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 2:1) to give compound **13b** (2.90 g, yield: 68.7%).
$^1$H NMR (400 MHz, $CDCl_3$): $\delta$8.64 (d, 1H), 8.39-8.35 (m, 1H), 7.18 (d, 1H), 4.14 (s, 3H), 3.95 (s, 3H).

Step c:

Methyl 3-(*N*-(6-((1*H*-pyrazol-1-yl)methyl)-4-methoxybenzo[d]isoxazol-3-yl)sulfamoyl)-4-methoxybenzoate

Under a nitrogen atmosphere, at 0 °C, sodium hydride (60%, 82 mg, 2.05 mmol) was added to a solution of **intermediate 1** (100 mg, 0.41 mmol) in *N*,*N*-dimethylformamide (2 mL), and the reaction system was stirred for 1 h. Then, compound **13b** (217 mg, 0.82 mmol) was added to the system, and stirring was continued at 0 °C for 1 h. After the reaction was completed, the mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:tetrahydrofuran = 10:1) to give compound **13c** (68 mg, yield: 35.2%).
$^1$HNMR (400 MHz, DMSO-$d_6$): $\delta$ 10.67 (s, 1H), 8.36 (d, 1H), 8.22-8.18 (m, 1H), 7.87 (d, 1H), 7.50 (d, 1H), 7.35 (d, 1H), 6.85 (s, 1H), 6.75 (s, 1H), 6.30 (t, 1H), 5.44 (s, 2H), 3.86 (s, 3H), 3.85 (s, 3H), 3.79 (s, 3H).
MS m/z (ESI): 473.1 [M+H]$^+$.

Step d:

*N*-(6-((1*H*-Pyrazol-1-yl)methyl)-4-methoxybenzo[*d*]isoxazol-3-yl)-5-(2-hydroxypropan-2-yl)-2-methoxybenzenesulfonamide

Under a nitrogen atmosphere, methylmagnesium iodide (237 mg, 3.18 mmol) was added to a solution of compound **13c** (150 mg, 0.32 mmol) in tetrahydrofuran (7 mL) at 20 °C, and the reaction system was stirred at 20 °C for 2 h. After the reaction was completed, the mixture was quenched with a saturated aqueous ammonium chloride solution and extracted with dichloromethane (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **13** (21 mg, yield: 14.0%).
$^1$HNMR (400 MHz, DMSO-$d_6$): $\delta$10.02 (s, 1H), 7.92 (d, 1H), 7.87 (d, 1H), 7.64 (d, 1H), 7.49 (d, 1H), 7.10 (d, 1H), 6.83 (s, 1H), 6.73 (s, 1H), 6.30 (t, 1H), 5.43 (s, 2H), 5.17 (s, 1H), 3.80 (s, 3H),
**[0450]** 3.74 (s, 3H), 1.39 (s, 6H).
MS m/z (ESI): 473.1 [M+H]$^+$.

**Example 20**

Synthesis of Compound 14

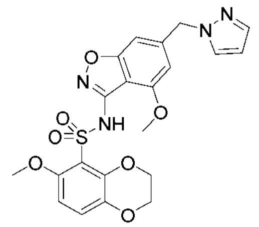

14

*N*-(6-((1*H*-Pyrazol-1-yl)methyl)-4-methoxybenzo[*d*]isoxazol-3-yl)-6-methoxy-2,3-dihydrobenzo[*b*][1,4]dioxine-5-sulfonamide

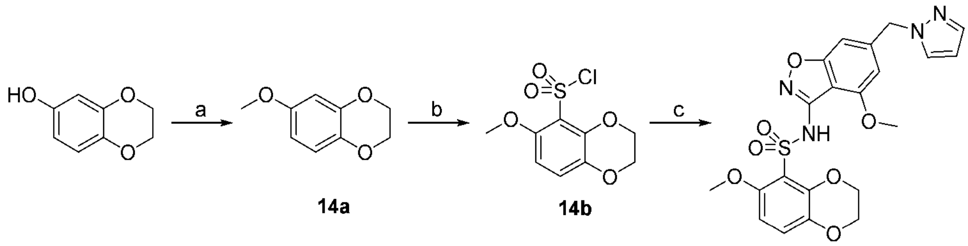


14a 14b 14

Step a:

6-Methoxy-2,3-dihydrobenzo[*b*][1,4]dioxine

6-Hydroxy-1,4-benzodioxane (500 mg, 3.29 mmol), iodomethane (700 mg, 4.93 mmol), and potassium carbonate (908 mg, 6.57 mmol) were dissolved in a solution of *N*,*N*-dimethylformamide (20 mL). The reaction system was stirred at 20 °C for 16 h. After the reaction was completed, the reaction mixture was filtered, and the filtrate was concentrated under reduced pressure. The resulting crude product was purified by flash silica gel column chromatography (petroleum ether:ethyl acetate = 3:1) to give compound **14a** (410 mg, yield: 75.1%).
$^1$H NMR (400 MHz, $CDCl_3$): 66.79 (d, 1H), 6.46-6.41 (m, 2H), 4.26-4.20 (m, 4H), 3.75 (s, 3H).

Step b:

6-Methoxy-2,3-dihydrobenzo[*b*][1,4]dioxine-5-sulfonyl chloride

Under a nitrogen atmosphere, *n*-butyllithium (2.5 M in *n*-hexane, 3.5 mL, 8.75 mmol) was added dropwise to a solution of compound **14a** (1.20 g, 7.22 mmol) in tetrahydrofuran (25 mL) at 0 °C, and the reaction system was stirred at 0 °C for 30 min and then cooled to -78 °C. Subsequently, sulfuryl chloride (1.46 g, 10.82 mmol) was added, and stirring was continued at - 78 °C for 30 min. After the reaction was completed, the mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **14b** (0.25 g, yield: 13.1%).
$^1$H NMR (400 MHz, $CDCl_3$): $\delta$7.15 (d, 1H), 6.57 (d, 1H), 4.47-4.45 (m, 2H), 4.31-4.29 (m, 2H),

3.86 (s, 3H).

Step c:

*N*-(6-((1*H*-Pyrazol-1-yl)methyl)-4-methoxybenzo[*d*]isoxazol-3-yl)-6-methoxy-2,3-dihydrobenzo[*b*][1,4]dioxine-5-sulfonamide

Under a nitrogen atmosphere, at 25 °C, sodium hydride (60%, 246 mg, 6.14 mmol) was added to a solution of intermediate 1 (150 mg, 0.61 mmol) in N,N-dimethylformamide (2 mL), and the reaction system was stirred for 1 h. Then,

compound 14b (195 mg, 0.73 mmol) was added to the system, and stirring was continued at 25 °C for 1 h. After the reaction was completed, the mixture was diluted with water and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **14** (21 mg, yield: 7.2%).

$^1$HNMR (400 MHz, DMSO-$d_6$): $\delta$ 9.73 (s, 1H), 7.87 (d, 1H), 7.50 (d, 1H), 7.04 (d, 1H), 6.84 (s, 1H), 6.75 (s, 1H), 6.63 (d, 1H), 6.31-6.30 (m, 1H), 5.45 (s, 2H), 4.20-4.15 (m, 4H), 3.86 (s, 3H), 3.69 (s, 3H).

MS m/z (ESI): 473.0 [M+H]$^+$.

**Example 21**

Synthesis of Compound 15

*N*-(6-((1*H*-Pyrazol-1-yl)methyl)-4-methoxybenzo[*d*]isoxazol-3-yl)-7-methoxyspiro[chromane-4,1'-cyclobutane]-8-sulfonamide

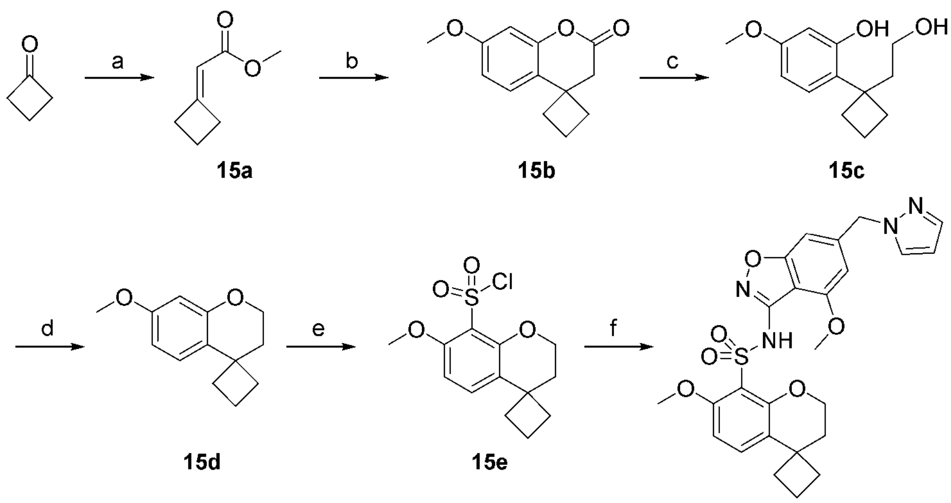


15a 15b 15c 15d 15e 15

Step a:

Methyl 2-cyclobutylideneacetate

Sodium hydride (60%, 2.08 g, 52.00 mmol) was added to a solution of trimethyl phosphonoacetate (9.46 g, 51.95 mmol) in tetrahydrofuran (80 mL) at 0 °C. The reaction system was stirred at 0 °C for 1 h. Then, cyclobutanone (2.60 g, 37.10 mmol) was added, and the reaction system was stirred at 25 °C for another 2 h. After the reaction was completed, the mixture was quenched with a saturated aqueous ammonium chloride solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 20:1) to give compound **15a** (3.00 g, yield: 64.1%).

$^1$H NMR (400 MHz, $CDCl_3$) $\delta$5.59-5.58 (m, 1H), 3.67 (s, 3H), 3.12 (t, 2H), 2.83 (t, 2H), 2.12-2.04 (m, 2H)

Step b:

7-Methoxyspiro[chromane-4,1'-cyclobutan]-2-one

3-Methoxyphenol (6.88 g, 55.42 mmol), compound **15a** (3.50 g, 27.74 mmol), and 98% concentrated sulfuric acid (0.54 g, 5.40 mmol) were mixed. The reaction system was then heated to 130 °C and stirred for 2 h. After the reaction was completed, the mixture was quenched with water and extracted with ethyl acetate (100 mL × 3). The organic layers were

combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 20:1) to give compound **15b** (0.74 g, yield: 12.2%).

$^1H$ NMR (400 MHz, $CDCl_3$): $\delta$7.38 (d, 1H), 6.78-6.75 (m, 1H), 6.64 (d, 1H), 3.82 (s, 3H), 2.89 (s, 2H), 2.40-2.37 (m, 2H),2.13-2.03 (m, 4H).

Step c:

2-(1-(2-Hydroxyethyl)cyclobutyl)-5-methoxyphenol

Lithium aluminum hydride (136 mg, 3.39 mmol) was added to a solution of compound **15b** (740 mg, 3.39 mmol) in tetrahydrofuran (20 mL) at 0 °C. The reaction system was stirred at 0 °C for 1 h. After the reaction was completed, the mixture was quenched with water and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 2:1) to give compound **15c** (570 mg, yield: 75.6%).

$^1H$ NMR (400 MHz, $CDCl_3$): $\delta$6.85 (d, 1H), 6.50-6.47 (m, 1H), 6.42 (d, 1H),3.79 (s, 3H), 3.67 (t, 2H), 2.55-2.47 (m, 2H), 2.34-2.29 (m, 2H), 2.22-2.07(m, 3H). 1.88-1.85 (m, 1H).

Step d:

7-Methoxyspiro[chromane-4,1'-cyclobutane]

Compound **15c** (570 mg, 2.56 mmol) and p-toluenesulfonic acid (88 mg, 0.51 mmol) were dissolved in a solution of toluene (10 mL). The reaction system was heated to 110 °C and stirred for 3 h. After the reaction was completed, the reaction mixture was concentrated under reduced pressure. The resulting crude product was purified by flash silica gel column chromatography (petroleum ether:ethyl acetate = 9:1) to give compound **15d** (360 mg, yield: 68.7%).

$^1H$ NMR (400 MHz, $CDCl_3$): $\delta$ 7.39 (d, 1H), 6.55-6.53 (m, 1H), 6.32 (d, 1H), 4.12 (t, 2H), 3.75 (s, 3H), 2.43-2.38 (m, 2H), 2.09-1.93 (m, 6H).

Step e:

7-Methoxyspiro[chromane-4,1'-cyclobutane]-8-sulfonyl chloride

Under a nitrogen atmosphere, *n*-butyllithium (2.5 M in *n*-hexane, 0.9 mL, 2.25 mmol) was added dropwise to a solution of compound **15d** (300 mg, 1.47 mmol) in tetrahydrofuran (10 mL) at 0 °C, and the reaction system was stirred at 0 °C for 1 h and then cooled to -78 °C. Subsequently, sulfuryl chloride (297 mg, 2.20 mmol) was added, and stirring was continued at - 78 °C for 30 min. After the reaction was completed, the mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 2:1) to give compound **15e** (190 mg, yield: 42.7%).

$^1H$ NMR (400 MHz, DMSO-$d_6$): $\delta$ 7.47 (d, 1H), 6.58 (d, 1H), 4.04-4.02 (m, 2H), 3.67 (s, 3H), 2.32-2.30 (m, 2H), 2.01-1.88 (m, 6H).

Step f:

*N*-(6-((1*H*-Pyrazol-1-yl)methyl)-4-methoxybenzo[*d*]isoxazol-3-yl)-7-methoxyspiro[chromane-4,1'-cyclobutane]-8-sulfonamide

Sodium hydride (60%, 82 mg, 2.05 mmol) was added to a solution of **intermediate 1** (100 mg, 0.41 mmol) in *N*,*N*-dimethylformamide (1 mL) at 0 °C. The reaction system was stirred for 15 min. Then, compound **15e** (186 mg, 0.61 mmol) was added to the system, and stirring was continued at 0 °C for 30 min. After the reaction was completed, the reaction system was diluted with water, adjusted to pH = 7 with a 1 N aqueous hydrochloric acid solution, and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous formic acid solution, mobile phase B: acetonitrile; gradient ratio: phase B 5%-95%) to give compound **15** (100

mg, yield: 47.8%).

$^1$HNMR (400 MHz, DMSO-$d_6$): $\delta$9.49 (s, 1H), 7.87 (d, 1H), 7.73 (d, 1H), 7.49 (d, 1H), 6.83 (s, 1H), 6.76-6.72 (m, 2H), 6.30 (s, 1H), 5.45 (s, 2H), 4.05 (t, 2H), 3.87 (s, 3H), 3.73 (s, 3H), 2.33-2.28 (m, 2H), 2.02-1.87 (m, 6H).

MS m/z (ESI): 511.0 [M+H]$^+$.

**Example 22**

Synthesis of Compound 16

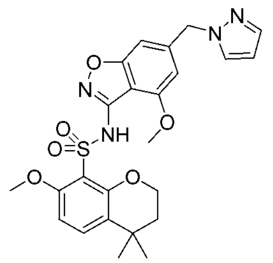

**16**

*N*-(6-((1*H*-Pyrazol-1-yl)methyl)-4-methoxybenzo[*d*]isoxazol-3-yl)-7-methoxy-4,4-dimethylchromane-8-sulfonamide

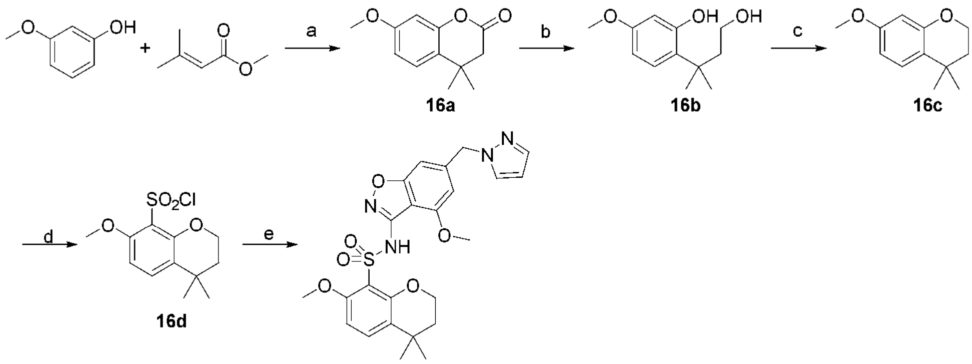


16a 16b 16c 16d 16

Step a:

7-Methoxy-4,4-dimethylchroman-2-one

3-Methoxyphenol (8.69 g, 70.00 mmol), methyl 3,3-dimethylacrylate (4.00 g, 35.04 mmol), and 98% concentrated sulfuric acid (0.69 g, 6.90 mmol) were mixed. The reaction system was then heated to 130 °C and stirred for 3 h. After the reaction was completed, the mixture was quenched with water and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **16a** (2.30 g, yield: 31.8%).

$^1$H NMR (400 MHz, $CDCl_3$): $\delta$7.22 (d, 1H), 6.74-6.71 (m, 1H), 6.64 (d, 1H), 3.82 (s, 3H), 2.62 (s, 2H), 1.35 (s, 6H).

Step b:

2-(4-Hydroxy-2-methylbutan-2-yl)-5-methoxyphenol

Lithium aluminum hydride (0.33 g, 8.73 mmol) was added to a solution of compound **16a** (1.80 g, 8.73 mmol) in tetrahydrofuran (20 mL) at 0 °C. The reaction system was stirred at 0 °C for 1 h. After the reaction was completed, the mixture was quenched with water and extracted with ethyl acetate (100 mL × 3). The organic layers were combined,

washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **16b** (1.39 g, yield: 75.7%).

$^1$H NMR (400 MHz, $CDCl_3$): $\delta$7.09 (d, 1H), 6.41-6.39 (m, 1H), 6.24 (d, 1H), 3.74 (s, 3H), 3.54 (t, 2H), 2.18 (t, 2H), 1.38 (s, 6H).

Step c:

7-Methoxy-4,4-dimethylchromane

Compound **16b** (320 mg, 1.52 mmol) and p-toluenesulfonic acid (53 mg, 0.31 mmol) were dissolved in a solution of toluene (10 mL). The reaction system was heated to 120 °C and stirred for 2 h. After the reaction was completed, the reaction mixture was concentrated under reduced pressure. The resulting crude product was purified by flash silica gel column chromatography (petroleum ether:ethyl acetate = 9:1) to give compound **16c** (150 mg, yield: 51.3%).

$^1$H NMR (400 MHz, $CDCl_3$): $\delta$ 7.15 (d, 1H), 6.49-6.46 (m, 1H), 6.34 (d, 1H), 4.18 (t, 2H), 3.74 (s, 3H), 1.81 (t, 2H), 1.30 (s, 6H).

Step d:

7-Methoxy-4,4-dimethylchromane-8-sulfonyl chloride

Under a nitrogen atmosphere, *n*-butyllithium (2.5 M in *n*-hexane, 1.1 mL, 2.75 mmol) was added dropwise to a solution of compound **16c** (450 mg, 2.34 mmol) in tetrahydrofuran (3 mL) at 0 °C, and the reaction system was stirred at 0 °C for 1 h and then cooled to -78 °C. Subsequently, sulfuryl chloride (474 mg, 3.51 mmol) was added, and stirring was continued at - 78 °C for 1 h. After the reaction was completed, the mixture was quenched with a saturated aqueous ammonium chloride solution and extracted with ethyl acetate (70 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **16d** (125 mg, yield: 18.4%).

$^1$H NMR (400 MHz, $CDCl_3$): $\delta$ 7.42 (d, 1H), 6.50 (d, 1H), 4.32 (t, 2H), 3.87 (s, 3H), 1.81 (t, 2H), 1.26 (s, 6H).

Step e:

*N*-(6-((1*H*-Pyrazol-1-yl)methyl)-4-methoxybenzo[*d*]isoxazol-3-yl)-7-methoxy-4,4-dimethylchromane-8-sulfonamide

Under a nitrogen atmosphere, at 25 °C, sodium hydride (60%, 62 mg, 1.55 mmol) was added to a solution of **intermediate 1** (75 mg, 0.31 mmol) in *N*,*N*-dimethylformamide (3 mL), and the reaction system was stirred for 1 h. Then, compound **16d** (112 mg, 0.38 mmol) was added to the system, and stirring was continued at 25 °C for 1 h. After the reaction was completed, the mixture was diluted with water and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **16** (30 mg, yield: 19.6%).

$^1$HNMR (400 MHz, DMSO-$d_6$): $\delta$ 9.51 (s, 1H), 7.87 (d, 1H), 7.50-7.48 (m, 2H), 6.83 (s, 1H), 6.75 (s, 1H), 6.67 (d, 1H), 6.31 (t, 1H), 5.45 (s, 2H), 4.08 (t, 2H), 3.87 (s, 3H), 3.71 (s, 3H), 1.67 (t, 2H), 1.20 (s, 6H).

MS m/z (ESI): 499.0 $[M+H]^+$.

**Example 23**

Synthesis of Compound 17

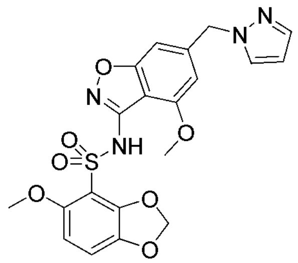

17

*N*-(6-((1*H*-Pyrazol-1-yl)methyl)-4-methoxybenzo[*d*]isoxazol-3-yl)-5-methoxybenzo[*d*][1,3]dioxole-4-sulfonamide

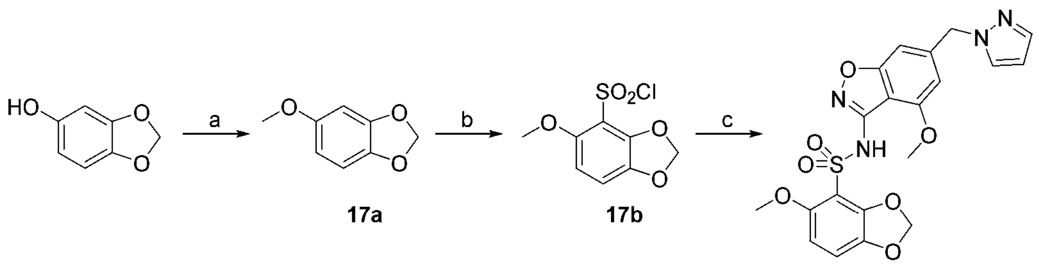


17a 17b 17

Step a:

5-Methoxybenzo[*d*][1,3]dioxole

Sesamol (2.00 g, 14.48 mmol), iodomethane (3.09 g, 21.77 mmol), and potassium carbonate (4.10 g, 29.67 mmol) were dissolved in acetone (30 mL). The reaction system was stirred at 25 °C for 16 h. After the reaction was completed, the reaction system was diluted with water and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **17a** (1.60 g, yield: 72.6%).

$^1H$ NMR (400 MHz, $CDCl_3$): $\delta$6.71 (d, 1H), 6.49 (d, 1H), 6.33-6.31 (m, 1H), 5.91 (s, 2H), 3.75 (s, 3H).

Step b:

5-Methoxybenzo[*d*][1,3]dioxole-4-sulfonyl chloride

Under a nitrogen atmosphere, *n*-butyllithium (1.6 M in *n*-hexane, 0.98 mL, 1.58 mmol) was added dropwise to a solution of compound **17a** (200 mg, 1.31 mmol) in tetrahydrofuran (10 mL) at 0 °C, and the reaction system was stirred at 0 °C for 1 h and then cooled to -78 °C. Subsequently, sulfuryl chloride (355 mg, 2.63 mmol) was added, and stirring was continued at - 78 °C for 1 h. After the reaction was completed, the mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **17b** (110 mg, yield: 33.4%).

$^1H$ NMR (400 MHz, DMSO-$d_6$): $\delta$6.76 (d, 1H), 6.34 (d, 1H), 5.91 (s, 2H), 3.66 (s, 3H).

Step c:

*N*-(6-((1*H*-Pyrazol-1-yl)methyl)-4-methoxybenzo[*d*]isoxazol-3-yl)-5-methoxybenzo[*d*][1,3]dioxole-4-sulfonamide

Sodium hydride (60%, 164 mg, 4.09 mmol) was added to a solution of **intermediate 1** (200 mg, 0.82 mmol) in *N*,*N*-dimethylformamide (2 mL) at 0 °C. The reaction system was stirred at 0 °C for 10 min. Then, compound **17b** (411 mg, 1.64 mmol) was added, and stirring was continued for 50 min. After the reaction was completed, the reaction mixture was

warmed to room temperature, quenched with a saturated aqueous ammonium chloride solution, and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **17** (92 mg, yield: 24.5%).

$^1$HNMR (400 MHz, DMSO-$d_6$): δ10.28 (s, 1H), 7.88 (d, 1H), 7.50 (d, 1H), 7.08 (d, 1H), 6.86 (s, 1H), 6.75 (s, 1H), 6.49 (d, 1H), 6.31-6.30 (m, 1H), 6.07 (s, 2H), 5.45 (s, 2H), 3.82 (s, 3H), 3.66 (s, 3H).

MS m/z (ESI): 459.0 [M+H]$^+$.

**Example 24**

Synthesis of Compound 18

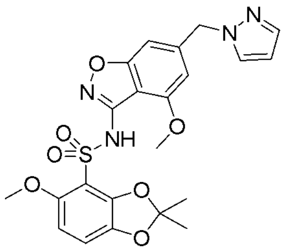

**18**

*N*-(6-((1*H*-Pyrazol-1-yl)methyl)-4-methoxybenzo[*d*]isoxazol-3-yl)-5-methoxy-2,2-dimethylbenzo[*d*][1,3]dioxole-4-sulfonamide

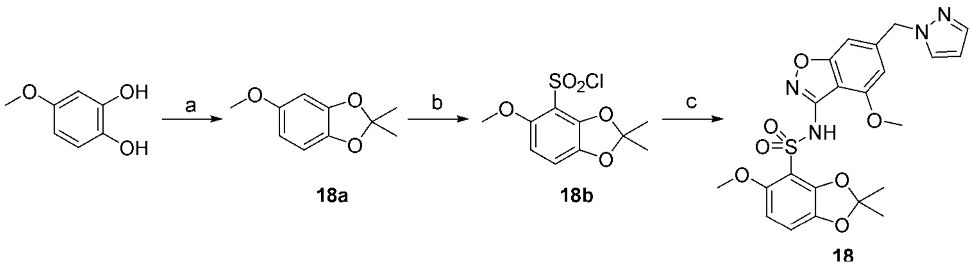


Step a:

5-Methoxy-2,2-dimethylbenzo[*d*][1,3]dioxole

4-Methoxybenzene-1,2-diol (1.00 g, 7.14 mmol), 2,2-dimethoxypropane (1.49 g, 14.27 mmol), and *p*-toluenesulfonic acid (0.12 g, 0.71 mmol) were dissolved in a solution of toluene (30 mL). The reaction system was heated to 120 °C and stirred for 16 h. After the reaction was completed, the reaction mixture was concentrated under reduced pressure. The resulting crude product was purified by flash silica gel column chromatography (petroleum ether:ethyl acetate = 20:1) to give compound **18a** (0.69 g, yield: 53.7%).

$^1$H NMR (400 MHz, DMSO-$d_6$): δ6.69 (d, 1H), 6.50(d, 1H), 6.31-6.28 (m, 1H), 3.66 (s, 3H), 1.60 (s, 6H).

Step b:

5-Methoxy-2,2-dimethylbenzo[*d*][1,3]dioxole-4-sulfonyl chloride

Under a nitrogen atmosphere, *n*-butyllithium (2.5 M in n-hexane, 4.3 mL, 10.75 mmol) was added dropwise to a

solution of compound **18a** (1.60 g, 8.84 mmol) in tetrahydrofuran (20 mL) at 0 °C, and the reaction system was stirred at 0 °C for 1 h and then cooled to -78 °C. Subsequently, sulfuryl chloride (2.40 g, 17.78 mmol) was added, and stirring was continued at - 78 °C for 1 h. After the reaction was completed, the mixture was quenched with a saturated aqueous ammonium chloride solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **18b** (0.47 g, yield: 19.0%).

$^1$H NMR (400 MHz, $CDCl_3$): $\delta$6.92 (d, 1H), 6.39 (d, 1H), 3.96 (s, 3H), 1.78 (s, 6H).

Step c:

*N*-(6-((1*H*-Pyrazol-1-yl)methyl)-4-methoxybenzo[*d*]isoxazol-3-yl)-5-methoxy-2,2-dimethylbenzo[*d*][1,3]dioxole-4-sulfonamide

Sodium hydride (60%, 115 mg, 2.87 mmol) was added to a solution of **intermediate 1** (140 mg, 0.57 mmol) in *N*,*N*-dimethylformamide (2 mL) at 0 °C. The reaction system was stirred at 0 °C for 10 min. Then, compound **18b** (320 mg, 1.15 mmol) was added, and stirring was continued for 50 min. After the reaction was completed, the reaction mixture was warmed to room temperature, quenched with a saturated aqueous ammonium chloride solution, and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:methanol = 9:1) to give compound **18** (30 mg, yield: 10.8%).

$^1$HNMR (400 MHz, DMSO-$d_6$): $\delta$10.18 (s, 1H), 7.81 (d, 1H), 7.50 (d, 1H), 6.97 (d, 1H), 6.85 (s,

1H), 6.74 (s, 1H), 6.45 (d, 1H), 6.31-6.30 (m, 1H), 5.45 (s, 2H), 3.83 (s, 3H), 3.66 (s, 3H), 1.53 (s, 6H).

MS m/z (ESI): 487.0 [M+H]$^+$.

**Example 25**

Synthesis of Compound 19

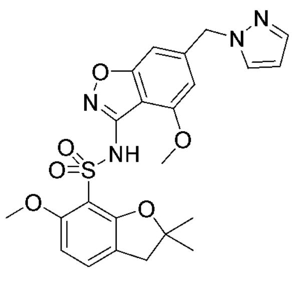

**19**

*N*-(6-((1*H*-Pyrazol-1-yl)methyl)-4-methoxybenzo[*d*]isoxazol-3-yl)-6-methoxy-2,2-dimethyl-2,3-dihydrobenzofuran-7-sulfonamide

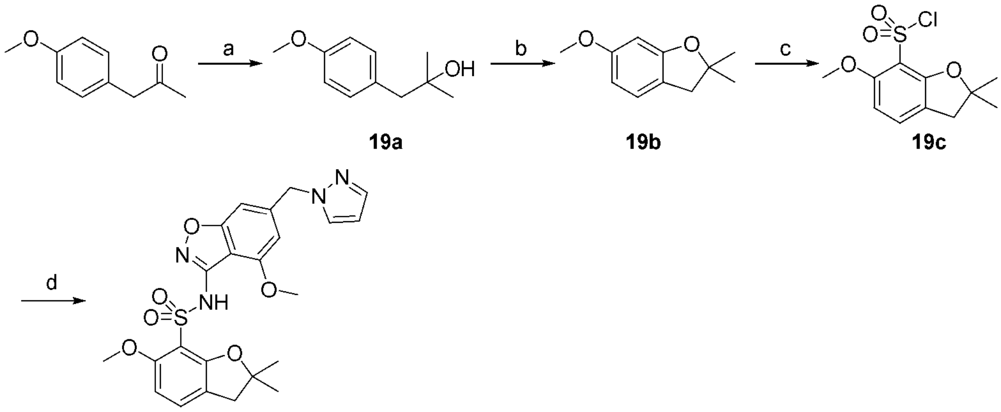


19a 19b 19c

19

Step a:

1-(4-Methoxyphenyl)-2-methylpropan-2-ol

Under a nitrogen atmosphere, methylmagnesium chloride (3.0 M in tetrahydrofuran, 9.10 mL, 27.30 mmol) was added dropwise to a solution of *p*-methoxyphenylacetone (1.50 g, 9.14 mmol) in tetrahydrofuran (30 mL), and the reaction system was stirred at 0 °C for 16 h. After the reaction was completed, the mixture was quenched with water and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **19a** (1.05 g, yield: 63.8%).

$^1$H NMR (400 MHz, $CDCl_3$) $\delta$ 7.15 (d, 2H), 6.87(d, 2H), 3.82 (s, 3H), 2.72(s, 2H), 1.20 (s, 6H).

Step b:

6-Methoxy-2,2-dimethyl-2,3-dihydrobenzofuran

Under a hydrogen atmosphere, lithium carbonate (1.23 g, 16.65 mmol), (diacetoxyiodo)benzene (5.36 g, 16.64 mmol), and palladium acetate (0.12 g, 0.53 mmol) were added to a solution of compound **19a** (2.00 g, 11.10 mmol) in perfluorobenzene (25 mL), and the reaction system was heated to 120 °C and stirred for 36 h. After the reaction was completed, the reaction mixture was filtered under reduced pressure, and the filtrate was concentrated under reduced pressure. The resulting crude product was purified by flash silica gel column chromatography (petroleum ether:ethyl acetate = 9:1) to give compound **19b** (0.55 g, yield: 27.8%).

$^1$H NMR (400 MHz, $CDCl_3$): $\delta$6.92 (d, 1H), 6.32-6.26(m, 2H), 3.68 (s, 3H), 2.87(s, 2H), 1.39 (s, 6H).

Step c:

6-Methoxy-2,2-dimethyl-2,3-dihydrobenzofuran-7-sulfonyl chloride

Under a nitrogen atmosphere, *n*-butyllithium (2.5 M in *n*-hexane, 0.8 mL, 2.00 mmol) was added dropwise to a solution of compound **19b** (250 mg, 1.40 mmol) in tetrahydrofuran (5 mL) at 0 °C, and the reaction system was stirred at 0 °C for 1 h and then cooled to -78 °C. Subsequently, sulfuryl chloride (227 mg, 1.68 mmol) was added, and stirring was continued at - 78 °C for 1 h. After the reaction was completed, the mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **19c** (50 mg, yield: 12.9%).

$^1$H NMR (400 MHz, $CDCl_3$): $\delta$ 7.30 (d, 1H), 6.43 (d, 1H), 3.96 (s, 3H), 2.98 (s, 2H), 1.55 (s, 6H).

Step d:

*N*-(6-((1*H*-Pyrazol-1-yl)methyl)-4-methoxybenzo[*d*]isoxazol-3-yl)-6-methoxy-2,2-dimethyl-2,3-dihydrobenzofuran-7-sulfonamide

Under a nitrogen atmosphere, at 0 °C, sodium hydride (60%, 50 mg, 1.24 mmol) was added to a solution of **intermediate 1** (100 mg, 0.41 mmol) in *N*,*N*-dimethylformamide (2 mL), and the reaction system was stirred for 1 h. Then, compound **19c** (170 mg, 0.61 mmol) was added to the system, and the reaction mixture was warmed to room temperature and stirred for 1 h. After the reaction was completed, the mixture was diluted with water and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **19** (5 mg, yield: 2.5%).

$^1$HNMR (400 MHz, DMSO-$d_6$): $\delta$ 9.55(s, 1H), 7.87(d, 1H), 7.50 (d, 1H), 7.29 (d, 1H), 6.83 (s, 1H), 6.76 (s, 1H), 6.53 (d, 1H), 6.30 (t, 1H), 5.45(s, 2H), 3.88 (s, 3H), 3.73 (s, 3H), 2.88(s, 2H), 1.20 (s, 6H).

MS m/z (ESI): 485.0 [M+H]$^+$.

**Example 26**

Synthesis of Compound 20

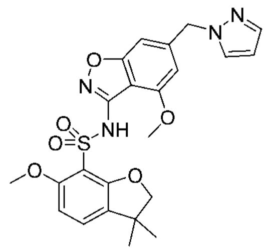

**20**

*N*-(6-((1*H*-Pyrazol-1-yl)methyl)-4-methoxybenzo[*d*]isoxazol-3-yl)-6-methoxy-3,3-dimethyl-2,3-dihydrobenzofuran-7-sulfonamide

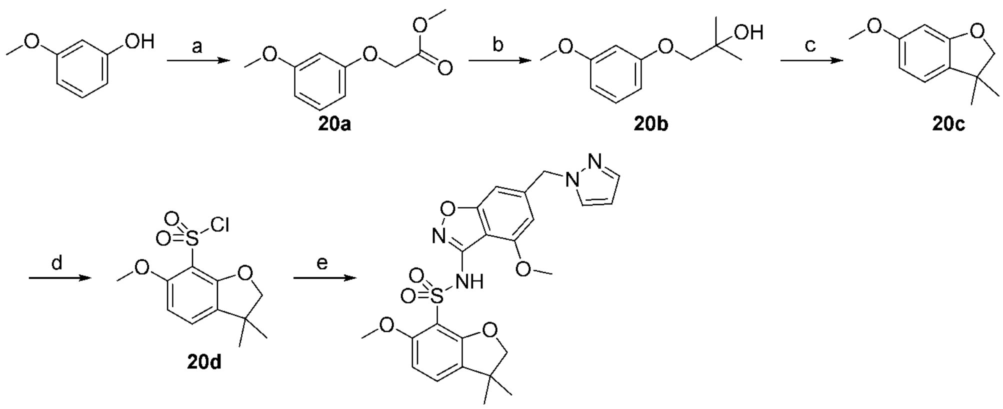


**20a** **20b** **20c** **20d** **20**

Step a:

Methyl 2-(3-methoxyphenoxy)acetate

3-Methoxyphenol (8.00 g, 64.44 mmol), ethyl bromoacetate (16.13 g, 96.59 mmol), and potassium carbonate

(17.80 g, 128.79 mmol) were dissolved in acetone (100 mL). The reaction system was heated to 60 °C and stirred for 16 h. After the reaction was completed, the reaction mixture was filtered under reduced pressure, and the filtrate was concentrated under reduced pressure. The resulting crude product was then purified by flash silica gel column chromatography (petroleum ether:ethyl acetate = 3:1) to give compound **20a** (12.30 g, yield: 97.3%).

$^1$H NMR (400 MHz, $CDCl_3$): $\delta$7.20 (t, 1H), 6.59-6.49 (m, 3H), 4.62 (s, 2H), 4.29 (q, 2H), 3.80 (s, 3H) 1.32 (t, 3H).

Step b:

1-(3-Methoxyphenoxy)-2-methylpropan-2-ol

Methylmagnesium chloride (3.0 M in *n*-hexane, 4.76 mL, 14.27 mmol) was added dropwise to a solution of compound **20a** (1.00 g, 4.76 mmol) in tetrahydrofuran (10 mL) at 0 °C, and the reaction system was stirred at 0 °C for 1 h. After the reaction was completed, the reaction mixture was quenched with water and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **20b** (0.82 g, yield: 82.0%).

$^1$H NMR (400 MHz, $CDCl_3$): $\delta$7.18 (t, 1H), 6.54-6.48 (m, 3H), 3.80 (s, 3H), 3.78 (s, 2H), 1.34 (s, 6H).

Step c:

6-Methoxy-3,3-dimethyl-2,3-dihydrobenzofuran

Under a nitrogen atmosphere, phosphorus pentoxide (19.23 g, 135.48 mmol) was added to a solution of compound **20b** (7.60 g, 38.73 mmol) in methanesulfonic acid (150 mL), and the reaction system was stirred at 25 °C for 2 h. After the reaction was completed, the reaction mixture was quenched with ice water and extracted with ethyl acetate (200 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 9:1) to give compound **20c** (1.90 g, yield: 27.5%).

MS m/z (ESI): 179.1 $[M+H]^+$.

Step d:

6-Methoxy-3,3-dimethyl-2,3-dihydrobenzofuran-7-sulfonyl chloride

Under a nitrogen atmosphere, *n*-butyllithium (2.5 M in *n*-hexane, 0.54 mL, 1.35 mmol) was added dropwise to a solution of compound **20c** (200 mg, 1.12 mmol) in tetrahydrofuran (10 mL) at 0 °C, and the reaction system was stirred at 0 °C for 1 h and then cooled to -78 °C. Subsequently, sulfuryl chloride (227 mg, 1.68 mmol) was added, and stirring was continued at - 78 °C for 1 h. After the reaction was completed, the mixture was quenched with a saturated aqueous ammonium chloride solution and extracted with ethyl acetate (10 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **20d** (30 mg, yield: 9.7%).

$^1$H NMR (400 MHz, $CDCl_3$): $\delta$7.23 (d, 1H), 6.36 (d, 1H), 3.89 (s, 3H), 2.90 (s, 2H), 1.48 (s, 6H).

Step e:

*N*-(6-((1*H*-Pyrazol-1-yl)methyl)-4-methoxybenzo[*d*]isoxazol-3-yl)-6-methoxy-3,3-dimethyl-2,3-dihydrobenzofuran-7-sulfonamide

Sodium hydride (60%, 106 mg, 2.66 mmol) was added to a solution of **intermediate 1** (130 mg, 0.53 mmol) in *N*, *N*-dimethylformamide (4 mL) at 0 °C. The reaction system was stirred at 25 °C for 1 h. Then, compound **20d** (198 mg, 0.72 mmol) was added, and stirring was continued for 1 h. After the reaction was completed, the reaction mixture was quenched with water and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile;

gradient ratio: phase B 5%-95%) to give compound **20** (75 mg, yield: 29.1%).

$^1$HNMR (400 MHz, DMSO-$d_6$): $\delta$9.56 (s, 1H), 7.69 (d, 1H), 7.50 (d, 1H), 7.29 (d, 1H), 6.83 (s, 1H), 6.75 (s, 1H), 6.52 (d, 1H), 6.30 (t, 1H), 5.45 (s, 2H), 3.88 (s, 3H), 3.73 (s, 3H), 2.88 (s, 2H), 1.20 (s, 6H).

MS m/z (ESI): 485.0 $[M+H]^+$.

**Example 27**

Synthesis of Compound 21

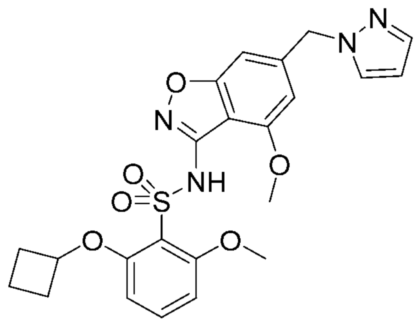

**21**

*N*-(6-((1*H*-Pyrazol-1-yl)methyl)-4-methoxybenzo[*d*]isoxazol-3-yl)-2-cyclobutoxy-6-methoxybenzenesulfonamide

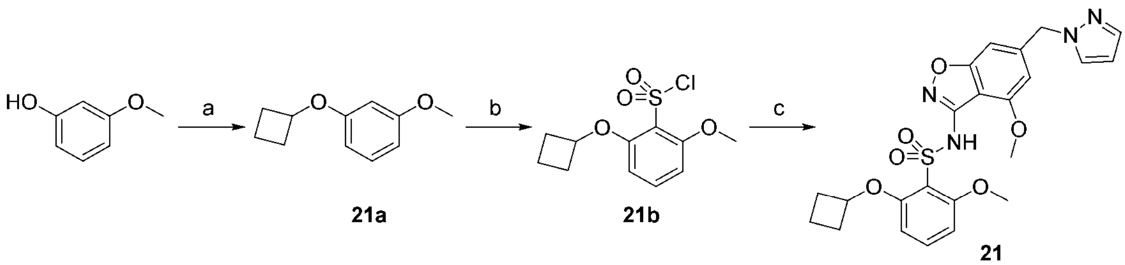


**21a** **21b** **21**

Step a:

1-Cyclobutoxy-3-methoxybenzene

Potassium carbonate (2.24 g, 16.21 mmol) and bromocyclobutane (1.31 g, 9.78 mmol) were added to a solution of 3-methoxyphenol (1.00 g, 8.06 mmol) in *N*,*N*-dimethylformamide (10 mL). The reaction system was heated to 100 °C and stirred for 40 h. After the reaction was completed, the reaction mixture was cooled to room temperature, diluted with water (50 mL), and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 20:1) to give compound **21a** (1.00 g, yield: 69.7%).

$^1$H NMR (400 MHz, DMSO-$d_6$): $\delta$7.18-7.12 (m, 1H), 6.51-6.48 (m, 1H), 6.43-6.36 (m, 2H), 4.71-4.61 (m, 1H), 3.72 (s, 3H), 2.46-2.36 (m, 2H), 2.08-1.95 (m, 2H), 1.82-1.58 (m, 2H).

Step b:

2-Cyclobutoxy-6-methoxybenzenesulfonyl chloride

Under a nitrogen atmosphere, *n*-butyllithium (2.5 M in *n*-hexane, 2.4 mL, 6.00 mmol) was added dropwise to a

solution of compound **21a** (900 mg, 5.05 mmol) in tetrahydrofuran (20 mL) at 0 °C, and the reaction system was stirred at 0 °C for 1 h and then cooled to -78 °C. Subsequently, sulfuryl chloride (1.36 g, 10.08 mmol) was added, and stirring was continued at - 78 °C for 2 h. After the reaction was completed, the mixture was quenched with a saturated aqueous ammonium chloride solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 5:1) to give compound **21b** (480 mg, yield: 34.3%).

$^1$H NMR (400 MHz, DMSO-$d_6$): $\delta$7.25-7.19 (m, 1H), 6.63 (d, 1H), 6.47 (d, 1H), 4.64 (t, 1H), 3.72 (s, 3H), 2.39-2.29 (m, 2H), 2.13-2.00 (m, 2H), 1.80-1.54 (m, 2H).

Step c:

*N*-(6-((1*H*-Pyrazol-1-yl)methyl)-4-methoxybenzo[*d*]isoxazol-3-yl)-2-cyclobutoxy-6-methoxybenzenesulfonamide

Sodium hydride (60%, 82 mg, 2.05 mmol) was added to a solution of **intermediate 1** (100 mg, 0.41 mmol) in *N*,*N*-dimethylformamide (3 mL) at 0 °C. The reaction system was stirred at 0 °C for 1 h. Then, compound **21b** (227 mg, 0.82 mmol) was added, and stirring was continued for 2 h. After the reaction was completed, the reaction mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **21** (45 mg, yield: 22.7%).

$^1$HNMR (400 MHz, DMSO-$d_6$): $\delta$9.53 (s, 1H), 7.88 (d, 1H), 7.50 (d, 1H), 7.45 (t, 1H), 6.82 (s, 1H), 6.77-6.74 (m, 2H), 6.54 (d, 1H), 6.31 (t, 1H), 5.45 (s, 2H), 4.73-4.64 (m, 1H), 3.88 (s, 3H), 3.78 (s, 3H), 2.27-2.21 (m, 2H), 1.94-1.81 (m, 2H), 1.58-1.46 (m, 2H).

MS m/z (ESI): 485.0 [M+H]$^+$.

**Example 28**

Synthesis of Compound 22

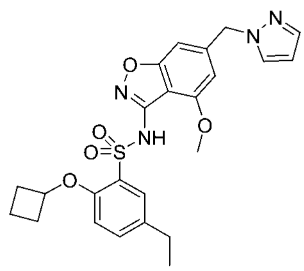

**22**

N-(6-((1*H*-Pyrazol-1-yl)methyl)-4-methoxybenzo[*d*]isoxazol-3-yl)-2-cyclobutoxy-5-ethylbenzenesulfonamide

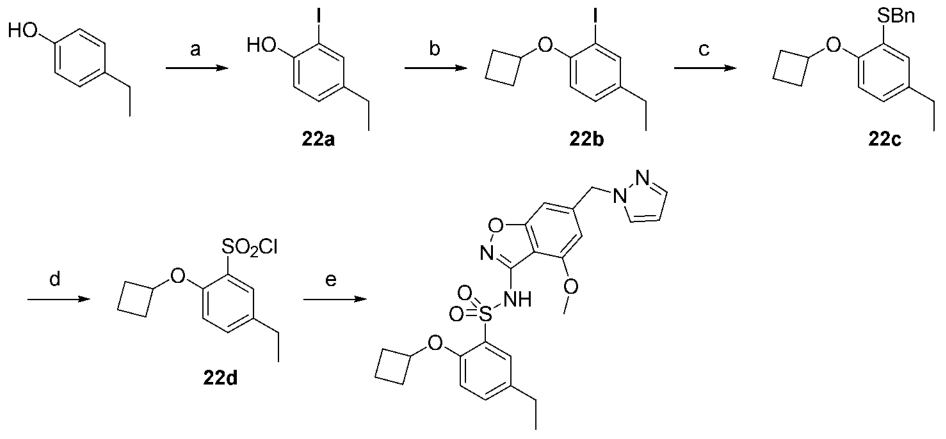


22a 22b 22c 22d 22

Step a:

4-Ethyl-2-iodophenol

*p*-Toluenesulfonic acid (2.82 g, 16.38 mmol) was added to a solution of 4-ethylphenol (2.00 g, 16.37 mmol) in acetonitrile (50 mL) at 25 °C. After the mixture was reacted for 10 min, N-iodosuccinimide (3.69 g, 16.40 mmol) was added, and the reaction system was stirred for 16 h. After the reaction was completed, the reaction mixture was quenched with an aqueous sodium sulfite solution (100 mL) and acidified with a hydrochloric acid solution (1 M), and then the phases were separated. The organic phase was collected, and the aqueous phase was extracted with ethyl acetate (100 mL × 2). The organic layers were combined and dried over anhydrous sodium sulfate. After filtration, the mother liquor was concentrated under reduced pressure. The resulting crude product was purified by flash silica gel column chromatography (petroleum ether:ethyl acetate = 9:1) to give compound **22a** (2.80 g, yield: 68.9%).

$^1$H NMR (400 MHz, DMSO-$d_6$): $\delta$10.00 (s, 1H), 7.49 (d, 1H), 7.03-7.01 (m, 1H), 6.79 (d, 1H), 2.49 (q, 2H), 1.11 (t, 3H).

Step b:

1-Cyclobutoxy-4-ethyl-2-iodobenzene

Compound **22a** (1.00 g, 4.03 mmol), bromocyclobutane (0.66 g, 4.84 mmol), and cesium carbonate (2.63 g, 8.06 mmol) were dissolved in a solution of *N*,*N*-dimethylformamide (15 mL). The reaction system was heated to 100 °C and stirred for 16 h. After the reaction was completed, the reaction mixture was cooled to room temperature, diluted with water (30 mL), and extracted with ethyl acetate (50 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 9:1) to give compound **22b** (1.10 g, yield: 90.3%).

$^1$H NMR (400 MHz, $CDCl_3$): $\delta$7.60 (d, 1H), 7.07-7.05 (m, 1H), 6.59 (d, 1H), 4.66-4.63 (m, 1H), 2.54 (q, 2H), 2.47-2.43 (m, 2H), 2.27-2.22 (m, 2H), 1.89-1.85 (m, 1H), 1.71-1.66 (m, 1H),1.19 (t, 3H).

Step c:

Benzyl(2-cyclobutoxy-5-ethylphenyl)sulfane

Under a nitrogen atmosphere, benzyl mercaptan (90 mg, 0.73 mmol), potassium carbonate (182 mg, 1.32 mmol), 1,4-diazabicyclo[2.2.2]octane (7.4 mg, 0.066 mmol), and copper(I) iodide (6.1 mg, 0.033 mmol) were added to a solution of compound **22b** (200 mg, 0.66 mmol) in N,N-dimethylformamide (6 mL). The reaction system was heated to 100 °C and stirred for 16 h. After the reaction was completed, the reaction mixture was cooled to room temperature, diluted with water (20 mL), and extracted with ethyl acetate (20 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 9:1) to give compound **22c** (140 mg, yield: 70.9%).

$^1$H NMR (400 MHz, $CDCl_3$): 67.24-7.14 (m, 5H), 6.98 (s, 1H), 6.87 (d, 1H), 6.54 (d, 1H), 4.60-4.56 (m, 1H), 4.03 (s,

2H), 2.47-2.35 (m, 4H), 2.17-2.12 (m, 2H), 1.79-1.77 (m, 1H), 1.61-1.59 (m, 1H), 1.07 (t, 3H).

Step d:

2-Cyclobutoxy-5-ethylbenzenesulfonyl chloride

N-Chlorosuccinimide (1.05 g, 7.84 mmol) was added to compound **22c** (780 mg, 2.61 mmol) in a mixed solution (glacial acetic acid and water in a ratio of 10:1, 10 mL) at 25 °C. The reaction system was stirred at 25 °C for 3 h. After the reaction was completed, the mixture was quenched with water (10 mL) and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 9:1) to give compound **22d** (460 mg, yield: 64.1%).

$^1$HNMR (400 MHz, $CDCl_3$): $\delta$7.75 (d, 1H), 7.44-7.41 (m, 1H), 6.85 (d, 1H), 4.87-4.82 (m, 1H), 2.65 (q, 2H), 2.53-2.47 (m, 2H), 2.38-2.30 (m, 2H), 1.95-1.93 (m, 1H), 1.76-1.72 (m, 1H), 1.24 (t, 3H).

Step e:

*N*-(6-((1*H*-Pyrazol-1-yl)methyl)-4-methoxybenzo[*d*]isoxazol-3-yl)-2-cyclobutoxy-5-ethylbenzenesulfonamide

Sodium hydride (60%, 82 mg, 2.05 mmol) was added to a solution of **intermediate 1** (100 mg, 0.41 mmol) in *N*,*N*-dimethylformamide (2 mL) at 0 °C. The reaction system was stirred at 0 °C for 10 min. Then, compound **22d** (169 mg, 0.61 mmol) was added, and stirring was continued for 50 min. After the reaction was completed, the reaction mixture was quenched with a saturated aqueous ammonium chloride solution, and the system was adjusted to pH = 5 with a 1 N aqueous hydrochloric acid solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:methanol = 20:1) to give compound **22** (39 mg, yield: 19.7%).

$^1$HNMR (400 MHz, DMSO-$d_6$): $\delta$9.73 (s, 1H), 7.87-7.81 (m, 1H), 7.67 (d, 1H), 7.49-7.48 (m, 1H), 7.42-7.39 (m, 1H), 6.88 (d, 1H), 6.84 (s, 1H), 6.75 (s, 1H), 6.30-6.29 (m, 1H), 5.44 (s, 2H), 4.69-4.66 (m, 1H), 3.81 (s, 3H), 2.60 (q, 2H), 2.23-2.20 (m, 2H), 1.80-1.75 (m, 2H), 1.55-1.51 (m, 2H),1.15 (t, 3H).

MS m/z (ESI): 483.0 $[M+H]^+$.

**Example 29**

Synthesis of Compound 23

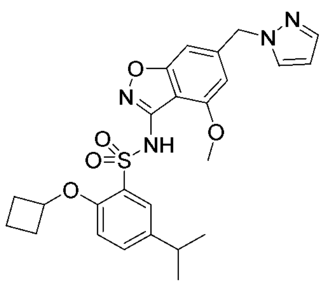

**23**

*N*-(6-((1*H*-Pyrazol-1-yl)methyl)-4-methoxybenzo[d]isoxazol-3-yl)-2-cyclobutoxy-5-isopropylbenzenesulfonamide

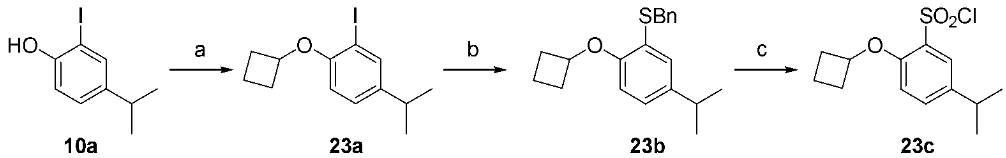


10a 23a 23b 23c

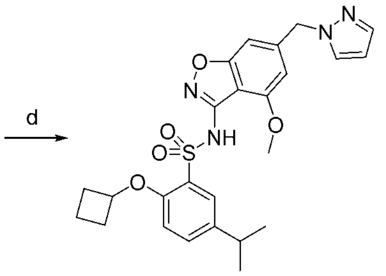


23

Step a:

1-Cyclobutoxy-2-iodo-4-isopropylbenzene

Bromocyclobutane (525 mg, 3.89 mmol) and cesium carbonate (2.11 g, 6.49 mmol) were added to a solution of compound **10a** (850 mg, 3.24 mmol) in N,N-dimethylformamide (10 mL). The reaction system was heated to 100 °C and stirred for 16 h. After the reaction was completed, the reaction mixture was cooled to room temperature, diluted with water (50 mL), and extracted with ethyl acetate (100 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 30:1) to give compound **23a** (884 mg, yield: 86.2%).

$^1$H NMR (400 MHz, DMSO-$d_6$): $\delta$7.60 (d, 1H), 7.19-7.16 (m, 1H), 6.75 (d, 1H), 4.74-4.65 (m, 1H), 2.85-2.73 (m, 1H), 2.48-2.38 (m, 2H), 2.11-1.98 (m, 2H), 1.84-1.58 (m, 2H), 1.16 (s, 3H), 1.14 (s, 3H).

Step b:

Benzyl(2-cyclobutoxy-5-isopropylphenyl)sulfane

Under a nitrogen atmosphere, benzyl mercaptan (0.52 g, 4.16 mmol), potassium carbonate (0.88 g, 6.40 mmol), 1,4-diazabicyclo[2.2.2]octane (0.04 g, 0.32 mmol), and copper(I) iodide (0.03 g, 0.16 mmol) were added to a solution of compound **23a** (1.00 g, 3.16 mmol) in N,N-dimethylformamide (10 mL). The reaction system was heated to 100 °C and stirred for 16 h. After the reaction was completed, the reaction mixture was cooled to room temperature, diluted with water (50 mL), and extracted with dichloromethane (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:dichloromethane = 10:1) to give compound **23b** (0.63 g, yield: 63.7%).

$^1$H NMR (400 MHz, DMSO-$d_6$): $\delta$7.37-7.20 (m, 5H), 7.06 (d, 1H), 6.97-6.94 (m, 1H), 6.71 (d, 1H), 4.71-4.62 (m, 1H), 4.15 (s, 2H), 2.81-2.72 (m, 1H), 2.45-2.36 (m, 2H), 2.09-1.96 (m, 2H), 1.83-1.58 (m, 2H), 1.12 (s, 3H), 1.10 (s, 3H).

Step c:

2-Cyclobutoxy-5-isopropylbenzenesulfonyl chloride

*N*-Chlorosuccinimide (95 mg, 0.71 mmol) was added to compound **23b** (37 mg, 0.12 mmol) in a mixed solution (glacial acetic acid and water in a ratio of 10:1, 3 mL) at 25 °C. The reaction system was stirred at 25 °C for 3 h. After the reaction was completed, water (50 mL) was added, and stirring was continued for 20 min. The reaction mixture was then filtered under reduced pressure to give compound **23c** (20 mg, yield: 58.5%).

$^1$HNMR (400 MHz, DMSO-$d_6$): $\delta$7.57 (d, 1H), 7.14-7.12 (m, 1H), 6.73 (d, 1H), 4.70-4.61 (m, 1H), 2.86-2.78 (m, 1H), 2.37-2.35 (m, 2H), 2.12-2.02 (m, 2H), 1.81-1.56 (m, 2H), 1.17 (s, 3H), 1.15 (s, 3H).

Step d:

*N*-(6-((1*H*-Pyrazol-1-yl)methyl)-4-methoxybenzo[*d*]isoxazol-3-yl)-2-cyclobutoxy-5-isopropylbenzenesulfonamide

Sodium hydride (60%, 82 mg, 2.05 mmol) was added to a solution of **intermediate 1** (100 mg, 0.41 mmol) in *N*,*N*-dimethylformamide (3 mL) at 0 °C. The reaction system was stirred at 0 °C for 1 h. Then, compound **23c** (236 mg, 0.82 mmol) was added, and stirring was continued for 2 h. After the reaction was completed, the reaction mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **23** (40 mg, yield: 19.7%).

$^1$HNMR (400 MHz, DMSO-$d_6$): $\delta$9.79 (s, 1H), 7.87 (d, 1H), 7.69 (d, 1H), 7.50-7.44 (m, 2H), 6.91-6.85 (m, 2H), 6.76 (s, 1H), 6.31-6.30 (m, 1H), 5.45(s, 2H), 4.69-4.64 (m, 1H), 3.79 (s, 3H), 2.94-2.90 (m, 1H), 2.23-2.17 (m, 2H), 1.80-1.73 (m, 2H), 1.57-1.48 (m, 2H), 1.19 (s, 3H), 1.17 (s, 3H).

MS m/z (ESI): 497.0 [M+H]$^+$.

**Example 30**

Synthesis of Compound 24

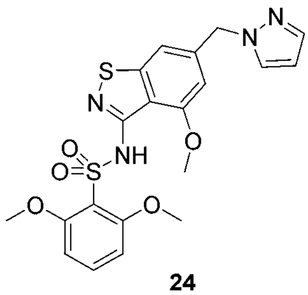

**24**

*N*-(6-((1*H*-Pyrazol-1-yl)methyl)-4-methoxybenzo[*d*]isothiazol-3-yl)-2,6-dimethoxybenzenesulfonamide

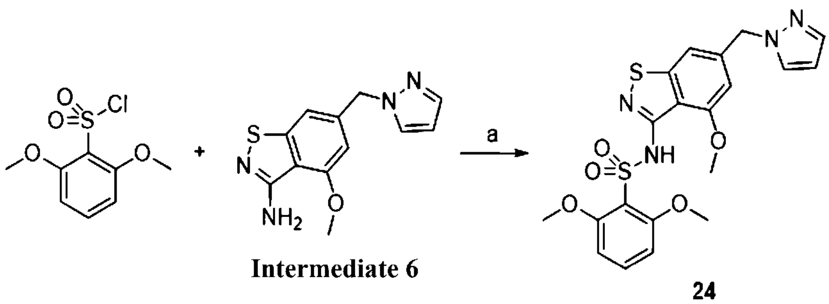


Intermediate 6

**24**

Step a:

*N*-(6-((1*H*-Pyrazol-1-yl)methyl)-4-methoxybenzo[*d*]isothiazol-3-yl)-2,6-dimethoxybenzenesulfonamide

Sodium hydride (60%, 111 mg, 2.77 mmol) was added to a solution of **intermediate 6** (120 mg, 0.46 mmol) in *N*,*N*-dimethylformamide (2 mL) at 0 °C. The reaction system was stirred at 0 °C for 1 h. Then, 2,6-dimethoxybenzenesulfonyl chloride (148 mg, 0.62 mmol) was added, and the mixture was warmed to room temperature and stirred for another 1 h. After the reaction was completed, the reaction mixture was quenched with water and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was

purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **24** (20 mg, yield: 9.4%).

$^{1}$HNMR (400 MHz, DMSO-$d_6$): $\delta$9.38 (s, 1H), 7.89 (d, 1H), 7.50 (d, 1H), 7.45 (t, 1H), 7.27 (s, 1H), 6.95 (s, 1H), 6.72 (d, 2H), 6.31 (t, 1H), 5.46 (s, 2H), 4.06 (s, 3H), 3.73 (s, 6H).

MS m/z (ESI): 460.9 [M+H]$^+$.

**Example 31**

Synthesis of Compound 25

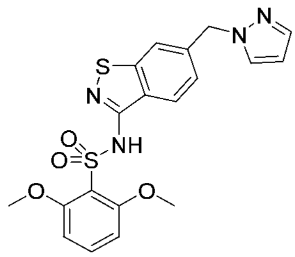

**25**

*N*-(6-((1*H*-Pyrazol-1-yl)methyl)benzo[*d*]isothiazol-3-yl)-2,6-dimethoxybenzenesulfonamide

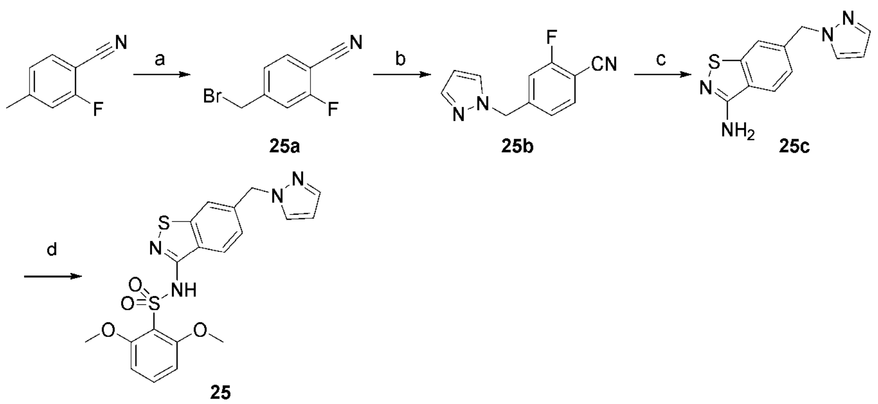


Step a:

4-(Bromomethyl)-2-fluorobenzonitrile

Dibenzoyl peroxide (0.09 g, 0.37 mmol) and N-bromosuccinimide (1.98 g, 11.12 mmol) were added to a solution of 2-fluoro-4-methylbenzonitrile (1.00 g, 7.40 mmol) in chloroform (20 mL). The reaction system was heated to 75 °C and stirred for 16 h. After the reaction was completed, the reaction mixture was diluted with water (100 mL) and extracted with dichloromethane (300 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 15:1) to give compound **25a** (0.78 g, yield: 49.2%).

$^{1}$H NMR (400 MHz, DMSO-$d_6$): $\delta$7.97-7.92 (m, 1H), 7.65 (d, 1H), 7.53-7.50 (m, 1H), 4.77 (s, 2H).

Step b:

4-((1*H*-Pyrazol-1-yl)methyl)-2-fluorobenzonitrile

Pyrazole (201 mg, 2.95 mmol) and cesium carbonate (963 mg, 2.96 mmol) were added to a solution of compound **25a** (575 mg, 2.69 mmol) in acetonitrile (15 mL) at 25 °C. The reaction system was stirred at 25 °C for 16 h. After the reaction was completed, the reaction mixture was diluted with water (50 mL) and extracted with dichloromethane (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 2:1) to give compound **25b** (522 mg, yield: 96.6%).
$^1$H NMR (400 MHz, DMSO-$d_6$): $\delta$7.92-7.89 (m, 2H), 7.53-7.52 (m, 1H), 7.27 (d, 1H), 7.15-7.12 (m, 1H), 6.33 (d, 1H), 5.48 (s, 2H).

Step c:

6-((1*H*-Pyrazol-1-yl)methyl)benzo[*d*]isothiazol-3-amine

Under a nitrogen atmosphere, sodium sulfide (27 mg, 0.35 mmol) was added to a solution of compound **25b** (70 mg, 0.35 mmol) in dimethyl sulfoxide (2 mL). The reaction system was heated to 70 °C and stirred for 7 h. After the reaction was completed, the reaction system was cooled to 0 °C. Subsequently, aqueous ammonia (1 mL) and an aqueous sodium hypochlorite solution (1 mL) were slowly added to the reaction system, and stirring was continued for 16 h. The reaction mixture was diluted with water (50 mL) and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:tetrahydrofuran = 8:1) to give compound **25c** (49 mg, yield: 61.1%).
$^1$HNMR (400 MHz, $CDCl_3$): $\delta$7.66 (d, 1H), 7.61-7.55 (m, 2H), 7.46 (d, 1H), 7.22 (d, 1H), 6.33 (t, 1H), 5.48 (s, 2H).
MS m/z (ESI): 231.2 $[M+H]^+$.

Step d:

*N*-(6-((1*H*-Pyrazol-1-yl)methyl)benzo[*d*]isothiazol-3-yl)-2,6-dimethoxybenzenesulfonamide

Sodium hydride (60%, 87 mg, 2.17 mmol) was added to a solution of compound **25c** (100 mg, 0.43 mmol) in *N*,*N*-dimethylformamide (3 mL) at 0 °C. The reaction system was stirred at 0 °C for 1 h. Then, 2,6-dimethoxybenzenesulfonyl chloride (206 mg, 0.87 mmol) was added, and stirring was continued for 1 h. After the reaction was completed, the reaction mixture was quenched with a saturated aqueous ammonium chloride solution and extracted with dichloromethane (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **25** (84 mg, yield: 44.9%).
$^1$HNMR (400 MHz, DMSO-$d_6$): $\delta$11.30 (s, 1H), 8.36 (d, 1H), 7.88 (d, 1H), 7.82 (s, 1H), 7.49 (d, 1H), 7.45 (t, 1H), 7.29 (d, 1H), 6.72 (s, 1H), 6.70 (s, 1H), 6.30 (t, 1H), 5.50 (s, 2H), 3.67 (s, 6H).
MS m/z (ESI): 430.9 $[M+H]^+$.

## Example 32

Synthesis of Compound 26

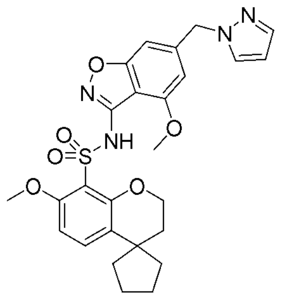

26

*N*-(6-((1*H*-Pyrazol-1-yl)methyl)-4-methoxybenzo[*d*]isoxazol-3-yl)-7-methoxyspiro[chromane-4,1'-cyclopentane]-8-sulfonamide

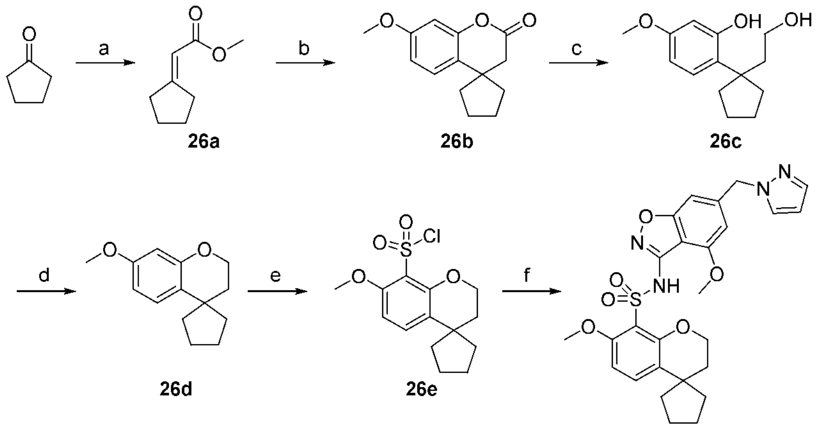


26a 26b 26c 26d 26e 26

Step a:

Methyl 2-cyclopentylideneacetate

Under a nitrogen atmosphere, sodium hydride (60%, 3.21 g, 80.25 mmol) was added to a solution of trimethyl phosphonoacetate (15.14 g, 83.14 mmol) in tetrahydrofuran (150 mL) at 0 °C, and the reaction system was stirred at 0 °C for 1 h. Then, a solution of cyclopentanone (5.00 g, 59.44 mmol) in tetrahydrofuran (50 mL) was added dropwise, and the reaction system was warmed to 25 °C and stirred for another 2 h. After the reaction was completed, the mixture was quenched with a saturated aqueous ammonium chloride solution and extracted with ethyl acetate (200 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 20:1) to give compound **26a** (3.66 g, yield: 43.9%).

$^1$H NMR (300 MHz, $CDCl_3$) $\delta$5.81-5.78 (m, 1H), 3.68 (s, 3H), 2.76 (t, 2H), 2.43 (t, 2H), 1.79-1.61 (m, 4H)

Step b:

7-Methoxyspiro[chromane-4,1'-cyclopentan]-2-one

3-Methoxyphenol (6.48 g, 52.20 mmol), compound **26a** (3.66 g, 26.11 mmol), and 98% concentrated sulfuric acid (0.51 g, 5.10 mmol) were mixed. The reaction system was then heated to 130 °C and stirred for 3 h. After the reaction was completed, the mixture was quenched with water and extracted with ethyl acetate (150 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 20:1) to give compound **26b** (1.90 g, yield: 31.3%).

$^1$H NMR (300 MHz, $CDCl_3$): $\delta$7.14 (d, 1H), 6.70-6.62 (m, 2H), 3.79 (s, 3H), 2.66 (s, 2H), 1.93-1.59 (m, 8H).

Step c:

2-(1-(2-Hydroxyethyl)cyclopentyl)-5-methoxyphenol

Under a nitrogen atmosphere, lithium aluminum hydride (0.27 g, 7.24 mmol) was added to a solution of compound **26b** (1.60 g, 6.89 mmol) in tetrahydrofuran (30 mL) at 0 °C, and the reaction system was stirred at 0 °C for 1 h. After the reaction was completed, the mixture was quenched with a saturated aqueous ammonium chloride solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 1:1) to give compound **26c** (1.60 g, yield: 98.3%).

$^1$H NMR (300 MHz, $CDCl_3$): 7.06 (d, 1H), 6.43-6.37 (m, 1H), 6.28 (d, 1H), 3.74 (s, 3H), 3.52 (t, 2H), 2.04-1.93 (m, 6H), 1.72-1.63 (m, 4H).

Step d:

7-Methoxyspiro[chromane-4,1'-cyclopentane]

Compound **26c** (1.60 g, 6.77 mmol) and p-toluenesulfonic acid (0.23 g, 1.34 mmol) were dissolved in a solution of toluene (32 mL). The reaction system was heated to 110 °C and stirred for 2 h. After the reaction was completed, the reaction mixture was concentrated under reduced pressure. The resulting crude product was purified by flash silica gel column chromatography (petroleum ether:tetrahydrofuran = 20:1) to give compound **26d** (1.26 g, yield: 85.2%).

$^1$H NMR (300 MHz, $CDCl_3$): $\delta$7.13 (d, 1H), 6.49 (dd, 1H), 6.33 (d, 1H), 4.17 (t, 2H), 3.75 (s, 3H), 1.93-1.69 (m, 10H).

Step e:

7-Methoxyspiro[chromane-4,1'-cyclopentane]-8-sulfonyl chloride

Under a nitrogen atmosphere, n-butyllithium (2.5 M in n-hexane, 2.5 mL, 6.25 mmol) was added dropwise to a solution of compound **26d** (1.26 g, 5.77 mmol) in tetrahydrofuran (25 mL) at 0 °C,
and the reaction system was stirred at 0 °C for 1 h and then cooled to -78 °C. Subsequently, sulfuryl chloride (1.01 g, 7.48 mmol) was added, and stirring was continued at - 78 °C for 15 min. After the reaction was completed, the mixture was quenched with a saturated aqueous ammonium chloride solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 2:1) to give compound **26e** (0.77 g, yield: 42.1%).

$^1$H NMR (400 MHz, $CDCl_3$): $\delta$7.45 (d, 1H), 6.57 (d, 1H), 4.37 (t, 2H), 3.94 (s, 3H), 1.88-1.77 (m, 10H).

Step f:

*N*-(6-((1*H*-Pyrazol-1-yl)methyl)-4-methoxybenzo[*d*]isoxazol-3-yl)-7-methoxyspiro[chromane-4,1'-cyclopentane]-8-sulfonamide

Sodium hydride (60%, 82 mg, 2.05 mmol) was added to a solution of **intermediate 1** (100 mg, 0.41 mmol) in *N*,*N*-dimethylformamide (2 mL) at 0 °C. The reaction system was stirred for 30 min. Then, compound **26e** (195 mg, 0.62 mmol) was added to the system, and stirring was continued at 0 °C for 1 h. After the reaction was completed, the mixture was diluted with a 1 N aqueous hydrochloric acid solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous formic acid solution, mobile phase B: acetonitrile; gradient ratio: phase B 5%-95%) to give compound 26 (105 mg, yield: 48.9%).

$^1$HNMR (400 MHz, DMSO-$d_6$): $\delta$9.47 (s, 1H), 7.87 (d, 1H), 7.50 (d, 1H), 7.44 (d, 1H), 6.85 (s, 1H), 6.76 (s, 1H), 6.69 (d, 1H), 6.30 (t, 1H), 5.45 (s, 2H), 4.07 (t, 2H), 3.87 (s, 3H), 3.72 (s, 3H), 1.77-1.60 (m, 10H).

MS m/z (ESI): 525.0 $[M+H]^+$.

**Example 33**

Synthesis of Compound 27

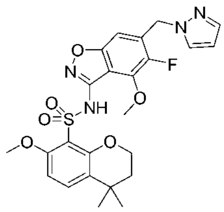

27

*N*-(6-((1*H*-Pyrazol-1-yl)methyl)-5-fluoro-4-methoxybenzo[*d*]isoxazol-3-yl)-7-methoxy-4,4-dimethylchromane-8-sulfonamide

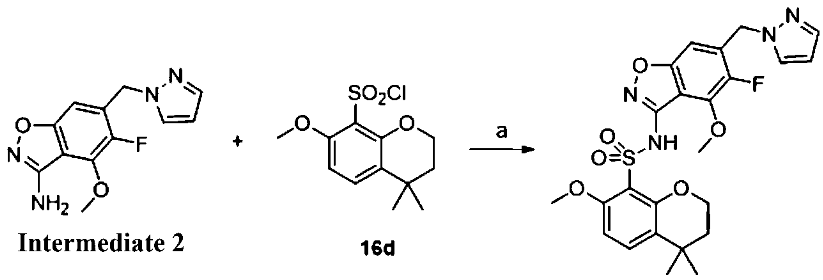


Intermediate 2 16d 27

Step a:

*N*-(6-((1*H*-Pyrazol-1-yl)methyl)-5-fluoro-4-methoxybenzo[*d*]isoxazol-3-yl)-7-methoxy-4,4-dimethylchromane-8-sulfonamide

Sodium hydride (60%, 61 mg, 1.52 mmol) was added to a solution of **intermediate 2** (80 mg, 0.31 mmol) in *N*,*N*-dimethylformamide (1 mL) at 0 °C. The reaction system was stirred at 0 °C for 15 min. Then, compound **16d** (133 mg, 0.46 mmol) was added to the system, and stirring was continued for 15 min. After the reaction was completed, the reaction mixture was warmed to room temperature, quenched with a 1 N aqueous hydrochloric acid solution, and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **27** (88 mg, yield: 55.8%).

$^1$HNMR (400 MHz, DMSO-$d_6$): $\delta$10.00 (s, 1H), 7.85 (d, 1H), 7.52-7.50 (m, 2H), 6.91 (d, 1H), 6.69 (d, 1H), 6.32-6.31 (m, 1H),5.50 (s, 2H), 4.10 (t, 2H), 4.03 (d, 3H),3.72 (s, 3H),1.70 (t, 2H),1.23 (s, 6H).

MS m/z (ESI): 517.1 [M+H]$^+$.

**Example 34**

Synthesis of Compound 28

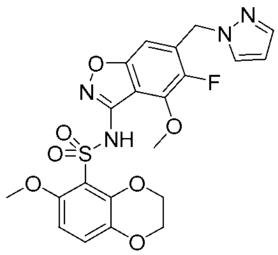

**28**

*N*-(6-((1*H*-Pyrazol-1-yl)methyl)-5-fluoro-4-methoxybenzo[*d*]isoxazol-3-yl)-6-methoxy-2,3-dihydrobenzo[*b*][1,4]dioxine-5-sulfonamide

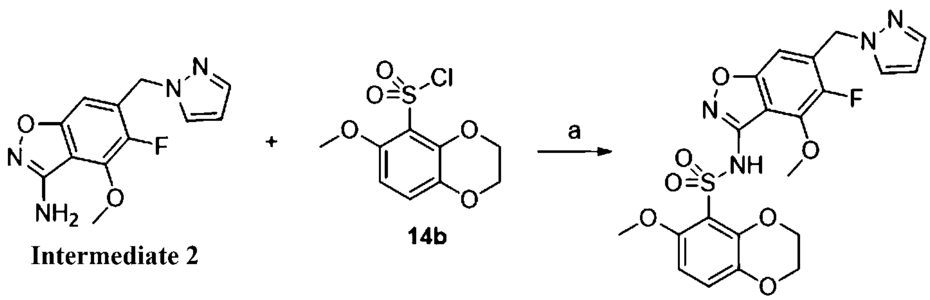


Intermediate 2

**14b**

**28**

Step a:

*N*-(6-((1*H*-Pyrazol-1-yl)methyl)-5-fluoro-4-methoxybenzo[*d*]isoxazol-3-yl)-6-methoxy-2,3-dihydrobenzo[*b*][1,4]dioxine-5-sulfonamide

Sodium hydride (60%, 61 mg, 1.52 mmol) was added to a solution of **intermediate 2** (80 mg, 0.31 mmol) in *N,N*-dimethylformamide (1 mL) at 0 °C. The reaction system was stirred at 0 °C for 15 min. Then, compound **14b** (121 mg, 0.46 mmol) was added to the system, and stirring was continued for 15 min. After the reaction was completed, the reaction mixture was warmed to room temperature, quenched with a 1 N aqueous hydrochloric acid solution, and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **28** (90 mg, yield: 60.2%).

$^1$HNMR (400 MHz, DMSO-$d_6$): $\delta$10.23 (s, 1H), 7.86 (d, 1H), 7.50 (d, 1H), 7.07 (d, 1H), 6.92 (d, 1H), 6.65 (d, 1H), 6.31-6.30 (m, 1H), 5.51 (s, 2H), 4.22-4.16 (m, 4H), 4.04 (d, 3H),3.70 (s, 3H).

MS m/z (ESI): 491.1 [M+H]$^+$.

**Example 35**

Synthesis of Compound 29

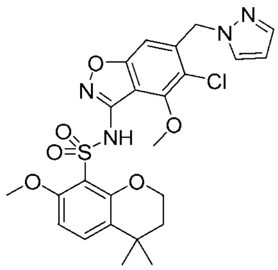

29

*N*-(6-((1*H*-Pyrazol-1-yl)methyl)-5-chloro-4-methoxybenzo[*d*]isoxazol-3-yl)-7-methoxy-4,4-dimethylchromane-8-sulfonamide

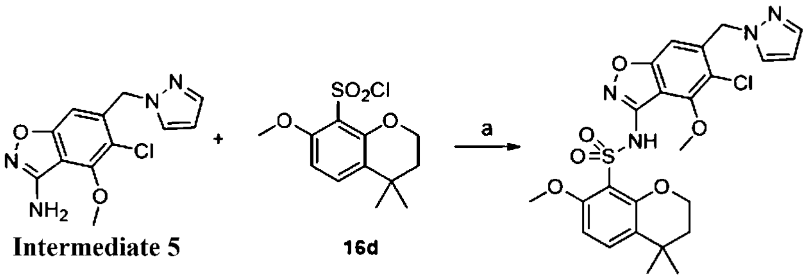


Intermediate 5

16d

29

Step a:

*N*-(6-((1*H*-Pyrazol-1-yl)methyl)-5-chloro-4-methoxybenzo[*d*]isoxazol-3-yl)-7-methoxy-4,4-dimethylchromane-8-sulfonamide

Sodium hydride (60%, 50 mg, 1.25 mmol) was added to a solution of **intermediate 5** (70 mg, 0.25 mmol) in *N*,*N*-dimethylformamide (1 mL) at 0 °C. The reaction system was stirred at 0 °C for 5 min. Then, compound **16d** (110 mg, 0.38 mmol) was added to the system, and stirring was continued for 10 min. After the reaction was completed, the reaction mixture was warmed to room temperature, quenched with a saturated aqueous ammonium chloride solution, and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **29** (89 mg, yield: 66.6%).

$^1$HNMR (400 MHz, DMSO-$d_6$): $\delta$10.30 (s, 1H), 7.88 (d, 1H), 7.54 (d, 1H), 7.52 (d, 1H), 6.79 (s, 1H), 6.70 (d, 1H), 6.34 (t, 1H), 5.57 (s, 2H), 4.12 (t, 2H), 3.91 (s, 3H), 3.72 (s, 3H), 1.71 (t, 2H), 1.25 (s, 6H).

MS m/z (ESI): 533.1 [M+H]$^+$.

**Example 36**

Synthesis of Compound 30

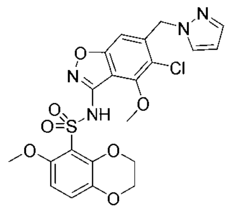

30

*N*-(6-((1*H*-Pyrazol-1-yl)methyl)-5-chloro-4-methoxybenzo[*d*]isoxazol-3-yl)-6-methoxy-2,3-dihydrobenzo[*b*][1,4]dioxine-5-sulfonamide

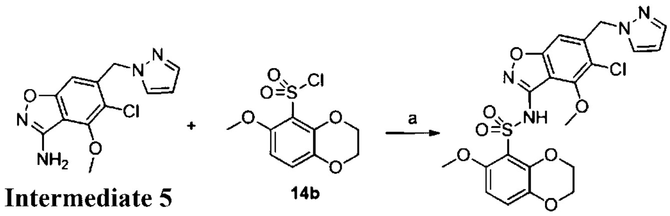


Intermediate 5

14b

30

Step a:

*N*-(6-((1*H*-Pyrazol-1-yl)methyl)-5-chloro-4-methoxybenzo[*d*]isoxazol-3-yl)-6-methoxy-2,3-dihydrobenzo[b][1,4]dioxine-5-sulfonamide

Sodium hydride (60%, 37 mg, 0.93 mmol) was added to a solution of **intermediate 5** (51 mg, 0.18 mmol) in *N*,*N*-dimethylformamide (1 mL) at 0 °C. The reaction system was stirred at 0 °C for 5 min. Then, compound **14b** (73 mg, 0.28 mmol) was added to the system, and stirring was continued for 10 min. After the reaction was completed, the reaction mixture was warmed to room temperature, quenched with a saturated aqueous ammonium chloride solution, and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **30** (63 mg, yield: 67.9%).
$^1$HNMR (400 MHz, DMSO-$d_6$): $\delta$10.53 (s, 1H), 7.88 (d, 1H), 7.55 (d, 1H), 7.08 (d, 1H), 6.79 (s, 1H), 6.67 (d, 1H), 6.34 (t, 1H), 5.57 (s, 2H), 5.20-5.16 (m, 4H), 3.94 (s, 3H), 3.71 (s, 3H).
MS m/z (ESI): 507.0 [M+H]$^+$.

**Example 37**

Synthesis of Compound 31

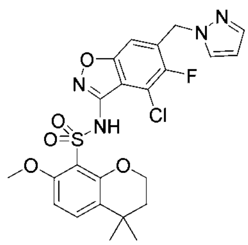

31

*N*-(6-((1*H*-Pyrazol-1-yl)methyl)-4-chloro-5-fluorobenzo[*d*]isoxazol-3-yl)-7-methoxy-4,4-dimethylchromane-8-sulfonamide

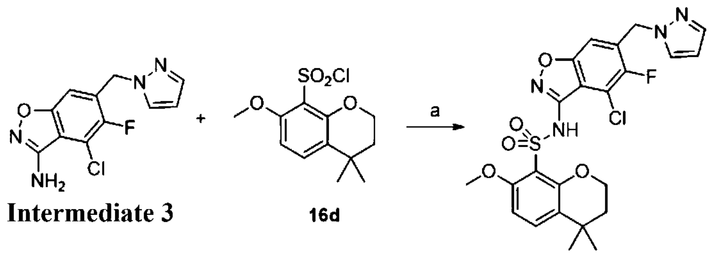


Intermediate 3 16d 31

Step a:

*N*-(6-((1*H*-Pyrazol-1-yl)methyl)-4-chloro-5-fluorobenzo[*d*]isoxazol-3-yl)-7-methoxy-4,4-dimethylchromane-8-sulfonamide

Sodium hydride (60%, 45 mg, 1.13 mmol) was added to a solution of **intermediate 3** (60 mg, 0.23 mmol) in *N*,*N*-dimethylformamide (1 mL) at 0 °C. The reaction system was stirred at 0 °C for 15 min. Then, compound **16d** (73 mg, 0.25 mmol) was added to the system, and stirring was continued for 15 min. After the reaction was completed, the reaction mixture was warmed to room temperature, quenched with a 1 N aqueous hydrochloric acid solution, and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous formic acid solution, mobile phase B: acetonitrile; gradient ratio: phase B 5%-95%) to give compound **31** (19 mg, yield: 16.2%).

$^1$HNMR (400 MHz, DMSO-$d_6$): $\delta$10.28 (s, 1H), 7.88 (d, 1H), 7.54-7.44 (m, 3H), 6.69 (d, 1H), 6.31 (t, 1H), 5.57 (s, 2H), 4.07 (t, 2H), 3.71 (s, 3H), 1.69 (t, 2H), 1.25 (s, 6H).

MS m/z (ESI): 521.1 $[M+H]^+$.

**Example 38**

Synthesis of Compound 32

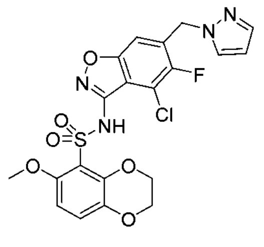

32

*N*-(6-((1*H*-Pyrazol-1-yl)methyl)-4-chloro-5-fluorobenzo[*d*]isoxazol-3-yl)-6-methoxy-2,3-dihydrobenzo[*b*][1,4]dioxine-5-sulfonamide

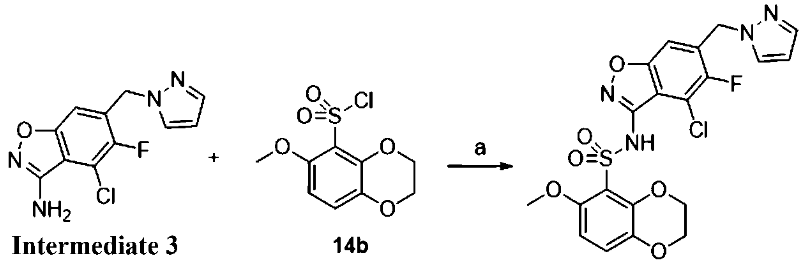


Intermediate 3 14b 32

Step a:

*N*-(6-((1*H*-Pyrazol-1-yl)methyl)-4-chloro-5-fluorobenzo[*d*]isoxazol-3-yl)-6-methoxy-2,3-dihydrobenzo[*b*][1,4]dioxine-5-sulfonamide

Sodium hydride (60%, 45 mg, 1.13 mmol) was added to a solution of **intermediate 3** (60 mg, 0.23 mmol) in *N*,*N*-dimethylformamide (1 mL) at 0 °C. The reaction system was stirred at 0 °C for 15 min. Then, compound **14b** (89 mg, 0.34 mmol) was added to the system, and stirring was continued for 15 min. After the reaction was completed, the reaction mixture was warmed to room temperature, quenched with a 1 N aqueous hydrochloric acid solution, and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous formic acid solution, mobile phase B: acetonitrile; gradient ratio: phase B 5%-95%) to give compound **32** (33 mg, yield: 29.6%).

$^1$HNMR (400 MHz, DMSO-$d_6$): $\delta$10.48 (s, 1H), 7.88 (d, 1H), 7.50 (d, 1H), 7.44 (d, 1H), 7.08 (d, 1H), 6.66 (d, 1H), 6.31 (t, 1H), 5.57 (s, 2H), 4.20-4.16 (m, 4H), 3.68 (s, 3H).

MS m/z (ESI): 495.0 [M+H]$^+$.

**Example 39**

Synthesis of Compound 33

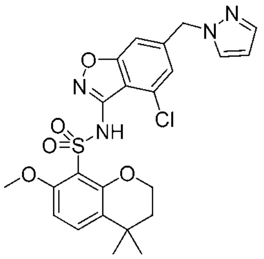

33

*N*-(6-((1*H*-Pyrazol-1-yl)methyl)-4-chlorobenzo[*d*]isoxazol-3-yl)-7-methoxy-4,4-dimethylchromane-8-sulfonamide

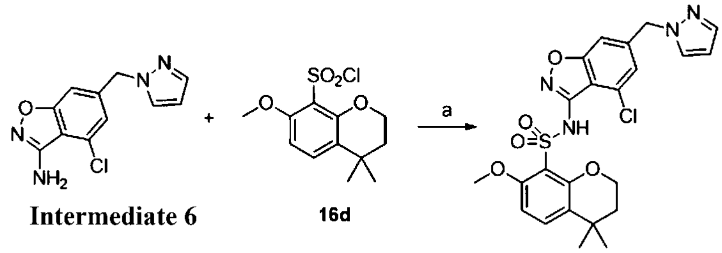


Intermediate 6 16d 33

Step a:

*N*-(6-((1*H*-Pyrazol-1-yl)methyl)-4-chlorobenzo[*d*]isoxazol-3-yl)-7-methoxy-4,4-dimethylchromane-8-sulfonamide

Sodium hydride (60%, 48 mg, 1.20 mmol) was added to a solution of **intermediate 6** (56 mg, 0.23 mmol) in N,N-dimethylformamide (1 mL) at 0 °C. The reaction system was stirred at 0 °C for 15 min. Then, compound **16d** (105 mg, 0.36 mmol) was added to the system, and stirring was continued for 15 min. After the reaction was completed, the reaction mixture was warmed to room temperature, quenched with water, and extracted with ethyl acetate (30 mL × 2). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **33** (79 mg, yield: 69.7%).

$^1$HNMR (400 MHz, DMSO-$d_6$): $\delta$10.09 (s, 1H), 7.90 (d, 1H), 7.52-7.45 (m, 3H), 7.25 (s, 1H), 6.68 (d, 1H), 6.31 (t, 1H), 5.50 (s, 2H), 4.06 (t, 2H), 3.70 (s, 3H), 1.68 (t, 2H), 1.24 (s, 6H).

MS m/z (ESI): 503.1 [M+H]$^+$.

**Example 40**

Synthesis of Compound 34

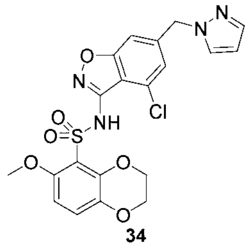

34

*N*-(6-((1*H*-Pyrazol-1-yl)methyl)-4-chlorobenzo[*d*]isoxazol-3-yl)-6-methoxy-2,3-dihydrobenzo[b][1,4]dioxine-5-sulfonamide

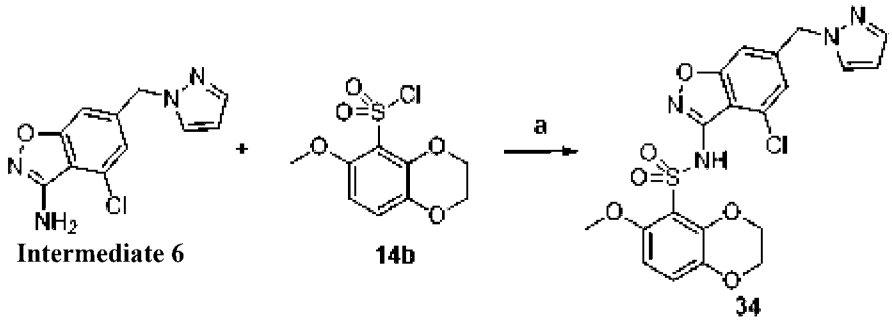


Intermediate 6 14b 34

Step a:

*N*-(6-((1*H*-Pyrazol-1-yl)methyl)-4-chlorobenzo[*d*]isoxazol-3-yl)-6-methoxy-2,3-dihydrobenzo[*b*][1,4]dioxine-5-sulfonamide

Sodium hydride (60%, 48 mg, 1.20 mmol) was added to a solution of **intermediate 6** (56 mg, 0.23 mmol) in *N*,*N*-dimethylformamide (1 mL) at 0 °C. The reaction system was stirred at 0 °C for 15 min. Then, compound 14b (96 mg, 0.36 mmol) was added to the system, and stirring was continued for 15 min. After the reaction was completed, the reaction mixture was warmed to room temperature, quenched with water, and extracted with ethyl acetate (30 mL × 2). The organic

layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **34** (52 mg, yield: 48.4%).

$^1$HNMR (400 MHz, DMSO-$d_6$): $\delta$10.31(s, 1H), 7.91 (d, 1H), 7.52 (d, 1H), 7.47 (s, 1H), 7.27 (s, 1H), 7.07 (d, 1H), 6.66 (d, 1H), 6.32 (t, 1H), 5.51 (s, 2H), 4.20-4.15 (m, 4H), 3.68 (s, 3H).

MS m/z (ESI): 477.0 [M+H]$^+$.

## Example 41

Synthesis of Compound 35

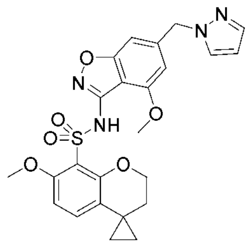

**35**

*N*-(6-((1*H*-Pyrazol-1-yl)methyl)-4-methoxybenzo[*d*]isoxazol-3-yl)-7-methoxyspiro[chromane-4,1'-cyclopropane]-8-sulfonamide

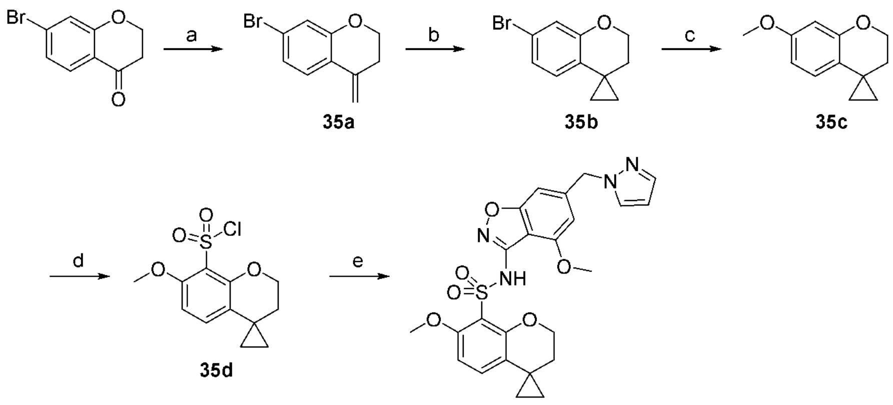


**35a** **35b** **35c** **35d** **35**

Step a:

7-Bromo-4-methylenechromane

Under a nitrogen atmosphere, at 0 °C, n-butyllithium (2.5 M in n-hexane, 13.74 mL, 34.35 mmol) was slowly added dropwise to a solution of methyltriphenylphosphonium bromide (12.59 g, 35.24 mmol) in tetrahydrofuran (80 mL), and the reaction system was stirred for 1 h. Then, a solution of 7-bromo-2,3-dihydrochromen-4-one (4.00 g, 17.62 mmol) in tetrahydrofuran (20 mL) was added dropwise to the system, and stirring was continued for 1 h. After the reaction was completed, the reaction mixture was quenched with a saturated aqueous ammonium chloride solution and extracted with ethyl acetate (200 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether) to give compound **35a** (2.43 g,

yield: 61.3%).

$^1$HNMR (400 MHz, DMSO-$d_6$): $\delta$7.60 (d, 1H), 7.08-7.05 (m, 2H), 5.63 (s, 1H), 4.99 (d, 1H), 4.19 (t, 2H), 2.64 (t, 2H).

Step b:

7-Bromospiro[chromane-4,1'-cyclopropane]

Under a nitrogen atmosphere, diiodomethane (11.57 g, 43.18 mmol) was slowly added dropwise to a solution of diethylzinc (1.0 M in *n*-hexane, 21.59 mL, 21.59 mmol) in dichloromethane (40 mL) at -78 °C, and the reaction system was warmed to 0 °C and stirred for 20 min. At 0 °C, trifluoroacetic acid (3.69 g, 32.39 mmol) was then added to the reaction system, and stirring was continued for 15 min. Then, a solution of compound **35a** (2.43 g, 10.80 mmol) in dichloromethane (12 mL) was added dropwise. After the reaction was completed, the reaction mixture was quenched with a saturated aqueous sodium bicarbonate solution and extracted with dichloromethane (150 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 99:1) to give compound **35b** (1.60 g, yield: 62.0%).

$^1$HNMR (400 MHz, $CDCl_3$): $\delta$6.97-6.91 (m, 2H), 6.50 (d, 1H), 4.27 (t, 2H), 1.84 (t, 2H), 1.04-1.01 (m, 2H), 0.86-0.83 (m, 2H).

Step c:

7-Methoxyspiro[chromane-4,1'-cyclopropane]

Sodium methoxide (30% in methanol, 16.26 g, 90.34 mmol) was added to a solution of compound **35b** (1.80 g, 7.53 mmol) and copper(I) bromide (0.43 g, 3.01 mmol) in *N,N*-dimethylformamide (10 mL). Subsequently, the reaction system was heated to 100 °C and stirred for 4 h. After the reaction was completed, the reaction mixture was quenched with an aqueous solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 96:4) to give compound **35c** (1.40 g, yield: 97.8%).

$^1$HNMR (400 MHz, DMSO-$d_6$): $\delta$6.61 (d, 1H), 6.38 (dd, 1H), 6.31 (d, 1H), 4.19 (t, 2H), 3.66 (s, 3H), 1.77 (t, 2H), 0.93-0.91 (m, 2H), 0.80-0.77 (m, 2H).

Step d:

7-Methoxyspiro[chromane-4,1'-cyclopropane]-8-sulfonyl chloride

Under a nitrogen atmosphere, at 0 °C, n-butyllithium (2.5 M in *n*-hexane, 0.64 mL, 1.16 mmol) was slowly added dropwise to a solution of compound **35c** (0.20 g, 1.05 mmol) in tetrahydrofuran (4 mL), and the reaction system was stirred for 1 h. Then, sulfuryl chloride (213 mg, 1.58 mmol) was added dropwise to the system, and stirring was continued for 30 min. After the reaction was completed, the reaction mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 3:1) to give compound **35d** (60 mg, yield: 19.8%).

$^1$HNMR (400 MHz, $CDCl_3$): $\delta$6.86 (d, 1H), 6.50 (d, 1H), 4.49 (t, 2H), 3.92 (s, 3H), 1.89 (t, 2H), 1.03-1.00 (m, 2H), 0.90-0.87 (m, 2H).

Step e:

*N*-(6-((1*H*-Pyrazol-1-yl)methyl)-4-methoxybenzo[*d*]isoxazol-3-yl)-7-methoxyspiro[chromane-4,1'-cyclopropane]-8-sulfonamide

Sodium hydride (60%, 41 mg, 1.03 mmol) was added to a solution of **intermediate 1** (50 mg, 0.20 mmol) in *N,N*-dimethylformamide (1 mL) at 0 °C. The reaction system was stirred at 0 °C for 15 min. Then, compound **35d** (59 mg, 0.20 mmol) was added to the system, and stirring was continued for 30 min. After the reaction was completed, the reaction mixture was warmed to room temperature, quenched with a 1 N aqueous hydrochloric acid solution, and extracted with

ethyl acetate (30 mL × 2). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous formic acid solution, mobile phase B: acetonitrile; gradient ratio: phase B 5%-95%) to give compound **35** (22 mg, yield: 21.6%).

$^1$HNMR (400 MHz, DMSO-$d_6$): $\delta$7.87 (d, 1H), 7.50 (d, 1H), 6.88 (d, 1H), 6.82 (s, 1H), 6.75 (s, 1H), 6.60 (d, 1H), 6.30 (t, 1H), 5.45 (s, 2H), 4.17 (t, 2H), 3.88 (s, 3H), 3.70 (s, 3H), 1.70 (t, 2H), 0.96-0.94 (m, 2H), 0.82-0.79 (m, 2H).

MS m/z (ESI): 497.1 [M+H]$^+$.

## Example 42

Synthesis of Compound 36

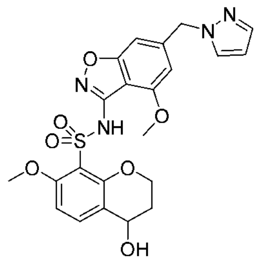

**36**

*N*(6-((1*H*Pyrazol-1-yl)methyl)-4-methoxybenzo[*d*]isoxazol-3-yl)-4-hydroxy-7-methoxychromane-8-sulfonamide

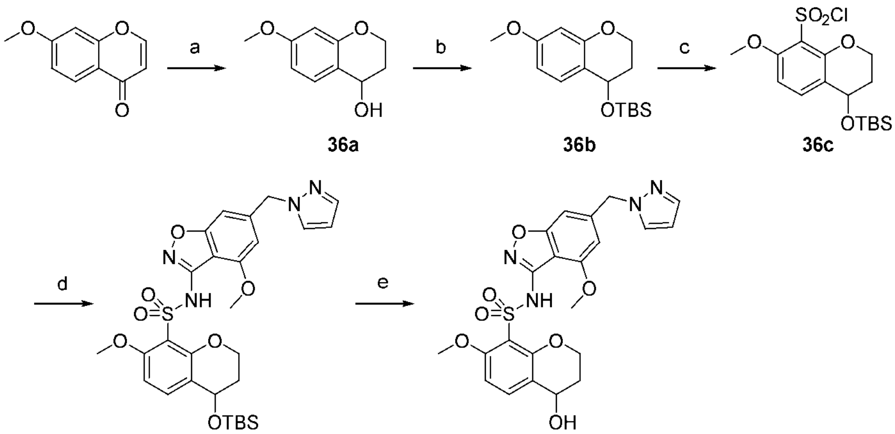


**36a** **36b** **36c**

**36d** **36**

Step a:

7-Methoxychroman-4-ol

Under a hydrogen atmosphere, palladium on carbon (900 mg, 10% w/w) was added to a solution of 7-methoxy-4*H*-chromen-4-one (9.10 g, 51.70 mmol) in methanol (100 mL), and the reaction system was stirred at 25 °C for 16 h. After the reaction was completed, the reaction mixture was filtered under reduced pressure, and the filtrate was concentrated under reduced pressure. The resulting crude product was purified by flash silica gel column chromatography (petroleum ether:ethyl acetate = 84:16) to give compound **36a** (7.90 g, yield: 84.9%).

Step b:

*tert*-Butyl((7-methoxychroman-4-yl)oxy)dimethylsilane

*tert*-Butyldimethylsilyl chloride (1.25 g, 8.32 mmol) was added to a solution of compound **36a** (1.00 g, 5.55 mmol) and 1*H*-imidazole (604 mg, 8.88 mmol) in *N,N*-dimethylformamide (20 mL). The reaction system was stirred for 16 h. After the reaction was completed, the reaction mixture was quenched with an aqueous solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 91:9) to give compound **36b** (1.40 g, yield: 85.7%).

$^1$HNMR (400 MHz, DMSO-$d_6$): $\delta$7.08 (d, 1H), 6.48 (dd, 1H), 6.31 (d, 1H), 4.78 (t, 1H), 4.21-4.16 (m, 2H), 3.70 (s, 3H), 2.04-1.96 (m, 1H), 1.85-1.79 (m, 1H), 0.87 (s, 9H), 0.15 (s, 3H), 0.12 (s, 3H).

Step c:

4-((*tert*-Butyldimethylsilyl)oxy)-7-methoxychromane-8-sulfonyl chloride

Under a nitrogen atmosphere, at 0 °C, *n*-butyllithium (2.5 M in *n*-hexane, 4.89 mL, 12.23 mmol) was slowly added dropwise to a solution of compound **36b** (3.00 g, 10.19 mmol) in tetrahydrofuran (60 mL), and the reaction system was stirred for 1 h. Then, the reaction system was cooled to -78 °C. Subsequently, sulfuryl chloride (1.65 g, 12.22 mmol) was added dropwise to the system, and stirring was continued for 15 min. After the reaction was completed, the reaction mixture was quenched with a saturated aqueous ammonium chloride solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 85:15) to give compound **36c** (1.40 g, yield: 35.0%).

$^1$HNMR (400 MHz, $CDCl_3$): δ7.43 (d, 1H), 6.58 (d, 1H), 4.75 (t, 1H), 4.48-4.45 (m, 2H), 3.96 (s, 3H), 2.12-2.08 (m, 1H), 2.07-1.97 (m, 1H), 0.90 (s, 9H), 0.16 (s, 3H), 0.14 (s, 3H).

Step d:

*N*-(6-((1*H*-Pyrazol-1-yl)methyl)-4-methoxybenzo[*d*]isoxazol-3-yl)-4-((*tert*-butyldimethylsilyl)oxy)-7-methoxychromane-8-sulfonamide

Sodium hydride (60%, 491 mg, 12.28 mmol) was added to a solution of **intermediate 1** (600 mg, 2.46 mmol) in *N*,*N*-dimethylformamide (3 mL) at 0 °C. The reaction system was stirred at 0 °C for 15 min. Then, compound **36c** (1.35 g, 3.44 mmol) was added to the system, and stirring was continued for 30 min. After the reaction was completed, the reaction mixture was warmed to room temperature, adjusted to pH = 6 with a 1 N aqueous hydrochloric acid solution, and extracted with ethyl acetate (100 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:tetrahydrofuran = 4:1) to give compound **36d** (680 mg, yield: 46.1%).

MS m/z (ESI): 601.1 $[M+H]^+$.

Step e:

*N*-(6-((1*H*-Pyrazol-1-yl)methyl)-4-methoxybenzo[*d*]isoxazol-3-yl)-4-hydroxy-7-methoxychromane-8-sulfonamide

Tetrabutylammonium fluoride trihydrate (70 mg, 0.25 mmol) was added to a solution of compound **36d** (50 mg, 0.08 mmol) in tetrahydrofuran (5 mL). The reaction system was stirred for 16 h. After the reaction was completed, the reaction mixture was quenched with a saturated aqueous ammonium chloride solution and extracted with ethyl acetate (20 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **36** (30 mg, yield: 74.1%).

$^1$HNMR (400 MHz, DMSO-$d_6$): δ7.87 (d, 1H), 7.50 (d, 1H), 7.42 (d, 1H), 6.81 (s, 1H), 6.75 (s, 1H), 6.70 (d, 1H), 6.30 (t, 1H), 5.42 (s, 2H), 5.37 (d, 1H), 4.57-4.52 (m, 1H), 4.21-4.15 (m, 1H) 4.12-4.07 (m, 1H), 3.89 (s, 3H), 3.73 (s, 3H), 1.87-1.74 (m, 2H).

MS m/z (ESI): 487.0 [M+H]+.

**Example 43**

Synthesis of Compound 37

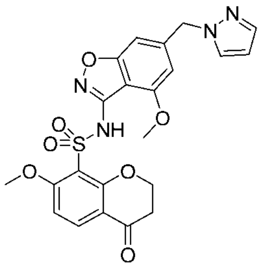

**37**

*N*-(6-((1*H*-Pyrazol-1-yl)methyl)-4-methoxybenzo[*d*]isoxazol-3-yl)-7-methoxy-4-oxochromane-8-sulfonamide

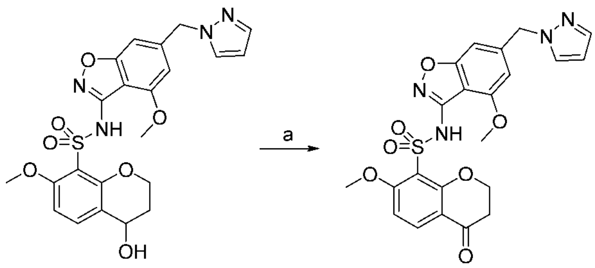


**36** **37**

Step a:

*N*-(6-((1*H*-Pyrazol-1-yl)methyl)-4-methoxybenzo[*d*]isoxazol-3-yl)-7-methoxy-4-oxochromane-8-sulfonamide

Manganese dioxide (26.8 mg, 0.31 mmol) was added to a solution of compound **36** (50 mg, 0.10 mmol) in chloroform (5 mL). The reaction system was heated to 60 °C and stirred for 16 h. After the reaction was completed, the reaction mixture was cooled to room temperature and filtered under reduced pressure, and the filtrate was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound 37 (2.9 mg, yield: 5.8%).
$^1$HNMR (400 MHz, DMSO-$d_6$): $\delta$10.24 (s, 1H), 7.95 (d, 1H), 7.87 (d, 1H), 7.50-7.49 (m, 1H), 6.92 (d, 1H), 6.82 (s, 1H), 6.73 (s, 1H), 6.30 (t, 1H), 5.44 (s, 2H), 4.47 (t, 2H), 3.85 (s, 3H), 3.83 (s, 3H), 2.72 (t, 2H).
MS m/z (ESI): 485.1 [M+H]+.

**Example 44**

Synthesis of Compound 38

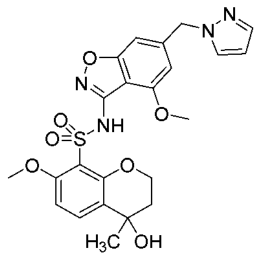

**38**

*N*-(6-((1*H*-Pyrazol-1-yl)methyl)-4-methoxybenzo[*d*]isoxazol-3-yl)-4-hydroxy-7-methoxy-4-methylchromane-8-sulfonamide

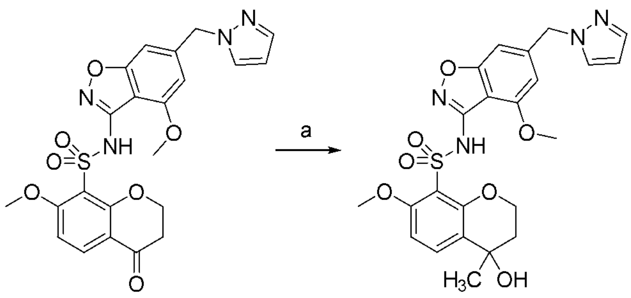


**37** **38**

Step a:

*N*-(6-((1*H*-Pyrazol-1-yl)methyl)-4-methoxybenzo[*d*]isoxazol-3-yl)-4-hydroxy-7-methoxy-4-methylchromane-8-sulfonamide

Under a nitrogen atmosphere, methylmagnesium bromide (1.0 M in tetrahydrofuran, 3.1 mL, 3.10 mmol) was added to a solution of compound **37** (150 mg, 0.31 mmol) in tetrahydrofuran (10 mL) at 0 °C, and the reaction system was stirred at 0 °C for 1 h. After the reaction was completed, the reaction mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **38** (60 mg, yield: 38.7%).

$^1$HNMR (400 MHz, DMSO-*d*$_6$): $\delta$9.39 (s, 1H), 7.87 (d, 1H), 7.61 (d, 1H), 7.50 (d, 1H), 6.82 (s, 1H), 6.76 (s, 1H), 6.71 (d, 1H), 6.30 (t, 1H), 5.45 (s, 2H), 5.18 (s, 1H), 4.18-4.13 (m, 2H), 3.88 (s, 3H), 3.73 (s, 3H), 1.87-1.81(m, 2H), 1.39 (s, 3H).

MS m/z (ESI): 501.1 [M+H]$^+$.

**Example 45**

Synthesis of Compound 39

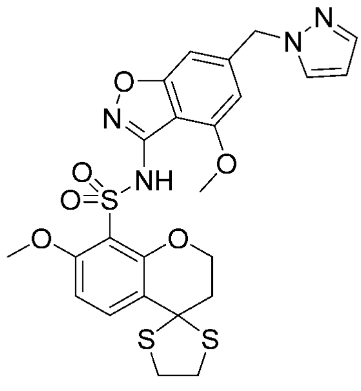

**39**

*N*-(6-((1*H*-Pyrazol-1-yl)methyl)-4-methoxybenzo[*d*]isoxazol-3-yl)-7-methoxyspiro[chromane-4,2'-[1,3]dithiolane]-8-sulfonamide

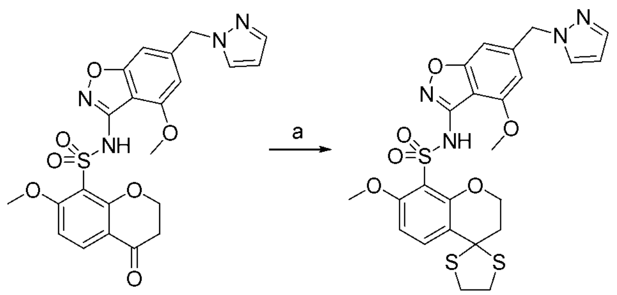


**37** **39**

Step a:

*N*-(6-((1*H*-Pyrazol-1-yl)methyl)-4-methoxybenzo[*d*]isoxazol-3-yl)-7-methoxyspiro[chromane-4,2'-[1,3]dithiolane]-8-sulfonamide

1,2-Ethanedithiol (12 mg, 0.12 mmol) and boron trifluoride diethyl etherate (18 mg, 0.12 mmol) were added to a solution of compound 37 (30 mg, 0.06 mmol) in dichloromethane (4 mL) at 30 °C. The reaction system was stirred at 30 °C for 16 h. After the reaction was completed, the reaction mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **39** (8.9 mg, yield: 25.6%).

$^1$HNMR (400 MHz, DMSO-$d_6$): δ9.77 (s, 1H), 7.94 (d, 1H), 7.87 (d, 1H), 7.50 (d, 1H), 6.85 (s, 1H), 6.79-6.75 (m, 2H), 6.30 (t, 1H), 5.45 (s, 2H), 4.20-4.19 (m, 2H), 3.84 (s, 3H), 3.74 (s, 3H), 3.64-3.57 (m, 2H), 3.51-3.45 (m, 2H), 2.41-2.38 (m, 2H).

MS m/z (ESI): 561.1 [M+H]$^+$.

**Example 46**

Synthesis of Compound 40

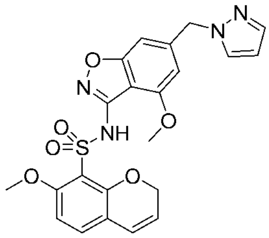

**40**

*N*-(6-((1*H*-Pyrazol-1-yl)methyl)-4-methoxybenzo[*d*]isoxazol-3-yl)-7-methoxy-2*H*-chromene-8-sulfonamide

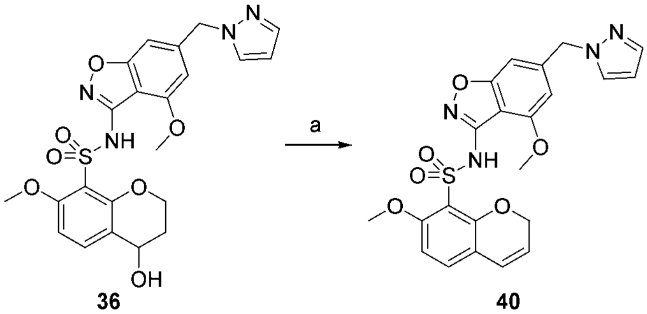


**36** **40**

Step a:

*N*-(6-((1*H*-Pyrazol-1-yl)methyl)-4-methoxybenzo[*d*]isoxazol-3-yl)-7-methoxy-2*H*-chromene-8-sulfonamide

Burgess reagent (88 mg, 0.37 mmol) was added to a solution of compound **36** (60 mg, 0.12 mmol) in toluene (2 mL). The reaction system was heated to 80 °C and stirred for 3 h. After the reaction was completed, the reaction mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **40** (4.3 mg, yield: 7.4%).

$^1$HNMR (400 MHz, DMSO-$d_6$): δ7.87 (d, 1H), 7.50 (d, 1H), 7.17 (d, 1H), 6.79 (s, 1H), 6.71 (s, 1H), 6.63 (d, 1H), 6.45-6.43 (m, 1H), 6.30 (t, 1H), 5.78-5.74 (m, 1H), 5.44 (s, 2H), 4.63-4.61 (m, 2H), 3.86 (s, 3H), 3.72 (s, 3H).

MS m/z (ESI): 469.1 [M+H]$^+$.

**Example 47**

Synthesis of Compound 41

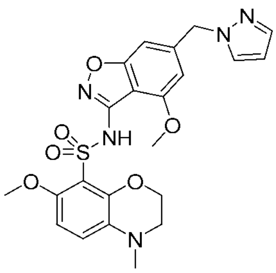

**41**

*N*-(6-((1*H*-Pyrazol-1-yl)methyl)-4-methoxybenzo[*d*]isoxazol-3-yl)-7-methoxy-4-methyl-3,4-dihydro-2*H*-benzo[*b*][1,4]oxazine-8-sulfonamide

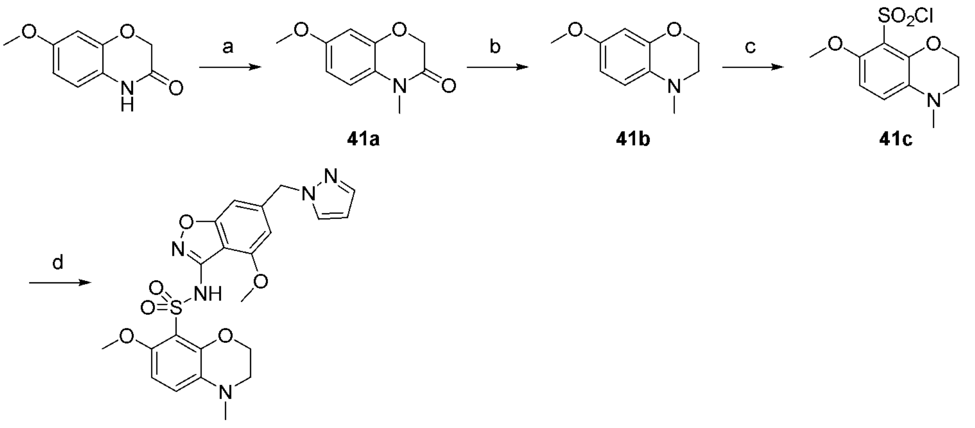


41a 41b 41c

41

Step a:

7-Methoxy-4-methyl-2*H*-benzo[*b*][1,4]oxazin-3(4*H*)-one

Under a nitrogen atmosphere, sodium hydride (60%, 0.58 g, 14.50 mmol) was added to a solution of 7-methoxy-2*H*-benzo[*b*][1,4]oxazin-3(4*H*)-one (1.84 g, 10.27 mmol) in *N,N*-dimethylformamide (30 mL) at 0 °C, and the reaction system was stirred at 0 °C for 30 min. Then, iodomethane (1.61 g, 11.34 mmol) was added to the system, and stirring was continued for 3 h. After the reaction was completed, the reaction mixture was warmed to room temperature, quenched with ice water (50 mL), and extracted with ethyl acetate (100 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **41a** (1.86 g, yield: 93.7%).

$^1$HNMR (400 MHz, DMSO-$d_6$): $\delta$7.06 (d, 1H), 6.66-6.61 (m, 2H), 4.61 (s, 2H), 3.72 (s, 3H), 3.24 (s, 3H).

Step b:

7-Methoxy-4-methyl-3,4-dihydro-2*H*-benzo[*b*][1,4]oxazine

Under a nitrogen atmosphere, a borane-dimethyl sulfide complex solution (19 mL) was added to a solution of compound **41a** (1.86 g, 9.63 mmol) in tetrahydrofuran (40 mL) at 30 °C, and the reaction system was heated to 75 °C, stirred for 3 h, and then cooled to 0 °C. The reaction mixture was quenched with ice water (40 mL) and extracted with ethyl acetate (100 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 6:1) to give compound **41b** (1.54 g, yield: 89.3%).

$^1$HNMR (400 MHz, $CDCl_3$): $\delta$6.62 (d, 1H), 6.45-6.41 (m, 2H), 4.32-4.30 (m, 2H), 3.73 (s, 3H), 3.18-3.15 (m, 2H), 2.82 (s, 3H).

Step c:

7-Methoxy-4-methyl-3,4-dihydro-2*H*-benzo[b][1,4]oxazine-8-sulfonyl chloride

Under a nitrogen atmosphere, *n*-butyllithium (2.5 M in *n*-hexane, 0.67 mL, 1.68 mmol) was added dropwise to a solution of compound **41b** (200 mg, 1.16 mmol) in tetrahydrofuran (6 mL) at 0 °C, and the reaction system was stirred at 0 °C for 1 h and then cooled to -78 °C. Subsequently, sulfuryl chloride (181 mg, 1.34 mmol) was added, and stirring was continued at - 78 °C for 15 min. After the reaction was completed, the mixture was quenched with a saturated aqueous ammonium chloride solution and extracted with ethyl acetate (20 mL × 3). The organic layers were combined, washed with

a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 4:1) to give compound **41c** (80 mg, yield: 25.8%).

$^1$HNMR (400 MHz, $CDCl_3$): $\delta$6.87 (d, 1H), 6.53 (d, 1H), 4.49-4.47 (m, 2H), 3.89 (s, 3H), 3.29-3.27 (m, 2H), 2.88 (s, 3H).

Step d:

*N*-(6-((1*H*-Pyrazol-1-yl)methyl)-4-methoxybenzo[*d*]isoxazol-3-yl)-7-methoxy-4-methyl-3,4-dihydro-2*H*-benzo[*b*][1,4]oxazine-8-sulfonamide

Sodium hydride (60%, 49 mg, 1.23 mmol) was added to a solution of **intermediate 1** (60 mg, 0.25 mmol) in *N*,*N*-dimethylformamide (1 mL) at 0 °C. The reaction system was stirred at 0 °C for 15 min. Then, compound **41c** (82 mg, 0.30 mmol) was added to the system, and stirring was continued for 15 min. After the reaction was completed, the mixture was warmed to room temperature. A 1 N aqueous hydrochloric acid solution (20 mL) was added to the reaction mixture, and the resulting mixture was extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **41** (9.2 mg, yield: 7.7%).

$^1$HNMR (400 MHz, DMSO-$d_6$): $\delta$7.87 (d, 1H), 7.50 (d, 1H), 6.84-6.81 (m, 2H), 6.74 (s, 1H), 6.57 (d, 1H), 6.30 (t, 1H), 5.44 (s, 2H), 4.16 (t, 2H), 3.89 (s, 3H), 3.65 (s, 3H), 3.10 (t, 2H), 2.76 (s, 3H).

MS m/z (ESI): 486.1 [M+H]$^+$.

**Example 48**

Synthesis of Compound 42

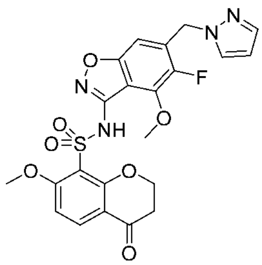

**42**

*N*-(6-((1*H*-Pyrazol-1-yl)methyl)-5-fluoro-4-methoxybenzo[*d*]isoxazol-3-yl)-7-methoxy-4-oxochromane-8-sulfonamide

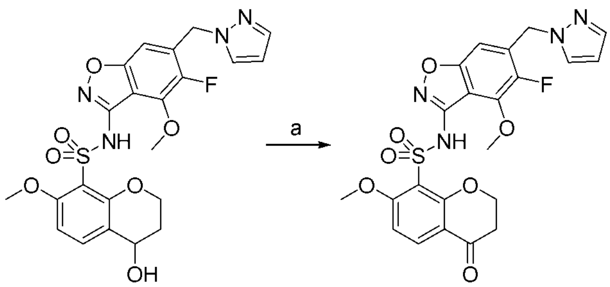


**107** **42**

Step a:

*N*-(6-((1*H*-Pyrazol-1-yl)methyl)-5-fluoro-4-methoxybenzo[*d*]isoxazol-3-yl)-7-methoxy-4-oxochromane-8-sulfonamide

Manganese dioxide (258.5 mg, 2.97 mmol) was added to a solution of compound **107** (100 mg, 0.20 mmol) in chloroform (10 mL). The reaction system was heated to 60 °C and stirred for 16 h. After the reaction was completed, the reaction mixture was cooled to room temperature and filtered under reduced pressure, and the filtrate was concentrated under reduced pressure. The resulting crude product was purified by high-performance liquid chromatography (Welchrom C18, 20 × 150 mm, 5 μm; mobile phase A: 5 mM aqueous formic acid solution, mobile phase B: acetonitrile; gradient ratio: phase B 5%-95%) to give compound **42** (80 mg, yield: 80.3%).

$^1$HNMR (400 MHz, DMSO-$d_6$): $\delta$10.64 (s, 1H), 7.96 (s, 1H), 7.85 (d, 1H), 7.49 (d, 1H), 6.93-6.91 (m, 2H), 6.30 (t, 1H), 5.50 (s, 2H), 4.49-4.47 (m, 2H), 4.04 (s, 3H), 3.85 (s, 3H), 2.67 (t, 2H).

MS m/z (ESI): 503.1 [M+H]$^+$.

**Example 49**

Synthesis of Compound 43

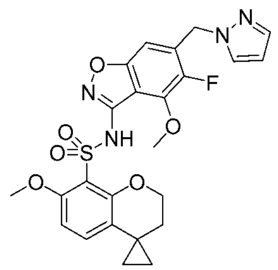

**43**

*N*-(6-((1*H*-Pyrazol-1-yl)methyl)-5-fluoro-4-methoxybenzo[*d*]isoxazol-3-yl)-7-methoxyspiro[chromane-4,1'-cyclopropane]-8-sulfonamide

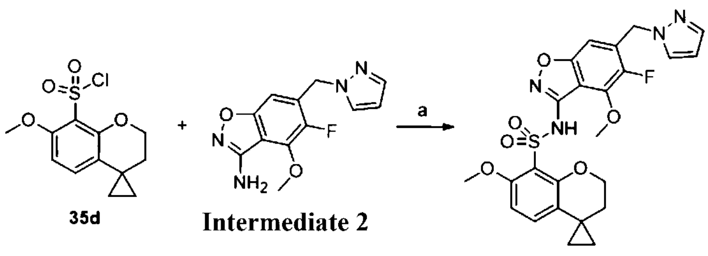


Step a:

*N*-(6-((1*H*-Pyrazol-1-yl)methyl)-5-fluoro-4-methoxybenzo[*d*]isoxazol-3-yl)-7-methoxyspiro[chromane-4,1'-cyclopropane]-8-sulfonamide

Sodium hydride (60%, 38 mg, 0.95 mmol) was added to a solution of **intermediate 2** (50 mg, 0.19 mmol) in *N*,*N*-dimethylformamide (1 mL) at 0 °C. The reaction system was stirred at 0 °C for 15 min. Then, compound **35d** (66 mg, 0.23 mmol) was added to the system, and stirring was continued for 15 min. After the reaction was completed, the mixture was quenched with a saturated aqueous ammonium chloride solution, adjusted to pH = 5 with a 1 N aqueous hydrochloric acid solution, and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **43** (29.3 mg, yield: 29.9%).

$^1$HNMR (400 MHz, DMSO-$d_6$): $\delta$10.01 (s, 1H), 7.86 (d, 1H), 7.50-7.49 (m, 1H), 6.91-6-89 (m, 2H), 6.61 (d, 1H), 6.31 (t, 1H), 5.50 (s, 2H), 4.19-4.17 (m, 2H), 4.06 (d, 3H), 3.70 (s, 3H), 6.92 (d, 1H), 1.73 (t, 2H), 0.98-0.96 (m, 2H), 0.83-0.81 (m, 2H).

MS m/z (ESI): 515.1 [M+H]$^+$.

**Example 50**

Synthesis of Compound 44

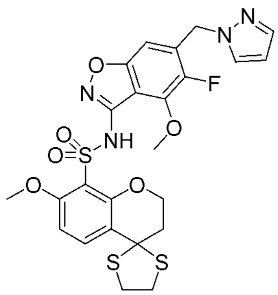

**44**

*N*-(6-((1*H*-Pyrazol-1-yl)methyl)-5-fluoro-4-methoxybenzo[*d*]isoxazol-3-yl)-7-methoxyspiro[chromane-4,2'-[1,3]dithiolane]-8-sulfonamide

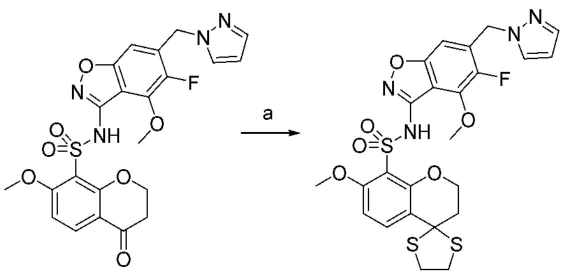


**42** **44**

Step a:

*N*-(6-((1*H*-Pyrazol-1-yl)methyl)-5-fluoro-4-methoxybenzo[*d*]isoxazol-3-yl)-7-methoxyspiro[chromane-4,2'-[1,3]dithiolane]-8-sulfonamide

1,2-Ethanedithiol (24 mg, 0.24 mmol) and boron trifluoride diethyl etherate (34 mg, 0.24 mmol) were added to a solution of compound **42** (60 mg, 0.12 mmol) in dichloromethane (5 mL) at 25 °C. The reaction system was stirred at 25 °C for 16 h. After the reaction was completed, the reaction mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous formic acid solution, mobile phase B: acetonitrile; gradient ratio: phase B 5%-95%) to give compound **44** (25 mg, yield: 36.2%).

$^1$HNMR (400 MHz, DMSO-$d_6$): $\delta$10.25 (s, 1H), 7.96 (d, 1H), 7.86 (d, 1H), 7.50 (d, 1H), 6.93-6.91 (m, 1H), 6.79 (d, 1H), 6.31 (t, 1H), 5.51 (s, 2H), 4.22 (t, 2H), 4.01 (d, 3H), 3.75 (s, 3H), 3.65-3.58 (m, 2H), 3.52-3.46(m, 2H), 2.42 (t, 2H).

MS m/z (ESI): 579.1 [M+H]$^+$.

**Example 51**

Synthesis of Compound 45

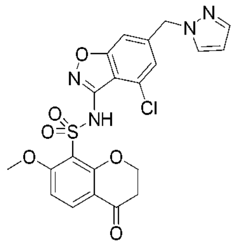

45

*N*-(6-((1*H*-Pyrazol-1-yl)methyl)-4-chlorobenzo[*d*]isoxazol-3-yl)-7-methoxy-4-oxochromane-8-sulfonamide

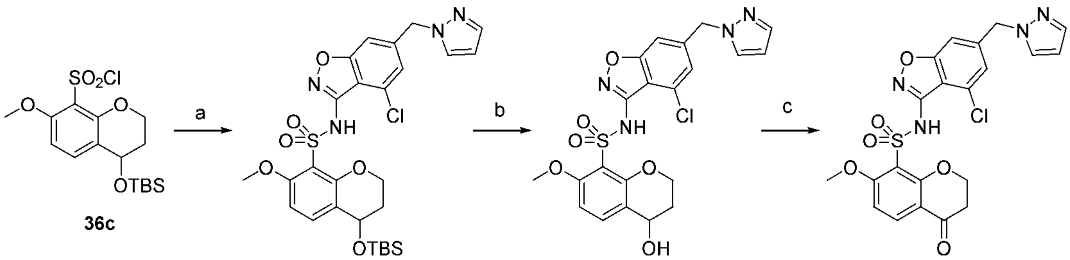


36c 45a 45b 45

Step a:

*N*-(6-((1*H*-Pyrazol-1-yl)methyl)-4-chlorobenzo[*d*]isoxazol-3-yl)-4-((tert-butyldimethylsilyl)oxy)-7-methoxychromane-8-sulfonamide

Sodium hydride (60%, 113 mg, 2.83 mmol) was added to a solution of **intermediate 6** (140 mg, 0.56 mmol) in N,N-dimethylformamide (1.4 mL) at 0 °C. The reaction system was stirred at 0 °C for 15 min. Then, compound **36c** (288 mg, 0.73 mmol) was added to the system, and stirring was continued for 15 min. After the reaction was completed, the reaction mixture was warmed to room temperature, quenched with a saturated aqueous ammonium chloride solution, and extracted with ethyl acetate (100 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 1:1) to give compound **45a** (225 mg, yield: 66.0%).

MS m/z (ESI): 605.6 $[M+H]^+$.

Step b:

*N*-(6-((1*H*-Pyrazol-1-yl)methyl)-4-chlorobenzo[*d*]isoxazol-3-yl)-4-hydroxy-7-methoxychromane-8-sulfonamide

Tetrabutylammonium fluoride trihydrate (312 mg, 1.12 mmol) was added to a solution of compound **45a** (225 mg, 0.37 mmol) in tetrahydrofuran (10 mL). The reaction system was stirred for 16 h. After the reaction was completed, the reaction mixture was quenched with a saturated aqueous ammonium chloride solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 4:1) to give compound **45b** (160 mg, yield: 87.7%).

MS m/z (ESI): 491.0 $[M+H]^+$.

Step c:

*N*-(6-((1*H*-Pyrazol-1-yl)methyl)-4-chlorobenzo[*d*]isoxazol-3-yl)-7-methoxy-4-oxochromane-8-sulfonamide

Manganese dioxide (425 mg, 4.89 mmol) was added to a solution of compound **45b** (160 mg, 0.33 mmol) in chloroform (10 mL). The reaction system was heated to 60 °C and stirred for 4 h. After the reaction was completed, the reaction mixture was cooled to room temperature and filtered under reduced pressure, and the filtrate was concentrated under reduced pressure. The resulting crude product was purified by high-performance liquid chromatography (Welchrom C18, 20 × 150 mm, 5 μm; mobile phase A: 5 mM aqueous formic acid solution, mobile phase B: acetonitrile; gradient ratio: phase B 5%-95%) to give compound **45** (90 mg, yield: 56.5%).
$^1$HNMR (400 MHz, DMSO-$d_6$): $\delta$10.66 (s, 1H), 7.97 (d, 1H), 7.90 (d, 1H), 7.51 (d, 1H), 7.45-7.43 (m, 1H), 7.25 (s, 1H), 6.93 (d, 1H), 6.31 (t, 1H), 5.50 (s, 2H), 4.47 (t, 2H), 3.83 (s, 3H), 2.72 (t, 2H).
MS m/z (ESI): 489.0 [M+H]$^+$.

**Example 52**

Synthesis of Compound 46

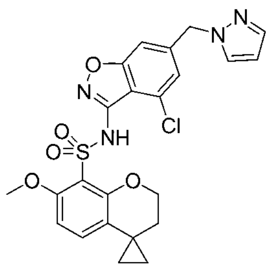

**46**

*N*-(6-((1*H*-Pyrazol-1-yl)methyl)-4-chlorobenzo[*d*]isoxazol-3-yl)-7-methoxyspiro[chromane-4,1'-cyclopropane]-8-sulfonamide

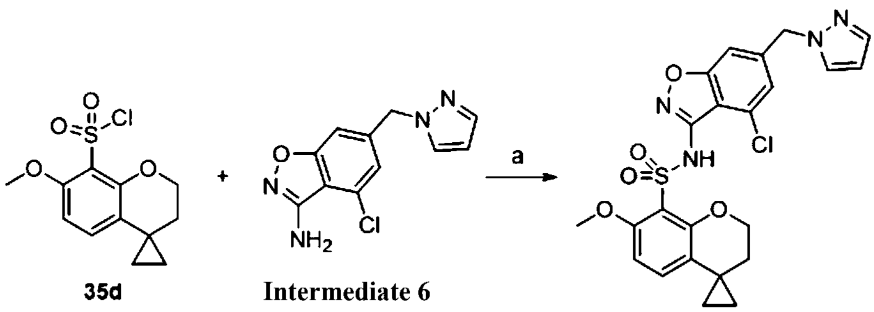


**35d** **Intermediate 6** **46**

Step a:

*N*-(6-((1*H*-Pyrazol-1-yl)methyl)-4-chlorobenzo[*d*]isoxazol-3-yl)-7-methoxyspiro[chromane-4,1'-cyclopropane]-8-sulfonamide

Sodium hydride (60%, 40 mg, 1.00 mmol) was added to a solution of **intermediate 6** (50 mg, 0.20 mmol) in *N*,*N*-dimethylformamide (1 mL) at 0 °C. The reaction system was stirred at 0 °C for 15 min. Then, compound 35d (76 mg, 0.26 mmol) was added to the system, and stirring was continued for 15 min. After the reaction was completed, the mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium

bicarbonate solution, mobile phase B: acetonitrile; gradient ratio: phase B 5%-95%) to give compound **46** (18 mg, yield: 17.9%).

$^1$HNMR (400 MHz, DMSO-$d_6$): $\delta$10.11 (s, 1H), 7.90 (d, 1H), 7.51 (d, 1H), 7.46 (s, 1H), 7.26 (s, 1H), 6.91 (d, 1H), 6.61 (d, 1H), 6.32 (t, 1H), 5.51 (s, 2H), 4.17 (t, 2H), 3.69 (s, 3H), 1.73 (t, 2H), 0.99-0.97 (m, 2H), 0.84-0.81 (m, 2H).

MS m/z (ESI): 501.0 [M+H]$^+$.

**Example 53**

Synthesis of Compound 47

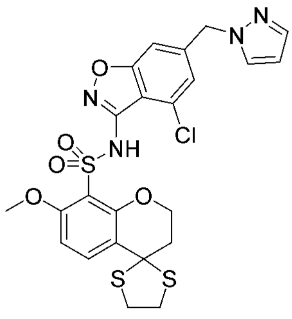

**47**

*N*-(6-((1*H*-Pyrazol-1-yl)methyl)-4-chlorobenzo[*d*]isoxazol-3-yl)-7-methoxyspiro[chromane-4,2'-[1,3]dithiolane]-8-sulfonamide

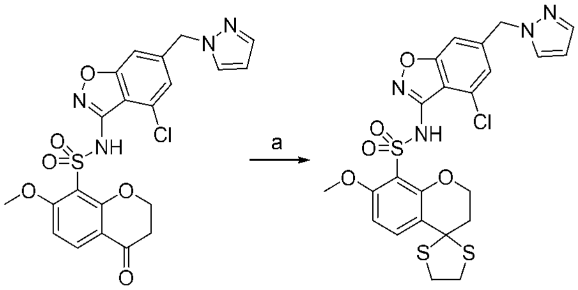


**45** **47**

Step a:

*N*-(6-((1*H*-Pyrazol-1-yl)methyl)-4-chlorobenzo[*d*]isoxazol-3-yl)-7-methoxyspiro[chromane-4,2'-[1,3]dithiolane]-8-sulfonamide

1,2-Ethanedithiol (50 mg, 0.53 mmol) and boron trifluoride diethyl etherate (72 mg, 0.53 mmol) were added to a solution of compound **45** (65 mg, 0.13 mmol) in dichloromethane (5 mL) at 25 °C. The reaction system was stirred at 25 °C for 16 h. After the reaction was completed, the reaction mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound 47 (13.9 mg, yield: 18.5%).

$^1$HNMR (400 MHz, DMSO-$d_6$): $\delta$10.30 (s, 1H), 7.96 (d, 1H), 7.91 (d, 1H), 7.51 (d, 1H), 7.46 (s, 1H), 7.25 (s, 1H), 6.78 (d, 1H), 6.31 (t, 1H), 5.50 (s, 2H), 4.21-4.19 (m, 2H), 3.73 (s, 3H), 3.62-3.59 (m, 2H), 3.52-3.46 (m, 2H), 2.41 (t, 2H).

MS m/z (ESI): 565.0 [M+H]$^+$.

**Example 54**

Synthesis of Compound 49

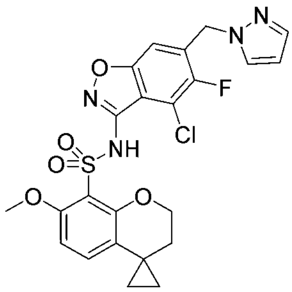

**49**

*N*-(6-((1*H*-Pyrazol-1-yl)methyl)-4-chloro-5-fluorobenzo[*d*]isoxazol-3-yl)-7-methoxyspiro[chromane-4,1'-cyclopropane]-8-sulfonamide

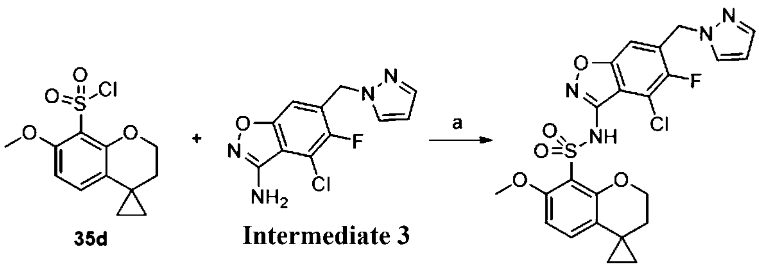


**35d** Intermediate 3 **49**

Step a:

*N*-(6-((1*H*-Pyrazol-1-yl)methyl)-4-chloro-5-fluorobenzo[*d*]isoxazol-3-yl)-7-methoxyspiro[chromane-4,1'-cyclopropane]-8-sulfonamide

Sodium hydride (60%, 36 mg, 0.90 mmol) was added to a solution of **intermediate 3** (50 mg, 0.19 mmol) in *N*,*N*-dimethylformamide (1 mL) at 0 °C. The reaction system was stirred at 0 °C for 15 min. Then, compound **35d** (68 mg, 0.24 mmol) was added to the system, and stirring was continued for 15 min. After the reaction was completed, the mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **49** (7.0 mg, yield: 7.2%).

$^1$HNMR (400 MHz, DMSO-$d_6$): $\delta$10.33 (s, 1H), 7.88 (d, 1H), 7.51 (d, 1H), 7.42 (s, 1H), 6.90 (d, 1H), 6.61 (d, 1H), 6.31 (t, 1H),5.56 (s, 2H), 4.19 (t, 2H), 3.70 (s, 3H), 1.74 (t, 2H), 0.99-0/97 (m, 2H), 0.84-0.82 (m, 2H).

MS m/z (ESI): 519.0 [M+H]$^+$.

**Example 55**

Synthesis of Compound 51

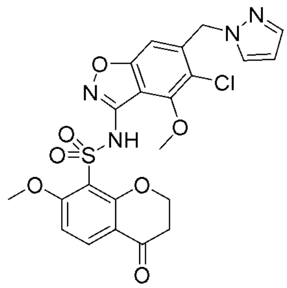

51

*N*-(6-((1*H*-Pyrazol-1-yl)methyl)-5-chloro-4-methoxybenzo[*d*]isoxazol-3-yl)-7-methoxy-4-oxochromane-8-sulfonamide

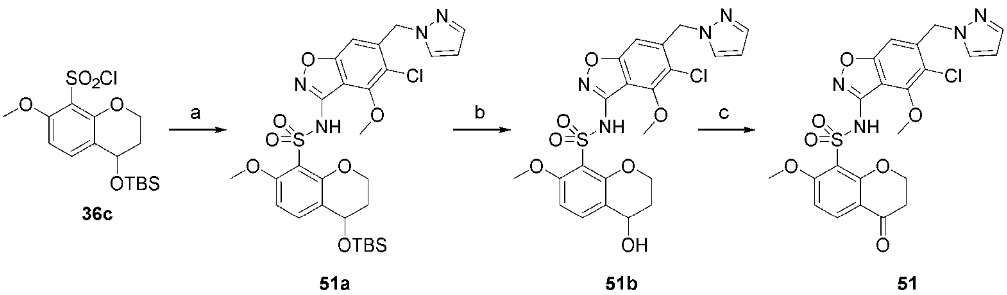

Step a:

*N*-(6-((1*H*-Pyrazol-1-yl)methyl)-5-chloro-4-methoxybenzo[*d*]isoxazol-3-yl)-4-((*tert*-butyldimethylsilyl)oxy)-7-methoxychromane-8-sulfonamide

Sodium hydride (60%, 86 mg, 2.15 mmol) was added to a solution of **intermediate 5** (120 mg, 0.43 mmol) in *N*,*N*-dimethylformamide (2 mL) at 0 °C.

The reaction system was stirred at 0 °C for 15 min. Then, compound **36c** (212 mg, 0.54 mmol) was added to the system, and stirring was continued for 15 min. After the reaction was completed, the reaction mixture was warmed to room temperature, quenched with a saturated aqueous ammonium chloride solution, and extracted with ethyl acetate (50 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:methanol = 49: 1) to give compound **51a** (171 mg, yield: 62.5%).

Step b:

*N*-(6-((1*H*-Pyrazol-1-yl)methyl)-5-chloro-4-methoxybenzo[*d*]isoxazol-3-yl)-4-hydroxy-7-methoxychromane-8-sulfonamide

Tetrabutylammonium fluoride trihydrate (226 mg, 0.81 mmol) was added to a solution of compound **51a** (171 mg, 0.27 mmol) in tetrahydrofuran (15 mL). The reaction system was stirred for 18 h. After the reaction was completed, the reaction mixture was quenched with a saturated aqueous ammonium chloride solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:methanol = 19:1) to give compound **51b** (109 mg, yield: 77.7%).

$^1$H NMR (400 MHz, $CDCl_3$) $\delta$ 8.17 (s, 1H), 7.61 (d, 1H), 7.51 (d, 1H), 7.42 (d, 1H), 6.56 (d, 1H), 6.54 (s, 1H), 6.36 (t, 1H), 5.51 (s, 2H), 4.71-4.69 (m, 1H), 4.44-4.40 (m, 1H), 4.33-4.27 (m, 1H), 4.13 (s, 3H), 3.89 (s, 3H), 1.82 (d, 1H), 1.28-1.24 (m, 1H).

Step c:

*N*-(6-((1*H*-Pyrazol-1-yl)methyl)-5-chloro-4-methoxybenzo[*d*]isoxazol-3-yl)-7-methoxy-4-oxochromane-8-sulfonamide

Manganese dioxide (260 mg, 2.99 mmol) was added to a solution of compound **51b** (104 mg, 0.20 mmol) in chloroform (10 mL). The reaction system was stirred at 30 °C for 18 h. After the reaction was completed, the reaction mixture was cooled to room temperature and filtered under reduced pressure, and the filtrate was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **51** (9 mg, yield: 8.7%).
$^1$HNMR (400 MHz, DMSO-$d_6$): $\delta$10.90 (s, 1H), 7.99 (d, 1H), 7.88 (d, 1H), 7.55 (d, 1H), 6.95 (d, 1H), 6.78 (s, 1H), 6.34 (t, 1H), 5.57 (s, 2H), 4.50 (t, 2H), 3.95 (s, 3H), 3.88 (s, 3H), 2.74 (t, 2H).
MS m/z (ESI): 519.0 [M+H]$^+$.

**Example 56**

Synthesis of Compound 52

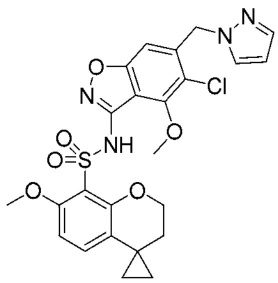

**52**

*N*-(6-((1*H*-Pyrazol-1-yl)methyl)-5-chloro-4-methoxybenzo[*d*]isoxazol-3-yl)-7-methoxyspiro[chromane-4,1'-cyclopropane]-8-sulfonamide

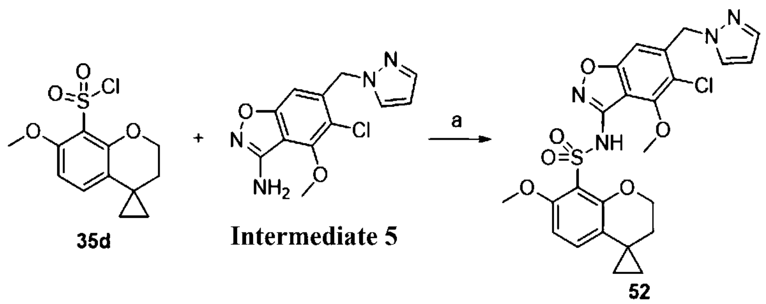


Step a:

*N*-(6-((1*H*-Pyrazol-1-yl)methyl)-5-chloro-4-methoxybenzo[*d*]isoxazol-3-yl)-7-methoxyspiro[chromane-4,1'-cyclopropane]-8-sulfonamide

Sodium hydride (60%, 36 mg, 0.90 mmol) was added to a solution of **intermediate 5** (50 mg, 0.18 mmol) in *N*,*N*-dimethylformamide (1 mL) at 0 °C. The reaction system was stirred at 0 °C for 15 min. Then, compound **35d** (68 mg, 0.24 mmol) was added to the system, and stirring was continued for 15 min. After the reaction was completed, the mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **52** (7 mg, yield: 7.3%).

$^{1}$HNMR (400 MHz, DMSO-$d_6$): $\delta$10.33 (s, 1H), 7.88 (d, 1H), 7.55 (d, 1H), 6.90 (d, 1H), 6.76 (s, 1H), 6.61 (d, 1H), 6.35 (t, 1H), 5.56 (s, 2H), 4.19 (t, 2H), 3.97 (s, 3H), 3.70 (s, 3H), 1.74 (t, 2H), 0.99-0.97 (m, 2H), 0.84-0.82 (m, 2H).

MS m/z (ESI): 531.0 $[M+H]^+$.

**Example 57**

Synthesis of Compound 53

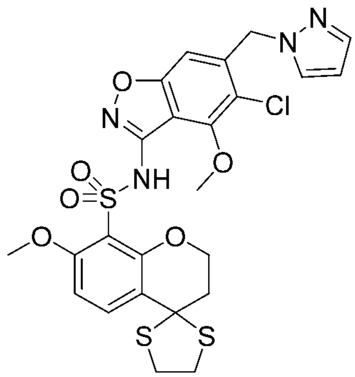

**53**

*N*-(6-((1*H*-Pyrazol-1-yl)methyl)-5-chloro-4-methoxybenzo[*d*]isoxazol-3-yl)-7-methoxyspiro[chromane-4,2'-[1,3]dithiolane]-8-sulfonamide

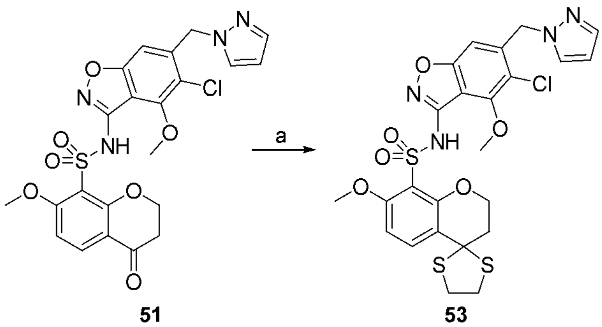


Step a:

*N*-(6-((1*H*-Pyrazol-1-yl)methyl)-5-chloro-4-methoxybenzo[*d*]isoxazol-3-yl)-7-methoxyspiro[chromane-4,2'-[1,3]dithiolane]-8-sulfonamide

1,2-Ethanedithiol (76 mg, 0.81 mmol) and boron trifluoride diethyl etherate (115 mg, 0.81 mmol) were added to a solution of compound **51** (60 mg, 0.12 mmol) in dichloromethane (6 mL) at 25 °C. The reaction system was stirred at 25 °C for 18 h. After the reaction was completed, the reaction mixture was quenched with a saturated aqueous ammonium chloride solution and extracted with dichloromethane (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **53** (25.3 mg, yield: 36.8%).

$^{1}$HNMR (400 MHz, DMSO-$d_6$): $\delta$10.53 (s, 1H), 7.98 (d, 1H), 7.88 (d, 1H), 7.55 (d, 1H), 6.82-6.80 (m, 2H), 6.35 (t,

1H), 5.57 (s, 2H), 4.25 (t, 2H), 3.91 (s, 3H), 3.76 (s, 3H), 3.66-3.59 (m, 2H), 3.53-3.46 (m, 2H), 2.46-2.42 (m, 2H).
MS m/z (ESI): 595.0 $[M+H]^+$.

**Example 58**

Synthesis of Compound 72

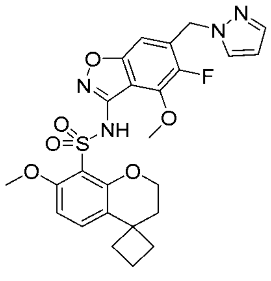

72

*N*-(6-((1*H*-Pyrazol-1-yl)methyl)-5-fluoro-4-methoxybenzo[*d*]isoxazol-3-yl)-7-methoxyspiro[chromane-4,1'-cyclobutane]-8-sulfonamide

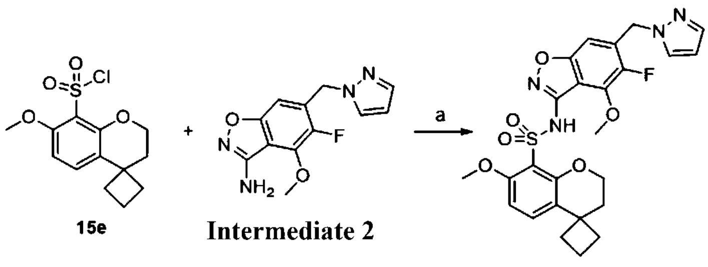


15e Intermediate 2 72

Step a:

*N*-(6-((1*H*-Pyrazol-1-yl)methyl)-5-fluoro-4-methoxybenzo[*d*]isoxazol-3-yl)-7-methoxyspiro[chromane-4,1'-cyclobutane]-8-sulfonamide

Sodium hydride (60%, 46 mg, 1.15 mmol) was added to a solution of **intermediate** 2 (60 mg, 0.23 mmol) in N,N-dimethylformamide (1 mL) at 0 °C. The reaction system was stirred at 0 °C for 15 min. Then, compound **15e** (68 mg, 0.24 mmol) was added to the system, and stirring was continued for 15 min. After the reaction was completed, the mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **72** (45 mg, yield: 37.2%).
$^1$HNMR (400 MHz, DMSO-$d_6$): $\delta$10.00 (s, 1H), 7.85 (d, 1H), 7.74 (d, 1H), 7.50 (d, 1H), 6.91 (d, 1H), 6.74 (d, 1H), 6.31 (t, 1H), 5.50 (s, 2H), 4.06-4.03 (m, 5H), 3.73 (s, 3H), 2.33-2.29 (m, 2H), 2.03-1.88 (m, 6H).
MS m/z (ESI): 529.1 $[M+H]^+$.

**Example 59**

Synthesis of Compound 73

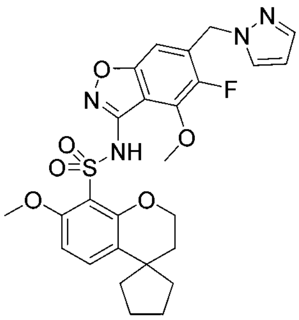

**73**

*N*-(6-((1*H*-Pyrazol-1-yl)methyl)-5-fluoro-4-methoxybenzo[*d*]isoxazol-3-yl)-7-methoxyspiro[chromane-4,1'-cyclopentane]-8-sulfonamide

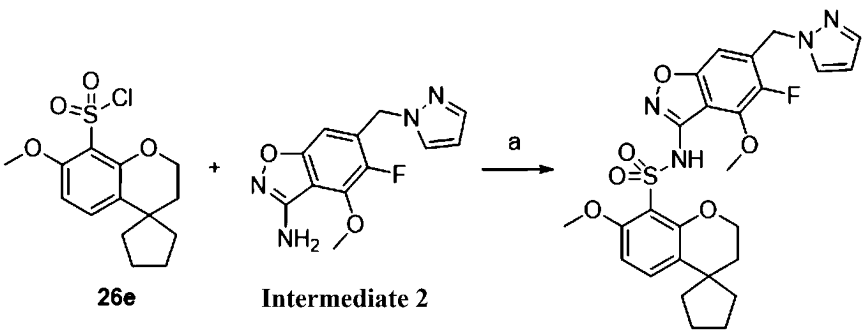


**26e** **Intermediate 2** **73**

Step a:

*N*-(6-((1*H*-Pyrazol-1-yl)methyl)-5-fluoro-4-methoxybenzo[*d*]isoxazol-3-yl)-7-methoxyspiro[chromane-4,1'-cyclopentane]-8-sulfonamide

Sodium hydride (60%, 53 mg, 1.33 mmol) was added to a solution of **intermediate 2** (70 mg, 0.27 mmol) in *N*,*N*-dimethylformamide (1 mL) at 0 °C. The reaction system was stirred at 0 °C for 15 min. Then, compound **26e** (110 mg, 0.35 mmol) was added to the system, and stirring was continued for 15 min. After the reaction was completed, the mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **73** (44.7 mg, yield: 30.9%).

$^1$HNMR (400 MHz, DMSO-$d_6$): δ9.99 (s, 1H), 7.85 (d, 1H), 7.50 (d, 1H), 7.45 (d, 1H), 6.91 (d, 1H), 6.69 (d, 1H), 6.31 (t, 1H), 5.50 (s, 2H), 4.08 (t, 2H), 4.03 (d, 3H), 3.72 (s, 3H), 1.79-1.67 (m, 10H).

MS m/z (ESI): 543.1 [M+H]$^+$.

**Example 60**

Synthesis of Compound 101

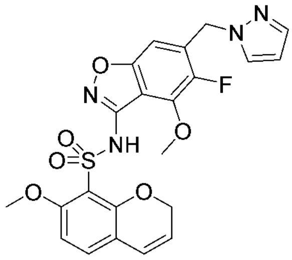

**101**

*N*-(6-((1*H*-Pyrazol-1-yl)methyl)-5-fluoro-4-methoxybenzo[*d*]isoxazol-3-yl)-7-methoxy-2*H*-chromene-8-sulfonamide

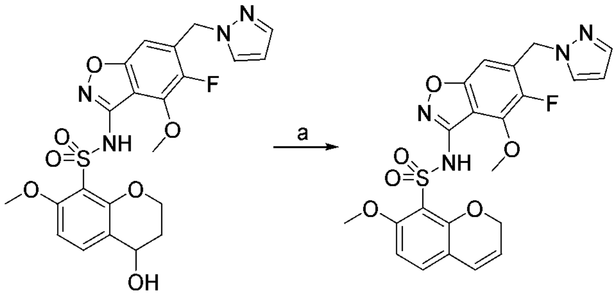


**107** **101**

Step a:

*N*-(6-((1*H*-Pyrazol-1-yl)methyl)-5-fluoro-4-methoxybenzo[*d*]isoxazol-3-yl)-7-methoxy-2*H*-chromene-8-sulfonamide

*p*-Toluenesulfonyl chloride (121 mg, 0.63 mmol) was added to a solution of compound **107** (80 mg, 0.16 mmol) in pyridine (8 mL). The reaction system was heated to 110 °C and stirred for 4 h. After the reaction was completed, the mixture was quenched with a saturated aqueous ammonium chloride solution, adjusted to pH = 5 with a 1 N aqueous hydrochloric acid solution, and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **101** (20.1 mg, yield: 26.1%).

$^1$HNMR (400 MHz, DMSO-$d_6$): $\delta$10.29 (s, 1H), 7.86 (d, 1H), 7.50 (d, 1H), 7.23 (d, 1H), 6.92 (s, 1H), 6.68 (d, 1H), 6.46 (d, 1H), 6.31 (t, 1H), 5.81-5.76 (m, 1H), 5.50 (s, 2H), 4.69 (br, 2H), 4.04 (d, 3H), 3.75 (s, 3H).

MS m/z (ESI): 487.0 [M+H]$^+$.

**Example 61**

Synthesis of Compound 106

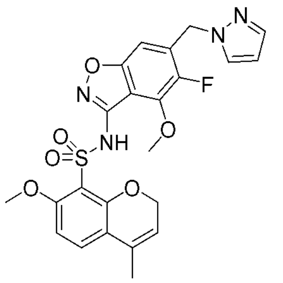

**106**

*N*-(6-((1*H*-Pyrazol-1-yl)methyl)-5-fluoro-4-methoxybenzo[*d*]isoxazol-3-yl)-7-methoxy-4-methyl-2*H*-chromene-8-sulfonamide

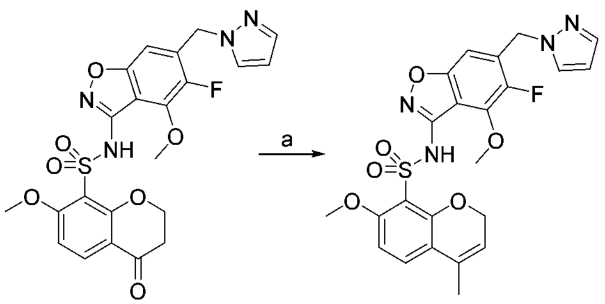


**42** **106**

Step a:

*N*-(6-((1*H*-Pyrazol-1-yl)methyl)-5-fluoro-4-methoxybenzo[*d*]isoxazol-3-yl)-7-methoxy-4-methyl-2*H*-chromene-8-sulfonamide

Under a nitrogen atmosphere, methylmagnesium bromide (1.0 M in tetrahydrofuran, 2.6 mL, 2.60 mmol) was added to a solution of compound **42** (130 mg, 0.26 mmol) in tetrahydrofuran (5 mL) at 0 °C, and the reaction system was stirred at 0 °C for 1 h. After the reaction was completed, the mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **106** (7.6 mg, yield: 5.9%).
$^1$HNMR (400 MHz, DMSO-$d_6$): $\delta$10.26 (s, 1H), 7.85 (d, 1H), 7.50 (d, 1H), 7.33-7.20 (m, 1H), 6.88-6.86 (m, 1H), 6.66-6.64 (m, 1H), 6.30 (t, 1H), 5.60-5.58 (m, 1H), 5.49 (s, 2H), 4.61-4.59 (m, 2H), 4.07 (s, 3H), 3.74 (s, 3H), 1.94 (s, 3H).
MS m/z (ESI): 501.1 [M+H]$^+$.

**Example 62**

Synthesis of Compound 107

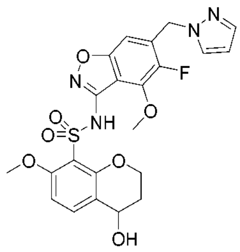

107

*N*-(6-((1*H*-Pyrazol-1-yl)methyl)-5-fluoro-4-methoxybenzo[*d*]isoxazol-3-yl)-4-hydroxy-7-methoxychromane-8-sulfonamide

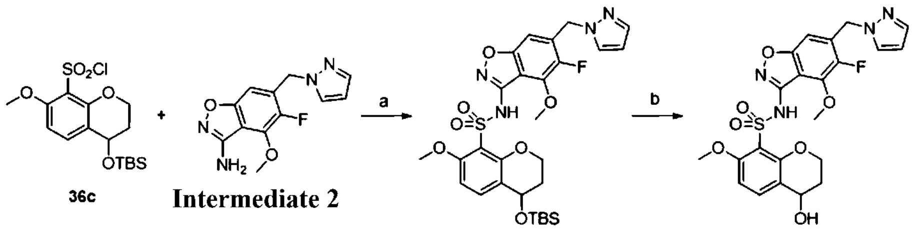


36c Intermediate 2 107a 107

Step a:

*N*-(6-((1*H*-Pyrazol-1-yl)methyl)-5-fluoro-4-methoxybenzo[*d*]isoxazol-3-yl)-4-((*tert*butyldimethylsilyl)oxy)-7-methoxychromane-8-sulfonamide

Sodium hydride (60%, 61 mg, 1.53 mmol) was added to a solution of **intermediate 2** (80 mg, 0.31 mmol) in N,N-dimethylformamide (1 mL) at 0 °C. The reaction system was stirred at 0 °C for 15 min. Then, compound **36c** (156 mg, 0.40 mmol) was added to the system, and stirring was continued for 15 min. After the reaction was completed, the mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 2:1) to give compound **107a** (135 mg, yield: 71.5%).
MS m/z (ESI): 619.7 $[M+H]^+$.

Step b:

*N*-(6-((1*H*-Pyrazol-1-yl)methyl)-5-fluoro-4-methoxybenzo[*d*]isoxazol-3-yl)-4-hydroxy-7-methoxychromane-8-sulfonamide

Tetrabutylammonium fluoride trihydrate (949 mg, 3.40 mmol) was added to a solution of compound **107a** (700 mg, 1.13 mmol) in tetrahydrofuran (10 mL). The reaction system was stirred for 16 h. After the reaction was completed, the reaction mixture was quenched with an aqueous solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 4:1) to give compound **107** (300 mg, yield: 52.6%).
$^1$HNMR (400 MHz, DMSO-$d_6$): δ10.01 (s, 1H), 7.85 (d, 1H), 7.49 (d, 1H), 7.40-7.38 (m, 1H), 6.83-6.81 (m, 1H), 6.68-6.66 (m, 1H), 6.30 (t, 1H), 5.48 (s, 2H), 5.36-5.34 (m, 1H), 4.56-4.54 (m, 1H), 4.20-4.09 (m, 2H), 4.09 (s, 3H), 3.70 (s, 3H), 1.91-1.76 (m, 2H).
MS m/z (ESI): 505.1 $[M+H]^+$.

**Example 63**

Synthesis of Compound 108

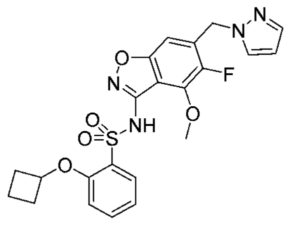

108

*N*-(6-((1*H*-Pyrazol-1-yl)methyl)-5-fluoro-4-methoxybenzo[*d*]isoxazol-3-yl)-2-cyclobutoxybenzenesulfonamide

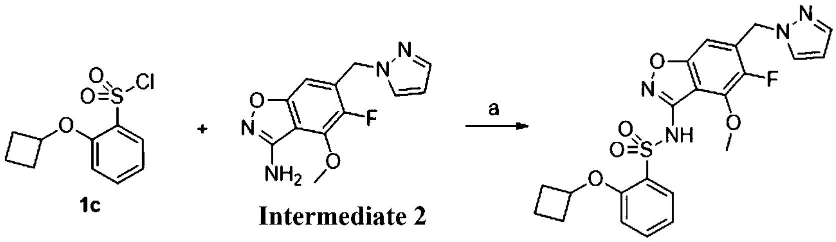


1c

Intermediate 2

108

Step a:

*N*-(6-((1*H*-Pyrazol-1-yl)methyl)-5-fluoro-4-methoxybenzo[*d*]isoxazol-3-yl)-2-cyclobutoxybenzenesulfonamide

Sodium hydride (60%, 22.9 mg, 0.57 mmol) was added to a solution of **intermediate 2** (50 mg, 0.19 mmol) in *N,N*-dimethylformamide (2 mL) at 0 °C. The reaction system was stirred at 0 °C for 1 h. Then, compound **1c** (94 mg, 0.38 mmol) was added to the system, and stirring was continued for 1 h. After the reaction was completed, the mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **108** (35 mg, yield: 39.2%).

$^1$HNMR (400 MHz, DMSO-$d_6$): δ 10.37 (s, 1H), 7.85-7.82 (m, 2H), 7.59-7.55 (m, 1H), 7.49 (d, 1H), 7.09 (t, 1H), 6.98 (d, 1H), 6.93 (d, 1H), 6.30 (t, 1H), 5.50 (s, 2H), 4.74-4.67 (m, 1H), 3.98 (d, 3H), 2.26-2.19 (m, 2H), 1.83-1.73 (m, 2H), 1.62-1.47 (m, 2H).

MS m/z (ESI): 472.9 [M+H]$^+$.

**Example 64**

Synthesis of Compound 103

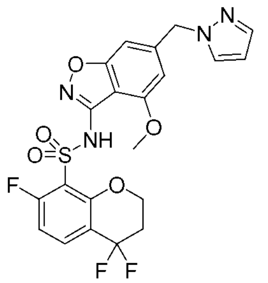

103

*N*-(6-((1*H*-Pyrazol-1-yl)methyl)-4-methoxybenzo[*d*]isoxazol-3-yl)-4,4,7-trifluorochromane-8-sulfonamide

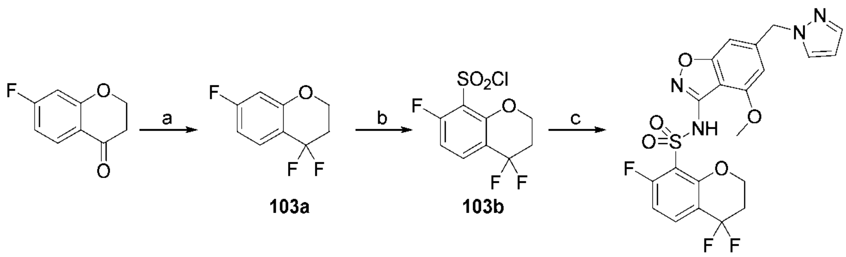


Step a:

4,4,7- Trifluorochromane

A solution system of 7-fluorochroman-4-one (2.50 g, 15.05 mmol) in bis(2-methoxyethyl)aminosulfur trifluoride (12 mL) was heated to 70 °C and stirred for 16 h. After the reaction was completed, the reaction mixture was quenched with ice water (40 mL) and extracted with dichloromethane (200 mL × 2). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether) to give compound **103a** (1.35 g, yield: 47.7%).

$^1$HNMR (400 MHz, $CDCl_3$): $\delta$7.58-7.54 (m, 1H), 6.75-6.70 (m, 1H), 6.61-6.57 (m, 1H), 4.36 (t, 2H), 2.50-2.41 (m, 2H).

Step b:

4,4,7-Trifluorochromane-8-sulfonyl chloride

Under a nitrogen atmosphere, *n*-butyllithium (2.5 M in n-hexane, 3.44 mL, 8.60 mmol) was added dropwise to a solution of compound **103a** (1.35 g, 7.18 mmol) in tetrahydrofuran (15 mL) at -78 °C, and the reaction system was stirred at -78 °C for 1 h. Then, sulfuryl chloride (1.16 g, 8.60 mmol) was added, and stirring was continued at -78 °C for 15 min. After the reaction was completed, the mixture was quenched with a saturated aqueous ammonium chloride solution and extracted with ethyl acetate (100 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 19:1) to give compound **103b** (800 mg, yield: 38.9%).

$^1$HNMR (400 MHz, $CDCl_3$): $\delta$7.95-7.91 (m, 1H), 6.96-6.91 (m, 1H), 4.64 (t, 2H), 2.63-2.53 (m, 2H).

Step c:

*N*-(6-((1*H*-Pyrazol-1-yl)methyl)-4-methoxybenzo[*d*]isoxazol-3-yl)-4,4,7-trifluorochromane-8-sulfonamide

Sodium hydride (60%, 98.26 mg, 2.46 mmol) was added to a solution of **intermediate 1** (200 mg, 0.82 mmol) in

*N,N*-dimethylformamide (2 mL) at 0 °C. The reaction system was stirred at 0 °C for 15 min. Then, a solution of compound **103b** (399.01 mg, 1.35 mmol) in *N,N*-dimethylformamide (1 mL) was added to the system, and stirring was continued for 15 min. After the reaction was completed, the mixture was quenched with a saturated aqueous ammonium chloride solution and extracted with dichloromethane (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:methanol = 19:1) to give compound 103 (360 mg, yield: 88.9%).

$^1$HNMR (400 MHz, DMSO-$d_6$): δ 11.09 (brs, 1H), 7.91-7.87 (m, 2H), 7.50 (d, 1H), 7.08 (t, 1H), 6.87 (s, 1H), 6.74 (s, 1H), 6.30 (t, 1H), 5.45 (s, 2H), 4.30 (t, 2H), 3.77 (s, 3H), 2.55-2.46 (m, 2H).

MS m/z (ESI): 494.8 [M+H]$^+$.

**Example 65**

Synthesis of Compound 102

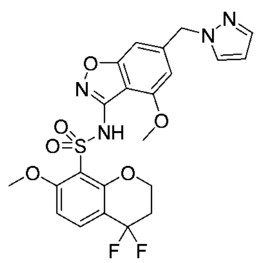

**102**

*N*-(6-((1*H*-Pyrazol-1-yl)methyl)-4-methoxybenzo[*d*]isoxazol-3-yl)-4,4-difluoro-7-methoxychromane-8-sulfonamide

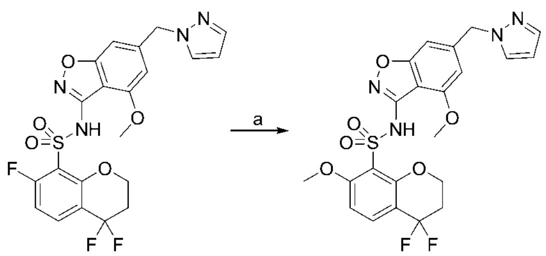


**103** **102**

Step a:

*N*-(6-((1*H*-Pyrazol-1-yl)methyl)-4-methoxybenzo[*d*]isoxazol-3-yl)-4,4-difluoro-7-methoxychromane-8-sulfonamide

Sodium methoxide (33% in methanol, 1 mL) was added to a solution of compound **103** (100 mg, 0.20 mmol) in methanol (2 mL). The reaction system was heated to 65 °C and stirred for 1 h. After the reaction was completed, the solution was quenched and adjusted to pH = 5 with a 1 N aqueous hydrochloric acid solution, and extracted with dichloromethane (50 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **102** (23 mg, yield: 22.5%).

$^1$HNMR (400 MHz, DMSO-$d_6$): δ 10.13 (s, 1H), 7.87 (d, 1H), 7.76 (d, 1H), 7.50 (d, 1H), 6.91 (d, 1H), 6.85 (s, 1H), 6.75 (s, 1H), 6.30 (t, 1H), 5.45 (s, 2H), 4.29 (t, 2H), 3.82 (s, 3H), 3.81 (s, 3H), 2.50-2.43 (m, 2H).

MS m/z (ESI): 506.8 [M+H]$^+$.

**Example 66**

Synthesis of Compound 105

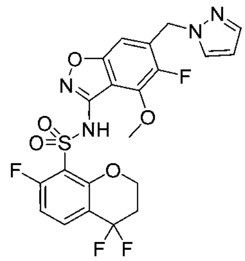

105

*N*-(6-((1*H*-Pyrazol-1-yl)methyl)-5-fluoro-4-methoxybenzo[*d*]isoxazol-3-yl)-4,4,7-trifluorochromane-8-sulfonamide

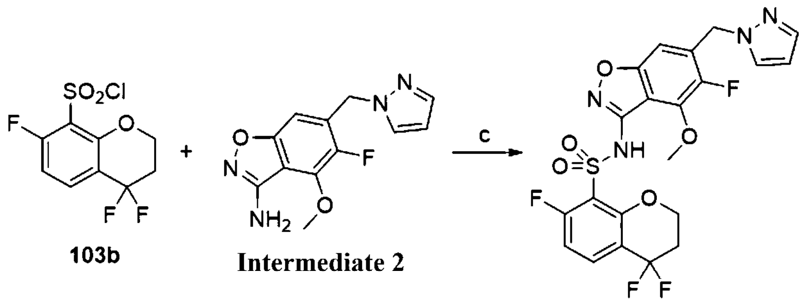


103b Intermediate 2 105

Step a:

*N*-(6-((1*H*-Pyrazol-1-yl)methyl)-5-fluoro-4-methoxybenzo[*d*]isoxazol-3-yl)-4,4,7-trifluorochromane-8-sulfonamide

Sodium hydride (60%, 76.30 mg, 1.91 mmol) was added to a solution of **intermediate 2** (100 mg, 0.38 mmol) in *N,N*-dimethylformamide (1 mL) at 0 °C. The reaction system was stirred at 0 °C for 15 min. Then, compound **103b** (164.00 mg, 0.57 mmol) was added to the system, and stirring was continued for 15 min. After the reaction was completed, the mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:tetrahydrofuran = 85:15) to give compound **105** (120 mg, yield: 61.4%).

$^1$HNMR (400 MHz, DMSO-$d_6$): δ 11.34 (brs, 1H), 7.92-7.86 (m, 2H), 7.50-7.49 (m, 1H), 7.08 (t, 1H), 6.95 (d, 1H), 6.31 (t, 1H), 5.51 (s, 2H), 4.37-4.34 (m, 2H), 4.00 (d, 3H), 2.60-2.52 (m, 2H).

MS m/z (ESI): 512.8 [M+H]$^+$.

**Example 67**

Synthesis of Compound 104

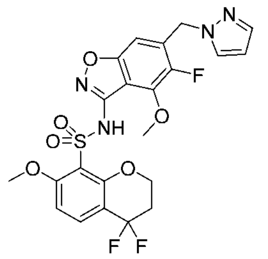

104

*N*-(6-((1*H*-Pyrazol-1-yl)methyl)-5-fluoro-4-methoxybenzo[*d*]isoxazol-3-yl)-4,4-difluoro-7-methoxychromane-8-sulfonamide

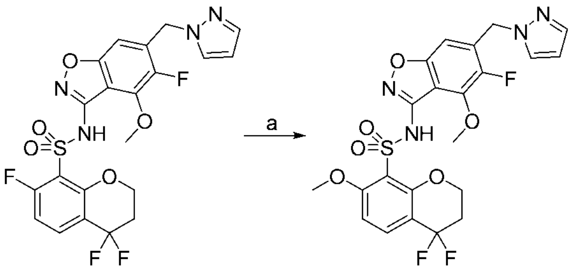


105 104

Step a:

*N*-(6-((1*H*-Pyrazol-1-yl)methyl)-5-fluoro-4-methoxybenzo[*d*]isoxazol-3-yl)-4,4-difluoro-7-methoxychromane-8-sulfonamide

Sodium methoxide (33% in methanol, 2 mL) was added to a solution of compound **105** (80 mg, 0.16 mmol) in methanol (2 mL). The reaction system was heated to 65 °C and stirred for 2 h. After the reaction was completed, the solution was quenched and adjusted to pH = 5 with a 1 N aqueous hydrochloric acid solution, and extracted with ethyl acetate (50 mL × 2). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **104** (46 mg, yield: 56.2%).

$^1$HNMR (400 MHz, DMSO-$d_6$): δ 10.54 (s, 1H), 7.86 (d, 1H), 7.77 (d, 1H), 7.50-7.49 (m, 1H), 6.93-6.91 (m, 2H), 6.31 (t, 1H), 5.51 (s, 2H), 4.31 (t, 2H), 4.02 (d, 3H), 3.82 (s, 3H), 2.54-2.46 (m, 2H).

MS m/z (ESI): 524.9 [M+H]$^+$.

**Example 68**

Synthesis of Compound 109

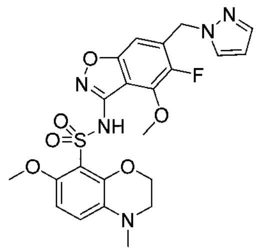

109

*N*-(6-((1*H*-Pyrazol-1-yl)methyl)-5-fluoro-4-methoxybenzo[*d*]isoxazol-3-yl)-7-methoxy-4-methyl-3,4-dihydro-2*H*-benzo[*b*][1,4]oxazine-8-sulfonamide

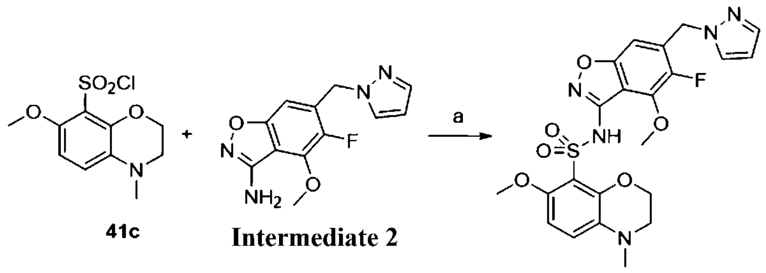


41c Intermediate 2 109

Step a:

*N*-(6-((1*H*-Pyrazol-1-yl)methyl)-5-fluoro-4-methoxybenzo[*d*]isoxazol-3-yl)-7-methoxy-4-methyl-3,4-dihydro-2*H*-benzo[*b*][1,4]oxazine-8-sulfonamide

Sodium hydride (60%, 72.00 mg, 1.80 mmol) was added to a solution of **intermediate 2** (94 mg, 0.36 mmol) in *N,N*-dimethylformamide (3 mL) at 0 °C. The reaction system was stirred at 0 °C for 10 min. Then, compound 41c (120.00 mg, 0.43 mmol) was added to the system, and stirring was continued for 15 min. After the reaction was completed, the system was quenched with a saturated aqueous ammonium chloride solution, adjusted to pH = 4 with a 0.5 N diluted aqueous hydrochloric acid solution, and then extracted with ethyl acetate (15 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **109** (62 mg, yield: 34.4%). $^1$HNMR (400 MHz, DMSO-$d_6$): δ 9.98 (brs, 1H), 7.86 (d, 1H), 7.50 (d, 1H), 6.90-6.87 (m, 2H), 6.58 (d, 1H), 6.31 (t, 1H), 5.50 (s, 2H), 4.20 (t, 2H), 4.06 (d, 3H), 3.66 (s, 3H), 3.12 (t, 2H), 2.77 (s, 3H).

MS m/z (ESI): 504.1 $[M+H]^+$.

**Example 69**

Synthesis of Compound 112

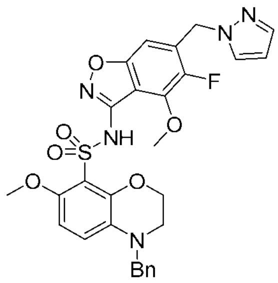

112

*N*-(6-((1*H*-Pyrazol-1-yl)methyl)-5-fluoro-4-methoxybenzo[*d*]isoxazol-3-yl)-4-benzyl-7-methoxy-3,4-dihydro-2*H*-benzo[*b*][1,4]oxazine-8-sulfonamide

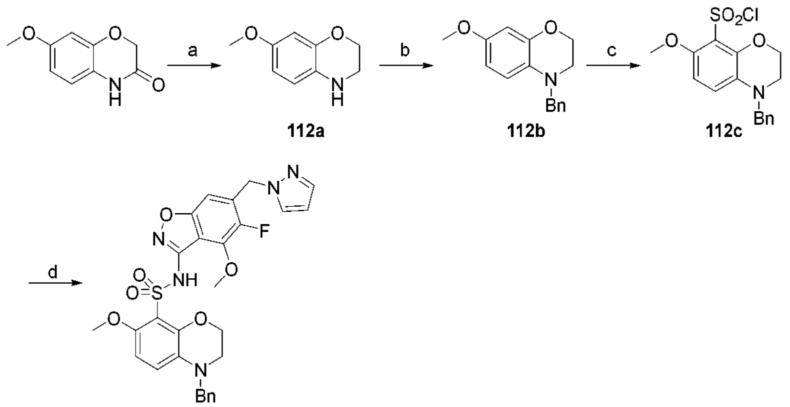


112a 112b 112c

112

Step a:

7-Methoxy-3,4-dihydro-2*H*-benzo[*b*][1,4]oxazine

Under a nitrogen atmosphere, a borane solution (1 M in tetrahydrofuran, 13.40 mL, 13.40 mmol) was added to a solution of 7-methoxy-2*H*-benzo[*b*][1,4]oxazin-3(4*H*)-one (1.20 g, 6.70 mmol) in tetrahydrofuran (40 mL) at 0 °C, and the reaction system was heated to 75 °C and stirred for 4 h. After the reaction was completed, the reaction mixture was concentrated under reduced pressure. The resulting crude product was purified by flash silica gel column chromatography (petroleum ether:ethyl acetate = 2:1) to give compound **112a** (0.82 g, yield: 74.1%).

MS m/z (ESI): 166.0 $[M+H]^+$.

Step b:

4-Benzyl-7-methoxy-3,4-dihydro-2*H*-benzo[*b*][1,4]oxazine

Under a nitrogen atmosphere, a solution of benzyl bromide (1.20 g, 7.02 mmol) in dichloromethane (30 mL) was added dropwise to a solution of compound **112a** (900 mg, 5.45 mmol) and *N,N*-diisopropylethylamine (2.10 g, 16.25 mmol) in dichloromethane (50 mL), and the reaction system was stirred at room temperature for 2 h. After the reaction was completed, the reaction mixture was concentrated under reduced pressure. The resulting crude product was purified by flash silica gel column chromatography (petroleum ether:ethyl acetate = 1:1) to give compound **112b** (1.2 g, yield: 86.3%).

MS m/z (ESI): 256.2 $[M+H]^+$.

Step c:

4-Benzyl-7-methoxy-3,4-dihydro-2*H*-benzo[*b*][1,4]oxazine-8-sulfonyl chloride

Under a nitrogen atmosphere, *n*-butyllithium (2.5 M in n-hexane, 0.4 mL, 1.00 mmol) was added dropwise to a solution of compound **112b** (200 mg, 0.78 mmol) in tetrahydrofuran (5 mL) at 0 °C, and the reaction system was stirred at 0 °C for 1 h and then cooled to -78 °C. Subsequently, sulfuryl chloride (127 mg, 0.94 mmol) was added, and stirring was continued at - 78 °C for 15 min.After the reaction was completed, the mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 1:1) to give compound **112c** (23 mg, yield: 8.3%).
$^1$HNMR (400 MHz, $CDCl_3$): δ7.41-7.30 (m, 5H), 6.91 (d, 1H), 6.50 (d, 1H), 4.51 (t, 2H), 4.46 (s, 2H), 3.91 (s, 3H), 3.45 (t, 2H).
MS m/z (ESI): 354.3 $[M+H]^+$.

Step d:

*N*-(6-((1*H*-Pyrazol-1-yl)methyl)-5-fluoro-4-methoxybenzo[*d*]isoxazol-3-yl)-4-benzyl-7-methoxy-3,4-dihydro-2*H*-benzo [*b*][1,4]oxazine-8-sulfonamide

Sodium hydride (60%, 72 mg, 1.80 mmol) was added to a solution of **intermediate 2** (94 mg, 0.36 mmol) in *N,N*-dimethylformamide (3 mL) at 0 °C. The reaction system was stirred at 0 °C for 10 min. Then, compound **112c** (153 mg, 0.43 mmol) was added to the system, and stirring was continued for 15 min. After the reaction was completed, the system was quenched with a saturated aqueous ammonium chloride solution, adjusted to pH = 4 with a 0.5 N aqueous hydrochloric acid solution, and extracted with ethyl acetate (20 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 4:1) to give compound **112** (110 mg, yield: 52.9%).
$^1$HNMR (400 MHz, DMSO-$d_6$): δ9.99 (s, 1H), 7.86 (d, 1H), 7.50 (d, 1H), 7.32-7.24 (m, 5H), 6.92 (d, 1H), 6.84 (d, 1H), 6.55 (d, 1H), 6.31 (t, 1H), 5.51 (s, 2H), 4.42 (s, 2H), 4.19 (t, 2H), 4.04 (d, 3H), 3.63 (s, 3H), 3.30-3.28 (m, 2H).
MS m/z (ESI): 580.2 $[M+H]^+$.

**Example 70**

Synthesis of Compound 111

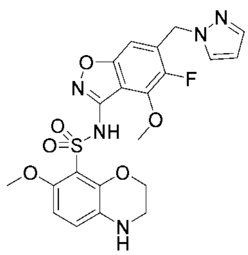

**111**

*N*-(6-((1*H*-Pyrazol-1-yl)methyl)-5-fluoro-4-methoxybenzo[*d*]isoxazol-3-yl)-7-methoxy-3,4-dihydro-2*H*-benzo[*b*][1,4]oxazine-8-sulfonamide

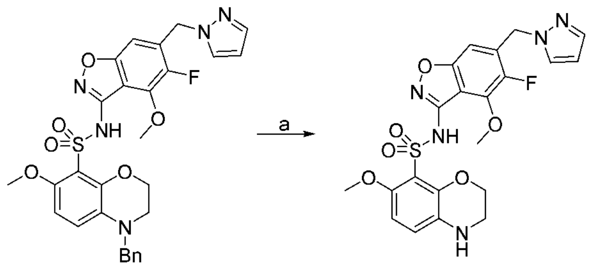


112

111

Step a:

*N*-(6-((1*H*-Pyrazol-1-yl)methyl)-5-fluoro-4-methoxybenzo[*d*]isoxazol-3-yl)-7-methoxy-3,4-dihydro-2*H*-benzo[*b*][1,4]oxazine-8-sulfonamide

Under a hydrogen atmosphere, palladium on carbon (138 mg, 1.30 mmol), palladium hydroxide (364 mg, 2.59 mmol), and 1,1,2-trichloroethane (575 mg, 2.59 mmol) were added to a solution of compound **112** (500 mg, 0.86 mmol) in methanol (20 mL), and the reaction system was stirred at 25 °C for 2 h. After the reaction was completed, the reaction system was filtered through celite, and the filtrate was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **111** (390 mg, yield: 92.4%).

$^1$HNMR (400 MHz, DMSO-$d_6$): δ 9.86 (brs, 1H), 7.85 (d, 1H), 7.50 (d, 1H), 6.89 (d, 1H), 6.72 (d, 1H), 6.53 (d, 1H), 6.31 (t, 1H), 5.66 (s, 1H), 5.55 (s, 2H), 4.08 (s, 3H), 4.08-4.05 (m, 2H), 3.63 (s, 3H), 3.19-3.17 (m, 2H).

MS m/z (ESI): 490.1 [M+H]$^+$.

**Example 71**

Synthesis of Compound 110

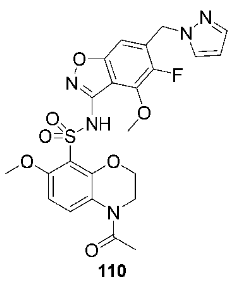

110

*N*-(6-((1*H*-Pyrazol-1-yl)methyl)-5-fluoro-4-methoxybenzo[*d*]isoxazol-3-yl)-4-acetyl-7-methoxy-3,4-dihydro-2*H*-benzo[*b*][1,4]oxazine-8-sulfonamide

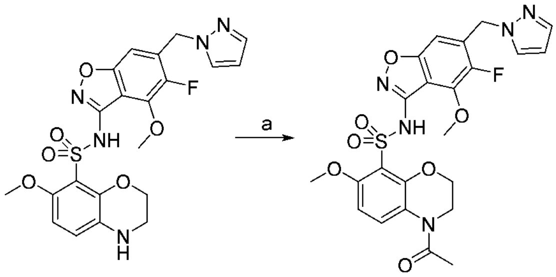


111

110

Step a:

*N*-(6-((1*H*-Pyrazol-1-yl)methyl)-5-fluoro-4-methoxybenzo[*d*]isoxazol-3-yl)-4-acetyl-7-methoxy-3,4-dihydro-2*H*-benzo[*b*][1,4]oxazine-8-sulfonamide

*N,N*-Diisopropylethylamine (392 mg, 3.03 mmol) was added to a solution of compound 111 (99 mg, 0.20 mmol) and acetic anhydride (248 mg, 2.43 mmol) in dichloromethane (50 mL). The reaction system was stirred at 25 °C for 18 h. After the reaction was completed, the reaction system was diluted with water and extracted with dichloromethane (10 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **110** (17 mg, yield: 15.8%).

$^1$HNMR (400 MHz, DMSO-$d_6$): δ 10.28 (brs, 1H), 8.03 (brs, 1H), 7.85 (d, 1H), 7.50 (d, 1H), 6.90-6.71 (m, 2H), 6.31 (t, 1H), 5.50 (s, 2H), 4.27-4.25 (m, 2H), 4.04 (s, 3H), 3.78-3.74 (m, 5H), 2.19 (s, 3H).

MS m/z (ESI): 532.1 [M+H]$^+$.

**Example 72**

Synthesis of Compound 114

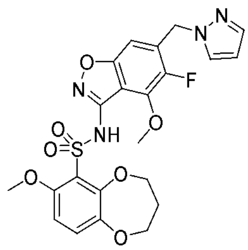

**114**

*N*-(6-((1*H*-Pyrazol-1-yl)methyl)-5-fluoro-4-methoxybenzo[*d*]isoxazol-3-yl)-7-methoxy-3,4-dihydro-2*H*-benzo[*b*][1,4]dioxepine-6-sulfonamide

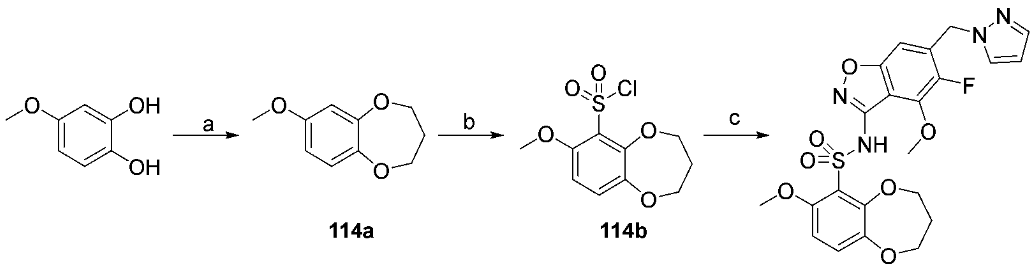


**114a** **114b** **114**

Step a:

7-Methoxy-3,4-dihydro-2*H*-benzo[*b*][1,4]dioxepine

Under a nitrogen atmosphere, 1,3-dibromopropane (562 mg, 2.78 mmol) was added dropwise to a solution of 4-methoxybenzene-1,2-diol (300 mg, 2.14 mmol) and potassium carbonate (1.04 g, 7.52 mmol) in ethanol (6 mL) at 20 °C, and the reaction system was heated to 85 °C and stirred for 16 h. After the reaction was completed, the mixture was diluted with an aqueous solution and extracted with ethyl acetate (30 mL × 3). The organic layers were combined, washed with a saturated aqueous NaCl solution, and dried over anhydrous sodium sulfate. After filtration, the mother liquor was

concentrated under reduced pressure. The resulting crude product was purified by flash silica gel column chromatography (petroleum ether:tetrahydrofuran = 50:1) to give compound **114a** (184 mg, yield: 47.7%).

$^1$HNMR (400 MHz, $CDCl_3$): δ6.83 (d, 1H), 6.48 (d, 1H), 6.43-6.40 (m, 1H), 4.12 (t, 2H), 4.06 (t, 2H), 3.76 (s, 3H), 2.13-2.06 (m, 2H).

Step b:

7-Methoxy-3,4-dihydro-2*H*-benzo[*b*][1,4]dioxepine-6-sulfonyl chloride

Under a nitrogen atmosphere, n-butyllithium (2.5 M in n-hexane, 0.49 mL, 1.23 mmol) was added dropwise to a solution of compound **114a** (184 mg, 1.02 mmol) in tetrahydrofuran (5 mL) at 0 °C, and the reaction system was stirred at 0 °C for 1 h and then cooled to -78 °C. Subsequently, sulfuryl chloride (152 mg, 1.13 mmol) was added, and stirring was continued at - 78 °C for 10 min. After the reaction was completed, the mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 5:1) to give compound **114b** (119 mg, yield: 41.8%).

$^1$HNMR (400 MHz, $CDCl_3$): δ7.22 (d, 1H), 6.59 (d, 1H), 4.29 (t, 2H), 4.11 (t, 2H), 3.86 (s, 3H), 2.22-2.17 (m, 2H).

Step c:

*N*-(6-((1*H*-Pyrazol-1-yl)methyl)-5-fluoro-4-methoxybenzo[*d*]isoxazol-3-yl)-7-methoxy-3,4-dihydro-2*H*-benzo[*b*][1,4]dioxepine-6-sulfonamide

Sodium hydride (60%, 53 mg, 1.33 mmol) was added to a solution of **intermediate 2** (70 mg, 0.27 mmol) in *N,N*-dimethylformamide (1 mL) at 0 °C. The reaction system was stirred at 0 °C for 1 h. Then, compound **114b** (112 mg, 0.40 mmol) was added to the system, and stirring was continued for 1 h. After the reaction was completed, the mixture was quenched with a saturated aqueous ammonium chloride solution and extracted with ethyl acetate (10 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous ammonium bicarbonate solution, mobile phase B: acetonitrile; gradient ratio: phase B 5%-95%) to give compound **114** (17 mg, yield: 12.6%).

$^1$HNMR (400 MHz, DMSO-$d_6$): δ10.38 (s, 1H), 7.86 (d, 1H), 7.50 (d, 1H), 7.23 (d, 1H), 6.91 (d, 1H), 6.80 (d, 1H), 6.31 (t, 1H), 5.50 (s, 2H), 4.07-4.05 (m, 2H), 4.02-4.00 (m, 5H), 3.33 (s, 3H), 2.06-2.04 (m, 2H).

MS m/z (ESI): 505.0 $[M+H]^+$.

**Example 73**

Synthesis of Compound 115

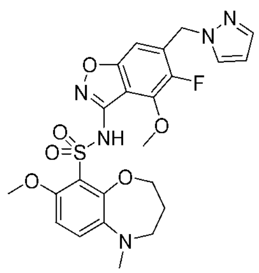

**115**

*N*-(6-((1*H*-Pyrazol-1-yl)methyl)-5-fluoro-4-methoxybenzo[*d*]isoxazol-3-yl)-8-methoxy-5-methyl-2,3,4,5-tetrahydrobenzo[b][1,4]oxazepine-9-sulfonamide

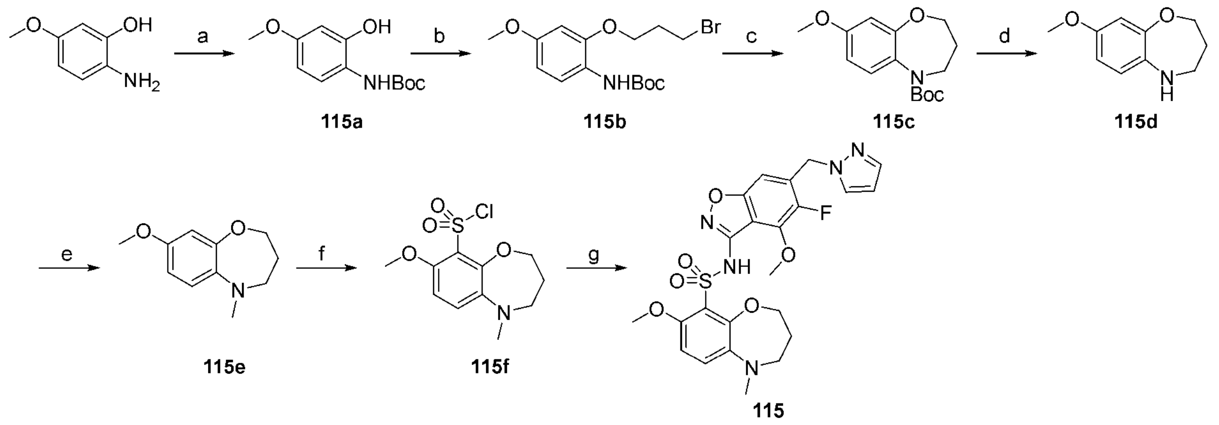


Step a:

*tert-Butyl* (2-hydroxy-4-methoxyphenyl)carbamate

Under a nitrogen atmosphere, a solution of di-tert-butyl dicarbonate (6.58 g, 30.15 mmol) in tetrahydrofuran (10 mL) was added dropwise to a solution of 2-amino-5-methoxyphenol (4.00 g, 28.75 mmol) in tetrahydrofuran (80 mL), and the reaction system was stirred at 25 °C for 5 h. After the reaction was completed, the mixture was diluted with an aqueous solution and extracted with ethyl acetate (70 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 9:1) to give compound **115a** (6.20 g, yield: 90.1%).
MS m/z (ESI): 184.0 $[M+H-56]^+$.

Step b:

*tert-Butyl* (2-(3-bromopropoxy)-4-methoxyphenyl)carbamate

A solution of compound **115a** (3.80 g, 15.88 mmol), 1,3-dibromopropane (14.45 g, 71.57 mmol), and potassium carbonate (17.58 g, 127.20 mmol) in acetonitrile (100 mL) was heated to 60 °C and stirred for 1 h. After the reaction was completed, the mixture was diluted with an aqueous solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 19:1) to give compound **115b** (3.00 g, yield: 52.4%).
MS m/z (ESI): 260.0 $[M+H-Boc]^+$.

Step c:

*tert*-Butyl 8-methoxy-3,4-dihydrobenzo[b][1,4]oxazepine-5(2H)-carboxylate

Under a nitrogen atmosphere, sodium hydride (60%, 0.56 g, 14.00 mmol) was added to a solution of compound **115b** (2.00 g, 5.55 mmol) in tetrahydrofuran (50 mL) at 0 °C, and the reaction system was stirred at 0 °C for 2 h. After the reaction was completed, the mixture was quenched with a saturated aqueous ammonium chloride solution and extracted with ethyl acetate (50 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 19:1) to give compound **115c** (1.16 g, yield: 74.8%).
$^1$HNMR (400 MHz, DMSO-$d_6$): δ7.09 (d, 1H), 6.59 (dd, 1H), 6.55 (d, 1H), 4.02-3.99 (m, 2H), 3.72 (s, 3H), 3.55-3.53 (m, 2H), 1.91-1.89 (m, 2H), 1.44-1.32 (m, 9H).
MS m/z (ESI): 224.2 $[M+H-56]^+$.

Step d:

8-Methoxy-2,3,4,5-tetrahydrobenzo[b][1,4]oxazepine

Trifluoroacetic acid (5 mL) was added to a solution of compound **115c** (1.16 g, 4.15 mmol) in dichloromethane (30 mL) at 0 °C. The reaction system was stirred at 25 °C for 5 h. After the reaction was completed, the mixture was quenched with a saturated aqueous sodium bicarbonate solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 3:1) to give compound **115d** (0.69 g, yield: 92.7%).
MS m/z (ESI): 180.3 $[M+H]^+$.

Step e:

8-Methoxy-5-methyl-2,3,4,5-tetrahydrobenzo[b][1,4]oxazepine

Under a nitrogen atmosphere, paraformaldehyde (0.83 g, 27.64 mmol) and acetic acid (20 mg) were added to a solution of compound **115d** (0.99 g, 5.52 mmol) and sodium cyanoborohydride (1.74 g, 27.69 mmol) in tetrahydrofuran (20 mL), and the reaction system was heated to 50 °C and stirred for 3 h. After the reaction was completed, the mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:ethyl acetate = 9:1) to give compound **115e** (0.88 g, yield: 82.4%).
$^1$HNMR (400 MHz, DMSO-$d_6$): δ6.79 (d, 1H), 6.54 (dd, 1H), 6.47 (d, 1H), 3.95 (t, 2H), 3.66 (s, 3H), 2.95 (t, 2H), 2.76 (s, 3H), 1.92-1.89 (m, 2H).
MS m/z (ESI): 194.2 $[M+H]^+$.

Step f:

8-Methoxy-5-methyl-2,3,4,5-tetrahydrobenzo[b][1,4]oxazepine-9-sulfonyl chloride

Under a nitrogen atmosphere, n-butyllithium (2.5 M in n-hexane, 1.99 mL, 4.98 mmol) was added dropwise to a solution of compound **115e** (875 mg, 4.53 mmol) in tetrahydrofuran (9 mL) at 0 °C, and the reaction system was stirred at 0 °C for 1 h and then cooled to -78 °C. Subsequently, sulfuryl chloride (733 mg, 5.43 mmol) was added, and stirring was continued at - 78 °C for 15 min. After the reaction was completed, the mixture was quenched with a saturated aqueous ammonium chloride solution and extracted with ethyl acetate (30 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (petroleum ether:tetrahydrofuran = 2:1) to give compound **115f** (410 mg, yield: 31.0%).
$^1$HNMR (400 MHz, $CDCl_3$): δ77.14 (d, 1H), 6.71 (d, 1H), 4.25 (t, 2H), 3.91 (s, 3H), 3.10 (t, 2H), 2.89 (s, 3H), 2.10-2.05 (m, 2H).
MS m/z (ESI): 292.0 $[M+H]^+$.

Step g:

*N*-(6-((1*H*-Pyrazol-1-yl)methyl)-5-fluoro-4-methoxybenzo[*d*]isoxazol-3-yl)-8-methoxy-5-methyl-2,3,4,5-tetrahydrobenzo[b][1,4]oxazepine-9-sulfonamide

Sodium hydride (60%, 38 mg, 0.95 mmol) was added to a solution of **intermediate 2** (50 mg, 0.19 mmol) in N,N-dimethylformamide (1 mL) at 0 °C. The reaction system was stirred at 0 °C for 15 min. Then, compound **115f** (72 mg, 0.25 mmol) was added to the system, and stirring was continued for 15 min. After the reaction was completed, the system was quenched with a saturated aqueous sodium chloride solution, then adjusted to pH = 5 with acetic acid, and extracted with ethyl acetate (10 mL × 3). The organic layers were combined, washed with a saturated aqueous NaCl solution, and dried over anhydrous sodium sulfate. After filtration, the mother liquor was concentrated under reduced pressure. The resulting crude product was purified by high-performance liquid chromatography (Welchrom C18, 20 × 150 mm, 5 μm; mobile phase A: 5 mM aqueous formic acid solution, mobile phase B: acetonitrile; gradient ratio: phase B 5%-95%) to give compound **115** (28.7 mg, yield: 29.1%).
$^1$HNMR (400 MHz, DMSO-$d_6$): δ10.06 (brs, 1H), 7.85 (d, 1H), 7.49 (d, 1H), 7.09-7.07 (m, 1H), 6.88-6.75 (m, 2H), 6.30 (t, 1H), 5.49 (s, 2H), 4.03 (s, 3H), 3.96 (t, 2H), 3.68 (s, 3H), 2.93 (t, 2H), 2.76 (s, 3H), 1.89-1.87 (m, 2H).
MS m/z (ESI): 518.0 $[M+H]^+$.

**Example 74**

Synthesis of Compound 120

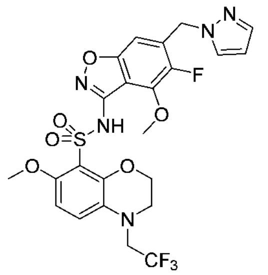

120

*N*-(6-((1*H*-Pyrazol-1-yl)methyl)-5-fluoro-4-methoxybenzo[*d*]isoxazol-3-yl)-7-methoxy-4-(2,2,2-trifluoroethyl)-3,4-dihydro-2*H*-benzo[*b*][1,4]oxazine-8-sulfonamide

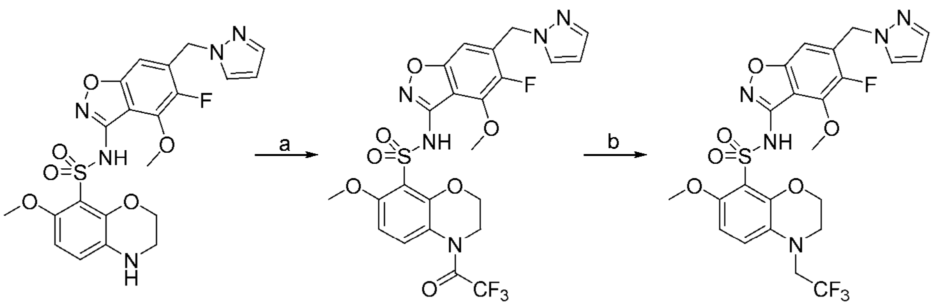


111 120a 120

Step a:

*N*-(6-((1*H*-Pyrazol-1-yl)methyl)-5-fluoro-4-methoxybenzo[*d*]isoxazol-3-yl)-7-methoxy-4-(2,2,2-trifluoroacetyl)-3,4-dihydro-2H-benzo[b][1,4]oxazine-8-sulfonamide

Under a nitrogen atmosphere, trifluoroacetic anhydride (103 mg, 0.49 mmol) was added to a solution of compound **111** (200 mg, 0.41 mmol) and triethylamine (62 mg, 0.61 mmol) in dichloromethane (3 mL) at 0 °C, and the reaction system was stirred at 25 °C for 1 h. After the reaction was completed, the mixture was quenched with an ice aqueous solution and extracted with dichloromethane (30 mL × 3). The organic layers were combined, washed with a saturated aqueous NaCl solution, and dried over anhydrous sodium sulfate. After filtration, the mother liquor was concentrated under reduced pressure. The resulting crude product was purified by flash silica gel column chromatography (dichloromethane:methanol = 19:1) to give compound **120a** (190 mg, yield: 79.4%).
MS m/z (ESI): 586.0 $[M+H]^+$.

Step b:

*N*-(6-((1*H*-Pyrazol-1-yl)methyl)-5-fluoro-4-methoxybenzo[*d*]isoxazol-3-yl)-7-methoxy-4-(2,2,2-trifluoroethyl)-3,4-dihydro-2*H*-benzo[*b*][1,4]oxazine-8-sulfonamide

Borane-tetrahydrofuran complex (1 M in tetrahydrofuran, 0.65 mL, 0.65 mmol) was added to a solution of compound **120a** (190 mg, 0.32 mmol) in tetrahydrofuran (5 mL) at 0 °C. The reaction system was heated to 60 °C and stirred for 6 h. After the reaction was completed, the mixture was quenched with a 3 M aqueous hydrochloric acid solution and extracted with dichloromethane (30 mL × 3). The organic layers were combined, washed with a saturated aqueous NaCl solution, and dried over anhydrous sodium sulfate. After filtration, the mother liquor was concentrated under reduced

pressure. The resulting crude product was purified by flash silica gel column chromatography (dichloromethane:methanol = 19:1) to give a crude product, which was then purified by high-performance liquid chromatography (Welchrom C18, 20 × 150 mm, 5 μm; mobile phase A: 5 mM aqueous formic acid solution, mobile phase B: acetonitrile; gradient ratio: phase B 5%-95%) to give compound **120** (31 mg, yield: 16.7%).

$^1$HNMR (400 MHz, DMSO-$d_6$): δ10.09 (brs, 1H), 7.86 (d, 1H), 7.50 (d, 1H), 7.06 (d, 1H), 6.89 (d, 1H), 6.64 (d, 1H), 6.30 (t, 1H), 5.50 (s, 2H), 4.16-4.09 (m, 4H), 4.05 (d, 3H), 3.66 (s, 3H), 3.37 (t, 2H).

MS m/z (ESI): 572.1 $[M+H]^+$.

**Example 75**

Synthesis of Compound 121

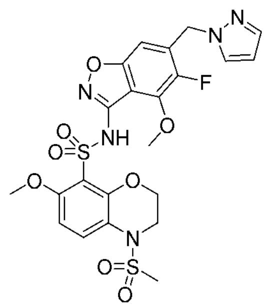

**121**

*N*-(6-((1*H*-Pyrazol-1-yl)methyl)-5-fluoro-4-methoxybenzo[*d*]isoxazol-3-yl)-7-methoxy-4-(methylsulfonyl)-3,4-dihydro-2*H*-benzo[*b*][1,4]oxazine-8-sulfonamide

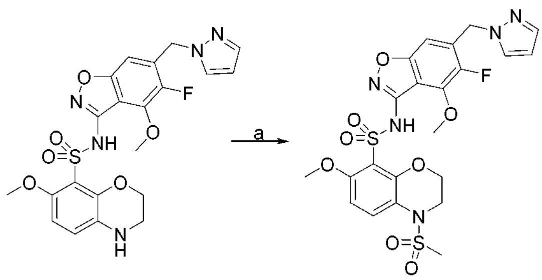


**111** **121**

Step a:

*N*-(6-((1*H*-Pyrazol-1-yl)methyl)-5-fluoro-4-methoxybenzo[*d*]isoxazol-3-yl)-7-methoxy-4-(methylsulfonyl)-3,4-dihydro-2*H*-benzo[*b*][1,4]oxazine-8-sulfonamide

Triethylamine (87 mg, 0.86 mmol) and methanesulfonyl chloride (98 mg, 0.86 mmol) were added to a solution of compound **111** (140 mg, 0.29 mmol) in dichloromethane (25 mL). The reaction system was stirred at room temperature for 16 h. After the reaction was completed, the mixture was diluted with an aqueous solution and extracted with dichloromethane (30 mL × 3). The organic layers were combined, washed with a saturated aqueous NaCl solution, and dried over anhydrous sodium sulfate. After filtration, the mother liquor was concentrated under reduced pressure. The resulting crude product was recrystallized from ethanol to give compound **121** (32 mg, yield: 19.7%).

$^1$HNMR (400 MHz, DMSO-$d_6$): δ10.44 (s, 1H), 7.86 (d, 1H), 7.69 (d, 1H), 7.50 (d, 1H), 6.93 (d, 1H), 6.79 (d, 1H), 6.31 (t, 1H), 5.50 (s, 2H), 4.27 (t, 2H), 4.02 (d, 3H), 3.75-3.72 (m, 5H), 3.07 (s, 3H).

MS m/z (ESI): 568.0 $[M+H]^+$.

Table 2. Typical compounds prepared in the present disclosure

| Compound No. | Structural formula | Chemical name | MS: (M + H)+ |
|---|---|---|---|
| 1 | 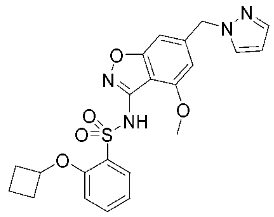 | *N*-(6-((1*H*-Pyrazol-1-yl)methyl)-4-methoxybenzo[*d*]isoxazol-3-yl)-2-cyclobutoxybenzenesulfonamide | 455.0 |
| 2 | 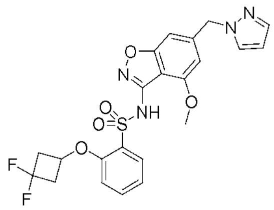 | *N*-(6-((1*H*-Pyrazol-1-yl)methyl)-4-methoxybenzo[*d*]isoxazol-3-yl)-2-(3,3-difluorocyclobutoxy)benzenesulfonamide | 491.0 |
| 3 | 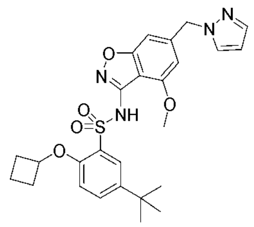 | *N*-(6-((1*H*-Pyrazol-1-yl)methyl)-4-methoxybenzo[*d*]isoxazol-3-yl)-5-(tert-butyl)-2-cyclobutoxybenzenesulfonamide | 511.1 |
| 4 | 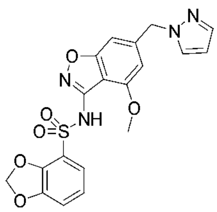 | *N*-(6-((1*H*-Pyrazol-1-yl)methyl)-4-methoxybenzo[*d*]isoxazol-3-yl)benzo[d][1,3]dioxole-4-sulfonamide | 428.9 |
| 5 | 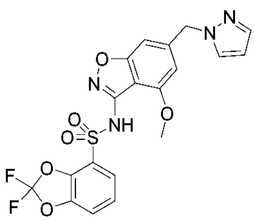 | *N*-(6-((1*H*-Pyrazol-1-yl)methyl)-4-methoxybenzo[*d*]isoxazol-3-yl)-2,2-difluorobenzo[*d*][1,3]dioxole-4-sulfonamide | 464.9 |
| 6 | 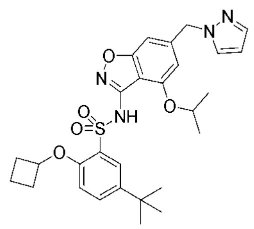 | *N*-(6-((1*H*-Pyrazol-1-yl)methyl)-4-isopropoxybenzo[*d*]isoxazol-3-yl)-5-(*tert*-butyl)-2-cyclobutoxybenzenesulfonamide | 539.2 |

(continued)

| Compound No. | Structural formula | Chemical name | MS: (M + H)+ |
|---|---|---|---|
| 7 | 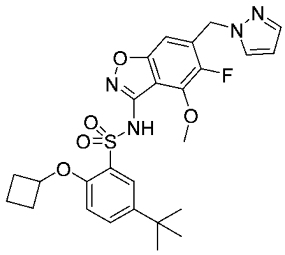 | *N*-(6-((1*H*-Pyrazol-1-yl)methyl)-5-fluoro-4-methoxybenzo [*d*]isoxazol-3-yl)-5-(tert-butyl)-2-cyclobutoxybenzenesulfonamide | 529.0 |
| 8 | 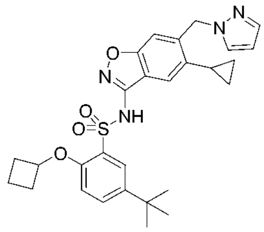 | *N*-(6-((1*H*-Pyrazol-1-yl)methyl)-5-*cyclopropylbenzo*[*d*]isoxazol-3-yl)-5-(*tert*-butyl)-2-cyclobutoxybenzenesulfonamide | 521.1 |
| 9 | 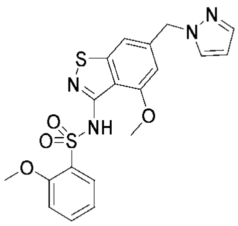 | *N*-(6-((1*H*-Pyrazol-1-yl)methyl)-4-methoxybenzo[*d*]isothiazol-3-yl)-2-methoxybenzenesulfonamide | 430.9 |
| 10 | 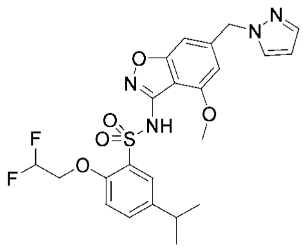 | *N*-(6-((1*H*-Pyrazol-1-yl)methyl)-4-methoxybenzo[*d*]isoxazol-3-yl)-2-(2,2-difluoroethoxy)-5-isopropylbenzenesulfonamide | 507.0 |
| 11 | 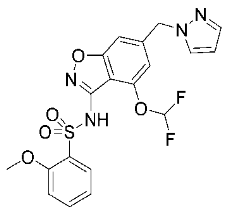 | *N*-(6-((1*H*-Pyrazol-1-yl)methyl)-4-(difluoromethoxy)benzo [*d*]isoxazol-3-yl)-2-methoxybenzenesulfonamide | 450.9 |
| 12 | 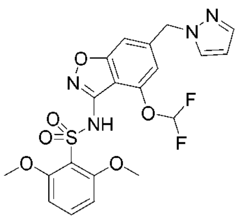 | *N*-(6-((1*H*-Pyrazol-1-yl)methyl)-4-(difluoromethoxy)benzo [*d*]isoxazol-3-yl)-2,6-dimethoxybenzenesulfonamide | 481.0 |

(continued)

| Compound No. | Structural formula | Chemical name | MS: (M + H)+ |
|---|---|---|---|
| 13 | 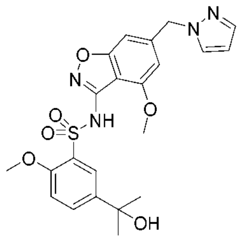 | *N*-(6-((1*H*-Pyrazol-1-yl)methyl)-4-methoxybenzo[*d*]isoxazol-3-yl)-5-(2-hydroxypropan-2-yl)-2-methoxybenzenesulfonamide | 473.1 |
| 14 | 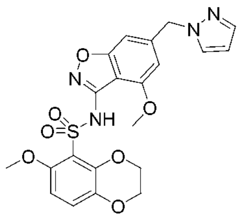 | *N*-(6-((1*H*-Pyrazol-1-yl)methyl)-4-methoxybenzo[*d*]isoxazol-3-yl)-6-methoxy-2,3-dihydrobenzo[*b*][1,4]dioxine-5-sulfonamide | 473.0 |
| 15 | 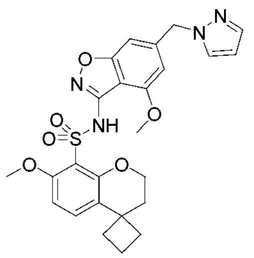 | N-(6-((1H-Pyrazol-1-yl)methyl)-4-methoxybenzo[d]isoxazol-3-yl)-7-methoxyspiro[chromane-4,1'-cyclobutane]-8-sulfonamide | 511.0 |
| 16 | 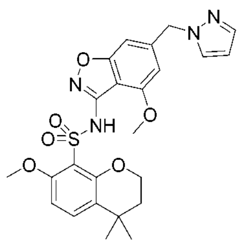 | *N*-(6-((1*H*-Pyrazol-1-yl)methyl)-4-methoxybenzo[*d*]isoxazol-3-yl)-7-methoxy-4,4-dimethylchromane-8-sulfonamide | 499.0 |
| 17 | 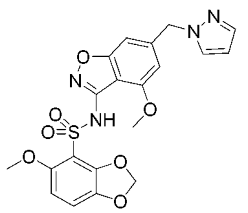 | *N*-(6-((1*H*-Pyrazol-1-yl)methyl)-4-methoxybenzo[*d*]isoxazol-3-yl)-5-methoxybenzo[*d*][1,3]dioxole-4-sulfonamide | 459.0 |
| 18 | 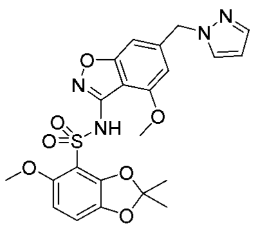 | *N*-(6-((1*H*-Pyrazol-1-yl)methyl)-4-methoxybenzo[*d*]isoxazol-3-yl)-5-methoxy-2,2-dimethylbenzo[*d*][1,3]dioxole-4-sulfonamide | 487.0 |

(continued)

| Compound No. | Structural formula | Chemical name | MS: (M + H)+ |
|---|---|---|---|
| 19 | 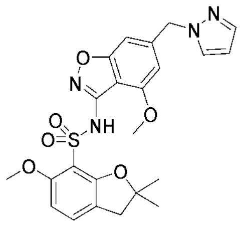 | *N*-(6-((1*H*-Pyrazol-1-yl)methyl)-4-methoxybenzo[*d*]isoxazol-3-yl)-6-methoxy-2,2-dimethyl-2,3-dihydrobenzofuran-7-sulfonamide | 485.0 |
| 20 | 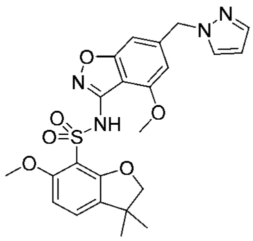 | *N*-(6-((1*H*-Pyrazol-1-yl)methyl)-4-methoxybenzo[*d*]isoxazol-3-yl)-6-methoxy-3,3-dimethyl-2,3-dihydrobenzofuran-7-sulfonamide | 485.0 |
| 21 | 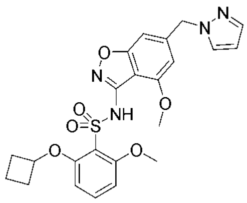 | *N*-(6-((1*H*-Pyrazol-1-yl)methyl)-4-methoxybenzo[*d*]isoxazol-3-yl)-2-cyclobutoxy-6-methoxybenzenesulfonamide | 485.0 |
| 22 | 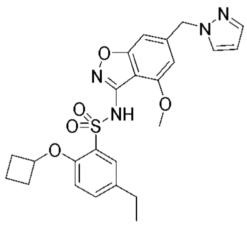 | *N*-(6-((1*H*-Pyrazol-1-yl)methyl)-4-methoxybenzo[*d*]isoxazol-3-yl)-2-cyclobutoxy-5-ethylbenzenesulfonamide | 483.0 |
| 23 | 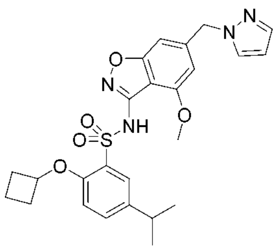 | *N*-(6-((1*H*-Pyrazol-1-yl)methyl)-4-methoxybenzo[*d*]isoxazol-3-yl)-2-cyclobutoxy-5-isopropylbenzenesulfonamide | 497.0 |
| 24 | 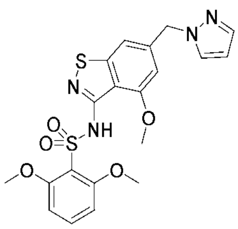 | *N*-(6-((1*H*-Pyrazol-1-yl)methyl)-4-methoxybenzo[*d*]isothiazol-3-yl)-2,6-dimethoxybenzenesulfonamide | 460.9 |

(continued)

| Compound No. | Structural formula | Chemical name | MS: (M + H)+ |
|---|---|---|---|
| 25 | 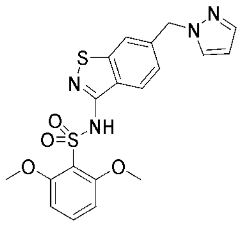 | *N*-(6-((1*H*-Pyrazol-1-yl)methyl)benzo[*d*]isothiazol-3-yl)-2,6-dimethoxybenzenesulfonamide | 430.9 |
| 26 | 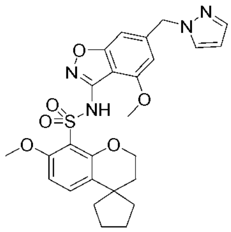 | *N*-(6-((1*H*-Pyrazol-1-yl)methyl)-4-methoxybenzo[*d*]isoxazol-3-yl)-7-methoxyspiro[chromane-4,1'-cyclopentane]-8-sulfonamide | 525.0 |
| 27 | 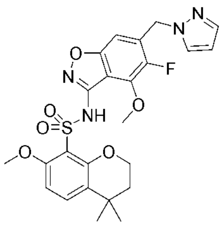 | *N*-(6-((1*H*-Pyrazol-1-yl)methyl)-5-fluoro-4-methoxybenzo[*d*]isoxazol-3-yl)-7-methoxy-4,4-dimethylchromane-8-sulfonamide | 517.1 |
| 28 | 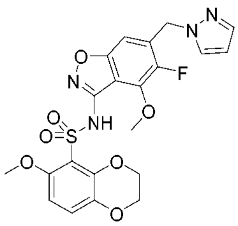 | *N*-(6-((1*H*-Pyrazol-1-yl)methyl)-5-fluoro-4-methoxybenzo[*d*]isoxazol-3-yl)-6-methoxy-2,3-dihydrobenzo[*b*][1,4]dioxine-5-sulfonamide | 491.1 |
| 29 | 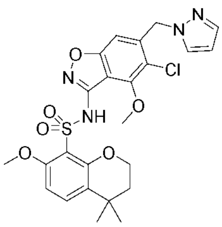 | *N*-(6-((1*H*-Pyrazol-1-yl)methyl)-5-chloro-4-methoxybenzo[*d*]isoxazol-3-yl)-7-methoxy-4,4-dimethylchromane-8-sulfonamide | 533.1 |
| 30 | 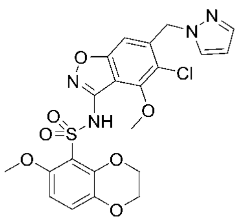 | *N*-(6-((1*H*-Pyrazol-1-yl)methyl)-5-chloro-4-methoxybenzo[*d*]isoxazol-3-yl)-6-methoxy-2,3-dihydrobenzo[*b*][1,4]dioxine-5-sulfonamide | 507.0 |

(continued)

| Compound No. | Structural formula | Chemical name | MS: (M + H)+ |
|---|---|---|---|
| 31 | 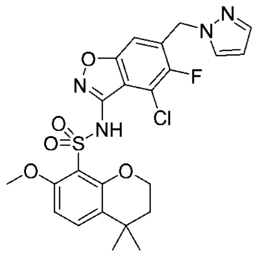 | *N*-(6-((1*H*-Pyrazol-1-yl)methyl)-4-chloro-5-fluorobenzo[*d*]isoxazol-3-yl)-7-methoxy-4,4-dimethylchromane-8-sulfonamide | 521.1 |
| 32 | 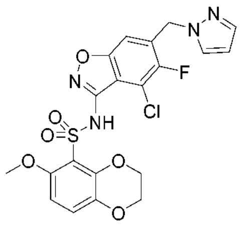 | *N*-(6-((1*H*-Pyrazol-1-yl)methyl)-4-chloro-5-fluorobenzo[*d*]isoxazol-3-yl)-6-methoxy-2,3-dihydrobenzo[*b*][1,4]dioxine-5-sulfonamide | 495.0 |
| 33 | 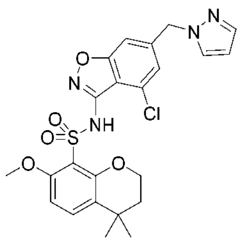 | *N*-(6-((1*H*-Pyrazol-1-yl)methyl)-4-chlorobenzo[*d*]isoxazol-3-yl)-7-methoxy-4,4-dimethylchromane-8-sulfonamide | 503.1 |
| 34 | 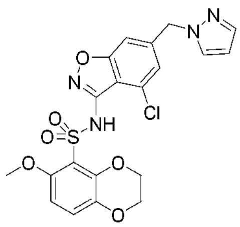 | *N*-(6-((1*H*-Pyrazol-1-yl)methyl)-4-chlorobenzo[*d*]isoxazol-3-yl)-6-methoxy-2,3-dihydrobenzo[*b*][1,4]dioxine-5-sulfonamide | 477.0 |
| 35 | 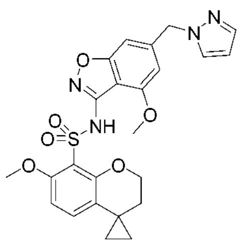 | *N*-(6-((1*H*-Pyrazol-1-yl)methyl)-4-methoxybenzo[*d*]isoxazol-3-yl)-7-methoxyspiro[chromane-4,1'-cyclopropane]-8-sulfonamide | 497.1 |
| 36 | 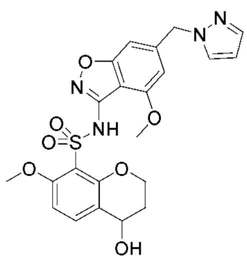 | *N*-(6-((1*H*-Pyrazol-1-yl)methyl)-4-methoxybenzo[*d*]isoxazol-3-yl)-4-hydroxy-7-methoxychromane-8-sulfonamide | 487.0 |

(continued)

| Compound No. | Structural formula | Chemical name | MS: (M + H)+ |
|---|---|---|---|
| 37 | 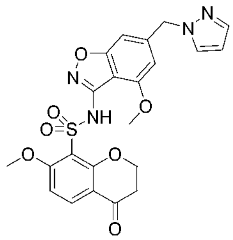 | *N*-(6-((1*H*-Pyrazol-1-yl)methyl)-4-methoxybenzo[*d*]isoxazol-3-yl)-7-methoxy-4-oxochromane-8-sulfonamide | 485.1 |
| 38 | 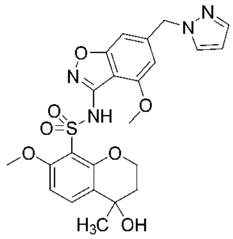 | *N*-(6-((1*H*-Pyrazol-1-yl)methyl)-4-methoxybenzo[*d*]isoxazol-3-yl)-4-hydroxy-7-methoxy-4-methylchromane-8-sulfonamide | 501.1 |
| 39 | 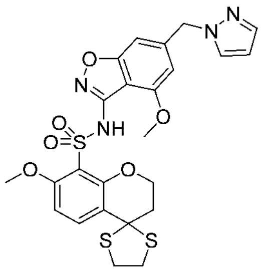 | *N*-(6-((1*H*-Pyrazol-1-yl)methyl)-4-methoxybenzo[*d*]isoxazol-3-yl)-7-methoxyspiro[chromane-4,2'-[1,3]dithiolane]-8-sulfonamide | 561.1 |
| 40 | 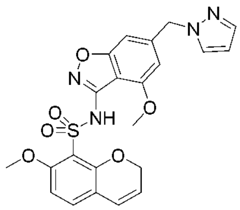 | *N*-(6-((1*H*-Pyrazol-1-yl)methyl)-4-methoxybenzo[*d*]isoxazol-3-yl)-7-methoxy-2H-chromene-8-sulfonamide | 469.1 |
| 41 | 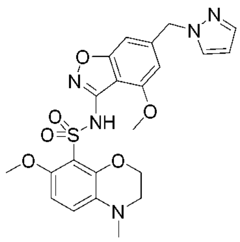 | *N*-(6-((1*H*-Pyrazol-1-yl)methyl)-4-methoxybenzo[*d*]isoxazol-3-yl)-7-methoxy-4-methyl-3,4-dihydro-2H-benzo[*b*][1,4]oxazine-8-sulfonamide | 486.1 |
| 42 | 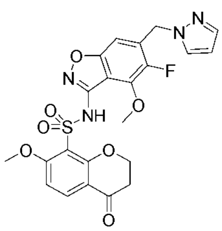 | *N*-(6-((1*H*-Pyrazol-1-yl)methyl)-5-fluoro-4-methoxybenzo[*d*]isoxazol-3-yl)-7-methoxy-4-oxochromane-8-sulfonamide | 503.1 |

(continued)

| Compound No. | Structural formula | Chemical name | MS: (M + H)+ |
|---|---|---|---|
| 43 | 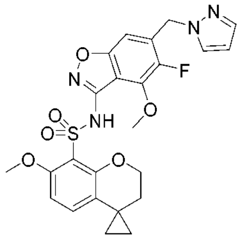 | *N*-(6-((1*H*-Pyrazol-1-yl)methyl)-5-fluoro-4-methoxybenzo [*d*]isoxazol-3-yl)-7-methoxyspiro[chromane-4,1'-cyclopropane]-8-sulfonamide | 515.1 |
| 44 | 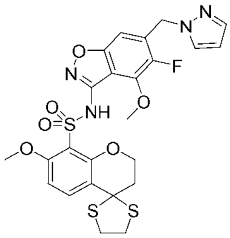 | *N*-(6-((1*H*-Pyrazol-1-yl)methyl)-5-fluoro-4-methoxybenzo [*d*]isoxazol-3-yl)-7-methoxyspiro[chromane-4,2'-[1,3] dithiolane]-8-sulfonamide | 579.1 |
| 45 | 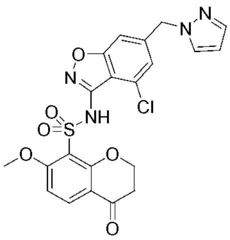 | *N*-(6-((1*H*-Pyrazol-1-yl)methyl)-4-chlorobenzo[*d*]isoxazol-3-yl)-7-methoxy-4-oxochromane-8-sulfonamide | 489.0 |
| 46 | 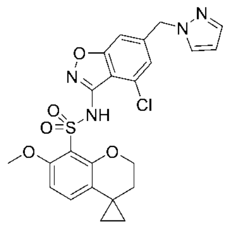 | *N*-(6-((1*H*-Pyrazol-1-yl)methyl)-4-chlorobenzo[*d*]isoxazol-3-yl)-7-methoxyspiro[chromane-4,1'-cyclopropane]-8-sulfonamide | 501.0 |
| 47 | 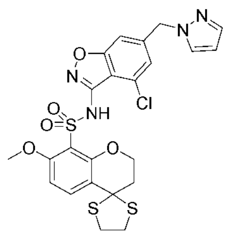 | *N*-(6-((1*H*-Pyrazol-1-yl)methyl)-4-chlorobenzo[*d*]isoxazol-3-yl)-7-methoxyspiro[chromane-4,2'-[1,3]dithiolane]-8-sulfonamide | 565.0 |
| 48 | 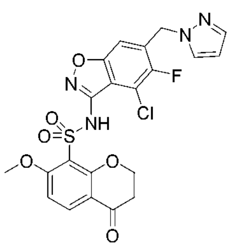 | *N*-(6-((1*H*-Pyrazol-1-yl)methyl)-4-chloro-5-fluorobenzo[*d*] isoxazol-3-yl)-7-methoxy-4-oxochromane-8-sulfonamide | 507.0 |

(continued)

| Compound No. | Structural formula | Chemical name | MS: (M + H)⁺ |
|---|---|---|---|
| 49 | 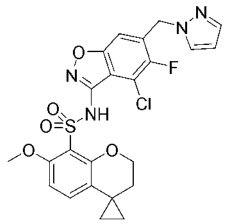 | *N*-(6-((1*H*-Pyrazol-1-yl)methyl)-4-chloro-5-fluorobenzo[*d*]isoxazol-3-yl)-7-methoxyspiro[chromane-4,1'-cyclopropane]-8-sulfonamide | 519.0 |
| 50 | 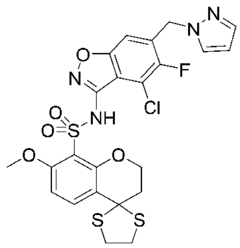 | *N*-(6-((1*H*-Pyrazol-1-yl)methyl)-4-chloro-5-fluorobenzo[*d*]isoxazol-3-yl)-7-methoxyspiro[chromane-4,2'-[1,3]dithiolane]-8-sulfonamide | 583.0 |
| 51 | 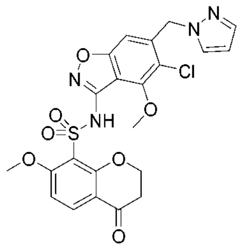 | *N*-(6-((1*H*-Pyrazol-1-yl)methyl)-5-chloro-4-methoxybenzo[*d*]isoxazol-3-yl)-7-methoxy-4-oxochromane-8-sulfonamide | 519.0 |
| 52 | 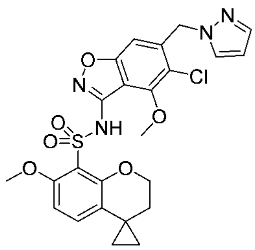 | *N*-(6-((1*H*-Pyrazol-1-yl)methyl)-5-chloro-4-methoxybenzo[*d*]isoxazol-3-yl)-7-methoxyspiro[chromane-4,1'-cyclopropane]-8-sulfonamide | 531.0 |
| 53 | 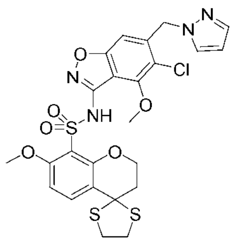 | *N*-(6-((1*H*-Pyrazol-1-yl)methyl)-5-chloro-4-methoxybenzo[*d*]isoxazol-3-yl)-7-methoxyspiro[chromane-4,2'-[1,3]dithiolane]-8-sulfonamide | 595.0 |
| 54 | 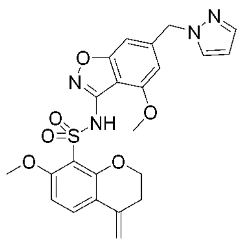 | *N*-(6-((1*H*-Pyrazol-1-yl)methyl)-4-methoxy-benzo[*d*]isoxazol-3-yl)-7-methoxy-4-methylenechromane-8-sulfonamide | 483.1 |

(continued)

| Compound No. | Structural formula | Chemical name | MS: (M + H)+ |
|---|---|---|---|
| 55 | 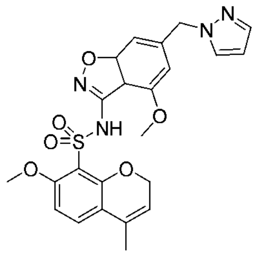 | *N*-(6-((1*H*-Pyrazol-1-yl)methyl)-4-methoxy-3a,7a-dihydrobenzo[*d*]isoxazol-3-yl)-7-methoxy-4-methyl-2*H*-chromene-8-sulfonamide | 483.1 |
| 56 | 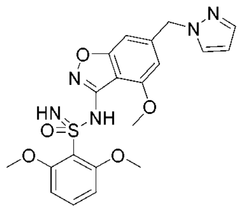 | *N*-(6-((1*H*-Pyrazol-1-yl)methyl)-4-methoxybenzo[*d*]isoxazol-3-yl)-2,6-dimethoxybenzenesulfonimidamide | 444.1 |
| 57 | 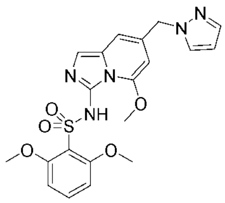 | *N*-(7-((1*H*-Pyrazol-1-yl)methyl)-5-methoxyimidazo[1,5-*a*]pyridin-3-yl)-2,6-dimethoxybenzenesulfonamide | 444.1 |
| 58 | 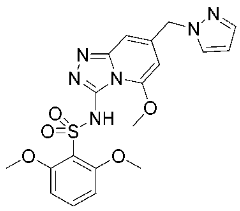 | *N*-(7-((1*H*-Pyrazol-1-yl)methyl)-5-methoxy-[1,2,4]triazolo[4,3-*a*]pyridin-3-yl)-2,6-dimethoxybenzenesulfonamide | 445.1 |
| 59 | 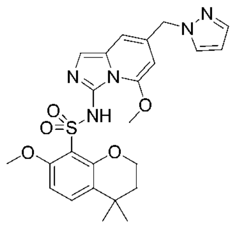 | *N*-(7-((1*H*-Pyrazol-1-yl)methyl)-5-methoxyimidazo[1,5-*a*]pyridin-3-yl)-7-methoxy-4,4-dimethylchromane-8-sulfonamide | 498.2 |
| 60 | 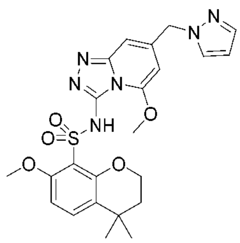 | *N*-(7-((1*H*-Pyrazol-1-yl)methyl)-5-methoxy-[1,2,4]triazolo[4,3-*a*]pyridin-3-yl)-7-methoxy-4,4-dimethylchromane-8-sulfonamide | 499.2 |

(continued)

| Compound No. | Structural formula | Chemical name | MS: (M + H)+ |
|---|---|---|---|
| 61 | 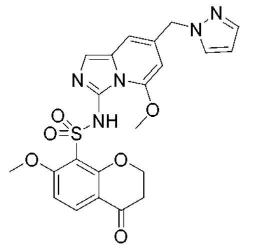 | *N*-(7-((1*H*-Pyrazol-1-yl)methyl)-5-methoxyimidazo[1,5-*a*]pyridin-3-yl)-7-methoxy-4-oxochromane-8-sulfonamide | 484.1 |
| 62 | 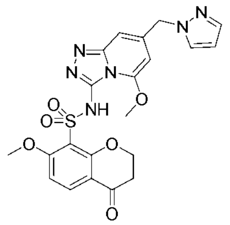 | *N*-(7-((1*H*-Pyrazol-1-yl)methyl)-5-methoxy-[1,2,4]triazolo[4,3-*a*]pyridin-3-yl)-7-methoxy-4-oxochromane-8-sulfonamide | 485.1 |
| 63 | 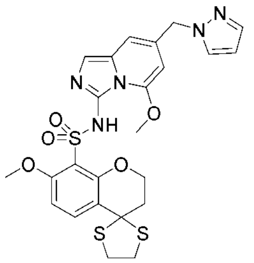 | *N*-(7-((1*H*-Pyrazol-1-yl)methyl)-5-methoxyimidazo[1,5-*a*]pyridin-3-yl)-7-methoxyspiro[chromane-4,2'-[1,3]dithiolane]-8-sulfonamide | 560.1 |
| 64 | 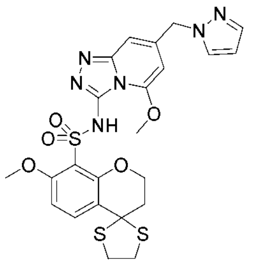 | *N*-(7-((1*H*-Pyrazol-1-yl)methyl)-5-methoxy-[1,2,4]triazolo[4,3-*a*]pyridin-3-yl)-7-methoxyspiro[chromane-4,2'-[1,3]dithiolane]-8-sulfonamide | 561.1 |
| 65 | 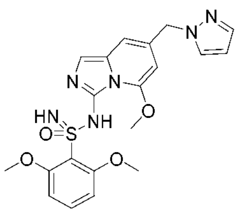 | *N*-(7-((1*H*-Pyrazol-1-yl)methyl)-5-methoxyimidazo[1,5-*a*]pyridin-3-yl)-2,6-dimethoxybenzenesulfonimidamide | 443.1 |
| 66 | 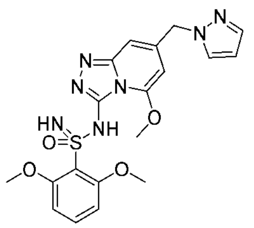 | *N*-(7-((1*H*-Pyrazol-1-yl)methyl)-5-methoxy-[1,2,4]triazolo[4,3-*a*]pyridin-3-yl)-2,6-dimethoxybenzenesulfonimidamide | 444.1 |

(continued)

| Compound No. | Structural formula | Chemical name | MS: (M + H)+ |
|---|---|---|---|
| 67 | 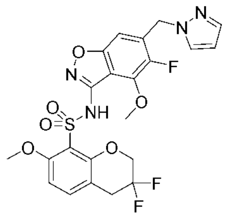 | *N*-(6-((1*H*-Pyrazol-1-yl)methyl)-5-fluoro-4-methoxybenzo[*d*]isoxazol-3-yl)-3,3-difluoro-7-methoxychromane-8-sulfonamide | 425.1 |
| 68 | 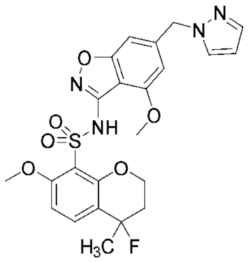 | *N*-(6-((1*H*-Pyrazol-1-yl)methyl)-4-methoxybenzo[*d*]isoxazol-3-yl)-4-fluoro-7-methoxy-4-methylchromane-8-sulfonamide | 503.1 |
| 69 | 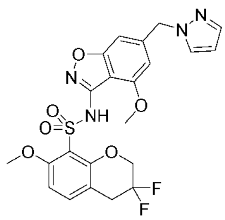 | *N*-(6-((1*H*-Pyrazol-1-yl)methyl)-4-methoxybenzo[*d*]isoxazol-3-yl)-3,3-difluoro-7-methoxychromane-8-sulfonamide | 507.1 |
| 70 | 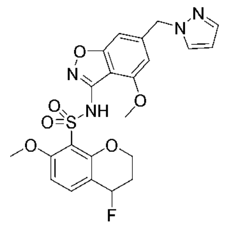 | *N*-(6-((1*H*-Pyrazol-1-yl)methyl)-4-methoxybenzo[*d*]isoxazol-3-yl)-4-fluoro-7-methoxychromane-8-sulfonamide | 491.1 |
| 71 | 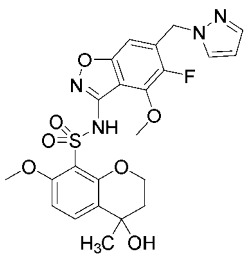 | *N*-(6-((1*H*-Pyrazol-1-yl)methyl)-5-fluoro-4-methoxybenzo[*d*]isoxazol-3-yl)-4-hydroxy-7-methoxy-4-methylchromane-8-sulfonamide | 519.1 |
| 72 | 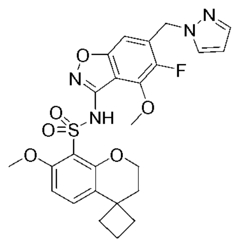 | *N*-(6-((1*H*-Pyrazol-1-yl)methyl)-5-fluoro-4-methoxybenzo[*d*]isoxazol-3-yl)-7-methoxyspiro[chromane-4,1'-cyclobutane]-8-sulfonamide | 529.1 |

(continued)

| Compound No. | Structural formula | Chemical name | MS: (M + H)+ |
|---|---|---|---|
| 73 | 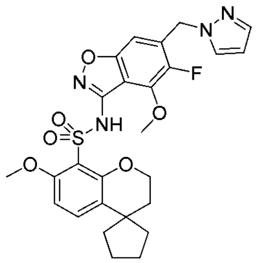 | *N*-(6-((1*H*-Pyrazol-1-yl)methyl)-5-fluoro-4-methoxybenzo[*d*]isoxazol-3-yl)-7-methoxyspiro[chromane-4,1'-cyclopentane]-8-sulfonamide | 543.1 |
| 74 | 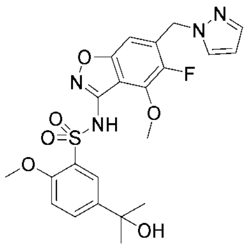 | *N*-(6-((1*H*-Pyrazol-1-yl)methyl)-5-fluoro-4-methoxybenzo[*d*]isoxazol-3-yl)-5-(2-hydroxypropan-2-yl)-2-methoxybenzenesulfonamide | 491.1 |
| 75 | 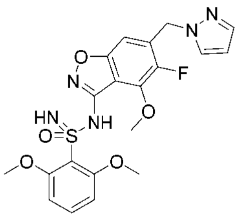 | *N*-(6-((1*H*-Pyrazol-1-yl)methyl)-5-fluoro-4-methoxybenzo[*d*]isoxazol-3-yl)-2,6-dimethoxybenzenesulfonimidamide | 462.1 |
| 76 | 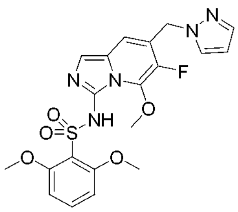 | *N*-(7-((1*H*-Pyrazol-1-yl)methyl)-6-fluoro-5-methoxyimidazo[1,5-*a*]pyridin-3-yl)-2,6-dimethoxybenzenesulfonamide | 462.1 |
| 77 | 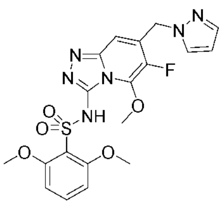 | *N*-(7-((1*H*-Pyrazol-1-yl)methyl)-6-fluoro-5-methoxy-[1,2,4]triazolo[4,3-*a*]pyridin-3-yl)-2,6-dimethoxybenzenesulfonamide | 463.1 |
| 78 | 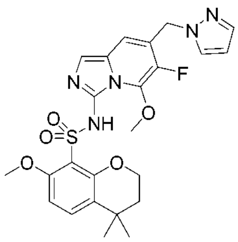 | *N*-(7-((1*H*-Pyrazol-1-yl)methyl)-6-fluoro-5-methoxyimidazo[1,5-*a*]pyridin-3-yl)-7-methoxy-4,4-dimethylchromane-8-sulfonamide | 516.2 |

(continued)

| Compound No. | Structural formula | Chemical name | MS: (M + H)+ |
|---|---|---|---|
| 79 | 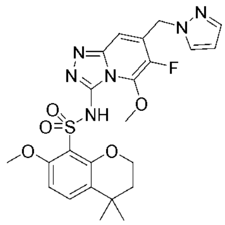 | *N*-(7-((1*H*-Pyrazol-1-yl)methyl)-6-fluoro-5-methoxy-[1,2,4]triazolo[4,3-*a*]pyridin-3-yl)-7-methoxy-4,4-dimethylchromane-8-sulfonamide | 517.2 |
| 80 | 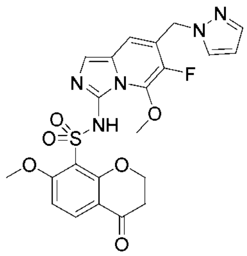 | *N*-(7-((1*H*-Pyrazol-1-yl)methyl)-6-fluoro-5-methoxyimidazo[1,5-*a*]pyridin-3-yl)-7-methoxy-4-oxochromane-8-sulfonamide | 502.1 |
| 81 | 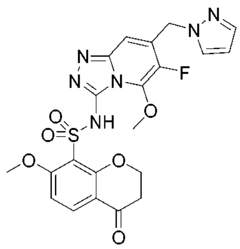 | *N*-(7-((1*H*-Pyrazol-1-yl)methyl)-6-fluoro-5-methoxy-[1,2,4]triazolo[4,3-*a*]pyridin-3-yl)-7-methoxy-4-oxochromane-8-sulfonamide | 503.1 |
| 82 | 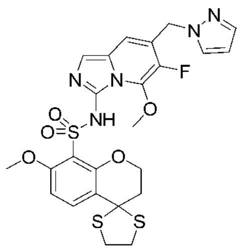 | *N*-(7-((1*H*-Pyrazol-1-yl)methyl)-6-fluoro-5-methoxyimidazo[1,5-*a*]pyridin-3-yl)-7-methoxyspiro[chromane-4,2'-[1,3]dithiolane]-8-sulfonamide | 578.1 |
| 83 | 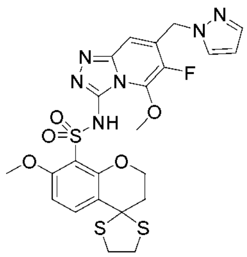 | *N*-(7-((1*H*-Pyrazol-1-yl)methyl)-6-fluoro-5-methoxy-[1,2,4]triazolo[4,3-*a*]pyridin-3-yl)-7-methoxyspiro[chromane-4,2'-[1,3]dithiolane]-8-sulfonamide | 579.1 |
| 84 | 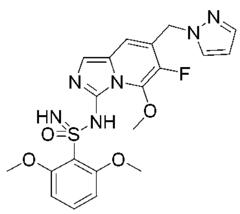 | *N*-(7-((1*H*-Pyrazol-1-yl)methyl)-6-fluoro-5-methoxyimidazo[1,5-*a*]pyridin-3-yl)-2,6-dimethoxybenzenesulfonimidamide | 461.1 |

(continued)

| Compound No. | Structural formula | Chemical name | MS: (M + H)+ |
|---|---|---|---|
| 85 | 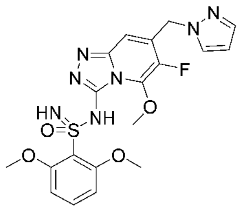 | *N*-(7-((1*H*-Pyrazol-1-yl)methyl)-6-fluoro-5-methoxy-[1,2,4]triazolo[4,3-*a*]pyridin-3-yl)-2,6-dimethoxybenzenesulfonimidamide | 462.1 |
| 86 | 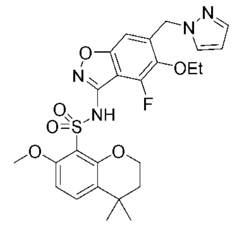 | *N*-(6-((1*H*-Pyrazol-1-yl)methyl)-5-ethoxy-4-fluorobenzo[d]isoxazol-3-yl)-7-methoxy-4,4-dimethylchromane-8-sulfonamide | 531.2 |
| 87 | 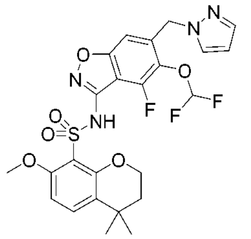 | *N*-(6-((1*H*-Pyrazol-1-yl)methyl)-5-(difluoromethoxy)-4-fluorobenzo[*d*]isoxazol-3-yl)-7-methoxy-4,4-dimethylchromane-8-sulfonamide | 553.1 |
| 88 | 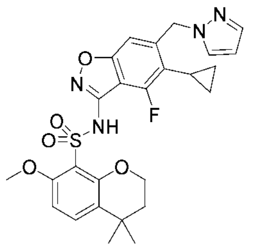 | *N*-(6-((1*H*-Pyrazol-1-yl)methyl)-5-cyclopropyl-4-fluorobenzo[d]isoxazol-3-yl)-7-methoxy-4,4-dimethylchromane-8-sulfonamide | 527.2 |
| 89 | 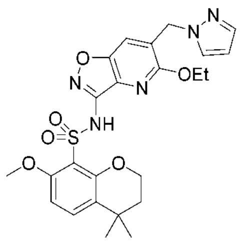 | *N*-(6-((1*H*-Pyrazol-1-yl)methyl)-5-ethoxyisoxazolo[4,5-*b*]pyridin-3-yl)-7-methoxy-4,4-dimethylchromane-8-sulfonamide | 514.2 |
| 90 | 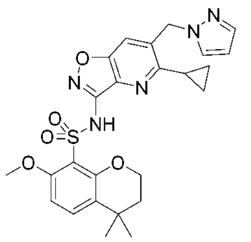 | *N*-(6-((1*H*-Pyrazol-1-yl)methyl)-5-cyclopropylisoxazolo[4,5-*b*]pyridin-3-yl)-7-methoxy-4,4-dimethylchromane-8-sulfonamide | 510.2 |

(continued)

| Compound No. | Structural formula | Chemical name | MS: (M + H)+ |
|---|---|---|---|
| 91 | 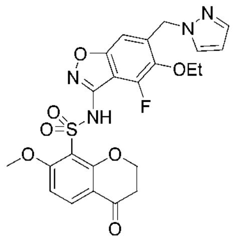 | *N*-(6-((1*H*-Pyrazol-1-yl)methyl)-5-ethoxy-4-fluorobenzo[*d*]isoxazol-3-yl)-7-methoxy-4-oxochromane-8-sulfonamide | 517.1 |
| 92 | 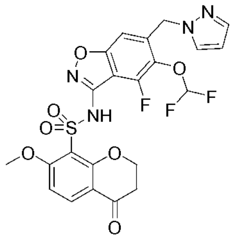 | *N*-(6-((1*H*-Pyrazol-1-yl)methyl)-5-(difluoromethoxy)-4-fluorobenzo[*d*]isoxazol-3-yl)-7-methoxy-4-oxochromane-8-sulfonamide | 539.1 |
| 93 | 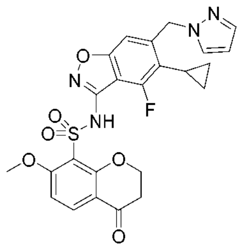 | *N*-(6-((1*H*-Pyrazol-1-yl)methyl)-5-cyclopropyl-4-fluorobenzo[*d*]isoxazol-3-yl)-7-methoxy-4-oxochromane-8-sulfonamide | 513.1 |
| 94 | 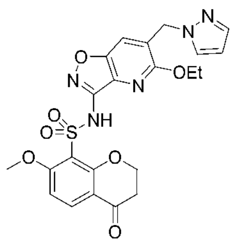 | *N*-(6-((1*H*-Pyrazol-1-yl)methyl)-5-ethoxyisoxazolo[4,5-*b*]pyridin-3-yl)-7-methoxy-4-oxochromane-8-sulfonamide | 500.1 |
| 95 | 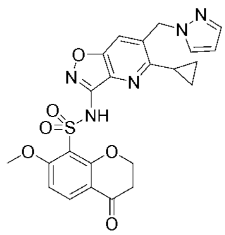 | *N*-(6-((1*H*-Pyrazol-1-yl)methyl)-5-cyclopropylisoxazolo[4,5-*b*]pyridin-3-yl)-7-methoxy-4-oxochromane-8-sulfonamide | 496.1 |
| 96 | 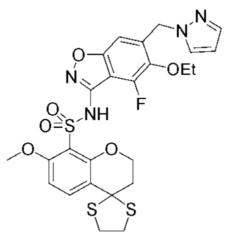 | *N*-(6-((1*H*-Pyrazol-1-yl)methyl)-5-ethoxy-4-fluorobenzo[*d*]isoxazol-3-yl)-7-methoxyspiro[chromane-4,2'-[1,3]dithiolane]-8-sulfonamide | 593.1 |

(continued)

| Compound No. | Structural formula | Chemical name | MS: (M + H)+ |
|---|---|---|---|
| 97 | 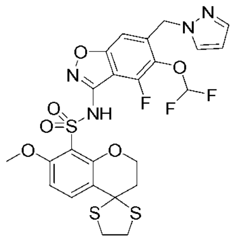 | *N*-(6-((1*H*-Pyrazol-1-yl)methyl)-5-(difluoromethoxy)-4-fluorobenzo[*d*]isoxazol-3-yl)-7-methoxyspiro[chromane-4,2'-[1,3]dithiolane]-8-sulfonamide | 615.1 |
| 98 | 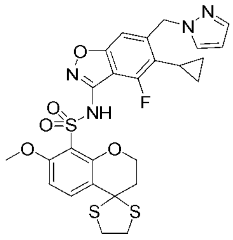 | *N*-(6-((1*H*-Pyrazol-1-yl)methyl)-5-cyclopropyl-4-fluorobenzo[*d*]isoxazol-3-yl)-7-methoxyspiro[chromane-4,2'-[1,3]dithiolane]-8-sulfonamide | 589.1 |
| 99 | 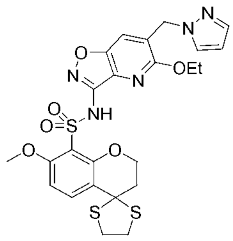 | *N*-(6-((1*H*-Pyrazol-1-yl)methyl)-5-ethoxyisoxazolo[4,5-*b*]pyridin-3-yl)-7-methoxyspiro[chromane-4,2'-[1,3]dithiolane]-8-sulfonamide | 576.1 |
| 100 | 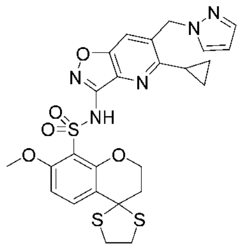 | *N*-(6-((1*H*-Pyrazol-1-yl)methyl)-5-cyclopropylisoxazolo[4,5-*b*]pyridin-3-yl)-7-methoxyspiro[chromane-4,2'-[1,3]dithiolane]-8-sulfonamide | 572.1 |
| 101 | 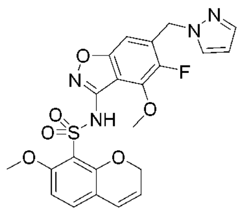 | *N*-(6-((1*H*-Pyrazol-1-yl)methyl)-5-fluoro-4-methoxybenzo[*d*]isoxazol-3-yl)-7-methoxy-2H-chromene-8-sulfonamide | 487.0 |
| 102 | 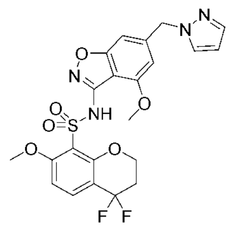 | *N*-(6-((1*H*-Pyrazol-1-yl)methyl)-4-methoxybenzo[*d*]isoxazol-3-yl)-4,4-difluoro-7-methoxychromane-8-sulfonamide | 506.8 |

(continued)

| Compound No. | Structural formula | Chemical name | MS: (M + H)+ |
|---|---|---|---|
| 103 | 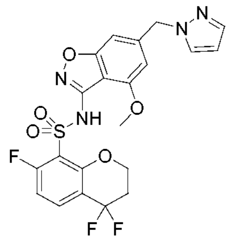 | *N*-(6-((1*H*-Pyrazol-1-yl)methyl)-4-methoxybenzo[*d*]isoxazol-3-yl)-4,4,7-trifluorochromane-8-sulfonamide | 494.8 |
| 104 | 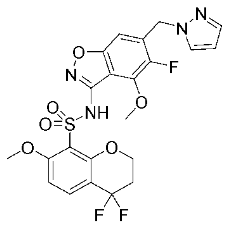 | *N*-(6-((1*H*-Pyrazol-1-yl)methyl)-5-fluoro-4-methoxybenzo [*d*]isoxazol-3-yl)-4,4-difluoro-7-methoxychromane-8-sulfonamide | 524.9 |
| 105 | 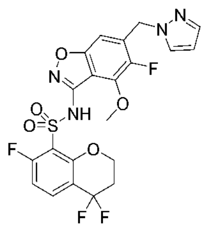 | *N*-(6-((1*H*-Pyrazol-1-yl)methyl)-5-fluoro-4-methoxybenzo [*d*]isoxazol-3-yl)-4,4,7-trifluorochromane-8-sulfonamide | 512.8 |
| 106 | 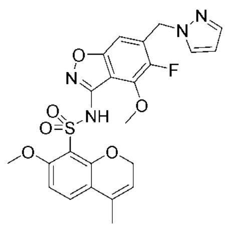 | *N*-(6-((1*H*-Pyrazol-1-yl)methyl)-5-fluoro-4-methoxybenzo [*d*]isoxazol-3-yl)-7-methoxy-4-methyl-2H-chromene-8-sulfonamide | 501.1 |
| 107 | 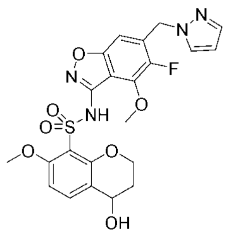 | *N*-(6-((1*H*-Pyrazol-1-yl)methyl)-5-fluoro-4-methoxybenzo [*d*]isoxazol-3-yl)-4-hydroxy-7-methoxychromane-8-sulfonamide | 505.1 |
| 108 | 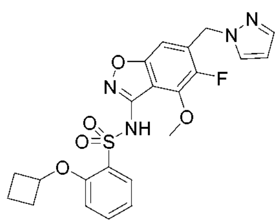 | *N*-(6-((1*H*-Pyrazol-1-yl)methyl)-5-fluoro-4-methoxybenzo [*d*]isoxazol-3-yl)-2-cyclobutoxybenzenesulfonamide | 472.9 |

(continued)

| Compound No. | Structural formula | Chemical name | MS: (M + H)⁺ |
|---|---|---|---|
| 109 | 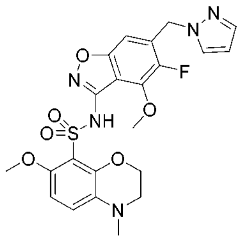 | *N*-(6-((1*H*-Pyrazol-1-yl)methyl)-5-fluoro-4-methoxybenzo[*d*]isoxazol-3-yl)-7-methoxy-4-methyl-3,4-dihydro-2H-benzo[*b*][1,4]oxazine-8-sulfonamide | 504.1 |
| 110 | 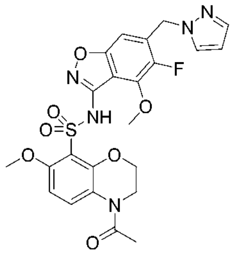 | *N*-(6-((1*H*-Pyrazol-1-yl)methyl)-5-fluoro-4-methoxybenzo[*d*]isoxazol-3-yl)-4-acetyl-7-methoxy-3,4-dihydro-2H-benzo[*b*][1,4]oxazine-8-sulfonamide | 532.1 |
| 111 | 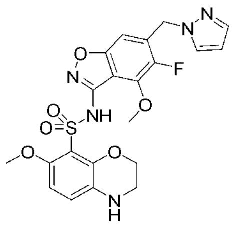 | *N*-(6-((1*H*-Pyrazol-1-yl)methyl)-5-fluoro-4-methoxybenzo[*d*]isoxazol-3-yl)-7-methoxy-3,4-dihydro-2H-benzo[b][1,4]oxazine-8-sulfonamide | 490.1 |
| 112 | 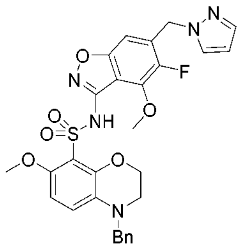 | *N*-(6-((1*H*-Pyrazol-1-yl)methyl)-5-fluoro-4-methoxybenzo[*d*]isoxazol-3-yl)-4-benzyl-7-methoxy-3,4-dihydro-2H-benzo[*b*][1,4]oxazine-8-sulfonamide | 580.2 |
| 113 | 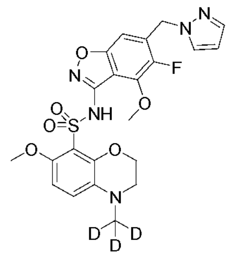 | *N*-(6-((1*H*-Pyrazol-1-yl)methyl)-5-fluoro-4-methoxybenzo[*d*]isoxazol-3-yl)-7-methoxy-4-(methyl-$d_3$)-3,4-dihydro-2*H*-benzo[*b*][1,4]oxazine-8-sulfonamide | 507.2 |
| 114 | 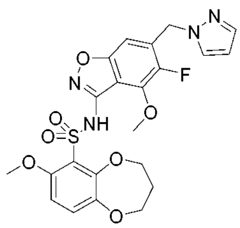 | *N*-(6-((1*H*-Pyrazol-1-yl)methyl)-5-fluoro-4-methoxybenzo[*d*]isoxazol-3-yl)-7-methoxy-3,4-dihydro-2*H*-benzo[*b*][1,4]dioxepine-6-sulfonamide | 505.0 |

(continued)

| Compound No. | Structural formula | Chemical name | MS: (M + H)+ |
|---|---|---|---|
| 115 | 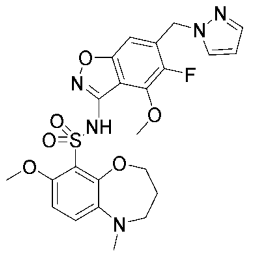 | *N*-(6-((1*H*-Pyrazol-1-yl)methyl)-5-fluoro-4-methoxybenzo[*d*]isoxazol-3-yl)-8-methoxy-5-methyl-2,3,4,5-tetrahydrobenzo[*b*][1,4]oxazepine-9-sulfonamide | 518.0 |
| 116 | 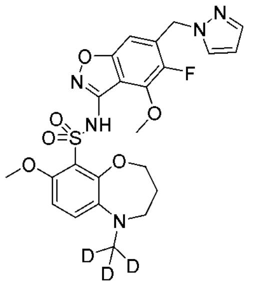 | *N*-(6-((1*H*-Pyrazol-1-yl)methyl)-5-fluoro-4-methoxybenzo[d]isoxazol-3-yl)-8-methoxy-5-(methyl-$d_3$)-2,3,4,5-tetrahydrobenzo[*b*][1,4]oxazepine-9-sulfonamide | 521.2 |
| 117 | 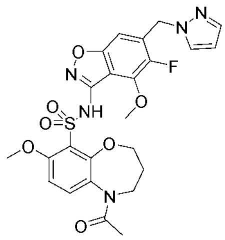 | *N*-(6-((1*H*-Pyrazol-1-yl)methyl)-5-fluoro-4-methoxybenzo[d]isoxazol-3-yl)-5-acetyl-8-methoxy-2,3,4,5-tetrahydrobenzo[*b*][1,4]oxazepine-9-sulfonamide | 546.1 |
| 118 | 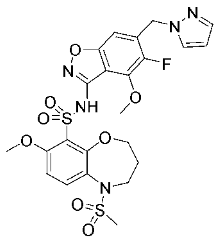 | *N*-(6-((1*H*-Pyrazol-1-yl)methyl)-5-fluoro-4-methoxybenzo[*d*]isoxazol-3-yl)-8-methoxy-5-(methylsulfonyl)-2,3,4,5-tetrahydrobenzo[*b*][1,4]oxazepine-9-sulfonamide | 582.1 |
| 119 | 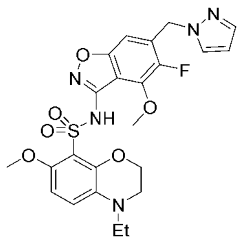 | *N*-(6-((1*H*-Pyrazol-1-yl)methyl)-5-fluoro-4-methoxybenzo[*d*]isoxazol-3-yl)-4-ethyl-7-methoxy-3,4-dihydro-2*H*-benzo[*b*][1,4]oxazine-8-sulfonamide | 518.1 |
| 120 | 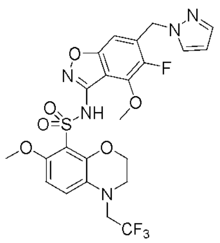 | *N*-(6-((1*H*-Pyrazol-1-yl)methyl)-5-fluoro-4-methoxybenzo[*d*]isoxazol-3-yl)-7-methoxy-4-(2,2,2-trifluoroethyl)-3,4-dihydro-2H-benzo[*b*][1,4]oxazine-8-sulfonamide | 572.1 |

(continued)

| Compound No. | Structural formula | Chemical name | MS: (M + H)+ |
|---|---|---|---|
| 121 | 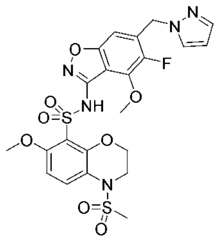 | *N*-(6-((1*H*-Pyrazol-1-yl)methyl)-5-fluoro-4-methoxybenzo[*d*]isoxazol-3-yl)-7-methoxy-4-(methylsulfonyl)-3,4-dihydro-2*H*-benzo[*b*][1,4]oxazine-8-sulfonamide | 568.0 |
| 122 | 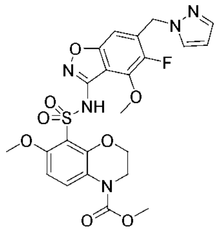 | Methyl 8-(*N*-(6-((1*H*-pyrazol-1-yl)methyl)-5-fluoro-4-methoxybenzo[*d*]isoxazol-3-yl)sulfamoyl)-7-methoxy-3,4-dihydro-4*H*-benzo[*b*][1,4]oxazine-4-carboxylate | 548.1 |
| 123 | 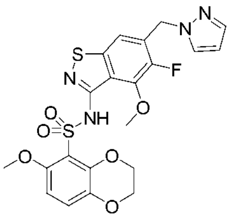 | *N*-(6-((1*H*-Pyrazol-1-yl)methyl)-5-fluoro-4-methoxybenzo[*d*]isothiazol-3-yl)-6-methoxy-2,3-dihydrobenzo[*b*][1,4]dioxine-5-sulfonamide | 507.1 |
| 124 | 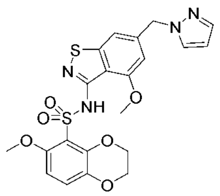 | *N*-(6-((1*H*-Pyrazol-1-yl)methyl)-4-methoxybenzo[d]isothiazol-3-yl)-6-methoxy-2,3-dihydrobenzo[*b*][1,4]dioxine-5-sulfonamide | 489.1 |
| 125 | 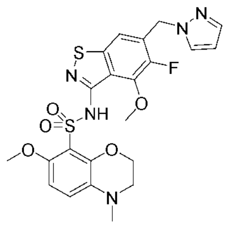 | *N*-(6-((1*H*-Pyrazol-1-yl)methyl)-5-fluoro-4-methoxybenzo[*d*]isothiazol-3-yl)-7-methoxy-4-methyl-3,4-dihydro-2*H*-benzo[*b*][1,4]oxazine-8-sulfonamide | 520.1 |
| 126 | 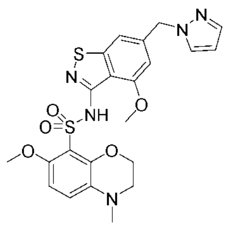 | *N*-(6-((1*H*-Pyrazol-1-yl)methyl)-4-methoxybenzo[*d*]isothiazol-3-yl)-7-methoxy-4-methyl-3,4-dihydro-2*H*-benzo[*b*][1,4]oxazine-8-sulfonamide | 502.1 |
| 127 | 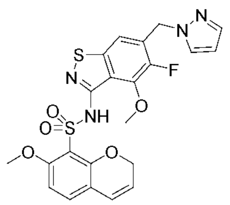 | *N*-(6-((1*H*-Pyrazol-1-yl)methyl)-5-fluoro-4-methoxybenzo[*d*]isothiazol-3-yl)-7-methoxy-2*H*-chromene-8-sulfonamide | 503.1 |

(continued)

| Compound No. | Structural formula | Chemical name | MS: (M + H)+ |
|---|---|---|---|
| 128 | 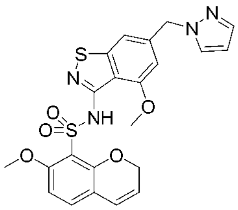 | *N*-(6-((1*H*-Pyrazol-1-yl)methyl)-4-methoxybenzo[*d*]isothiazol-3-yl)-7-methoxy-2*H*-chromene-8-sulfonamide | 485.1 |
| 129 | 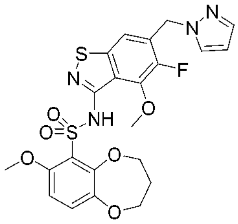 | *N*-(6-((1*H*-Pyrazol-1-yl)methyl)-5-fluoro-4-methoxybenzo[*d*]isothiazol-3-yl)-7-methoxy-3,4-dihydro-2*H*-benzo[*b*][1,4]dioxepine-6-sulfonamide | 521.1 |
| 130 | 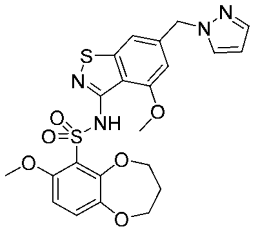 | *N*-(6-((1*H*-Pyrazol-1-yl)methyl)-4-methoxybenzo[*d*]isothiazol-3-yl)-7-methoxy-3,4-dihydro-2*H*-benzo[*b*][1,4]dioxepine-6-sulfonamide | 503.1 |
| 131 | 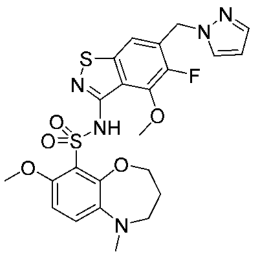 | *N*-(6-((1*H*-Pyrazol-1-yl)methyl)-5-fluoro-4-methoxybenzo[*d*]isothiazol-3-yl)-8-methoxy-5-methyl-2,3,4,5-tetrahydrobenzo[*b*][1,4]oxazepine-9-sulfonamide | 534.1 |
| 132 | 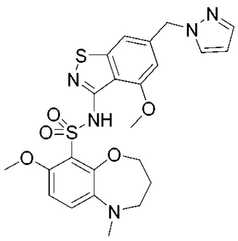 | *N*-(6-((1*H*-Pyrazol-1-yl)methyl)-4-methoxybenzo[*d*]isothiazol-3-yl)-8-methoxy-5-methyl-2,3,4,5-tetrahydrobenzo[*b*][1,4]oxazepine-9-sulfonamide | 516.1 |
| Note: Compounds other than Examples 1-75 included in Table 2 were prepared in a manner similar to that for Examples 1-75. | | | |

## Biological Evaluation

**Test Example 1:** Experiment for Determining Inhibitory Effects of Compounds on Proliferation of Breast Cancer Cell Line ZR-75-1

Experimental principle

ATP is an indicator of the metabolism of living cells. The CellTiter-Glo® Luminescent Cell Viability Assay is a homogeneous assay method for determining the number of viable cells by quantifying ATP.

Reagents and instruments

1) ZR-75-1 (Cell Bank of Chinese Academy of Sciences, TCHu126)
2) RPMI-1640 medium (Gibco, 11875-093)
3) Fetal Bovine Serum, New Zealand (Gibco,10091-148)
4) Penicillin-streptomycin (10,000 U/mL) (Gibco, 15140-122)
5) Trypsin-EDTA (0.25%), containing phenol red (Gibco, 25200-072)
6) DPBS (Shanghai BasalMedia, B210KJ)
7) Corning® 96-well Clear Flat Bottom Ultra-Low Attachment Microplate (Corning, Cat. No. 3474)
8) 330 μL 96 Holes Deep Well Plate, transparents, 96 Round Holes, V-shaped Bottom, Low Retention (Stellar Scientific, DP33VR-9-NS)
9) Corning® 96-well solid white flat-bottom polystyrene microplate (Corning, Cat. No. 3917)
10) CellTiter-Glo® Luminescent Cell Viability Assay (Promega, G7573)
11) Countstar® BioMed automatic cell counter
12) Trypan blue solution, 0.4% (Gibco, Cat. No. 15250061)
13) 37 °C constant-temperature incubator (Thermo, FORMA STERI-CYCLE i160 LK)
14) Biosafety cabinet (Thermo, 1379)

Experimental procedures:

Cell plating

1) The formula of the complete medium for ZR-75-1 was as follows: 500 mL RPMI-1640 + 10% FBS + 1% penicillin-streptomycin.
2) Thirty minutes before cell plating, the complete medium, serum, trypsin, and DPBS for use in ZR-75-1 cell culture were pre-warmed in a water bath at 37 °C.
3) The ZR-75-1 cells were taken out of the incubator, and the medium was removed by pipetting. The cells were rinsed once with 5 mL of DPBS, and then the DPBS was discarded.
4) The cells in the T75 culture flask were digested with 1-3 mL of (0.25%) trypsin-EDTA. After the trypsin was added, the culture flask was incubated in an incubator at 37 °C for 2-5 min, and dispersion was confirmed by microscopic examination. Tapping or agitating the culture flask was avoided to prevent disturbing the cells, which might cause cell clumping.
5) 9 mL of the complete medium was added to terminate the digestion, thus forming a cell suspension, which was then mixed well by pipetting.
6) Cell suspension counting: 20 μL of the cell suspension was pipetted and diluted with 20 μL of the 0.4% trypan blue solution (Gibco, Cat. No. 15250061). The total cell count and the percentage of cell viability were determined using the Countstar® BioMed automatic cell counter. Only cells with a cell viability of no less than 95% were used for the experiment.
7) The concentration of the cell suspension was adjusted to 2000 cells/well, and the cell suspension was added to a 96-well cell culture plate at 80 μL/well.

Treatment of cells with compounds

1) Twenty-four hours after cell plating, the cell plate was taken out.
2) Each compound was prepared with a complete medium. The stock solution was a 10 mM solution in DMSO, which was first diluted with the medium to 100 μM, then diluted to 1 μM, and finally subjected to a 5-fold serial dilution across a total of 9 concentration gradients.
3) 20 μL of the compound diluted with the medium was added to each well of the cell culture plate, which already contained 80 μL of the medium. The final concentration started at 200 nM, followed by a 5-fold serial dilution across a total of 9 concentration gradients. Two replicate wells were set for each compound, and each plate could be used to test 3 compounds, with the DMSO content controlled within 0.5%.
4) The culture medium was replenished once every three days with a complete medium containing the same

concentration of the test compound, with 20 μL/well added each time.

Plate reading assay

After 10 days of incubation with the compound, the cell culture plate was taken out, and the CellTiter-Glo® reagent was added at 50 μL/well. The mixture was mixed well by shaking on a shaker for 20 min, then left to stand at room temperature for 20 min, and pipetted and transferred to a white plate at 100 μL/well. Then, readings were taken using a multi-mode microplate reader for assay.

Table 3. $IC_{50}$ ranges for inhibitory effects of compounds on proliferation of ZR-75-1 cells

| A:$IC_{50}$ < 0.5 nM; B: $IC_{50}$ ≥ 0.5 nM to < 2 nM; C: $IC_{50}$ ≥ 2 nM to < 10 nM; D: $IC_{50}$ ≥ 10 nM to < 50 nM; E: $IC_{50}$ ≥ 50 nM. | | |
|---|---|---|
| Compound No. | Structure | $IC_{50}$ (KAT 6A) |
| PF-9363 | 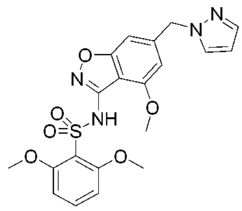 | B |
| PF-8144 | 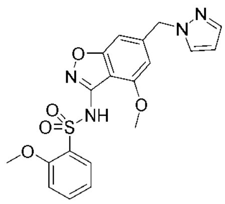 | B |
| PF-0128 | 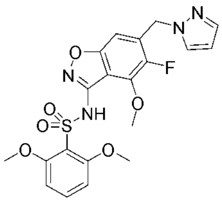 | B |
| 1 | 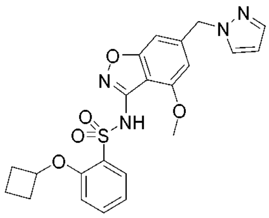 | B |
| 2 | 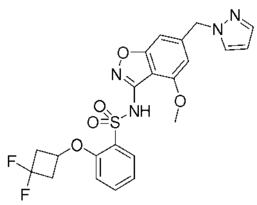 | C |
| 3 | 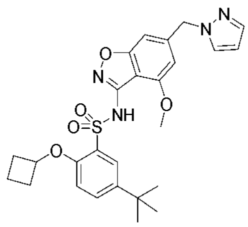 | C |

(continued)

| A:$IC_{50}$ < 0.5 nM; B: $IC_{50}$ ≥ 0.5 nM to < 2 nM; C: $IC_{50}$ ≥ 2 nM to < 10 nM; D: $IC_{50}$ ≥ 10 nM to < 50 nM; E: $IC_{50}$ ≥ 50 nM. | | |
|---|---|---|
| Compound No. | Structure | $IC_{50}$ (KAT 6A) |
| 4 | 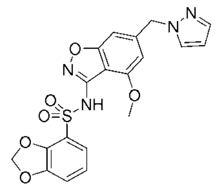 | D |
| 5 | 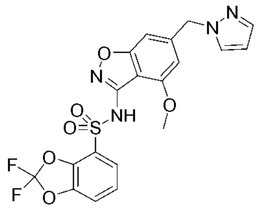 | E |
| 6 | 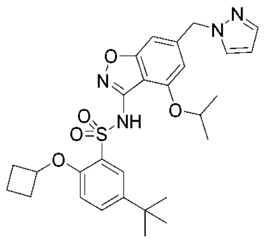 | C |
| 7 | 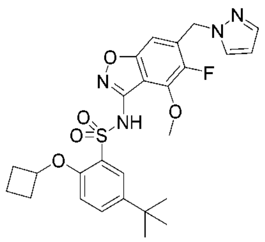 | B |
| 8 | 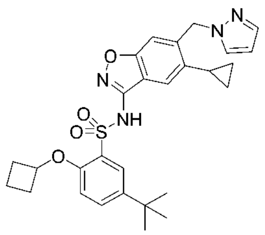 | D |
| 9 | 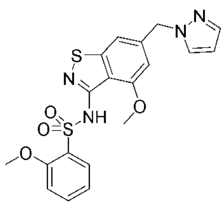 | E |

(continued)

| A:$IC_{50}$ < 0.5 nM; B: $IC_{50}$ ≥ 0.5 nM to < 2 nM; C: $IC_{50}$ ≥ 2 nM to < 10 nM; D: $IC_{50}$ ≥ 10 nM to < 50 nM; E: $IC_{50}$ ≥ 50 nM. | | |
|---|---|---|
| Compound No. | Structure | $IC_{50}$ (KAT 6A) |
| 10 | 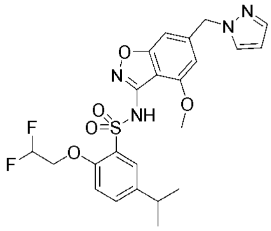 | C |
| 11 | 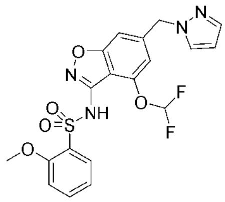 | C |
| 12 | 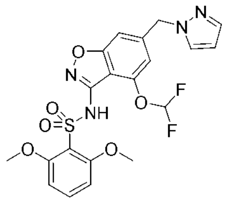 | C |
| 13 | 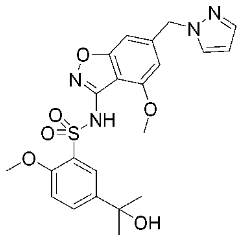 | B |
| 14 | 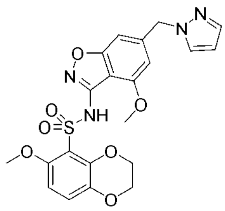 | B |
| 15 | 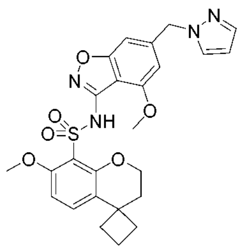 | A |

(continued)

| A:$IC_{50}$ < 0.5 nM; B: $IC_{50}$ ≥ 0.5 nM to < 2 nM; C: $IC_{50}$ ≥ 2 nM to < 10 nM; D: $IC_{50}$ ≥ 10 nM to < 50 nM; E: $IC_{50}$ ≥ 50 nM. | | |
|---|---|---|
| Compound No. | Structure | $IC_{50}$ (KAT 6A) |
| 16 | 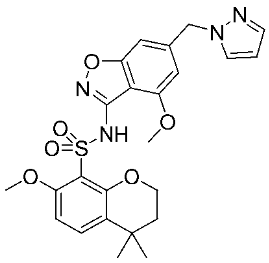 | A |
| 17 | 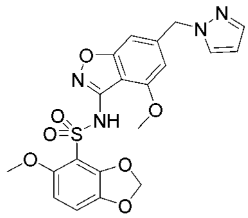 | B |
| 18 | 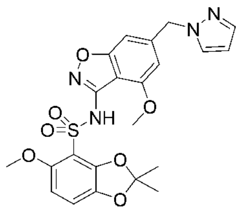 | C |
| 19 | 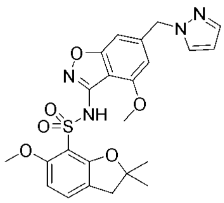 | C |
| 20 | 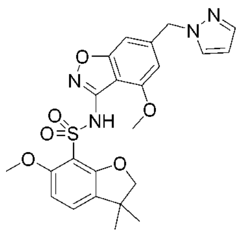 | B |
| 21 | 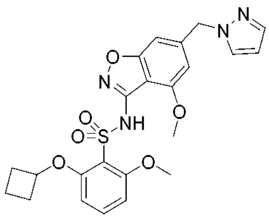 | C |

(continued)

| A:$IC_{50}$ < 0.5 nM; B: $IC_{50}$ ≥ 0.5 nM to < 2 nM; C: $IC_{50}$ ≥ 2 nM to < 10 nM; D: $IC_{50}$ ≥ 10 nM to < 50 nM; E: $IC_{50}$ ≥ 50 nM. | | |
|---|---|---|
| Compound No. | Structure | $IC_{50}$ (KAT 6A) |
| 22 | 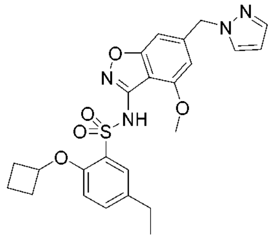 | C |
| 23 | 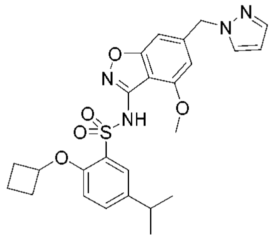 | C |
| 24 | 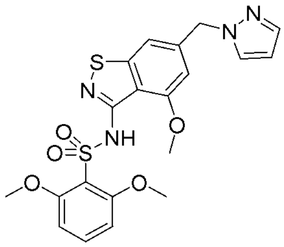 | D |
| 25 | 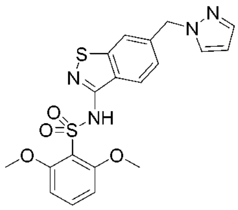 | E |
| 26 | 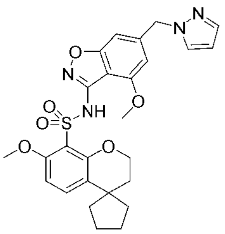 | A |
| 27 | 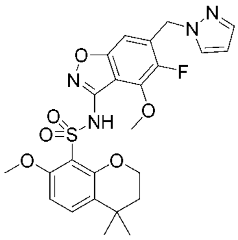 | A |

(continued)

| A:$IC_{50}$ < 0.5 nM; B: $IC_{50}$ ≥ 0.5 nM to < 2 nM; C: $IC_{50}$ ≥ 2 nM to < 10 nM; D: $IC_{50}$ ≥ 10 nM to < 50 nM; E: $IC_{50}$ ≥ 50 nM. | | |
|---|---|---|
| Compound No. | Structure | $IC_{50}$ (KAT 6A) |
| 28 | 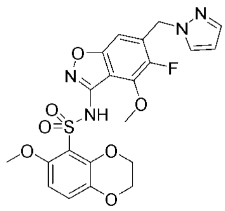 | A |
| 29 | 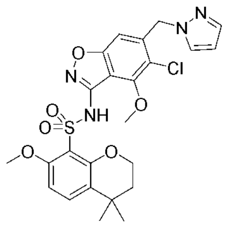 | D |
| 30 | 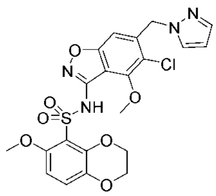 | D |
| 31 | 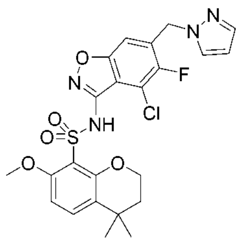 | B |
| 32 | 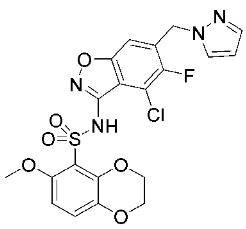 | C |
| 33 | 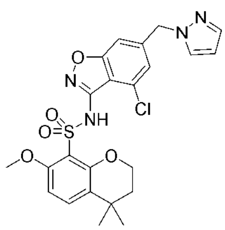 | B |

(continued)

| A:$IC_{50}$ < 0.5 nM; B: $IC_{50}$ ≥ 0.5 nM to < 2 nM; C: $IC_{50}$ ≥ 2 nM to < 10 nM; D: $IC_{50}$ ≥ 10 nM to < 50 nM; E: $IC_{50}$ ≥ 50 nM. | | |
|---|---|---|
| Compound No. | Structure | $IC_{50}$ (KAT 6A) |
| 34 | 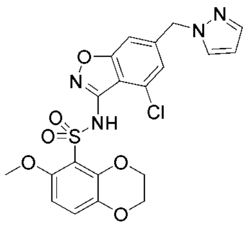 | C |
| 35 | 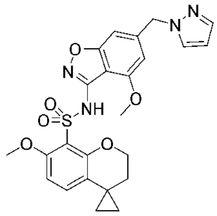 | A |
| 36 | 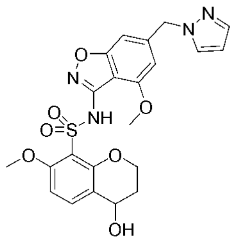 | A |
| 37 | 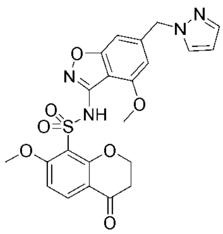 | A |
| 38 | 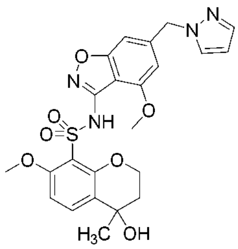 | A |
| 39 | 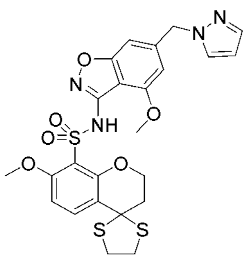 | A |

(continued)

| A:$IC_{50}$ < 0.5 nM; B: $IC_{50}$ ≥ 0.5 nM to < 2 nM; C: $IC_{50}$ ≥ 2 nM to < 10 nM; D: $IC_{50}$ ≥ 10 nM to < 50 nM; E: $IC_{50}$ ≥ 50 nM. | | |
|---|---|---|
| Compound No. | Structure | $IC_{50}$ (KAT 6A) |
| 40 | 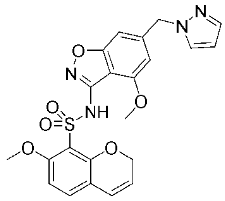 | A |
| 41 | 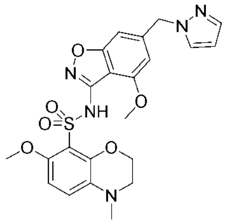 | B |
| 42 | 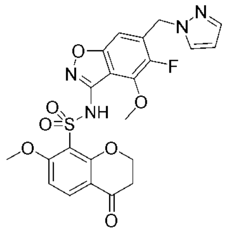 | A |
| 43 | 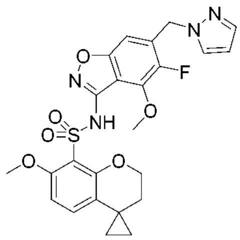 | A |
| 44 | 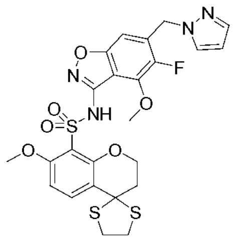 | A |
| 45 | 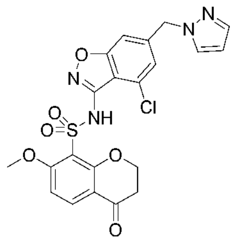 | C |

(continued)

| A:$IC_{50}$ < 0.5 nM; B: $IC_{50}$ ≥ 0.5 nM to < 2 nM; C: $IC_{50}$ ≥ 2 nM to < 10 nM; D: $IC_{50}$ ≥ 10 nM to < 50 nM; E: $IC_{50}$ ≥ 50 nM. | | |
|---|---|---|
| Compound No. | Structure | $IC_{50}$ (KAT 6A) |
| 46 | 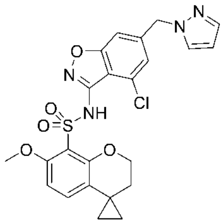 | C |
| 47 | 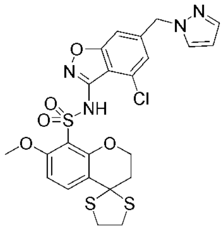 | B |
| 48 | 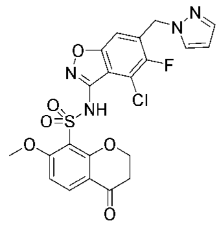 | B |
| 49 | 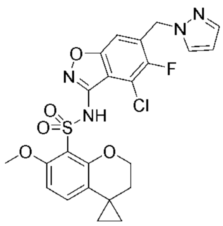 | C |
| 52 | 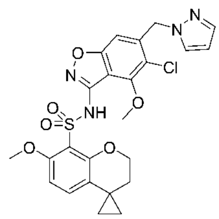 | E |
| 58 | 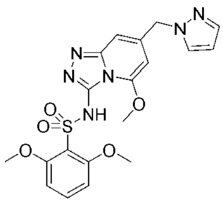 | E |

(continued)

| A:$IC_{50}$ < 0.5 nM; B: $IC_{50}$ ≥ 0.5 nM to < 2 nM; C: $IC_{50}$ ≥ 2 nM to < 10 nM; D: $IC_{50}$ ≥ 10 nM to < 50 nM; E: $IC_{50}$ ≥ 50 nM. | | |
|---|---|---|
| Compound No. | Structure | $IC_{50}$ (KAT 6A) |
| 72 | 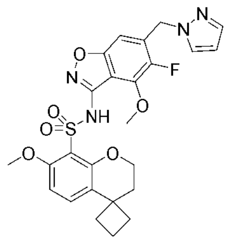 | B |
| 73 | 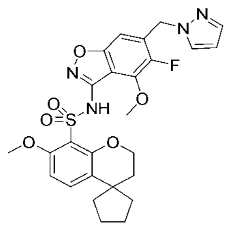 | B |
| 101 | 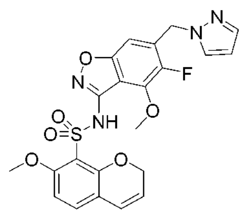 | A |
| 102 | 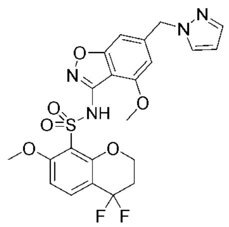 | B |
| 103 | 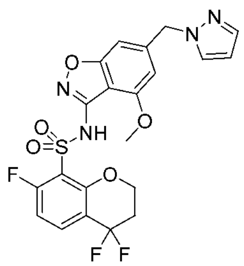 | D |
| 104 | 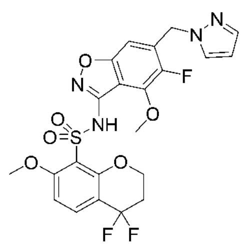 | A |

(continued)

| A:IC$_{50}$ < 0.5 nM; B: IC$_{50}$ ≥ 0.5 nM to < 2 nM; C: IC$_{50}$ ≥ 2 nM to < 10 nM; D: IC$_{50}$ ≥ 10 nM to < 50 nM; E: IC$_{50}$ ≥ 50 nM. | | |
|---|---|---|
| Compound No. | Structure | IC$_{50}$ (KAT 6A) |
| 105 | 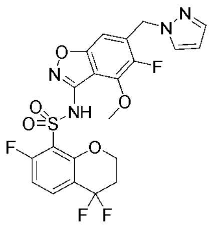 | D |
| 106 | 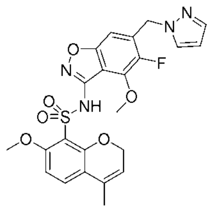 | A |
| 107 | 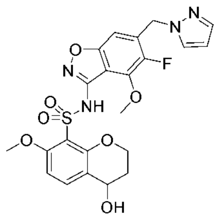 | B |
| 108 | 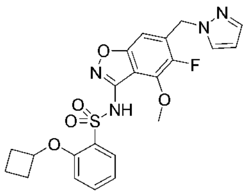 | B |
| 109 | 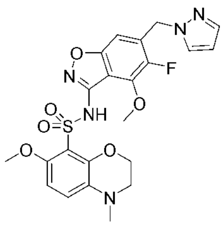 | A |
| 110 | 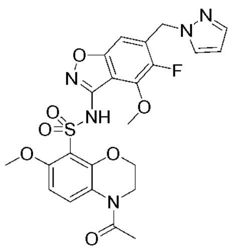 | B |

(continued)

| A:$IC_{50}$ < 0.5 nM; B: $IC_{50} \geq$ 0.5 nM to < 2 nM; C: $IC_{50} \geq$ 2 nM to < 10 nM; D: $IC_{50} \geq$ 10 nM to < 50 nM; E: $IC_{50} \geq$ 50 nM. | | |
|---|---|---|
| Compound No. | Structure | $IC_{50}$ (KAT 6A) |
| 111 | 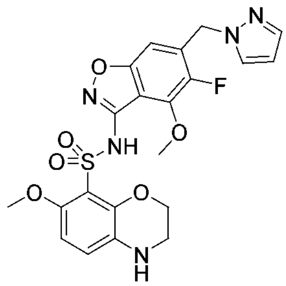 | A |
| 112 | 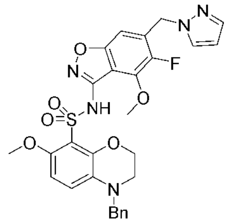 | C |
| 120 | 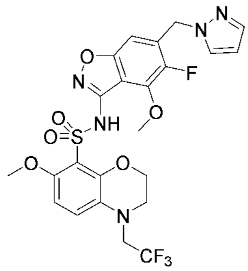 | C |
| 121 | 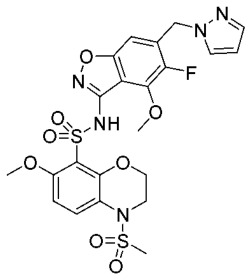 | C |

Conclusion: Under the conditions in the inhibition experiment on the proliferation of the breast cancer cell line ZR-75-1 with high expression of KAT6A/B, some of the compounds of the present disclosure were potent inhibitors, among which compounds 15-16, 26-28, 35-40, 42-44, 101, 104, 106, 109, and 111 were more potent inhibitors against KAT6A/B compared to the reference compounds PF-9363, PF-8144, and PF-0128. The reference compounds PF-9363, PF-8144, and PF-0128 are disclosed in the patent WO2020/254946A1; the reference compounds PF-9363 and PF-8144 are also disclosed in WO2022/013369A1.

**Test Example 2:** Selective Inhibitory Effects of Test Compounds on Human KAT6 Enzymes (Radioisotope Method)

<u>Test method</u>

1) Experimental reagents and instruments

1. KAT6A (ACTIVE MOTIF, Cat. No. 81223)
2. KAT5 (BPS, Cat. No. 79422)
3. KAT7 (ACTIVE MOTIF, Cat. No. 31889)
4. KAT6B (ACTIVE MOTIF, Cat. No. 81224)
5. Acetyl coenzyme A, [Acetyl-3H, 250 μCi (9.25 MBq] (PERKIN ELMER, Cat. No. NET290250UC)
6. Acetyl coenzyme A (CAYMAN, Cat. No. 16160)
7. 384-well Flashplate (Perkin Elmer, Cat. No. SMP410A001PK)
8. Garcinol (MCE, Cat. No. HY-107569)
9. Automatic microplate pipetting system (Precision, PRC384U, BioTek)
10. Ultrasonic nanoliter liquid handling system (Echo 650 Liquid Handler, Echo 650, Labcyte)
11. Centrifuge (Avanti J-15R, Beckman Coulter)
12. Liquid scintillation counter (Microplate Counter, Microbeta2, Perkin Elmer)

2) Reagent preparation

1. For KAT6A and KAT7, a 1× assay buffer was prepared as follows: 10 mM Tris-HCl pH 8.0, 0.01% Tween-20, and 1 mM DTT; for KAT6B and KAT5, a 1× assay buffer was prepared as follows: 50 mM Tris-HCl pH 8.0, 0.05% Tween-20, and 1 mM DTT.
2. Compound preparation: For KAT6A, KAT6B, and KAT7, the final concentration of the compounds started at 1 μM, followed by a 5-fold serial dilution across a total of 7 concentration gradients; for KAT5, the final concentration of the compounds started at 10 μM, followed by a 5-fold serial dilution across a total of 7 concentration gradients.
3. Enzyme solution preparation: The final concentrations were prepared using the respective 1× assay buffers, as shown in Table 4.
4. Substrate reaction solution preparation: The mixed substrates of H4 peptide and [3H]acetyl coenzyme A at final concentrations were prepared using the respective 1× assay buffers, as shown in Table 4.

3) Experimental procedures

1. The enzyme solution was transferred to 384-well reaction plates at 10 ilL/well. For each negative control well, 10 μL of the 1× assay buffer was transferred. The plates were then centrifuged at 1000 RPM for 1 min and incubated at room temperature for 15 min.
2. The substrate reaction solution was added at 10 μL/well, and the plates were centrifuged at 1000 RPM for 1 min to start the reaction.
3. The samples for KAT6A, KAT6B, KAT7, KAT8, and KAT5 were then incubated at room temperature for 60 min, 120 min, 45 min, 60 min, and 120 min, respectively.
4. Stop solution preparation: A stop solution was prepared by diluting 50 μM cold Ac-CoA in 1× buffer.
5. The stop solution was added at 10 μL/well, and the plates were centrifuged at 100 RPM for 1 min to stop the reaction.
6. The mixtures in the reaction plates were transferred at 25 μL/well to new plates (Streptavidin FlashPlate HTS PLUS, High Capacity, 384-well).
7. The plates were incubated at room temperature for at least 60 min and then
8. read using MicroBeta2.

Table 4. Final concentrations of prepared enzyme solutions and substrate reaction solutions

| Item | KAT5 (full-length) | KAT6A (488-778) | KAT6B (718-1008) | KAT7 (full-length) |
|---|---|---|---|---|
| [Enzyme] nM | 2.5 | 2.5 | 5 | 2 |
| [Biotin-H4(1-30)] nM | 400 | / | 400 | 600 |
| [Biotin-H3(1-21)] nM | / | 400 | / | / |
| [3H-Ac-CoA] nM | 800 | 250 | 560 | 550 |
| Stop reagent [Cold Ac-CoA] μM | 50 | 50 | 50 | 50 |

4) Data Analysis

Raw data were obtained in EXCEL, and the inhibition rates were calculated:

$$\text{Formula (1): } Y = (\text{Max signal} - \text{Sample signal})/(\text{Max signal} - \text{Min signal}) \times 100$$

Y: inhibition rate;
Max signal: reaction signal from enzyme and substrate;
Sample signal: signal from sample;
Min signal: signal from substrate only (negative control).

The inhibition rate data were imported into XLFit Excel, and $IC_{50}$ values were obtained using formula (2):

$$\text{Formula (2): } Y = \text{Bottom} + (\text{Top} - \text{Bottom})/(1 + ((IC_{50}/X) \times \text{HillSlope}))$$

X: compound concentration;
Top: value of the top plateau of the fitted regression curve;
Bottom: value of the bottom plateau of the fitted regression curve;
HillSlope: slope of the fitted regression curve.

The $IC_{50}$ value was substituted into formula (3) to obtain the enzymatic activity inhibition constant Ki:

$$\text{Formula (3): } Ki = IC_{50}/(1 + [S]/Km)$$

[S]: substrate concentration; Km: Michaelis constant.

5) Data results

Table 5. Inhibitory activity of the compounds of the present disclosure against KAT5/KAT6A/KAT6B/KAT7

| Compound No. | KAT6A/ Ki (nM) | KAT6B/ Ki (nM) | KAT5/ Ki (nM) | KAT5/ KAT6A | KAT5/ KAT6B | KAT7/ Ki (nM) | KAT7/ KAT6A | KAT7/ KAT6B |
|---|---|---|---|---|---|---|---|---|
| PF-8144 | 0.58 | 5.18 | 672.32 | 1159 | 130 | 85.66 | 148 | 17 |
| 28 | 0.20 | 0.90 | 648.64 | 3243 | 721 | 40.59 | 203 | 45 |
| 101 | 0.21 | 0.65 | 79.61 | 379 | 122 | 7.39 | 35 | 11 |
| 109 | 0.47 | 0.90 | 286.20 | 609 | 318 | 54.68 | 116 | 61 |
| 110 | 0.29 | 1.60 | 1150.35 | 3967 | 719 | 64.53 | 223 | 40 |
| 114 | 0.38 | 2.49 | 1854.26 | 4880 | 745 | 173.88 | 458 | 70 |
| 115 | 0.63 | 6.48 | 5038.76 | 7998 | 778 | 492.57 | 782 | 76 |
| 121 | 0.28 | 0.90 | 576.94 | 2061 | 641 | 96.05 | 343 | 107 |

Conclusion: Some of the compounds of the present disclosure were potent KAT6A/KAT6B inhibitors, among which compounds 28, 101, 109, 110, 114, and 121 were more potent inhibitors against KAT6A/KAT6B compared to the reference compound PF-8144; compounds 28, 109, 110, 114, 115, and 121 exhibited better selective inhibitory effects on KAT6A/KAT6B compared to KAT5 or KAT7; some of the compounds possessed both stronger inhibitory activity and better selectivity.

**Test Example 3:** Determination of Pharmacokinetic Data of Compounds in SD (Sprague Dawley) Rats

Assay objective

The pharmacokinetic characteristics of some of the compounds of the present disclosure were evaluated by a pharmacokinetic experiment in SD rats.

Test method

Female SD rats aged 6-8 weeks from Shanghai Jihui Laboratory Animal Care Co., Ltd. were selected. The test animals were fasted overnight before administration, and feeding was resumed 4 h after administration. The compounds were dissolved in 5% DMSO + 5% Tween 80 + 90% normal saline. For a single intravenous administration, the administration dose was 1 mg/kg, and the administration volume was 2 mL/kg. For a single intragastric administration, the administration dose was 2 mg/kg, and the administration volume was 10 mL/kg. Blood was collected at 0.083 h (for intravenous administration only), 0.25 h, 0.5 h, 1 h, 2 h, 4 h, 8 h, and 24 h after administration. At each indicated time point, about 150 μL of blood sample was collected from each rat via the vein and placed into an EDTA-$K_2$ tube. Each sample was centrifuged (2000 g, 4 °C, 5 min) within 15 min after sample collection to obtain plasma, which was then stored at -70 °C until analysis. The concentration of the corresponding test compound in the SD rat plasma was determined using UPLC-MS/MS.

Pharmacokinetic parameter results

Table 6. Pharmacokinetic test results of representative compounds of the present disclosure in SD rats

| Compound No. | Route/dose of administration (mg/kg) | CL (mL/min/Kg) | Vdss (L/Kg) | $AUC_{0\text{-inf}}$ (hr*ng/mL) | $T_{1/2}$ (h) | F (%) |
|---|---|---|---|---|---|---|
| PF-8144 | i.v. / 1 | 0.597 | 0.143 | 28318 | 3.16 | -- |
| | p.o./ 2 | -- | -- | 55494 | 3.79 | 98 |
| 28 | i.v. / 1 | 0.761 | 0.295 | 22573 | 5.87 | -- |
| | p.o./ 2 | -- | -- | 42761 | 6.73 | 94.7 |
| 42 | i.v. / 1 | 1.69 | 0.261 | 10249 | 3.79 | -- |
| | p.o./ 2 | -- | -- | 11273 | 3.31 | 55 |
| 101 | i.v. / 1 | 0.35 | 0.237 | 48124 | 8.88 | -- |
| | p.o./ 2 | -- | -- | 89071 | 9.21 | 92.5 |
| 104 | i.v. / 1 | 3.98 | 0.705 | 4200 | 2.83 | -- |
| | p.o./ 2 | -- | -- | 6109 | 2.13 | 72.7 |
| 109 | i.v. / 1 | 0.185 | 0.119 | 90718 | 8.57 | -- |
| | p.o./ 2 | -- | -- | 187103 | 9.73 | 96.0 |
| 114 | i.v. / 1 | 0.462 | 0.608 | 39220 | 17.2 | -- |
| | p.o./ 2 | -- | -- | 97873 | 13.0 | 144 |
| 115 | i.v. / 1 | 0.363 | 0.408 | 47541 | 14.3 | -- |
| | p.o./ 2 | -- | -- | 109392 | 15.9 | 105 |
| Note: CL represents clearance rate; Vdss represents apparent volume of distribution; $AUC_{0\text{-inf}}$ represents area under the curve ($_{0\text{-inf}}$); $T_{1/2}$ represents half-life; F represents bioavailability. | | | | | | |

Conclusion: Under the conditions in this experiment, the representative compounds disclosed herein exhibited high bioavailability and exposure levels, with some of the representative compounds demonstrating lower *in vivo* clearance rates, longer half-lives, and higher exposure levels compared to the reference compound PF-8144.

**Test Example 4:** Assay for Effects of Compounds on Histone Acetylation in Estrogen Receptor-Positive Breast Cancer Cells

Assay method: Western blotting method (immunoblotting assay)

Materials and method

The effects of the test compounds on the acetylation level of H3K23 in ZR-75-1 cells (Nanjing Cobioer, CBP60402) were assayed.

1. On day 1, the cells were plated at 400,000 cells/well (2 mL/well) and cultured overnight in an incubator at 37 °C with 5% $CO_2$.
2. On day 2, compounds were added starting at 5 μM, with a 10-fold serial dilution across a total of 3 concentration gradients, and DMSO was added to the negative control wells.
3. On day 6, after 5 days of incubation with compounds, protein samples were collected:
An ice box, pre-cooled PBS (4 °C), a cell lysis buffer RIPA (Beyotime, P0013B), and a protease inhibitor (100×) (Thermo Scientific™, 87786) were prepared. Then, 120 μL of the cell lysis buffer was added to each well. The mixture was placed on ice for about 15 min. Then, the cell lysate was taken out and centrifuged at 12,000 rpm for 20 min, and the supernatant was collected.
4. Protein quantification by BCA method (Beyotime, P0009)
Standards (8 concentrations) were prepared, and the samples and the standards were added to a 96-well plate. The samples were subjected to a 10-fold serial dilution (18 μL of PBS + 2 μL of sample supernatant). A working solution was prepared and then added. The prepared working solution was a BCA working solution (solution A:solution B = 50:1). After the addition of the working solution, the mixture was baked in an oven at 37 °C for 30 min. The absorbance was then measured using a microplate reader. Loading Buffer (Biofuraw™ LDS Sample Buffer, With Reducing, 180-8210D) was added to adjust the protein concentration to 1.4 μg/μL, followed by incubation in a metal bath at 100 °C for 10 min.
5. Electrophoresis and development
The samples were taken out and thawed on ice. Before loading, they were vortexed and centrifuged. A pre-cast gel (Tanon™ 4-20% Bis-tris gradient pre-cast gel, 180-9115H) was installed, and the samples were loaded. The electrophoresis system was run at 120 V for 40 min. Subsequently, the proteins were transferred to a PVDF membrane, and then the membrane was placed in 5% BSA for blocking for 1 h. Corresponding antibodies (Acetyl-Histone H3 (Lys23) (D6Y7M) Rabbit mAb (14932T, CST), Histone H4 (L64C1) Mouse mAb (2935T, CST), β-Actin Antibody (4967S, CST)) were added, and then the membrane was incubated overnight at 4 °C. The next day, the PVDF membrane was taken out and washed (8 min × 3) with TBST (with 0.5% Tween 20). Horseradish peroxidase-conjugated secondary antibodies (Anti-rabbit IgG, 7074P2 and Anti-mouse IgG, 7076P2, CST) were prepared and then incubated with the membrane on a shaker for 1 h. After the incubation was completed, the membrane was washed (8 min × 3) with TBST. After the washing, a luminescence solution (Tanon, 180-506) was added for exposure. After the development was completed, the strips that had been incubated with Acetyl-Histone H3 (Lys23) (D6Y7M) Rabbit mAb were washed twice with TBST, followed by protein elution (Thermo Fisher, 46430). Subsequently, the above-mentioned blocking was performed again, followed by incubation with Histone H4 (L64Cl) Mouse mAb and Anti-mouse IgG. Then, development was carried out again. The development results are shown in FIG. 1.
6. Results:

The test compound 28 and PF-8144 could both significantly reduce the acetylation level of H3K23.

**Test Example 5:** Anti-Tumor Effects of Test Compounds in $ER^+KAT6^{high}$ Breast Cancer Cell-Xenograft Tumor Model

Assay method: ZR-75-1 cells were subcutaneously inoculated into the right back of each mouse to evaluate the efficacy of the compounds in the subcutaneous xenograft tumor model of ZR-75-1 cells.

Materials and method:

1. Cell culture: ZR-75-1 cells were cultured as a monolayer *in vitro* under the following culture conditions: an RPMI 1640 medium supplemented with 10% fetal bovine serum and 1% Anti-anti (Antibiotic-Antimycotic), incubated in an incubator at 37 °C with 5% $CO_2$. The cells were digested with trypsin-EDTA twice a week for passaging as per conventional practice. When the cell saturation reached 80%-90% and the quantity met the requirement, the cells were harvested, counted, and inoculated.

2. Animals: Balb/c nude mice, female, 6-8 weeks old, weighing 18-22 g. A total of 128 mice were required (80 mice were enrolled). They were provided by Vital River.

3. Tumor inoculation: Two days before tumor inoculation, 17-beta estradiol tablets (0.18 mg) were subcutaneously administered, and 10 million ZR-75-1 tumor cells + matrigel (total 200 μL) were subcutaneously inoculated into the right back of each mouse. When the mean tumor volume reached about 137 $mm^3$, the mice were grouped for administration.

4. Experimental administration: The compounds were dissolved in 5% DMSO + 5% Tween 80 + 90% Saline, and administered intragastrically (PO) daily for five weeks.

5. Experimental indicators: The experimental indicators were to investigate whether the tumor growth was inhibited or delayed or the tumor was cured. Tumor diameters were measured twice weekly using a vernier caliper. The tumor volume was calculated using the following formula: $V = 0.5a \times b^2$, where a and b represent the long diameter and short diameter of the tumor, respectively.

6. The anti-tumor efficacy of the compounds was evaluated by TGI (%) or relative tumor proliferation rate T/C (%). TGI (%) refers to the tumor growth inhibition rate. Calculation of TGI (%): TGI (%) = [(1 - (mean tumor volume at the end of administration in a treatment group - mean tumor volume at the start of administration in the treatment group))/(mean tumor volume at the end of treatment in the solvent control group - mean tumor volume at the start of treatment in the solvent control group)] × 100%. The calculation formula for the relative tumor proliferation rate T/C (%) was as follows: T/C % = TRTV/CRTV × 100% (TRTV: RTV of the treatment group; CRTV: RTV of the negative control group). The relative tumor volume (RTV) was calculated based on the tumor measurement results using the following formula: RTV = Vt/V0, where V0 represents the mean tumor volume measured at the time of grouping for administration (i.e., PG-D0), and Vt represents the mean tumor volume at a certain measurement. TRTV and CRTV were derived from data measured on the same day.

7. Statistical analysis

The mean and standard error of mean (SEM) of tumor volume at each time point for each group were included. The differences in tumor volume and tumor weight among the groups were statistically analyzed. For comparisons among three or more groups, one-way ANOVA was used for analysis. If the F-value showed a significant difference, the Games-Howell method was applied for testing. If the F-value showed no significant difference, the Dunnett's (2-sided) method was applied for analysis. The analysis was performed using SPSS 17.0, and $p < 0.05$ was considered to indicate a significant difference.

For comparisons between different doses of the same drug or different drugs at the same dose, two-way ANOVA was used for analysis, and the Bonferroni method was applied for testing. The analysis was performed using GraphPad Prism 9.0, and $p < 0.05$ was considered to indicate a significant difference. On day 35 after the start of administration, the data between different doses of the same drug or different drugs at the same dose were analyzed using one-way ANOVA. If the F-value showed a significant difference, the Games-Howell method was applied for testing. If the F-value showed no significant difference, the Dunnett's (2-sided) method was applied for analysis. The analysis was performed using SPSS 17.0, and $p < 0.05$ was considered to indicate a significant difference.

Table 7. Animal experimental grouping and administration regimen

| Group | Number (n) | Compound | Dose (mg/kg) | Preparation volume (μL/g) | Route of administration | Duration |
|---|---|---|---|---|---|---|
| 1 | 8 | Control group (solvent) | -- | 10 | PO | QD*5W |
| 2 | 8 | PF-8144 | 0.01 | 10 | PO | QD*5W |
| 3 | 8 | PF-8144 | 0.1 | 10 | PO | QD*5W |
| 4 | 8 | PF-8144 | 2 | 10 | PO | QD*5W |
| 5 | 8 | Compound 28 | 0.01 | 10 | PO | QD*5W |
| 6 | 8 | Compound 28 | 0.1 | 10 | PO | QD*5W |
| 7 | 8 | Compound 28 | 2 | 10 | PO | QD*5W |
| 8 | 8 | Compound 101 | 0.01 | 10 | PO | QD*5W |
| 9 | 8 | Compound 101 | 0.1 | 10 | PO | QD*5W |
| 10 | 8 | Compound 101 | 2 | 10 | PO | QD*5W |

8. Results

In this experiment, the *in vivo* efficacy of the compounds in the human breast cancer ZR-75-1 cell xenograft tumor model was evaluated. The tumor volumes and body weights of the mice in each group at different time points are shown in FIGs. 2 and 3.

On day 35 after the start of the administration, the tumor volume of the tumor-bearing mice in the control group reached 1,424 $mm^3$. Compared to the control group (solvent), the mean tumor volumes for PF-8144 at 0.01 mg/kg, 0.1 mg/kg, and 2 mg/kg on day 35 after the grouping for administration were 1,035 $mm^3$ (T/C = 72.71%, TGI = 30.19%, p = 0.911), 580 $mm^3$ (T/C = 40.77%, TGI = 65.53%, p = 0.022), and 36 $mm^3$ (T/C = 2.51%, TGI = 107.85%, p = 0.001), respectively. The mean tumor volumes for compound 28 at 0.01 mg/kg, 0.1 mg/kg, and 2 mg/kg were 1,017 $mm^3$ (T/C = 76.14%, TGI = 30.94%, p = 0.654), 162 $mm^3$ (T/C = 11.36%, TGI = 98.07%, p = 0.002), and 12 $mm^3$ (T/C = 0.84%, TGI = 109.69%, p = 0.001), respectively. The mean tumor volumes for compound 101 at 0.01 mg/kg, 0.1 mg/kg, and 2 mg/kg were 264 $mm^3$ (T/C = 19.06%, TGI = 89.81%, p = 0.002), 25 $mm^3$ (T/C = 1.79%, TGI = 108.64%, p = 0.001), and 14 $mm^3$ (T/C = 0.96%, TGI = 109.56%, p = 0.001), respectively. Except for the 0.01 mg/kg dose groups of PF-8144 and compound 28, all administration groups exhibited significant anti-tumor effects. At the dose of 0.1 mg/kg, compound 28 and compound 101 were significantly superior in efficacy to PF-8144. Meanwhile, at all doses, the body weight changes in the compound 28 group and the compound 101 group showed no statistically significant difference compared to the PF-8144 group.

**Test Example 6**: Anti-Tumor Effects of Test Compounds in $ER^+KAT6^{high}$ Breast Cancer Cell-Xenograft Tumor Model

Assay method: ZR-75-1 cells were subcutaneously inoculated into the right back of each mouse to evaluate the efficacy of the compounds in the subcutaneous xenograft tumor model of ZR-75-1 cells.

Materials and method:

1. Cell culture: ZR-75-1 cells were cultured as a monolayer *in vitro* under the following culture conditions: an RPMI 1640 medium supplemented with 10% fetal bovine serum and 1% Anti-anti (Antibiotic-Antimycotic), incubated in an incubator at 37 °C with 5% $CO_2$. The cells were digested with trypsin-EDTA twice a week for passaging as per conventional practice. When the cell saturation reached 80%-90% and the quantity met the requirement, the cells were harvested, counted, and inoculated.
2. Animals: Balb/c nude mice, female, 6-8 weeks old, weighing 18-22 g. A total of 115 mice were required (72 mice were enrolled). They were provided by Vital River or other qualified suppliers.
3. Tumor inoculation: Two days before tumor inoculation, 17-beta estradiol tablets (0.18 mg) were subcutaneously administered, and 10 million ZR-75-1 tumor cells + matrigel (total 200 μL) were subcutaneously inoculated into the right back of each mouse. When the mean tumor volume reached about 131 $mm^3$, the mice were grouped for administration.
4. Experimental administration: The compounds were dissolved in 5% DMSO + 5% Tween 80 + 90% Saline or 10% DMSO + 10% Solutol + 80% (10% HP-β-CD in saline), and administered intragastrically (PO) daily for five weeks.
5. Experimental indicators: The experimental indicators were to investigate whether the tumor growth was inhibited or delayed or the tumor was cured. Tumor diameters were measured twice weekly using a vernier caliper. The tumor volume was calculated using the following formula: $V = 0.5a \times b^2$, where a and b represent the long diameter and short diameter of the tumor, respectively.
6. The anti-tumor efficacy of the compounds was evaluated by TGI (%) or relative tumor proliferation rate T/C (%). TGI (%) refers to the tumor growth inhibition rate. Calculation of TGI (%): TGI (%) = [(1 - (mean tumor volume at the end of administration in a treatment group - mean tumor volume at the start of administration in the treatment group))/(mean tumor volume at the end of treatment in the solvent control group - mean tumor volume at the start of treatment in the solvent control group)] × 100%. The calculation formula for the relative tumor proliferation rate T/C (%) was as follows: T/C % = TRTV/CRTV × 100% (TRTV: RTV of the treatment group; CRTV: RTV of the negative control group). The relative tumor volume (RTV) was calculated based on the tumor measurement results using the following formula: RTV = Vt/V0, where V0 represents the mean tumor volume measured at the time of grouping for administration (i.e., PG-D0), and Vt represents the mean tumor volume at a certain measurement. TRTV and CRTV were derived from data measured on the same day.
7. Statistical analysis

The mean and standard error of mean (SEM) of tumor volume at each time point for each group were included.

The differences in tumor volume and tumor weight among the groups were statistically analyzed. For comparisons among three or more groups, one-way ANOVA was used for analysis. If the F-value showed a significant difference, the Games-Howell method was applied for testing. If the F-value showed no significant difference, the Dunnett's (2-sided) method was applied for analysis. The analysis was performed using SPSS 17.0, and $p < 0.05$ was considered to indicate a significant difference.

For comparisons between different doses of the same drug or different drugs at the same dose, two-way ANOVA was used for analysis, and the Bonferroni method was applied for testing. The analysis was performed using GraphPad Prism 9.0, and $p < 0.05$ was considered to indicate a significant difference. On day 34 after the start of administration, the data between different doses of the same drug or different drugs at the same dose were analyzed using one-way ANOVA. If the F-value showed a significant difference, the Games-Howell method was applied for testing. If the F-value showed no significant difference, the Dunnett's (2-sided) method was applied for analysis. The analysis was performed using SPSS 17.0, and $p < 0.05$ was considered to indicate a significant difference.

Table 8. Animal experimental grouping and administration regimen

| Group | Number (n) | Compound | Dose (mg/kg) | Preparation volume (μL/g) | Route of administration | Duration |
|---|---|---|---|---|---|---|
| 1 | 8 | Control group (vehicle) | -- | 10 | PO | QD*5W |
| 2 | 8 | PF-8144 | 0.1 | 10 | PO | QD*5W |
| 3 | 8 | PF-8144 | 0.3 | 10 | PO | QD*5W |
| 4 | 8 | Compound 42 | 0.1 | 10 | PO | QD*5W |
| 5 | 8 | Compound | 0.3 | 10 | PO | QD*5W |
| | | 42 | | | | |
| 6 | 8 | Compound 114 | 0.1 | 10 | PO | QD*5W |
| 7 | 8 | Compound 114 | 0.3 | 10 | PO | QD*5W |
| 8 | 8 | Compound 115 | 0.1 | 10 | PO | QD*5W |
| 9 | 8 | Compound 115 | 0.3 | 10 | PO | QD*5W |

8. Results

In this experiment, the *in vivo* efficacy of the compounds in the human breast cancer ZR-75-1 cell xenograft tumor model was evaluated. The tumor volumes and body weights of the mice in each group at different time points are shown in FIGs. 4 and 5.

On day 34 after the start of the administration, the tumor volume of the tumor-bearing mice in the control group reached 1,126 $mm^3$. Compared to the control group (solvent), the mean tumor volumes for PF-8144 at 0.1 mg/kg and 0.3 mg/kg on day 34 after the grouping for administration were 407 $mm^3$ (T/C = 36.2%, TGI = 72.2%, $p < 0.001$) and 220 $mm^3$ (T/C = 19.5%, TGI = 91.07%, $p < 0.001$), respectively. The mean tumor volumes for compound 42 at 0.1 mg/kg and 0.3 mg/kg were 44 $mm^3$ (T/C = 3.9%, TGI = 108.67%, $p < 0.001$) and 25 $mm^3$ (T/C = 2.2%, TGI = 110.58%, $p < 0.001$), respectively. The mean tumor volumes for compound 114 at 0.1 mg/kg and 0.3 mg/kg were 403 $mm^3$ (T/C = 35.8%, TGI = 72.61%, $p < 0.001$) and 119 $mm^3$ (T/C = 10.5%, TGI = 101.21%, $p < 0.001$), respectively. The mean tumor volumes for compound 115 at 0.1 mg/kg and 0.3 mg/kg were 97 $mm^3$ (T/C = 8.6%, TGI = 103.38%, $p < 0.001$) and 27 $mm^3$ (T/C = 2.4%, TGI = 110.47%, $p < 0.001$), respectively. All administration groups exhibited significant anti-tumor effects. At the doses of 0.1 mg/kg and 0.3 mg/kg, compound 42 and compound 115 were significantly superior in efficacy to PF-8144. Meanwhile, at all doses, the body weight changes in the compound 42 group, the compound 114 group, and the compound 115 group showed no statistically significant difference compared to the PF-8144 group.

It will be appreciated that various modifications or changes may be made by those skilled in the art after reading the aforementioned content of the present disclosure, and such equivalent forms also fall within the scope defined by the appended claims of the present application.

## Claims

**1.** A compound of formula (I) or a pharmaceutically acceptable salt, an ester, a stereoisomer, a tautomer, a polymorph, a hydrate, a solvate, an N-oxide, an isotopically labeled compound, a metabolite, or a prodrug thereof:

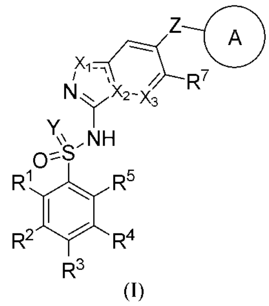

(I)

**characterized in that**:

the dashed line is an optional chemical bond;

$R^1$, $R^2$, and $R^3$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;

$R^4$ and $R^5$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, $C_{3-6}$ halocycloalkyl, $C_{3-6}$ cycloalkoxy, $C_{3-6}$ halocycloalkoxy, 6- to 10-membered aryl, 5- to 10-membered heteroaryl, and $C_{1-6}$ hydroxyalkyl, or $R^4$ and $R^5$, together with the carbon atoms to which they are attached, form 4- to 10-membered heterocyclyl, 5- to 6-membered heteroaryl, 7- to 12-membered spiroheterocyclyl, or 8- to 15-membered fused heterocyclyl, wherein the 4- to 10-membered heterocyclyl, 5- to 6-membered heteroaryl, 7- to 12-membered spiroheterocyclyl, or 8- to 15-membered fused heterocyclyl is optionally substituted with one or more substituents selected from: hydroxyl, amino, halogen, $C_{1-6}$ alkyl, $C_{1-6}$ haloalkyl, $C_{1-6}$ hydroxyalkyl, $C_{1-6}$ alkoxy, $C_{1-6}$ haloalkoxy, $-C(O)C_{1-6}$ alkyl, $-C(O)C_{3-6}$ cycloalkyl, $-C(O)C_{1-6}$ haloalkyl, $-C(O)C_{3-6}$ halocycloalkyl, $-C(O)OC_{1-6}$ alkyl, $-S(O)_2C_{1-6}$ alkyl, $-S(O)_2C_{3-6}$ cycloalkyl, $-S(O)_2C_{1-6}$ haloalkyl, $-S(O)_2C_{3-6}$ halocycloalkyl, $-PO(C_{1-6}$ alkyl$)_2$, $-PO(C_{3-6}$ cycloalkyl$)_2$, $-PO(C_{1-6}$ haloalkyl$)_2$, $-PO(C_{3-6}$ halocycloalkyl$)_2$, oxo, $=CH_2$, $=CHC_{1-6}$ alkyl, $=C(C_{1-6}$ alkyl$)_2$, $=CHC_{1-6}$ haloalkyl, $=C(C_{1-6}$ haloalkyl$)_2$, $-NHC_{1-6}$ alkyl, $-N(C_{1-6}$ alkyl$)_2$, cyano, benzyl, $C_{3-6}$ cycloalkyl, $C_{3-6}$ heterocyclyl, $C_{3-6}$ halocycloalkyl, $C_{3-6}$ haloheterocyclyl, $C_{3-6}$ cycloalkoxy, $C_{3-6}$ halocycloalkoxy, 6- to 10-membered aryl, 5- to 10-membered heteroaryl, and $C_{2-6}$ alkenyl;

ring A is selected from 3- to 8-membered heterocyclyl, 6- to 10-membered aryl, and 5- to 10-membered heteroaryl and 6- to 12-membered fused heterocyclyl containing at least one nitrogen atom; ring A is optionally substituted with one or more substituents selected from: halogen, amino, hydroxyl, cyano, $C_{1-6}$ alkyl, $C_{1-6}$ haloalkyl, $C_{1-6}$ alkoxy, 3- to 8-membered cycloalkoxy, $C_{1-6}$ haloalkoxy, 3- to 8-membered halocycloalkoxy, oxo, and $C_{2-6}$ alkenyl;

$X_1$ is O, S, C, or N;

$X_2$ is N or C;

$X_3$ is N or $CR_6$;

$R^6$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-8}$ cycloalkyl, $C_{3-8}$ halocycloalkyl, $C_{3-8}$ cycloalkoxy, and $C_{3-8}$ halocycloalkoxy;

$R^7$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-8}$ cycloalkyl, $C_{3-8}$ halocycloalkyl, $C_{3-8}$ cycloalkoxy, and $C_{3-8}$ halocycloalkoxy; and

Y is O or NH;

Z is $-CH_2-$, $-CF_2-$, -C(O)-, O, -S(O)-, $-S(O)_2-$, or -NH-.

**2.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to claim 1, **characterized in that** the compound is a compound of formula (II-1), (II-2), or (II-3),

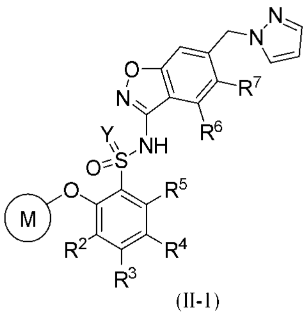
(II-1)

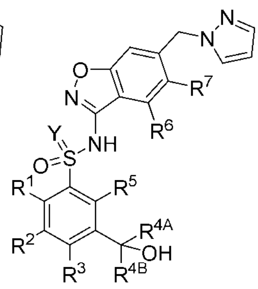
(II-2)

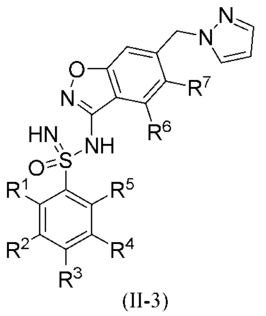
(II-3)

wherein:

$R^1$ is selected from $C_{1-4}$ alkoxy, $C_{1-4}$ haloalkoxy, $C_{3-6}$ cycloalkoxy, and $C_{3-6}$ halocycloalkoxy; preferably, $R^1$ is selected from methoxy, difluoromethoxy, trifluoromethoxy, - $OCH_2CHF_2$, cyclobutyloxy, and

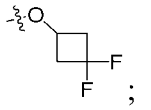
;

$R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy; preferably, $R^2$ and $R^3$ are identical or different and are each independently selected from a hydrogen atom, halogen, $C_{1-4}$ alkyl, and $C_{1-4}$ alkoxy; more preferably, $R^2$ and $R^3$ are identical or different and are each independently selected from a hydrogen atom and halogen; most preferably, both $R^2$ and $R^3$ are hydrogen atoms;

$R^4$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, $C_{3-6}$ halocycloalkyl, $C_{3-6}$ cycloalkoxy, $C_{3-6}$ halocycloalkoxy, and $C_{1-6}$ hydroxyalkyl; preferably, $R^4$ is selected from a hydrogen atom, $C_{1-6}$ alkyl, $C_{3-6}$ heterocyclyl, and $C_{1-6}$ hydroxyalkyl; more preferably, $R^4$ is selected from a hydrogen atom, ethyl, isopropyl, and *tert*-butyl;

$R^{4A}$ and $R^{4B}$ are identical or different and are each independently selected from $C_{1-6}$ alkyl and $C_{1-6}$ haloalkyl; preferably, both $R^{4A}$ and $R^{4B}$ are methyl;

$R^5$ is selected from a hydrogen atom, $C_{1-4}$ alkoxy, $C_{1-4}$ haloalkoxy, $C_{4-6}$ cycloalkoxy, and $C_{4-6}$ halocycloalkoxy; preferably, $R^5$ is selected from a hydrogen atom, methoxy, difluoromethoxy, trifluoromethoxy, $-OCH_2CHF_2$, cyclobutyloxy, and

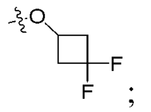
;

more preferably, $R^5$ is a hydrogen atom or methoxy;

$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy; preferably, $R^6$ and $R^7$ are identical or different and are each independently selected from a hydrogen atom, a chlorine atom, a fluorine atom, methoxy, ethoxy, isopropoxy, difluoromethoxy, and cyclopropyl;

ring M is selected from 4- to 7-membered cycloalkyl and 4- to 7-membered halocycloalkyl; preferably, ring M is selected from cyclobutyl and

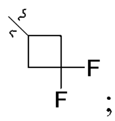
;

Y is O or NH; preferably, Y is O.

**3.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according

to claim 1, **characterized in that** the compound is a compound of formula (III),

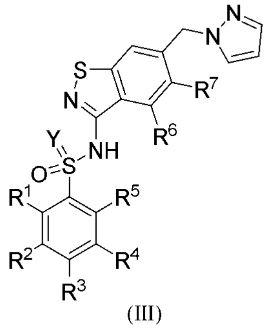

(III)

wherein:

$R^1$, $R^2$, and $R^3$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy; preferably, $R^1$ is selected from $C_{1-6}$ alkoxy, $C_{1-6}$ haloalkoxy, $C_{3-6}$ cycloalkoxy, and $C_{3-6}$ halocycloalkoxy, and $R^2$ and $R^3$ are identical or different and are each independently selected from a hydrogen atom, halogen, and $C_{1-6}$ alkyl; more preferably, $R^1$ is methoxy, and both $R^2$ and $R^3$ are hydrogen atoms;
$R^4$ and $R^5$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, $C_{3-6}$ cycloalkoxy, and $C_{1-6}$ hydroxyalkyl; preferably, $R^4$ is selected from a hydrogen atom, $C_{1-6}$ alkyl, $C_{3-6}$ heterocyclyl, and $C_{1-6}$ hydroxyalkyl, and $R^5$ is selected from a hydrogen atom, $C_{1-4}$ alkoxy, $C_{1-4}$ haloalkoxy, $C_{3-6}$ cycloalkoxy, and $C_{3-6}$ halocycloalkoxy; more preferably, $R^4$ is selected from a hydrogen atom, ethyl, isopropyl, *tert*-butyl, and

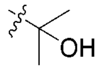,

and $R^5$ is selected from a hydrogen atom, methoxy, difluoromethoxy, trifluoromethoxy, $-OCH_2CHF_2$, cyclobutyloxy, and

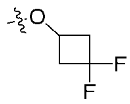;

most preferably, $R^4$ is a hydrogen atom, and $R^5$ is a hydrogen atom or methoxy;
$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy; preferably, $R^6$ and $R^7$ are identical or different and are each independently selected from a hydrogen atom, a chlorine atom, a fluorine atom, methoxy, ethoxy, isopropoxy, difluoromethoxy, and cyclopropyl;
Y is O or NH; preferably, Y is O.

**4.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to claim 1, **characterized in that** the compound is a compound of formula (IV),

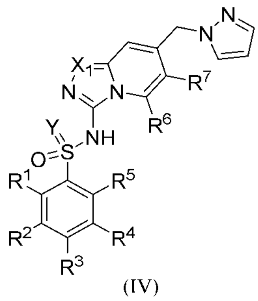

(IV)

wherein:

$R^1$, $R^2$, and $R^3$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy; preferably, $R^1$ is selected from $C_{1-6}$ alkoxy, $C_{1-6}$ haloalkoxy, $C_{3-6}$ cycloalkoxy, and $C_{3-6}$ halocycloalkoxy, and $R^2$ and $R^3$ are identical or different and are each independently selected from a hydrogen atom, halogen, and $C_{1-6}$ alkyl; more preferably, $R^1$ is methoxy, and both $R^2$ and $R^3$ are hydrogen atoms;

$R^4$ and $R^5$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, $C_{3-6}$ cycloalkoxy, and $C_{1-6}$ hydroxyalkyl; preferably, $R^4$ is selected from a hydrogen atom, $C_{1-6}$ alkyl, $C_{3-6}$ heterocyclyl, and $C_{1-6}$ hydroxyalkyl, and $R^5$ is selected from a hydrogen atom, $C_{1-4}$ alkoxy, $C_{1-4}$ haloalkoxy, $C_{3-6}$ cycloalkoxy, and $C_{3-6}$ halocycloalkoxy; more preferably, $R^4$ is selected from a hydrogen atom, ethyl, isopropyl, *tert*-butyl, and

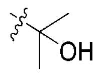,

and $R^5$ is selected from a hydrogen atom, methoxy, difluoromethoxy, trifluoromethoxy, $-OCH_2CHF_2$, cyclobutyloxy, and

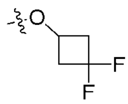;

most preferably, $R^4$ is a hydrogen atom, and $R^5$ is a hydrogen atom or methoxy;

$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy; preferably, $R^6$ and $R^7$ are identical or different and are each independently selected from a hydrogen atom, a chlorine atom, a fluorine atom, methoxy, ethoxy, isopropoxy, difluoromethoxy, and cyclopropyl;

$X_1$ is C or N;

Y is O or NH; preferably, Y is O.

**5.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to claim 1, **characterized in that**

the compound is a compound of formula (V),

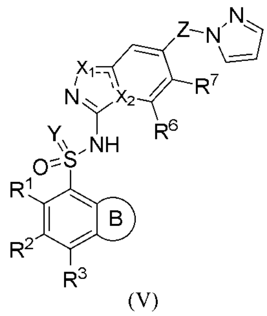

(V)

wherein:

the dashed line is an optional chemical bond;

$R^1$, $R^2$, $R^3$, $R^6$, $R^7$, Y, $X_1$, $X_2$, and Z are as defined in claim 1;

ring B is selected from 4- to 10-membered heterocyclyl, 5- to 6-membered heteroaryl, and 7- to 12-membered spiroheterocyclyl; ring B is optionally substituted with one or more substituents selected from: hydroxyl, amino, halogen, $C_{1-6}$ alkyl, $C_{1-6}$ haloalkyl, $C_{1-6}$ alkoxy, $C_{1-6}$ haloalkoxy, -C(O)$C_{1-6}$ alkyl, -C(O)$C_{3-6}$ cycloalkyl, -C(O)$C_{1-6}$ haloalkyl, -C(O)$C_{3-6}$ halocycloalkyl, -C(O)O$C_{1-6}$ alkyl, -$S(O)_2C_{1-6}$ alkyl, -$S(O)_2C_{3-6}$ cycloalkyl, -$S(O)_2C_{1-6}$ haloalkyl, - $S(O)_2C_{3-6}$ halocycloalkyl, -PO($C_{1-6}$ alkyl)$_2$, -PO($C_{3-6}$ cycloalkyl)$_2$, -PO($C_{1-6}$ haloalkyl)$_2$, -PO($C_{3-6}$ halocycloalkyl)$_2$, oxo, =$CH_2$, =CH$C_{1-6}$ alkyl, =C($C_{1-6}$ alkyl)$_2$, =CH$C_{1-6}$ haloalkyl, =C($C_{1-6}$ haloalkyl)$_2$, -NH$C_{1-6}$ alkyl, -N($C_{1-6}$ alkyl)$_2$, cyano, benzyl, $C_{3-6}$ cycloalkyl, $C_{3-6}$ heterocyclyl, $C_{3-6}$ halocycloalkyl, $C_{3-6}$ cycloalkoxy, $C_{3-6}$ halocycloalkoxy, 6- to 10-membered aryl, 5- to 10-membered heteroaryl, and $C_{2-6}$ alkenyl.

6. The compound or the pharmaceutically acceptable salt, the ester, the stereoisomer, the tautomer, the polymorph, the hydrate, the solvate, the N-oxide, the isotopically labeled compound, the metabolite, or the prodrug thereof according to claim 5, **characterized in that** in the compound of formula (V), ring B is

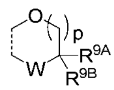,

wherein the dashed line represents a chemical bond shared by ring B and the benzene ring; W is selected from -O-, -$NR^{9C}$-, -$CR^{9D}R^{9E}$-,

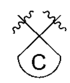,

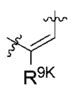, 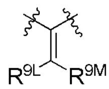,

and -C(O)-, and W is connected to a carbon atom ortho to the carbon atom where $R^3$ is located on the benzene ring;

p is 0, 1, or 2;

$R^{9A}$ and $R^{9B}$ are identical or different and are each independently selected from a hydrogen atom, hydroxyl, oxo, halogen, $C_{1-6}$ alkyl, $C_{1-6}$ haloalkyl, $C_{1-6}$ alkoxy, $C_{1-6}$ haloalkoxy, -C(O)$C_{1-6}$ alkyl, -C(O)$C_{1-6}$ haloalkyl, cyano, $C_{3-6}$ cycloalkyl, $C_{3-6}$ halocycloalkyl, $C_{3-6}$ cycloalkoxy, and $C_{3-6}$ halocycloalkoxy;

$R^{9C}$ is selected from a hydrogen atom, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, $C_{3-6}$ halocycloalkoxy, - C(O)$C_{1-6}$ alkyl, -C(O)$C_{3-6}$ cycloalkyl, -C(O)$C_{1-6}$ haloalkyl, -C(O)$C_{3-6}$ halocycloalkyl, -C(O)O$C_{1-6}$ alkyl, -$S(O)_2C_{1-6}$ alkyl, -$S(O)_2C_{3-6}$ cycloalkyl, -$S(O)_2C_{1-6}$ haloalkyl, -$S(O)_2C_{3-6}$ halocycloalkyl, - PO($C_{1-6}$ alkyl)$_2$, -PO($C_{3-6}$ cycloalkyl)$_2$, -PO($C_{1-6}$ haloalkyl)$_2$, -PO($C_{3-6}$ halocycloalkyl)$_2$, benzyl, 6- to 10-membered aryl, 5- to 10-membered heteroaryl, and $C_{2-6}$ alkenyl;

$R^{9D}$ and $R^{9E}$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, -C(O)$C_{1-6}$ alkyl, -C(O)$C_{1-6}$ haloalkyl, cyano, -NH$C_{1-6}$ alkyl,

-N($C_{1-6}$ alkyl)$_2$, $C_{3-6}$ cycloalkyl, $C_{3-6}$ halocycloalkyl, $C_{3-6}$ cycloalkoxy, $C_{3-6}$ halocycloalkoxy, and hydroxyl; ring C is selected from 3- to 8-membered cycloalkyl, 3- to 8-membered heterocyclyl, 6- to 12-membered spirocyclyl, 6- to 12-membered spiroheterocyclyl, and 5- to 12-membered bridged heterocyclyl; ring C is optionally substituted with one or more substituents selected from halogen, hydroxyl, amino, $C_{1-6}$ alkyl, and $C_{1-6}$ haloalkyl;

$R^{9K}$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, 6- to 10-membered aryl, 5- to 10-membered heteroaryl, and hydroxyl, wherein the $C_{3-6}$ cycloalkyl and $C_{3-6}$ heterocyclyl are optionally substituted with one or more substituents selected from: halogen, hydroxyl, methyl, and methoxy;

$R^{9M}$ and $R^{9L}$ are identical or different and are each independently selected from a hydrogen atom, $C_{1-6}$ alkyl, and $C_{1-6}$ haloalkyl.

**7.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to claim 6, **characterized in that** in the compound of formula (V), W is selected from -O-, -$NR^{9C}$-,

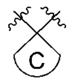, 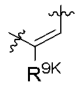,

and -C(O)-; relatively preferably, W is selected from -O-, -$NR^{9C}$-,

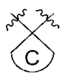, and 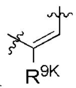;

more preferably, W is selected from -O- and -$NR^{9C}$-.

**8.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to claim 1, 5, or 6, **characterized in that** the compound is a compound of formula (VI-1), (VI-2), (VI-3), or (VI-4),

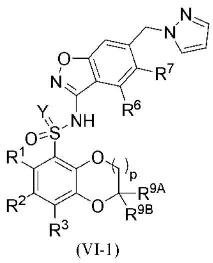
(VI-1)

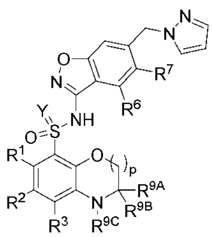
(VI-2)

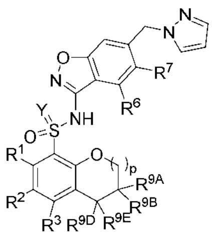
(VI-3)

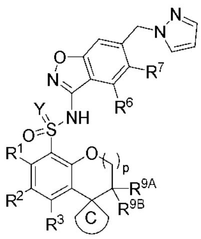
(VI-4)

wherein:

$R^1$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;

$R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;

$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;

$R^{9A}$ and $R^{9B}$ are identical or different and are each independently selected from a hydrogen atom, hydroxyl, oxo, halogen, $C_{1-6}$ alkyl, $C_{1-6}$ haloalkyl, $C_{1-6}$ alkoxy, $C_{1-6}$ haloalkoxy, -C(O)$C_{1-6}$ alkyl, -C(O)$C_{1-6}$ haloalkyl, cyano, $C_{3-6}$ cycloalkyl, $C_{3-6}$ halocycloalkyl, $C_{3-6}$ cycloalkoxy, and $C_{3-6}$ halocycloalkoxy;

$R^{9C}$ is selected from a hydrogen atom, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, $C_{3-6}$ halocycloalkoxy, - C(O)$C_{1-6}$ alkyl, -C(O)$C_{3-6}$ cycloalkyl, -C(O)$C_{1-6}$ haloalkyl, -C(O)$C_{3-6}$ halocycloalkyl, -C(O)O$C_{1-6}$ alkyl, -S(O)$_2C_{1-6}$ alkyl, -S(O)$_2C_{3-6}$ cycloalkyl, -S(O)$_2C_{1-6}$ haloalkyl,

-$S(O)_2C_{3-6}$ halocycloalkyl, - $PO(C_{1-6}$ alkyl$)_2$, -$PO(C_{3-6}$ cycloalkyl$)_2$, -$PO(C_{1-6}$ haloalkyl$)_2$, -$PO(C_{3-6}$ halocycloalkyl$)_2$, benzyl, 6- to 10-membered aryl, 5- to 10-membered heteroaryl, and $C_{2-6}$ alkenyl;

$R^{9D}$ and $R^{9E}$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, -$C(O)C_{1-6}$ alkyl, -$C(O)C_{1-6}$ haloalkyl, cyano, -$NHC_{1-6}$ alkyl, -$N(C_{1-6}$ alkyl$)_2$, $C_{3-6}$ cycloalkyl, $C_{3-6}$ halocycloalkyl, $C_{3-6}$ cycloalkoxy, $C_{3-6}$ halocycloalkoxy, and hydroxyl;

ring C is selected from 3- to 8-membered cycloalkyl, 3- to 8-membered heterocyclyl, 6- to 12-membered spirocyclyl, 6- to 12-membered spiroheterocyclyl, and 5- to 12-membered bridged heterocyclyl; ring C is optionally substituted with one or more substituents selected from halogen, hydroxyl, amino, $C_{1-6}$ alkyl, and $C_{1-6}$ haloalkyl;

Y is O or NH;

p is 0, 1, or 2.

**9.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-8, **characterized in that** $R^1$ is selected from a hydrogen atom, $C_{1-4}$ alkoxy, $C_{1-4}$ haloalkoxy, $C_{4-6}$ cycloalkoxy, and $C_{4-6}$ halocycloalkoxy.

**10.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-9, **characterized in that** $R^1$ is selected from a hydrogen atom, methoxy, difluoromethoxy, trifluoromethoxy, -$OCH_2CHF_2$, cyclobutyloxy, and

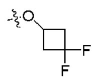 .

**11.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-10, **characterized in that** $R^1$ is cyclobutyloxy or methoxy.

**12.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-11, **characterized in that** $R^1$ is methoxy.

**13.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-12, **characterized in that** $R^2$ and $R^3$ are each independently selected from a hydrogen atom, halogen, $C_{1-4}$ alkyl, and $C_{1-4}$ alkoxy.

**14.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-13, **characterized in that** both $R^2$ and $R^3$ are hydrogen atoms.

**15.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-14, **characterized in that** $R^6$ and $R^7$ are identical or different and are each independently selected from a hydrogen atom, a chlorine atom, a fluorine atom, methoxy, ethoxy, isopropoxy, difluoromethoxy, and cyclopropyl.

**16.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-15, **characterized in that** $R^6$ and $R^7$ are identical or different and are each independently selected from a hydrogen atom, a chlorine atom, a fluorine atom, and methoxy.

**17.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-16, **characterized in that** $R^6$ is methoxy.

**18.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-17, **characterized in that** $R^7$ is a hydrogen atom or a fluorine atom.

**19.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-18, **characterized in that** $R^7$ is a fluorine atom.

**20.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-19, **characterized in that** $R^{9A}$ and $R^{9B}$ are identical or different and are each independently selected from a hydrogen atom, a fluorine atom, and methyl.

**21.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-20, **characterized in that** both $R^{9A}$ and $R^{9B}$ are hydrogen atoms.

**22.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-21, **characterized in that** $R^{9C}$ is selected from a hydrogen atom, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, $C_{3-6}$ halocycloalkoxy, heteroaryl, -C(O)$C_{1-6}$ alkyl, -C(O)$C_{3-6}$ cycloalkyl, -C(O)$C_{1-6}$ haloalkyl, -C(O)$C_{3-6}$ halocycloalkyl, -$S(O)_2C_{1-6}$ alkyl, -$S(O)_2C_{3-6}$ cycloalkyl, -$S(O)_2C_{1-6}$ haloalkyl, -$S(O)_2C_{3-6}$ halocycloalkyl, benzyl, and

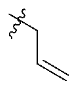.

**23.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-22, **characterized in that** $R^{9C}$ is a hydrogen atom.

**24.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-23, **characterized in that** $R^{9C}$ is methyl.

**25.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-23, **characterized in that** $R^{9C}$ is deuterated methyl.

**26.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-23, **characterized in that** $R^{9C}$ is ethyl.

**27.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-23, **characterized in that** $R^{9C}$ is trifluoroethyl.

**28.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-23, **characterized in that** $R^{9C}$ is -C(O)$OCH_3$.

**29.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-23, **characterized in that** $R^{9C}$ is benzyl.

**30.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according

to any one of claims 6-23, **characterized in that** $R^{9C}$ is $-C(O)CH_3$.

**31.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-23, **characterized in that** $R^{9C}$ is $-S(O)_2CH_3$.

**32.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-21, **characterized in that** $R^{9D}$ and $R^{9E}$ are identical or different and are each independently selected from a hydrogen atom, a fluorine atom, methyl, and hydroxyl.

**33.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-21 and 32, **characterized in that** $R^{9D}$ and $R^{9E}$ are identical or different and are each independently selected from a fluorine atom and methyl.

**34.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-21, **characterized in that** ring C is selected from

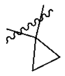, 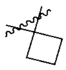, 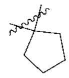, and 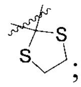;

ring C is optionally substituted with one or more substituents selected from halogen, hydroxyl, amino, $C_{1-6}$ alkyl, and $C_{1-6}$ haloalkyl; preferably, ring C is selected from

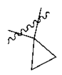, 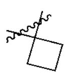, 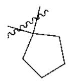, and 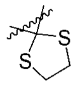.

**35.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 5-34, **characterized in that** Y is O.

**36.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-35, **characterized in that** p is 1.

**37.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 6-35, **characterized in that** p is 2.

**38.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to claim 1, 5, or 6, **characterized in that** the compound is a compound of formula (VII-1), (VII-2), or (VII-3),

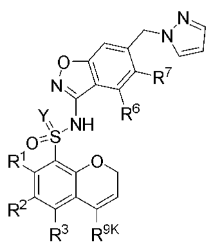
(VII-1)

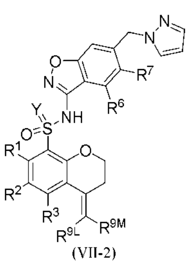
(VII-2)

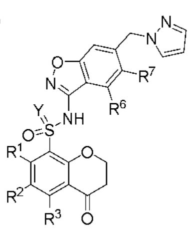
(VII-3)

wherein:

$R^1$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
$R^2$ and $R^3$ are identical or different and are each independently selected from a hydrogen atom, halogen, $C_{1-4}$ alkyl, $C_{1-4}$ haloalkyl, $C_{1-4}$ alkoxy, and $C_{1-4}$ haloalkoxy;
$R^6$ and $R^7$ are identical or different and are each independently selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ halocycloalkyl, $C_{3-6}$ cycloalkoxy, and $C_{3-6}$ halocycloalkoxy;
$R^{9K}$ is selected from a hydrogen atom, halogen, $C_{1-6}$ alkyl, $C_{1-6}$ haloalkyl, $C_{1-6}$ alkoxy, $C_{1-6}$ haloalkoxy, $C_{3-6}$ cycloalkyl, $C_{3-6}$ heterocyclyl, 6- to 10-membered aryl, 5- to 10-membered heteroaryl, and hydroxyl, wherein the $C_{3-6}$ cycloalkyl and $C_{3-6}$ heterocyclyl are optionally substituted with one or more substituents selected from: halogen, hydroxyl, methyl, and methoxy;
$R^{9M}$ and $R^{9L}$ are identical or different and are each independently selected from a hydrogen atom, $C_{1-6}$ alkyl, and $C_{1-6}$ haloalkyl;
Y is O or NH.

**39.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to claim 38, **characterized in that** $R^1$ is selected from a hydrogen atom, $C_{1-4}$ alkoxy, $C_{1-4}$ haloalkoxy, $C_{4-6}$ cycloalkoxy, and $C_{4-6}$ halocycloalkoxy.

**40.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to claim 38 or 39, **characterized in that** $R^1$ is selected from a hydrogen atom, methoxy, difluoromethoxy, trifluoromethoxy, - $OCH_2CHF_2$, cyclobutyloxy, and

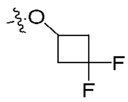

.

**41.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-40, **characterized in that** $R^1$ is methoxy.

**42.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-41, **characterized in that** $R^2$ and $R^3$ are identical or different and are each independently selected from a hydrogen atom, halogen, $C_{1-4}$ alkyl, and $C_{1-4}$ alkoxy.

**43.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-42, **characterized in that** $R^2$ and $R^3$ are identical or different and are each independently selected from a hydrogen atom and halogen.

**44.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-43, **characterized in that** both $R^2$ and $R^3$ are hydrogen atoms.

**45.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-44, **characterized in that** $R^6$ and $R^7$ are identical or different and are each independently selected from a hydrogen atom, a chlorine atom, a fluorine atom, methoxy, ethoxy, isopropoxy, difluoromethoxy, and cyclopropyl.

**46.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according

to any one of claims 38-45, **characterized in that** $R^6$ is methoxy.

**47.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-46, **characterized in that** $R^7$ is selected from a hydrogen atom and a fluorine atom.

**48.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-47, **characterized in that** $R^7$ is a fluorine atom.

**49.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-48, **characterized in that** $R^{9K}$ is selected from a hydrogen atom, halogen, $C_{1-6}$ alkyl, $C_{1-6}$ haloalkyl, and hydroxyl.

**50.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-49, **characterized in that** $R^{9K}$ is selected from a hydrogen atom, methyl, and hydroxyl.

**51.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-50, **characterized in that** $R^{9K}$ is a hydrogen atom.

**52.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-47, **characterized in that** $R^{9M}$ and $R^{9L}$ are identical or different and are each independently selected from a hydrogen atom and $C_{1-6}$ alkyl.

**53.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-47 and 52, **characterized in that** $R^{9M}$ and $R^{9L}$ are identical or different and are each independently selected from a hydrogen atom, a fluorine atom, and methyl.

**54.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 38-53, **characterized in that** Y is O.

**55.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of the preceding claims, **characterized in that** the compound is isotopically substituted; preferably, the isotopic substitution is a substitution with a deuterium atom.

**56.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of the preceding claims, **characterized in that** the structural formula of the compound is selected from Table 1.

**57.** A method for preparing a compound or a pharmaceutically acceptable salt thereof, **characterized in that** the method comprises:

scheme I:

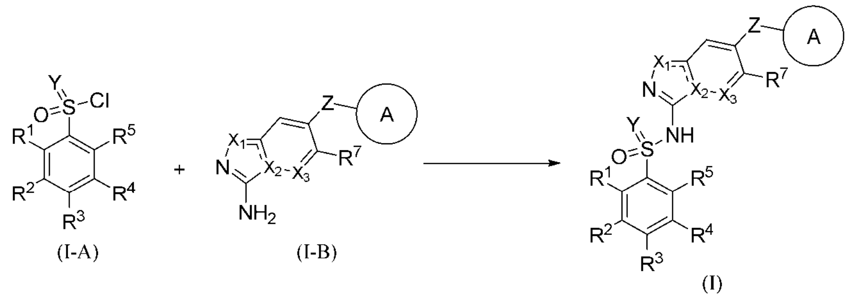

reacting a compound of formula (I-A) or a salt thereof with a compound of formula (I-B) or a salt thereof in the presence of a base to give a compound of formula (I) or a pharmaceutically acceptable salt thereof, wherein:

the dashed line is an optional chemical bond, and
ring A, $R^1$, $R^2$, $R^3$, $R^4$, $R^5$, $R^7$, $X_1$, $X_2$, $X_3$, Y, and Z are as defined in claim 1;

scheme II:

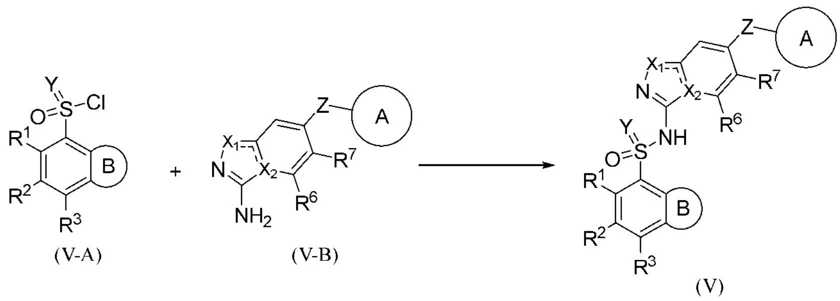

reacting a compound of formula (V-A) or a salt thereof with a compound of formula (V-B) or a salt thereof in the presence of a base to give a compound of formula (V) or a pharmaceutically acceptable salt thereof, wherein:

the dashed line is an optional chemical bond, and
ring A, ring B, $R^1$, $R^2$, $R^3$, $R^6$, $R^7$, $X_1$, $X_2$, Y, and Z are as defined in claim 5 or 6;

scheme III:

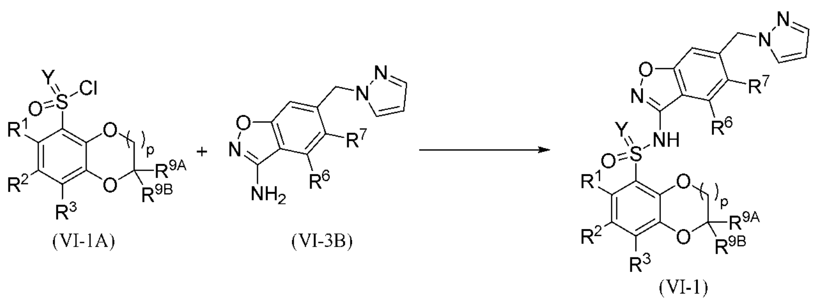

reacting a compound of formula (VI-1A) or a salt thereof with a compound of formula (VI-3B) or a salt thereof in the presence of a base to give a compound of formula (VI-1) or a pharmaceutically acceptable salt thereof,
wherein:

$R^1$, $R^2$, $R^3$, $R^6$, $R^7$, $R^{9A}$, $R^{9B}$, Y, and p are as defined in claim 8;

scheme IV:

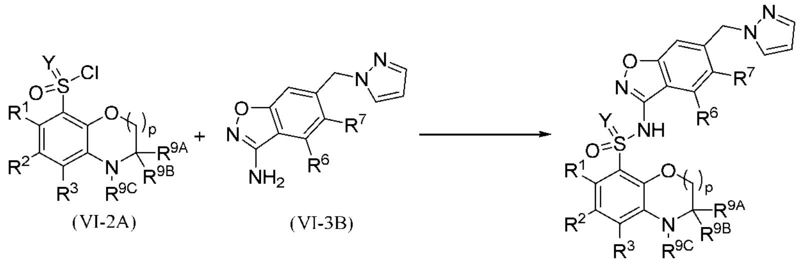

(VI-2A) (VI-3B) (VI-2)

reacting a compound of formula (VI-2A) or a salt thereof with a compound of formula (VI-3B) or a salt thereof in the presence of a base to give a compound of formula (VI-2) or a pharmaceutically acceptable salt thereof,
wherein:

$R^1$, $R^2$, $R^3$, $R^6$, $R^7$, $R^{9A}$, $R^{9B}$, $R^{9C}$, Y, and p are as defined in claim 8;

scheme V:

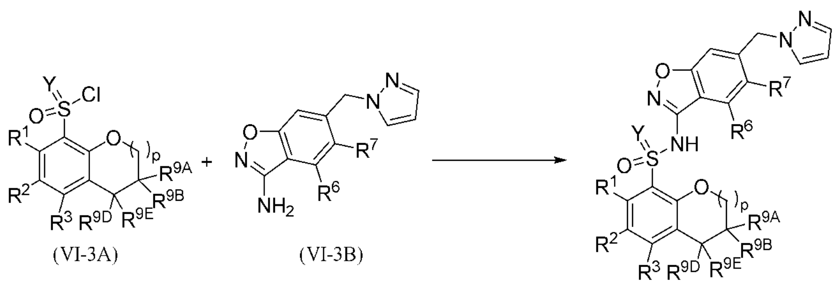

(VI-3A) (VI-3B) (VI-3)

reacting a compound of formula (VI-3A) or a salt thereof with a compound of formula (VI-3B) or a salt thereof in the presence of a base to give a compound of formula (VI-3) or a pharmaceutically acceptable salt thereof,
wherein:

$R^1$, $R^2$, $R^3$, $R^6$, $R^7$, $R^{9A}$, $R^{9B}$, $R^{9D}$, $R^{9E}$, Y, and p are as defined in claim 8;

scheme VI:

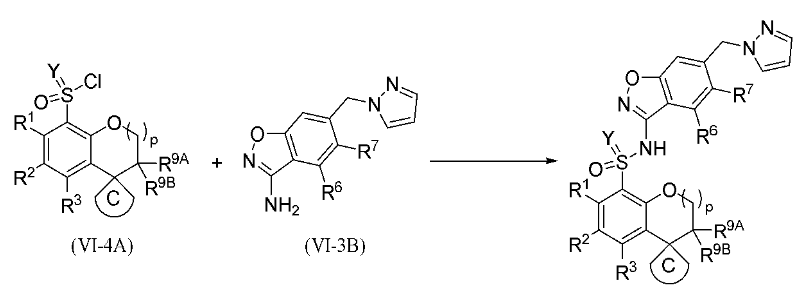

(VI-4A) (VI-3B) (VI-4)

reacting a compound of formula (VI-4A) or a salt thereof with a compound of formula (VI-3B) or a salt thereof in the presence of a base to give a compound of formula (VI-4) or a pharmaceutically acceptable salt thereof,
wherein:

ring C, $R^1$, $R^2$, $R^3$, $R^6$, $R^7$, $R^{9A}$, $R^{9B}$, Y, and p are as defined in claim 8;

scheme VII:

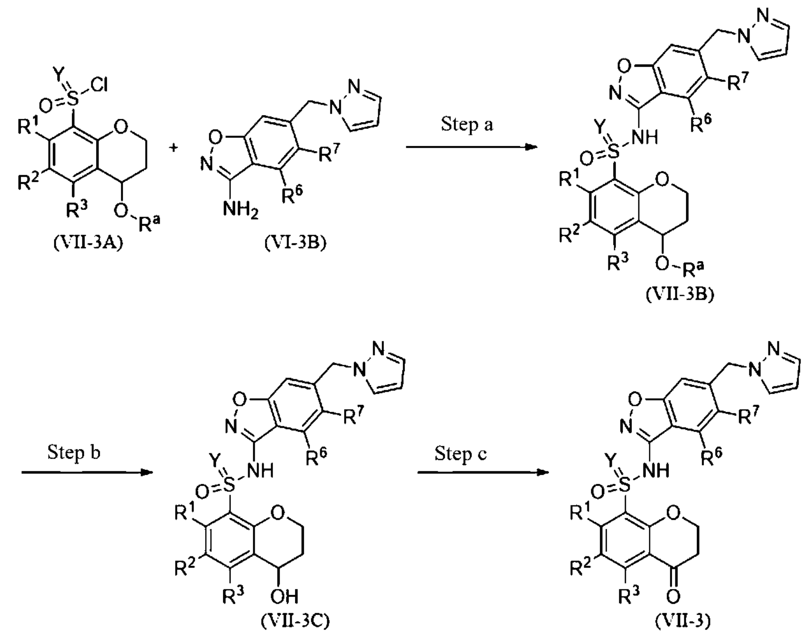

(VII-3A) (VI-3B) (VII-3B) (VII-3C) (VII-3)

step a: reacting a compound of formula (VII-3A) or a salt thereof with a compound of formula (VI-3B) or a salt thereof in the presence of a base to give a compound of formula (VII-3B) or a pharmaceutically acceptable salt thereof;
step b: reacting the compound of formula (VII-3B) or the salt thereof with a deprotecting reagent to remove a hydroxyl protecting group to give a compound of formula (VII-3C) or a pharmaceutically acceptable salt thereof; and
step c: reacting the compound of formula (VII-3C) or the salt thereof with an oxidizing reagent for oxidation to give a compound of formula (VII-3) or a pharmaceutically acceptable salt thereof;
wherein:

$R^1$, $R^2$, $R^3$, $R^6$, $R^7$, and Y are as defined in claim 38;
$R^a$ is the hydroxyl protecting group, preferably (trimethylsilyl)ethoxymethyl;
the deprotecting reagent in step b is an acid or a fluorine-containing reagent, preferably trifluoroacetic acid or tetrabutylammonium fluoride;
the oxidizing reagent in step c is a reagent for oxidizing a hydroxyl group to a ketone, preferably manganese dioxide or Dess-Martin reagent;
the base used in the reactions of the above schemes is selected from: triethylamine, diisopropylethylamine, pyridine, 2,4-dimethylpyridine, 2,6-dimethylpyridine, *n*-butyllithium, lithium bis(trimethylsilyl)amide, sodium hydride, sodium hydroxide, cesium carbonate, and potassium carbonate;
the reactions of the above schemes are preferably carried out in a solvent selected from: ethylene glycol dimethyl ether, methanol, ethanol, acetonitrile, *n*-butanol, toluene, tetrahydrofuran, dichloromethane, dimethyl sulfoxide, 1,4-dioxane, *N,N*-dimethylformamide, *N,N*-dimethylacetamide, 1,2-dibromoethane, toluene, pyridine, and a mixture thereof.

**58.** A pharmaceutical composition, **characterized in that** the pharmaceutical composition comprises the compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 1-56, and one or more pharmaceutically acceptable carriers, diluents, or excipients.

**59.** The compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 1-56, or the pharmaceutical composition according to claim 58, **characterized in that** the compound or the pharmaceutically acceptable salt, the ester, the stereoisomer, the tautomer, the polymorph, the hydrate, the solvate, the N-oxide, the isotopically labeled compound, the metabolite, or the prodrug thereof, or the pharmaceutical composition is for use as a drug, preferably for use in the treatment or prevention of a cancer in which KAT6A/B plays a role.

**60.** Use of the compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 1-56, or the pharmaceutical composition according to claim 58 in the preparation of a medicament for inhibiting a KAT, **characterized in that** the KAT is preferably KAT6, more preferably KAT6A and/or KAT6B.

**61.** The use according to claim 60, **characterized in that** the compound or the pharmaceutically acceptable salt, the ester, the stereoisomer, the tautomer, the polymorph, the hydrate, the solvate, the N-oxide, the isotopically labeled compound, the metabolite, the prodrug, or the isotopically substituted form thereof, or the pharmaceutical composition is used for treating or preventing a cancer, wherein the cancer is selected from lung cancer, breast cancer, rectal cancer, colon cancer, esophageal cancer, gastric cancer, liver cancer, gallbladder cancer, cholangiocarcinoma, kidney cancer, bladder cancer, urothelial cancer, head and neck cancer, nasopharyngeal cancer, prostate cancer, cervical cancer, endometrial cancer, ovarian cancer, pancreatic cancer, melanoma, bone cancer, mesothelioma, gastrointestinal stromal tumor, sarcoma, brain glioma, thyroid cancer, salivary gland tumor, glioblastoma, neuroblastoma, gastric myxoma, lymphoma, leukemia, plasmacytoma, sinoatrial node tumor, and tenosynovial giant cell tumor.

**62.** The use according to claim 61, **characterized in that** the cancer is selected from breast cancer, prostate cancer, lung cancer, pancreatic cancer, ovarian cancer, cervical cancer, endometrial cancer, bladder cancer, brain glioma, malignant lymphoma, liver cancer, and leukemia.

**63.** The use according to claim 61 or 62, **characterized in that** the breast cancer is $ER^+$ breast cancer or $ER^+$/$HER2^-$ breast cancer.

**64.** The use according to claim 61 or 62, **characterized in that** the lung cancer is non-small cell lung cancer.

**65.** The use according to claim 61 or 62, **characterized in that** the prostate cancer is castration-resistant prostate cancer.

**66.** A method for inhibiting KAT6A/B, **characterized in that** the method comprises administering the compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 1-56, or the pharmaceutical composition according to claim 58.

**67.** A method for treating or preventing a cancer in which KAT6A/B plays a role, **characterized in that** the method comprises administering to a subject in need thereof the compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 1-56, or the pharmaceutical composition according to claim 58, wherein the cancer is selected from lung cancer, breast cancer, rectal cancer, colon cancer, esophageal cancer, gastric cancer, liver cancer, gallbladder cancer, cholangiocarcinoma, kidney cancer, bladder cancer, urothelial cancer, head and neck cancer, nasopharyngeal cancer, prostate cancer, cervical cancer, endometrial cancer, ovarian cancer, pancreatic cancer, melanoma, bone cancer, mesothelioma, gastrointestinal stromal tumor, sarcoma, brain glioma, thyroid cancer, salivary gland tumor, glioblastoma, neuroblastoma, gastric myxoma, lymphoma, leukemia, plasmacytoma, sinoatrial node tumor, and tenosynovial giant cell tumor.

**68.** The method according to claim 67, **characterized in that** the cancer is selected from breast cancer, prostate cancer, lung cancer, pancreatic cancer, ovarian cancer, cervical cancer, endometrial cancer, bladder cancer, brain glioma, malignant lymphoma, liver cancer, and leukemia.

**69.** The method according to claim 67 or 68, **characterized in that** the breast cancer is $ER^+$ breast cancer or $ER^+$/$HER2^-$ breast cancer.

**70.** The method according to claim 67 or 68, **characterized in that** the lung cancer is non-small cell lung cancer.

**71.** The method according to claim 67 or 68, **characterized in that** the prostate cancer is castration-resistant prostate cancer.

**72.** A kit, **characterized in that** the kit comprises the compound or the pharmaceutically acceptable salt, the ester, the stereoisomer, the tautomer, the polymorph, the hydrate, the solvate, the N-oxide, the isotopically labeled compound, the metabolite, or the prodrug thereof according to any one of claims 1-56, or the pharmaceutical composition according to claim 58, and a package insert.

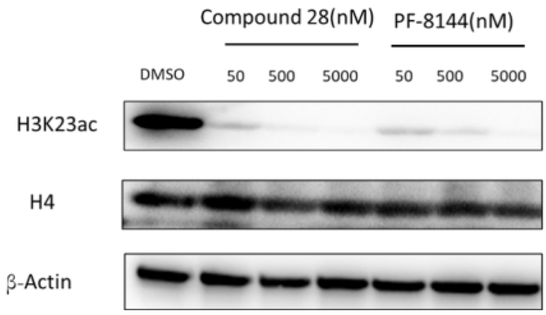
Compound 28(nM)
PF-8144(nM)
DMSO
50
500
5000
50
500
5000
H3K23ac
H4
β-Actin

FIG. 1

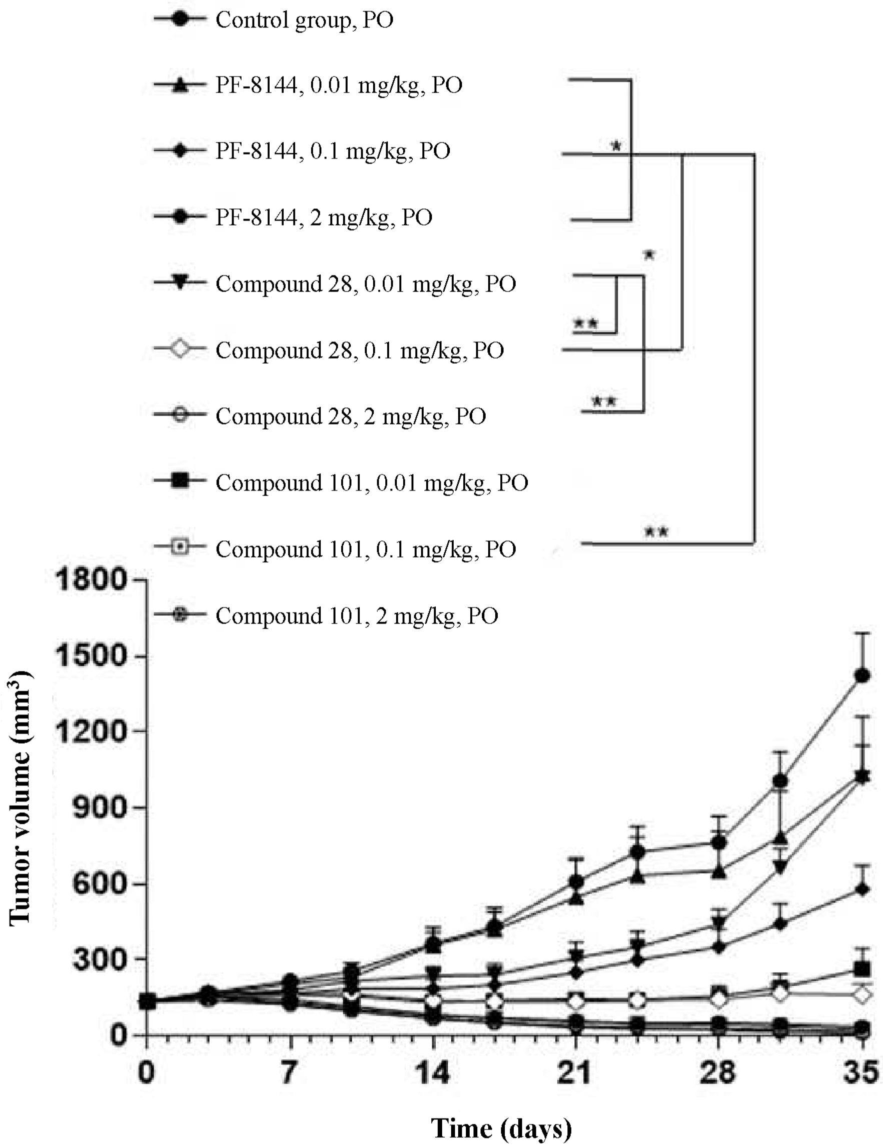
Control group, PO
PF-8144, 0.01 mg/kg, PO
PF-8144, 0.1 mg/kg, PO
PF-8144, 2 mg/kg, PO
Compound 28, 0.01 mg/kg, PO
Compound 28, 0.1 mg/kg, PO
Compound 28, 2 mg/kg, PO
Compound 101, 0.01 mg/kg, PO
Compound 101, 0.1 mg/kg, PO
Compound 101, 2 mg/kg, PO
*
*
**
**
**
Tumor volume (mm³)
1800
1500
1200
900
600
300
0
0
7
14
21
28
35
Time (days)

FIG. 2

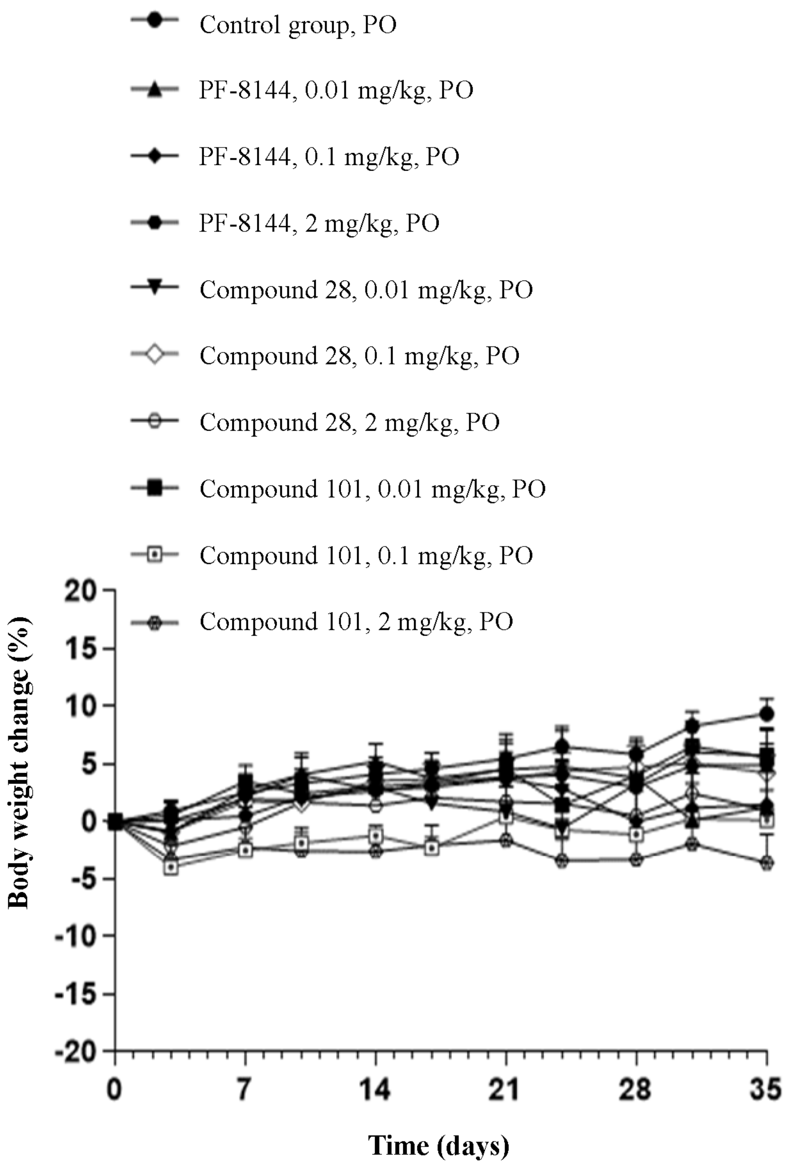
Control group, PO
PF-8144, 0.01 mg/kg, PO
PF-8144, 0.1 mg/kg, PO
PF-8144, 2 mg/kg, PO
Compound 28, 0.01 mg/kg, PO
Compound 28, 0.1 mg/kg, PO
Compound 28, 2 mg/kg, PO
Compound 101, 0.01 mg/kg, PO
Compound 101, 0.1 mg/kg, PO
Compound 101, 2 mg/kg, PO
Body weight change (%)
20
15
10
5
0
-5
-10
-15
-20
0
7
14
21
28
35
Time (days)

FIG. 3

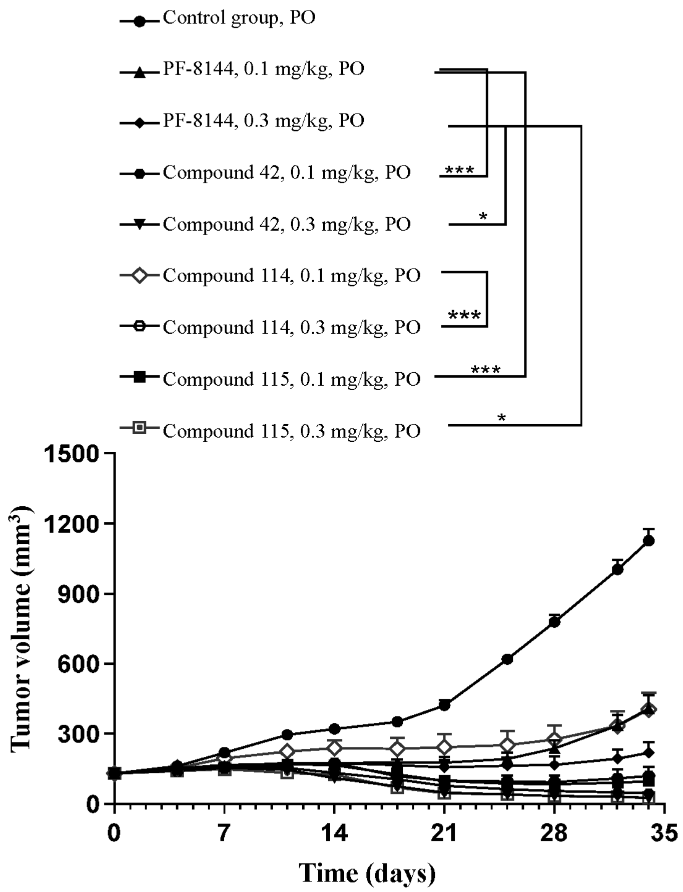
Control group, PO
PF-8144, 0.1 mg/kg, PO
PF-8144, 0.3 mg/kg, PO
Compound 42, 0.1 mg/kg, PO
Compound 42, 0.3 mg/kg, PO
Compound 114, 0.1 mg/kg, PO
Compound 114, 0.3 mg/kg, PO
Compound 115, 0.1 mg/kg, PO
Compound 115, 0.3 mg/kg, PO
***
*
***
***
*
Tumor volume (mm3)
1500
1200
900
600
300
0
0
7
14
21
28
35
Time (days)

FIG. 4

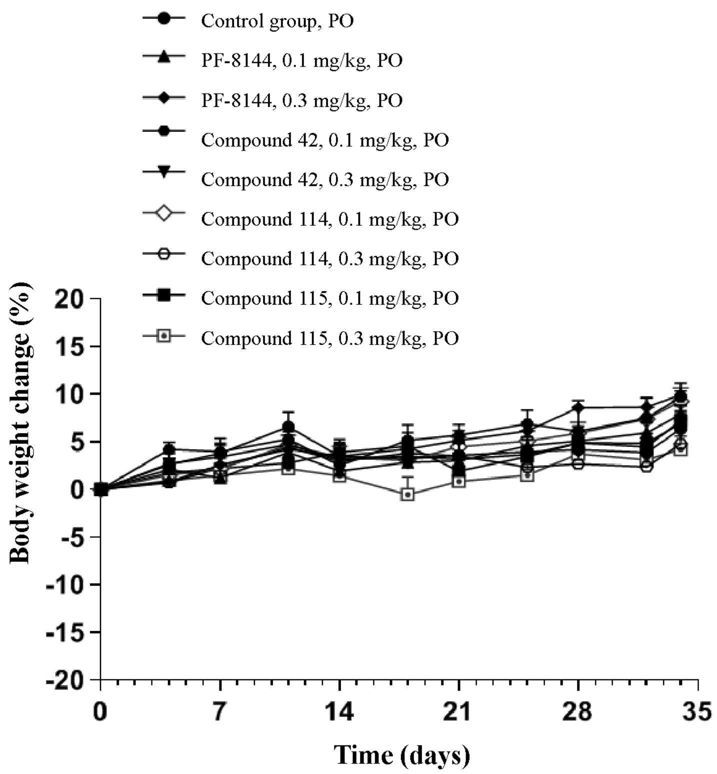
Control group, PO
PF-8144, 0.1 mg/kg, PO
PF-8144, 0.3 mg/kg, PO
Compound 42, 0.1 mg/kg, PO
Compound 42, 0.3 mg/kg, PO
Compound 114, 0.1 mg/kg, PO
Compound 114, 0.3 mg/kg, PO
Compound 115, 0.1 mg/kg, PO
Compound 115, 0.3 mg/kg, PO
Body weight change (%)
20
15
10
5
0
-5
-10
-15
-20
0
7
14
21
28
35
Time (days)

FIG. 5

## INTERNATIONAL SEARCH REPORT

International application No.
**PCT/CN2024/111949**

**A. CLASSIFICATION OF SUBJECT MATTER**

C07D 413/06(2006.01)i; C07D 413/02(2006.01)i; C07D 487/04(2006.01)i; C07D 519/00(2006.01)i; A61K 31/41(2006.01)i; A61K 31/423(2006.01)i; A61K 31/428(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, ENTXT, VEN, STN (Registry, Marpat, Caplus), CNKI, 万方, WANFANG: 磺酰胺, 赖氨酸乙酰转移酶, 肿瘤, 癌, sulfonamide, KAT6, cancer, tumor, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2023280182 A1 (HANGZHOU INNOGATE PHARMA CO., LTD.) 12 January 2023 (2023-01-12)<br>claims 1 and 15-18 | 1-2, 55-65, 72 |
| X | CN 114364672 A (PFIZER INC. et al.) 15 April 2022 (2022-04-15)<br>claims 1 and 26, description, paragraphs 0199, 0252-0263 and 0579, and embodiments 61, 73 and 99 | 5-65, 72 |
| X | American Chemical Society (ACS). "RN 2890672-86-1"<br>*STNext Registry Database*, 26 January 2023 (2023-01-26),<br>abstract | 1-2, 56 |
| PX | WO 2023192817 A1 (ISOSTERIX INC.) 05 October 2023 (2023-10-05)<br>claims 1 and 93-97, and description, paragraphs 0002, 0273-0274, 0305 and 0314-0316 | 4, 55-56, 58-65, 72 |
| A | WO 2023088233 A1 (INSILICO MEDICINE IP LIMITED) 25 May 2023 (2023-05-25)<br>entire document | 1-65, 72 |
| A | CN 112334466 A (CTXT PTY LTD.) 05 February 2021 (2021-02-05)<br>entire document | 1-65, 72 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 November 2024** | **26 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

## INTERNATIONAL SEARCH REPORT

International application No.
**PCT/CN2024/111949**

### C. DOCUMENTS CONSIDERED TO BE RELEVANT

| Category* | Citation of document, with indication, where appropriate, of the relevant passages | Relevant to claim No. |
|---|---|---|
| A | CN 115594695 A (JIANGSU HENGRUI MEDICINE CO., LTD. et al.) 13 January 2023 (2023-01-13)<br>entire document | 1-65, 72 |

Form PCT/ISA/210 (second sheet) (July 2022)

**INTERNATIONAL SEARCH REPORT**

International application No.
**PCT/CN2024/111949**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **66-71**
because they relate to subject matter not required to be searched by this Authority, namely:

Claim 66 sets forth a method for inhibiting KATA/B, and claims 67-71 set forth a method for treating or preventing a cancer in which KATA/B plays a role, which methods fall within disease treatment methods as defined in PCT Rule 39.1(iv), and therefore no search is carried out.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/111949**

| Patent document cited in search report | Publication date (day/month/year) | Patent family member(s) | Publication date (day/month/year) |
|---|---|---|---|
| WO 2023280182 A1 | 12 January 2023 | EP 4368620 A1 | 15 May 2024 |
| | | WO 2024008129 A1 | 11 January 2024 |
| | | JP 2024526311 A | 17 July 2024 |
| | | CA 3225068 A1 | 12 January 2023 |
| | | CN 118382622 A | 23 July 2024 |
| CN 114364672 A | 15 April 2022 | AU 2023274178 A1 | 15 February 2024 |
| | | PT 3986890 T | 17 January 2024 |
| | | KR 20220024671 A | 03 March 2022 |
| | | TW 202115048 A | 16 April 2021 |
| | | TWI 787620 B | 21 December 2022 |
| | | FI 3986890 T3 | 15 January 2024 |
| | | MD 3986890 T2 | 30 April 2024 |
| | | HUE 064683 T2 | 28 April 2024 |
| | | BR 112021025528 A2 | 19 April 2022 |
| | | RS 64931 B1 | 29 December 2023 |
| | | HRP 20231501 T1 | 01 March 2024 |
| | | LT 3986890 T | 11 December 2023 |
| | | EP 3986890 A1 | 27 April 2022 |
| | | EP 3986890 B1 | 15 November 2023 |
| | | IL 288802 A | 01 February 2022 |
| | | EP 4299135 A2 | 03 January 2024 |
| | | EP 4299135 A3 | 28 February 2024 |
| | | CR 20210627 A | 08 February 2022 |
| | | AU 2020296361 A1 | 06 January 2022 |
| | | CL 2021003372 A1 | 07 October 2022 |
| | | MX 2021016085 A | 20 April 2022 |
| | | DK 3986890 T3 | 18 December 2023 |
| | | CO 2021017504 A2 | 17 January 2022 |
| | | PL 3986890 T3 | 11 March 2024 |
| | | US 2020399258 A1 | 24 December 2020 |
| | | US 11492346 B2 | 08 November 2022 |
| | | UY 38752 A | 29 January 2021 |
| | | ECSP 21091615 A | 31 January 2022 |
| | | SI 3986890 T1 | 29 March 2024 |
| | | CA 3143666 A1 | 24 December 2020 |
| | | CA 3143666 C | 11 June 2024 |
| | | WO 2020254946 A1 | 24 December 2020 |
| | | ES 2969616 T3 | 21 May 2024 |
| | | PE 20220808 A1 | 20 May 2022 |
| | | JP 2022537285 A | 25 August 2022 |
| | | JP 7352662 B2 | 28 September 2023 |
| | | JP 2023175821 A | 12 December 2023 |
| | | US 2023174522 A1 | 08 June 2023 |
| WO 2023192817 A1 | 05 October 2023 | US 2023416260 A1 | 28 December 2023 |
| | | US 11976075 B2 | 07 May 2024 |
| | | AU 2023241822 A1 | 26 September 2024 |
| | | WO 2023192817 A9 | 03 October 2024 |
| | | US 2023303580 A1 | 28 September 2023 |
| | | TW 202400593 A | 01 January 2024 |
| WO 2023088233 A1 | 25 May 2023 | EP 4433474 A1 | 25 September 2024 |

Form PCT/ISA/210 (patent family annex) (July 2022)

**INTERNATIONAL SEARCH REPORT**
**Information on patent family members**

International application No.
**PCT/CN2024/111949**

| Patent document cited in search report | Publication date (day/month/year) | Patent family member(s) | Publication date (day/month/year) |
|---|---|---|---|
| | | CO 2024007129 A2 | 17 June 2024 |
| | | CL 2024001466 A1 | 13 September 2024 |
| | | PE 20241355 A1 | 03 July 2024 |
| | | US 2024150374 A1 | 09 May 2024 |
| | | US 12091406 B2 | 17 September 2024 |
| | | IL 312696 A | 01 July 2024 |
| | | KR 20240109267 A | 10 July 2024 |
| | | US 2024109880 A1 | 04 April 2024 |
| | | AR 127681 A1 | 21 February 2024 |
| | | CA 3237830 A1 | 25 May 2023 |
| | | MX 2024005839 A | 26 June 2024 |
| | | TW 202334163 A | 01 September 2023 |
| | | AU 2022390319 A1 | 30 May 2024 |
| CN 112334466 A | 05 February 2021 | ES 2967983 T3 | 06 May 2024 |
| | | BR 112020025869 A2 | 23 March 2021 |
| | | EA 202092451 A1 | 16 March 2021 |
| | | SG 11202010450 VA | 28 January 2021 |
| | | EP 3810602 A1 | 28 April 2021 |
| | | EP 3810602 B1 | 01 November 2023 |
| | | MD 3810602 T2 | 31 March 2024 |
| | | EP 4335439 A2 | 13 March 2024 |
| | | EP 4335439 A3 | 15 May 2024 |
| | | GEP 20227403 B | 10 August 2022 |
| | | SI 3810602 T1 | 29 February 2024 |
| | | UY 38270 A | 31 January 2020 |
| | | HRP 20231467 T1 | 01 March 2024 |
| | | CR 20210032 A | 11 February 2021 |
| | | JP 2024133637 A | 02 October 2024 |
| | | KR 20210022655 A | 03 March 2021 |
| | | NI 202000092 A | 23 March 2021 |
| | | JP 2021529738 A | 04 November 2021 |
| | | MA 52948 A | 28 April 2021 |
| | | MA 52948 B1 | 29 February 2024 |
| | | IL 279554 A | 31 January 2021 |
| | | IL 279554 B1 | 01 August 2024 |
| | | CL 2020003219 A1 | 11 June 2021 |
| | | WO 2019243491 A1 | 26 December 2019 |
| | | AU 2019289888 A1 | 12 November 2020 |
| | | CA 3101238 A1 | 26 December 2019 |
| | | PH 12020500673 A1 | 31 May 2021 |
| | | ECSP 20075270 A | 26 February 2021 |
| | | PT 3810602 T | 03 January 2024 |
| | | MX 2020012723 A | 14 May 2021 |
| | | RS 64932 B1 | 29 December 2023 |
| | | AR 114972 A1 | 11 November 2020 |
| | | LT 3810602 T | 25 January 2024 |
| | | GB 201810092 D0 | 08 August 2018 |
| | | TW 202016103 A | 01 May 2020 |
| | | TWI 826471 B | 21 December 2023 |
| | | CO 2020014586 A2 | 08 March 2021 |

Form PCT/ISA/210 (patent family annex) (July 2022)

**INTERNATIONAL SEARCH REPORT**
**Information on patent family members**

International application No.
**PCT/CN2024/111949**

| Patent document cited in search report | Publication date (day/month/year) | Patent family member(s) | Publication date (day/month/year) |
|---|---|---|---|
| | | PE 20210181 A1 | 29 January 2021 |
| | | US 2020039945 A1 | 06 February 2020 |
| | | FI 3810602 T3 | 21 December 2023 |
| | | DOP 2020000249 A | 28 February 2021 |
| | | US 2022153710 A1 | 19 May 2022 |
| | | HUE 064744 T2 | 28 April 2024 |
| | | CU 20200096 A7 | 06 August 2021 |
| | | DK 3810602 T3 | 11 December 2023 |
| | | PL 3810602 T3 | 04 March 2024 |
| CN 115594695 A | 13 January 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019103952 A
- WO 2020254946 A1
- WO 2022013369 A1


### Non-patent literature cited in the description


- **P.G.M. WUTS** ; **T.W. GREENE**. Protective Groups in Organic Synthesis. John Wiley, 2007
- Fieser and Fieser's Reagents for Organic Synthesis. John Wiley and Sons, 1991, vol. 1-15
- Rodd's Chemistry of Carbon Compounds. Elsevier Science Publishers, 1989, vol. 1-5
- Organic Reactions. John Wiley and Sons, 1991, vol. 1-40
- March's Advanced Organic Chemistry. Wiley
- Larock's Comprehensive Organic Transformations. Wiley-VCH, 1999
- **A.R. GENNARO**. Remington's The Science and Practice of Pharmacy. Lippincott, Williams & Wilkins, 2005